# EUROPEAN PATENT SPECIFICATION

(11) **EP 3 052 519 B1**
(45) Date of publication and mention of the grant of the patent: **25.11.2020**
(21) Application number: 14790422.1
(22) Date of filing: 03.10.2014
(51) Int. Cl.: C07K 14/435

(54) **PROTOXIN-II VARIANTS AND METHODS OF USE**
PROTOXIN-VII-VARIANTEN UND VERFAHREN ZUR VERWENDUNG
VARIANTS DE PROTOXINE II ET MÉTHODES D'UTILISATION

(30) Priority: 03.10.2013 US 201361886100 P
(43) Date of publication of application: 10.08.2016
(60) Divisional application: 20197420.1
(73) Proprietor: Janssen Biotech, Inc., Horsham, PA 19044 (US)
(72) Inventor: FLINSPACH, Mack, San Deigo, California 92121 (US); WICKENDEN, Alan, San Deigo, California 92121 (US); FELLOWS, Ross, San Diego, California 92121 (US); NEFF, Robert, San Deigo, California 92121 (US); LIU, Yi, San Deigo, California 92121 (US); HAGAN, Rebecca, San Diego, California 92121 (US); XU, Qinghao, San Diego, California 92121 (US)
(74) Representative: Carpmaels & Ransford LLP
(86) International application number: PCT/US2014/058972
(87) International publication number: WO 2015/051216

(56) References cited:
- WO-A2-2012/004664
- S. YANG ET AL: "Discovery of a selective NaV1.7 inhibitor from centipede venom with analgesic efficacy exceeding morphine in rodent pain models", PROCEEDINGS OF THE NATIONAL ACADEMY OF SCIENCES, vol. 110, no. 43, 30 September 2013 (2013-09-30), pages 17534-17539, XP055153073, ISSN: 0027-8424, DOI: 10.1073/pnas.1306285110
- SCHMALHOFER W A ET AL: "ProTx-II, a selective inhibitor of NaV1.7 sodium channels, blocks action potential propagation in nociceptors", MOLECULAR PHARMACOLOGY, AMERICAN SOCIETY FOR PHARMACOLOGY AND EXPERIMENTAL THERAPEUTICS, US, vol. 74, no. 5, 1 January 2008 (2008-01-01), pages 1476-1484, XP009120036, ISSN: 0026-895X, DOI: 10.1124/MOL.108.047670
- MIDDLETON RICHARD E ET AL: "Two tarantula peptides inhibit activation of multiple sodium channels", BIOCHEMISTRY, AMERICAN CHEMICAL SOCIETY, US, vol. 41, no. 50, 17 December 2002 (2002-12-17), pages 14734-14747, XP002537028, ISSN: 0006-2960, DOI: 10.1021/BI026546A [retrieved on 2002-11-19] cited in the application
- J. J. SMITH ET AL: "Molecular Interactions of the Gating Modifier Toxin ProTx-II with Nav1.5: IMPLIED EXISTENCE OF A NOVEL TOXIN BINDING SITE COUPLED TO ACTIVATION", JOURNAL OF BIOLOGICAL CHEMISTRY, vol. 282, no. 17, 5 March 2007 (2007-03-05), pages 12687-12697, XP055177873, ISSN: 0021-9258, DOI: 10.1074/jbc.M610462200

## Description

### FIELD OF THE INVENTION

The present invention relates to Protoxin-II variants, synthetic polynucleotides encoding them, and methods of making and using the foregoing.

### BACKGROUND OF THE INVENTION

Voltage-gated sodium channels (VGSC) are present in all excitable cells including cardiac and skeletal muscle cells and central and peripheral neurons. In neuronal cells, sodium channels are responsible for amplifying sub-threshold depolarizations and generating the rapid upstroke of the action potential. As such, sodium channels are essential to the initiation and propagation of electrical signals in the nervous system. Aberrant sodium channel function is thought to underlie a variety of medical disorders (Hübner and Jentsch, Hum Mol Genet 11:2435-45, 2002), including epilepsy (Yogeeswari et al., Curr Drug Targets 5:589-602, 2004), arrhythmia (Tfelt-Hansen et al., J Cardiovasc Electrophysiol 21:107-15, 2010), myotonia (Cannon and Bean, J Clin Invest 120:80-3, 2010), and pain (Cregg et al., J Physiol 588:1897-904, 2010). Sodium channels are typically a complex of various subunits, the principle one being the pore-forming alpha-subunit, which is alone sufficient for function.

Nine known members of the family of voltage-gated sodium channel alpha subunits exist in humans, Nav1.1 - Nav1.9. The Nav1.x subfamily can be pharmacologically subdivided into two groups, the tetrodotoxin (TTX) -sensitive and TTX-resistant. Nav1.7, (a.k.a. PN1 or hNE) is encoded by the SCN9A gene, is TTX-sensitive and is primarily expressed in peripheral sympathetic and sensory neurons. Nav1.7 accumulates at nerve fiber endings and amplifies small sub-threshold depolarizations and acts as a threshold channel that regulates excitability.

Nav1.7 function is implicated in various pain states, including acute, inflammatory and/or neuropathic pain. In man, gain of function mutations of Nav1.7 have been linked to primary erythermalgia (PE), a disease characterized by burning pain and inflammation of the extremities (Yang et al., J Med Genet 41:171-4, 2004), and paroxysmal extreme pain disorder (PEPD)(Fertleman et al., Neuron 52:767-74, 2006). Consistent with this observation, non-selective sodium channel blockers lidocaine, mexiletine and carbamazepine can provide symptomatic relief in these painful disorders (Legroux-Crespel et al., Ann Dermatol Venereol 130:429-33, 2003; Fertleman et al., Neuron 52:767-74, 2006).

Loss-of-function mutations of Nav1.7 in humans cause congenital indifference to pain (CIP), a rare autosomal recessive disorder characterized by a complete indifference or insensitivity to painful stimuli (Cox et al., Nature 444:894-8, 2006; Goldberg et al, Clin Genet 71:311-9, 2007; Ahmad et al., Hum Mol Genet 16:2114-21, 2007).

Single nucleotide polymorphisms in the coding region of SCN9A have been associated with increased nociceptor excitability and pain sensitivity. For example, a polymorphism rs6746030 resulting in R1150W substitution in human Nav1.7 has been associated with osteoarthritis pain, lumbar discectomy pain, phantom pain, and pancreatitis pain (Reimann et al., Proc Natl Acad Sci USA 107:5148-53, 2010). DRG neurons expressing the R1150W mutant Nav1.7 display increased firing frequency in response to depolarization (Estacion et al., Ann Neurol 66:862-6, 2009). A disabling form of fibromyalgia has been associated with SCN9A sodium channel polymorphism rs6754031, indicating that some patients with severe fibromyalgia may have a dorsal root ganglia sodium channelopathy (Vargas-Alarcon et al., BMC Musculoskelet Disord 13:23, 2012).

In mice, deletion of the SCN9A gene in nociceptive neurons leads to reduction in mechanical and thermal pain thresholds and reduction or abolition of inflammatory pain responses (Nassar et al., Proc Natl Acad Sci USA 101:12706-11, 2004). Ablating SCN9A in all sensory neurons abolished mechanical pain, inflammatory pain and reflex withdrawal responses to heat. Deleting SCN9A in both sensory and sympathetic neurons abolished mechanical, thermal and neuropathic pain, and recapitulated the pain-free phenotype seen in humans with Nav1.7 loss-of-function mutations (Minett et al., Nat Commun 3:791, 2012). Nav1.7 inhibitors or blockers may therefore be useful in the treatment of a wide range of pain associated with various disorders.

Spider venoms are known to contain a large number of sodium channel blocking peptides, including Huwentoxin-IV (HwTx-IV) (Peng et al., J Biol Chem 277:47564-71, 2002), Protoxin-I, Protoxin-II (Middleton et al., Biochemistry 41:14734-47, 2002) and Phrixotoxin-III (Bosmans et al., Mol Pharmacol 69:419-29, 2006). WO 2012/004664 and Yang et. al. (Yang et. al., Proc Natl Acad Sci USA, 110(43), 17534-17539, 2013) relate to peptides that inhibit the Nav1.7 sodium channel. There is a need for identification of additional Nav1.7 blockers for treatment of a wide range of pain indications. In particular, there is a need for new Nav1.7 blockers with selectivity for Nav1.7 over other voltage gated sodium channel isoforms.

### SUMMARY OF THE INVENTION

The invention provides an isolated Protoxin-II variant comprising an amino acid sequence selected from the group consisting of SEQ ID Nos:
40, 44, 56, 59, 65, 78, 109, 110, 111, 114, 115, 116, 117, 118, 119, 121, 122, 123, 129, 131, 132, 133, 134, 135, 136, 138, 139, 140, 141, 142, 145, 146, 147, 149, 150, 151, 152, 153, 154, 156, 158, 159, 172, 173, 175, 177, 178, 183, 184, 185, 186, 189, 190, 193, 196, 197, 199, 205, 207, 210, 211, 216, 217, 224, 230, 269, 270, 271, 272, 273, 274, 275, 277, 278, 279, 280, 281, 282, 283, 284, 287, 288, 289, 290, 291, 292, 297, 298, 299, 301, 302, 304, 305, 307, 308, 309, 310, 312, 314, 315, 318, 319,320, 321, 322, 323, 324, 325, and 326,
wherein the variant inhibits human Nav1.7 activity with an IC₅₀ value of 30x10⁻⁹ M or less, wherein the IC₅₀ value is measured using a FLIPR Tetra membrane depolarisation assay, the assay comprising fluorescence resonance energy transfer (FRET) in the presence of 25x10⁻⁶ M 3-veratroylveracevine in HEK293 cells stably expressing human Nav1.7 and using bis-1,3(diethylthiobarbituric acid)trimethine oxonol as an electron acceptor and trisodium 8-octadecyloxypyrene-1,3,6-trisulfonate as a donor by exciting the donor at 390- 420 nm and measuring FRET at 515-575 nm.

The invention also provides the protein conjugate comprising the Protoxin-II variant as defined by the claims conjugated to a half-life extending moiety; wherein the conjugate is a fusion protein comprising the Protoxin-II variant and a human serum albumin (HSA), albumin binding domain (ABD), or a Fc polypeptide; or the conjugate comprises polyethylene glycol (PEG) chemically coupled to the Protoxin-II variant.

The invention also provides an isolated polynucleotide encoding the Protoxin-II variant as defined by the claims.

The invention also provides a vector comprising the isolated polynucleotide as defined by the claims.

The invention also provides a host cell comprising the vector as defined by the claims.

The invention also provides a method of producing an isolated Protoxin-II variant, comprising culturing the host cell as defined by the claims and recovering the Protoxin-II variant produced by the host cell.

The invention also provides a pharmaceutical composition comprising the isolated Protoxin-II variant as defined by the claims and a pharmaceutically acceptable excipient.

The invention also provides the Protoxin-II variant as defined by the claims for use in treating Nav1.7-mediated pain in a subject in need thereof.

The disclosure provides an isolated Protoxin-II variant comprising the sequence
X₁X₂X₃CX₄X₅WX₆QX₇CX₈X₉X₁₀X₁₁X₁₂CCX₁₃X₁₄FX₁₅CX₁₆LWCX₁₇KKLW (SEQ ID NO: 403), wherein
- X₁: is G, P, A or deleted;
- X₂: is P, A or deleted;
- X₃: is S, Q, A, R or Y;
- X₄: is Q, R, K, A or S;
- X₅: is K, S, Q or R;
- X₆: is M or F;
- X₇: is T, S, R, K or Q;
- X₈: is D or T;
- X₉: is S, A or R;
- X₁₀: is E, R, N, K, T or Q;
- X₁₁: is R or K;
- X₁₂: is K, Q, S or A;
- X₁₃: is E, Q or D;
- X₁₄: is G or Q;
- X₁₅: is V or S;
- X₁₆: is R or T; and
- X₁₇: is K or R;
optionally having an N-terminal extension or a C-terminal extension,
wherein the polypeptide inhibits human Nav1.7 activity with an IC₅₀ value of about 1x10⁻⁷ M or less, wherein the IC₅₀ value is measured using a FLIPR® Tetra membrane depolarization assay using fluorescence resonance energy transfer (FRET) in the presence of 25x10⁻⁶ M 3-veratroylveracevine in HEK293 cells stably expressing human Nav1.7. The disclosure also provides an isolated Protoxin-II variant comprising the amino acid sequence that is 90%, 91%, 92%, 93%, 94%, 95%, 96%, 97%, 98% or 99% identical to the amino acid sequence of SEQ ID NO: 78 (GPQCQKWMQTCDRERKCCEGFVCTLWCRKKLW-COOH), wherein
the amino acid sequence has Q at position 1, Q at position 7 and F at position 19, when residue numbering is according to SEQ ID NO: 1;
the polypeptide inhibits human Nav1.7 activity with an IC₅₀ value of about 30x10⁻⁹ M or less, wherein the IC₅₀ value is measured using a FLIPR® Tetra membrane depolarization assay using fluorescence resonance energy transfer (FRET) in the presence of 25x10⁻⁶ M 3-veratroylveracevine in HEK293 cells stably expressing human Nav1.7; and
the polypeptide selectively inhibits Nav1.7.

Another embodiment of the invention is a polynucleotide encoding the Protoxin-II variant of the invention.

Another embodiment of the invention is a vector comprising the isolated polynucleotide of the invention.

Another embodiment of the invention is a host cell comprising the vector of the invention.

Another embodiment of the invention is a method for producing the Protoxin-II variant of the invention, comprising culturing the host cell of the invention and recovering the Protoxin-II variant from the cell.

Another embodiment of the invention is a pharmaceutical composition comprising the isolated Protoxin-II variant of the invention and a pharmaceutically acceptable excipient.

Another embodiment of the invention is a Protoxin-II variant as defined by the claims for use in treating Nav1.7-mediated pain in a subject, comprising administering to a subject an effective amount of the Protoxin-II variant of the invention to treat the Nav1.7-mediated pain.

### BRIEF DESCRIPTION OF THE DRAWINGS

**Figure 1** shows the genus amino acid sequence of Protoxin-II variants that inhibit Nav1.7 with an IC₅₀ value of 30 nM or less in a FLIPR Tetra assay. Residue numbering is according to wild-type Protoxin-II of SEQ ID NO: 1. Genus SEQ ID NO: 403.
**Figure 2** shows the IC₅₀ values for Nav1.7 and Nav1.6 inhibition in a QPatch assay, and selectivity of each variant calculated by ratio of IC₅₀(Nav1.6)/IC₅₀(Nav1.7) obtained in QPatch assay. SE: standard error.
**Figure 3** shows the sequences and the genus sequence of Protoxin-II variants that inhibit Nav1.7 with an IC₅₀ value of 30 nM or less in a FLIPR Tetra assay, and are over 30-fold selective over Nav1.6. Selectivity of each variant was calculated by ratio of IC₅₀(Nav1.6)/IC₅₀9av1.7) obtained in QPatch assay. Residue numbering is according to wild-type Protoxin-II of SEQ ID NO: 1.
**Figure 4A** shows efficacy of NV1D3034 (NV1D3034-OH) (SEQ ID NO: 78) against CFA-induced thermal hyperalgesia assessed by measurement of paw withdrawal latency in the Hargreaves test before (pre-CFA) and after CFA injection (0) and 1-day after peptide administration (1). ***P<0.001 vs. PBS, two-way ANOVA followed by Bonferroni's multiple comparison.
**Figure 4B** shows efficacy of NV1D3034 (NV1D3034-OH) (SEQ ID NO: 78) in CFA-induced thermal hyperalgesia expressed as percent MPE (maximum possible effect) (MPE%) at each dose on day1 following peptide administration. *P<0.05 vs PBS, one-way ANOVA followed by Bonferroni's multiple comparison.
**Figure 5A** shows efficacy of NV1D3368 (NV1D3368-OH) (SEQ ID NO: 198) against CFA-induced thermal hyperalgesia assessed by measurement of paw withdrawal latency in the Hargreaves test before (pre-CFA) and after CFA injection (0) and 1-day after peptide administration (1). **P<0.01 and ****P<0.0001 vs.
PBS, two-way ANOVA followed by Bonferroni's multiple comparison
**Figure 5B** shows efficacy of NV1D3368 (NV1D3368-OH) (SEQ ID NO: 198) in CFA-induced thermal hyperalgesia expressed as percent MPE (MPE%) at each dose on day1 following peptide administration. *P<0.05 and **P<0.01 vs PBS, one-way ANOVA followed by Bonferroni's multiple comparison.
**Figure 6A** shows efficacy of NV1D2775-OH (SEQ ID NO: 56) against CFA-induced thermal hyperalgesia assessed by measurement of paw withdrawal latency in the Hargreaves test before (pre-CFA) and after CFA injection (0) and 1-day after peptide administration (1). ****P<0.0001 vs. PBS, two-way ANOVA followed by Bonferroni's multiple comparison.
**Figure 6B** shows efficacy of NV1D2775-OH (SEQ ID NO: 56) in CFA-induced thermal hyperalgesia expressed as percent MPE (MPE%) at each dose on day1 following peptide administration.. ***P<0.001 and ****P<0.0001 vs PBS, one-way ANOVA followed by Bonferroni's multiple comparison.
**Figure 6C** shows efficacy of NV1D2775-OH (SEQ ID NO: 56) against CFA-induced tactile allodynia. Tactile thresholds of hind paw before (pre-CFA) and after CFA (0) and 1-day after peptide administration (1). ****P<0.0001 vs. PBS, two-way ANOVA followed by Bonferroni's multiple comparison.
**Figure 6D** shows efficacy of NV1D2775-OH (SEQ ID NO: 56) against CFA-induced tactile allodynia expressed as percent MPE (MPE%) on day1 following peptide. ***P<0.001 vs PBS, one-way ANOVA followed by Bonferroni's multiple comparison.
**Figure 7A** shows time course of NV1D2775-OH mediated reversal of thermal hyperalgesia in the CFA model as assessed by measurement of paw withdrawal latency in the Hargreaves test before and after CFA and at various time points post-peptide administration. **P<0.01 vs. PBS, two-way ANOVA followed by Bonferroni's multiple comparison. Shaded areas indicate compound delivery period (0-24hr).
**Figure 7B** shows time course of NV1D2775-OH mediated reversal of tactile allodynia in the CFA model as assessed by measurement of tactile threshold before and after CFA and at various time points post-peptide administration. **P<0.01 vs. PBS, two-way ANOVA followed by Bonferroni's multiple comparison. Shaded areas indicate compound delivery period (0-24hr).
**Figure 8** shows that NV1D2775-OH produced significant analgesia in the hotplate test. Thermal withdrawal latency was evaluated at 50 and 55°C pre- and post-pump implantation. Pump implantation had no impact on the latency in the control PBS group. One day after pump, NV1D2775-OH treated-mice exhibited prolonged latency compared to the PBS group. *P<0.05 and ****P<0.0001 vs. PBS, one-way ANOVA followed by Bonferroni's multiple comparison.
**Figure 9** shows that NV1D2775-OH pretreatment protected animals from carrageenan-induced thermal hyperalgesia. Paw withdrawal latencies were measured pre- and on day1 post-pump before intraplantar carrageenan injection. Latencies were measured again at 2, 3 and 4hr following carrageenan.

### DETAILED DESCRIPTION OF THE INVENTION

As used herein and in the claims, the singular forms "a," "and," and "the" include plural reference unless the context clearly dictates otherwise.

Unless defined otherwise, all technical and scientific terms used herein have the same meaning as commonly understood by one of ordinary skill in the art to which an invention belongs. Although any compositions and methods similar or equivalent to those described herein can be used in the practice or testing of the invention, exemplary compositions and methods are described herein.

The term "polypeptide" means a molecule that comprises at least two amino acid residues linked by a peptide bond to form a polypeptide. Small polypeptides of less than 50 amino acids may be referred to as "peptides". Polypeptides may also be referred as "proteins".

The term "polynucleotide" means a molecule comprising a chain of nucleotides covalently linked by a sugar-phosphate backbone or other equivalent covalent chemistry. Double and single-stranded DNAs and RNAs are typical examples of polynucleotides.

The term "complementary sequence" means a second isolated polynucleotide sequence that is antiparallel to a first isolated polynucleotide sequence and that comprises nucleotides complementary to the nucleotides in the first polynucleotide sequence.

The term "vector" means a non-natural polynucleotide capable of being duplicated within a biological system or that can be moved between such systems. Vector polynucleotides typically contain a cDNA encoding a protein of interest and additional elements, such as origins of replication, polyadenylation signal or selection markers, that function to facilitate the duplication or maintenance of these polynucleotides in a biological system. Examples of such biological systems may include a cell, virus, animal, plant, and reconstituted biological systems utilizing biological components capable of duplicating a vector. The polynucleotide comprising a vector may be DNA or RNA molecules or a hybrid of these.

The term "expression vector" means a vector that can be utilized in a biological system or a reconstituted biological system to direct the translation of a polypeptide encoded by a polynucleotide sequence present in the expression vector.

The term "variant" as used herein refers to a polypeptide or a polynucleotide that differs from wild type Protoxin-II polypeptide of SEQ ID NO: 1 or the polynucleotide encoding the wild type Protoxin-II having the sequence of SEQ ID NO: 107 by one or more modifications for example, substitutions, insertions or deletions of nucleotides or amino acids.

Throughout the specification, residues that are substituted in the Protoxin-II variants are numbered corresponding to their position in the wild-type Protoxin-II of SEQ ID NO: 1. For example, "Y1A" in the specification refers to the substitution of tyrosine at residue position that corresponds to the position 1 in the wild type Protoxin-II of SEQ ID NO:1 with alanine.

"Complementary DNA" or "cDNA" refers to a well-known synthetic polynucleotide that shares the arrangement of sequence elements found in native mature mRNA species with contiguous exons, with the intervening introns present in genomic DNA are removed. The codons encoding the initiator methionine may or may not be present in cDNA. cDNA may be synthesized for example by reverse transcription or synthetic gene assembly.

"Synthetic" or "non-natural" as used herein refers to a polynucleotide or a polypeptide molecule not present in nature.

"Nav1.7" (also referred to as hNE or PN1) or "hNav1.7" as used herein refers to the well-known human sodium channel protein type 9 subunit alpha having a sequence shown in GenBank accession number NP_002968.1 and in SEQ ID NO: 79.

The term "wild type Protoxin-II" or "wild type ProTx-II" as used herein refers to the tarantula *Thrixopelma pruriens* (Peruvian green velvet tarantula) toxin peptide having the amino acid sequence YCQKWMWTCDSERKCCEGMVCRLWCKKKLW-COOH (SEQ ID NO: 1) as described in Middleton et al., Biochemistry 41(50):14734-47, 2002.

The term "recombinant Protoxin-II" or recombinant ProTx-II" as used herein refers to the recombinant Protoxin-II obtained from expression and subsequent cleavage of a Protoxin-II fusion protein having the sequence of GPYCQKWMWTCDSERKCCEGMVCRLWCKKKLW-OH as shown in SEQ ID NO: 2. Recombinant Protoxin-II incorporates a two amino acid N-terminal extension (residues G and P) when compared to the wild type Protoxin-II.

"Blocks human Nav1.7 activity" or "inhibits human Nav1.7 activity" as used herein refers to the ability of the Protoxin-II variant of the invention to reduce membrane depolarization induced by veratridine (3-veratroylveracevine) with an IC₅₀ value of about 1x10⁻⁷ M or less in a FLIPR® Tetra membrane depolarization assay using fluorescence resonance energy transfer (FRET), where veratridine-induced depolarization is measured as a reduction in FRET signal using DISBAC2(3) ([bis-(1,3-diethylthiobarbituric acid) trimethine oxonol]) as an acceptor and PTS18 (trisodium 8-octadecyloxypyrene-1,3,6-trisulfonate) as a donor by exciting the donor at 390-420 nm and measuring FRET at 515-575 nm in a cell line stably expressing human Nav1.7.

"FLIPR® Tetra membrane depolarization assay" as used herein is the assay described in Example 3.

The term "substantially identical" as used herein means that the two Protoxin-II variant amino acid sequences being compared are identical or have "insubstantial differences". Insubstantial differences are substitutions of 1, 2, 3, 4, 5, 6, or 7 amino acids in the Protoxin-II variant amino acid sequence that do not adversely affect peptide properties. Amino acid sequences substantially identical to the Protoxin-II variants disclosed herein are within the scope of the application. In some instances, the sequence identity can be about 80%, 81%, 82%, 83%, 84%, 85%, 86%, 87%, 88%, 89%, 90%, 91%, 92%, 93%, 94%, 95%, 96%, 97%, 98%, 99% or higher. Percent identity can be determined for example by pairwise alignment using the default settings of the AlignX module of Vector NTI v.9.0.0 (Invitrogen, Carslbad, CA). The protein sequences of the present invention may be used as a query sequence to perform a search against public or patent databases, for example, to identify related sequences. Exemplary programs used to perform such searches are the XBLAST or BLASTP programs (http_//www_ncbi_nlm/nih_gov), or the GenomeQuest™ (GenomeQuest, Westborough, MA) suite using the default settings.

Conventional one and three-letter amino acid codes are used herein as shown in Table 1.

**Table 1.**

| Amino acid | Three letter code | One letter code |
|---|---|---|
| Alanine | Ala | A |
| Arginine | Arg | R |
| Asparagine | Asn | N |
| Aspartate | Asp | D |
| Cysteine | Cys | C |
| Glutamate | Glu | E |
| Glutamine | Gln | Q |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |

The present invention provides isolated Protoxin-II (ProTx-II) variant polypeptides as defined by the claims that inhibit human Nav1.7 activity, polynucleotides encoding them, vectors, host cells, and methods of using the polynucleotides and polypeptides of the invention. The polypeptides of the invention inhibit depolarization resulting from Nav1.7 activation, and therefore may be useful in the treatment of various conditions associated with pain and conditions associated with sensory or sympathetic neuron dysfunction.

The variants of the invention are potent inhibitors of Nav1.7. The current invention is based, at least in part, on the finding that certain residue substitutions in Protoxin-II enhance selectivity, synthetic yield and/or homogeneity without adversely affecting the potency of the generated Protoxin-II variants, specifically W7 and M19, and additionally residues Y1 and S11, and further additionally residues E12, R22 and (residue numbering according to SEQ ID NO: 1).

One embodiment of the invention is an isolated Protoxin-II variant, wherein the Protoxin-II variant inhibits human Nav1.7 activity with an IC₅₀ value of about 1x10⁻⁹ M or less, about 1x10⁻¹⁰ M or less, about 1x10⁻¹¹ M or less, or about 1x10⁻¹² M or less, wherein the IC₅₀ value is measured using a FLIPR® Tetra membrane depolarization assay using fluorescence resonance energy transfer (FRET) in the presence of 25x10⁻⁶ M 3-veratroylveracevine in HEK293 cells stably expressing human Nav1.7 and using bis-1,3(diethylthiobarbituric acid) trimethine oxonol as an electron acceptor and trisodium 8-octadecyloxypyrene-1,3,6-trisulfonate as a donor by exciting the donor at 390- 420 nm and measuring FRET at 515-575 nm.

The disclosure provides an isolated Protoxin-II variant comprising the sequence
X₁X₂X₃CX₄X₅WX₆QX₇CX₈X₉X₁₀X₁₁X₁₂CCX₁₃X₁₄FX₁₅CX₁₆LWCX₁₇KKLW (SEQ ID NO: 403), wherein
- X₁: is G, P, A or deleted;
- X₂: is P, A or deleted;
- X₃: is S, Q, A, R or Y;
- X₄: is Q, R, K, A or S;
- X₅: is K, S, Q or R;
- X₆: is M or F;
- X₇: is T, S, R, K or Q;
- X₈: is D or T;
- X₉: is S, A or R;
- X₁₀: is E, R, N, K, T or Q;
- X₁₁: is R or K;
- X₁₂: is K, Q, S or A;
- X₁₃: is E, Q or D;
- X₁₄: is G or Q;
- X₁₅: is V or S;
- X₁₆: is R or T; and
- X₁₇: is K or R;
optionally having an N-terminal extension or a C-terminal extension,
wherein the polypeptide inhibits human Nav1.7 activity with an IC₅₀ value of about 1x10⁻⁷ M or less, wherein the IC₅₀ value is measured using a FLIPR® Tetra membrane depolarization assay using fluorescence resonance energy transfer (FRET) in the presence of 25x10⁻⁶ M 3-veratroylveracevine in HEK293 cells stably expressing human Nav1.7.

The N-terminal extension can comprise the amino acid sequences of SEQ ID NOs: 372, 373, 374, 375, 376, 377, 378, 379, 380, 381, 382, 383, 384 or 385.

The C-terminal extension can comprise the amino acid sequence of SEQ ID NOs: 374, 386, 387, 388, 389, 390, 391, 392, 393, 394, 395, 396 or 397.

The N-terminal and/or the C-terminal extension may be conjugated to the Protoxin-II variant via a linker.

The linker can comprise the amino acid sequence of SEQ ID NOs: 383, 392, 398, 399, 400, 401 or 402.

The N-terminal extension can consist of the amino acid sequences of SEQ ID NOs: 372, 373, 374, 375, 376, 377, 378, 379, 380, 381, 382, 383, 384 or 385.

The C-terminal extension can consist of the amino acid sequence of SEQ ID NOs: 374, 386, 387, 388, 389, 390, 391, 392, 393, 394, 395, 396 or 397.

The linker can consist of the amino acid sequence of SEQ ID NOs: 383, 392, 398, 399, 400, 401 or 402.

The Protoxin-II variants of the invention are potent Nav1.7 inhibitors. Recombinant Protoxin-II (SEQ ID NO: 2) has an IC₅₀ value of about 4x10⁻⁹ M for human Nav1.7 in a veratridine-induced depolarization inhibition assay measuring decline in FRET (fluorescence resonance energy transfer) in cells stably expressing Nav1.7 using FLIPR® Tetra instrument (Molecular Devices) using experimental details described in Example 3. A Protoxin-II variant is "a potent" Nav1.7 inhibitor when the IC₅₀ value in the assay described above and in Experiment 3 is about 30x10⁻⁹ M or less i.e. within 10 fold of recombinant Protoxin-II. For clarity, an IC₅₀ of 30x10⁻⁹ M is identical to IC₅₀ of 3.0x10⁻⁸ M.

The Protoxin-II variant polypeptides of the invention may be produced by chemical synthesis, such as solid phase peptide synthesis, on an automated peptide synthesizer. Alternatively, the polypeptides of the invention may be obtained from polynucleotides encoding the polypeptides by the use of cell-free expression systems such as reticulocyte lysate based expression systems, or by recombinant expression systems. Those skilled in the art will recognize other techniques for obtaining the polypeptides of the invention. In an exemplary method, the Protoxin-II variants of the invention are generated by expressing them as human serum albumin (HSA) fusion proteins utilizing a glycine-rich linker such as (GGGGS)₄ (SEQ ID NO:80) or (GGGGS)₆ (SEQ ID NO: 81) coupled to a protease cleavable linker such as a recognition sequence for HRV3C protease (Recombinant type 14 3C protease from human rhinovirus) LEVLFQGP (HRV3C linker) (SEQ ID NO: 82), and cleaving the expressed fusion proteins with the HRV3C protease to release the recombinant Protoxin-II variant peptides. Hexahistidine (SEQ ID NO: 108) or other tags may be used to facilitate purification using well known methods.

Protoxin-II variants of the invention may be purified using methods described herein. In an exemplary method, Protoxin-II variants of the invention expressed as HSA fusion proteins and cleaved with HRV3C protease may be purified using sold phase extraction (SPE) as described herein.

Generation of the Protoxin-II variants optionally having N-terminal and/or C-terminal extensions, and Protoxin-II variant fusion proteins is typically achieved at the nucleic acid level. The polynucleotides may be synthesized using chemical gene synthesis according to methods described in U.S. Pat. No. 6,521,427 and 6,670,127, utilizing degenerate oligonucleotides to generate the desired variants, or by standard PCR cloning and mutagenesis. Libraries of variants may be generated by standard cloning techniques to clone the polynucleotides encoding the Protoxin-II variants into the vector for expression.

The Protoxin-II variants may incorporate additional N- and/or C-terminal amino acids when compared to the wild type Protoxin-II of SEQ ID NO: 1, for example resulting from cloning and/or expression schemes. For example, cleavage from HSA after expression of the variant as HSA-linker-HRV3C cleavable peptide-Protoxin-II variant fusion protein may result in the incorporation of additional two residues to the N-terminus of each Protoxin-II variant, such as G and P.

The Protoxin-II variants of the invention are tested for their ability to inhibit human Nav1.7 using methods described herein. An exemplary assay is a veratridine-induced depolarization inhibition assay measuring decline in FRET (fluorescence resonance energy transfer) in cells stably expressing Nav1.7. Another exemplary assay employs electrophysiological recordings to measure changes in Nav1.7-mediated currents using well known patch clamp techniques and as described herein.

Examples of isolated Protoxin-II variants are those comprising the amino acid sequence of SEQ ID NOs: 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 109, 110, 111, 112, 113, 114, 115, 116, 117, 118, 119, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154 ,155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225,226, 227, 228, 229, 230, 231, 232, 233, 234, 235, 236, 237, 238, 239, 240, 241, 242, 243, 244, 245, 246, 247, 248, 249, 250, 251, 252, 253, 254, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 35, 357, 358, 359, 360, 361, 362, 363, 364, 365, 366, 367, 368 367, 370 or 371.

The Protoxin-II variants of the invention may inhibit human Nav1.7 with an IC₅₀ value of about 1x10⁻⁹ or less, about 1x10⁻¹⁰ M or less, about 1x10⁻¹¹ M or less, or about 1x10⁻¹² M or less. Exemplary variants demonstrating the range of IC₅₀ values are variants having amino acid sequences shown in SEQ ID NOs: 30, 40, 44, 52, 56, 56, 59, 65, 78, 109, 110, 111, 117, 118, 119, 120, 121, 122, 123, 124, 125, 126, 127, 128, 129, 131, 132, 133, 134, 135, 136, 137, 138, 139, 140, 141, 142, 143, 144, 145, 146, 147, 148, 149, 150, 151, 152, 153, 154, 155, 156, 157, 158, 159, 162, 165, 166, 167, 168, 169, 170, 171, 172, 173, 174, 175, 177, 178, 179, 180, 182, 183, 184, 185, 186, 189, 190, 193, 195, 197, 199, 206, 207, 208, 209, 210, 211, 212, 213, 214, 215, 216, 217, 218, 224, 226, 227, 231, 232, 243, 244, 245, 247, 249, 252, 255, 258, 261, 263, 264, 265, 266, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 332, 334, 335, 336, 337, 339, 340, 341, 342, 346, 351, 358, 359, 364, 366, 367, or 368.

Table 2 and Table 3 show the sequences of select Protoxin-II variants.

**Table 2.**

| Protein name | Protoxin-II variant peptide name | SEQ ID NO: | Protein amino acid sequence |
|---|---|---|---|
| | wild type | 1 | YCQKWMWTCDSERKCCEGMVCRLWCKKKLW-COOH |
| | NV1D12 | 2 | GPYCQKWMWTCDSERKCCEGMVCRLWCKKKLW-COOH |
| | NV1D748 | 3 | GPACQKWMWTCDSERKCCEGMVCRLWCKKKLW-COOH |
| | NV1D751 | 4 | GPQCQKWMWTCDSERKCCEGMVCRLWCKKKLW-COOH |
| | NV1D2292 | 5 | GPRCQKWMWTCDSERKCCEGMVCRLWCKKKLW-COOH |
| | NV1D750 | 6 | GPSCQKWMWTCDSERKCCEGMVCRLWCKKKLW-COOH |
| | NV1D1328 | 7 | GPYCQKWFWTCDSERKCCEGMVCRLWCKKKLW-COOH |
| | NV1D774 | 8 | GPYCQKWMQTCDSERKCCEGMVCRLWCKKKLW-COOH |
| | NV1D786 | 9 | GPYCQKWMWTCDAERKCCEGMVCRLWCKKKLW-COOH |
| | NV1D2300 | 10 | GPYCQKWMWTCDRERKCCEGMVCRLWCKKKLW-COOH |
| | NV1D791 | 11 | GPYCQKWMWTCDSKRKCCEGMVCRLWCKKKLW-COOH |
| | NV1D1332 | 12 | GPYCQKWMWTCDSNRKCCEGMVCRLWCKKKLW-COOH |
| | NV1D2512 | 13 | GPYCQKWMWTCDSERKCCEGFVCRLWCKKKLW-COOH |
| | NV1D1336 | 14 | GPYCQKWMWTCDSERKCCEGLVCRLWCKKKLW-COOH |
| | NV1D1337 | 15 | GPYCQKWMWTCDSERKCCEGMVCTLWCKKKLW-COOH |
| | NV1D2308 | 16 | GPYCQKWMWTCDSERKCCEGMVCRLWCRKKLW-COOH |
| NV1G953 | NV1D2670 | 17 | GPACQKWMQTCDSERKCCEGMVCRLWCKKKLW-COOH |
| NV1G951 | NV1D2674 | 18 | GPACQKWMWTCDAERKCCEGMVCRLWCKKKLW-COOH |
| NV1G909 | NV1D2664 | 19 | GPACQKWMWTCDSERKCCEGFVCRLWCKKKLW-COOH |
| NV1G963 | NV1D2671 | 20 | GPQCQKWMQTCDSERKCCEGMVCRLWCKKKLW-COOH |
| NV1G949 | NV1D2675 | 21 | GPQCQKWMWTCDAERKCCEGMVCRLWCKKKLW-COOH |
| NV1G977 | NV1D2665 | 22 | GPQCQKWMWTCDSERKCCEGFVCRLWCKKKLW-COOH |
| NV1G957 | NV1D2668 | 23 | GPRCQKWMQTCDSERKCCEGMVCRLWCKKKLW-COOH |
| NV1G965 | NV1D2672 | 24 | GPRCQKWMWTCDAERKCCEGMVCRLWCKKKLW-COOH |
| NV1G973 | NV1D2662 | 25 | GPRCQKWMWTCDSERKCCEGFVCRLWCKKKLW-COOH |
| NV1G975 | NV1D2669 | 26 | GPSCQKWMQTCDSERKCCEGMVCRLWCKKKLW-COOH |
| NV1G971 | NV1D2673 | 27 | GPSCQKWMWTCDAERKCCEGMVCRLWCKKKLW-COOH |
| NV1G995 | NV1D2663 | 28 | GPSCQKWMWTCDSERKCCEGFVCRLWCKKKLW-COOH |
| NV1G961 | NV1D2676 | 29 | GPYCQKWMQTCDAERKCCEGMVCRLWCKKKLW-COOH |
| NV1G911 | NV1D2666 | 30 | GPYCQKWMQTCDSERKCCEGFVCRLWCKKKLW-COOH |
| | NV1D2816 | 31 | GPACQKWFQTCDSERKCCEGMVCRLWCKKKLW-COOH |
| NV1G905 | NV1D2735 | 32 | GPACQKWMQTCDSERKCCEGFVCRLWCKKKLW-COOH |
| NV1G919 | NV1D2739 | 33 | GPACQKWMWTCDAERKCCEGFVCRLWCKKKLW-COOH |
| NV1G979 | NV1D2731 | 34 | GPACQKWMQTCDAERKCCEGMVCRLWCKKKLW-COOH |
| | NV1D2810 | 35 | GPQCQKWFQTCDSERKCCEGMVCRLWCKKKLW-COOH |
| NV1G1099 | NV1D2732 | 36 | GPQCQKWMQTCDAERKCCEGMVCRLWCKKKLW-COOH |
| NV1G1011 | NV1D2740 | 37 | GPQCQKWMWTCDAERKCCEGFVCRLWCKKKLW-COOH |
| | NV1D2819 | 38 | GPRCQKWFWTCDAERKCCEGMVCRLWCKKKLW-COOH |
| NV1G1105 | NV1D2729 | 39 | GPRCQKWMQTCDAERKCCEGMVCRLWCKKKLW-COOH |
| NV1G1013 | NV1D2733 | 40 | GPRCQKWMQTCDSERKCCEGFVCRLWCKKKLW-COOH |
| | NV1D2814 | 41 | GPSCQKWFQTCDSERKCCEGMVCRLWCKKKLW-COOH |
| | NV1D2820 | 42 | GPSCQKWFWTCDAERKCCEGMVCRLWCKKKLW-COOH |
| NV1G983 | NV1D2730 | 43 | GPSCQKWMQTCDAERKCCEGMVCRLWCKKKLW-COOH |
| NV1G1003 | NV1D2734 | 44 | GPSCQKWMQTCDSERKCCEGFVCRLWCKKKLW-COOH |
| NV1G1009 | NV1D2738 | 45 | GPSCQKWMWTCDAERKCCEGFVCRLWCKKKLW-COOH |
| | NV1D2851 | 46 | GPYCQKWFKTCDAERKCCEGMVCRLWCKKKLW-COOH |
| | NV1D2850 | 47 | GPYCQKWFQTCDAERKCCEGMVCRLWCKKKLW-COOH |
| NV1G987 | NV1D2667 | 48 | GPYCQKWMWTCDAERKCCEGFVCRLWCKKKLW-COOH |
| | NV1D2867 | 49 | GPACQKWFQTCDAERKCCEGMVCRLWCKKKLW-COOH |
| | NV1D2881 | 50 | GPACQKWFQTCDSERKCCEGFVCRLWCKKKLW-COOH |
| | NV1D2882 | 51 | GPACQKWFQTCDSERKCCEGLVCRLWCKKKLW-COOH |
| NV1G899 | NV1D2774 | 52 | GPACQKWMQTCDAERKCCEGFVCRLWCKKKLW-COOH |
| NV1G1077 | NV1D2902 | 53 | GPACQKWMQTCDAERKCCEGLVCRLWCKKKLW-COOH |
| | NV1D2861 | 54 | GPQCQKWFQTCDAERKCCEGMVCRLWCKKKLW-COOH |
| | NV1D2870 | 55 | GPQCQKWFQTCDSERKCCEGLVCRLWCKKKLW-COOH |
| NV1G1007 | NV1D2775 | 56 | GPQCQKWMQTCDAERKCCEGFVCRLWCKKKLW-COOH |
| NV1G1067 | NV1D2893 | 57 | GPQCQKWMQTCDAERKCCEGLVCRLWCKKKLW-COOH |
| | NV1D2887 | 58 | GPRCQKWFWTCDAERKCCEGFVCRLWCKKKLW-COOH |
| NV1G1005 | NV1D2772 | 59 | GPRCQKWMQTCDAERKCCEGFVCRLWCKKKLW-COOH |
| NV1G1061 | NV1D2896 | 60 | GPRCQKWMQTCDAERKCCEGLVCRLWCKKKLW-COOH |
| | NV1D2877 | 61 | GPSCQKWFQTCDSERKCCEGFVCRLWCKKKLW-COOH |
| | NV1D2878 | 62 | GPSCQKWFQTCDSERKCCEGLVCRLWCKKKLW-COOH |
| | NV1D2889 | 63 | GPSCQKWFWTCDAERKCCEGFVCRLWCKKKLW-COOH |
| | NV1D2889 | 64 | GPSCQKWFWTCDAERKCCEGFVCRLWCKKKLW-COOH |
| NV1G1001 | NV1D2773 | 65 | GPSCQKWMQTCDAERKCCEGFVCRLWCKKKLW-COOH |
| | NV1D2890 | 66 | GPSCQKWFWTCDAERKCCEGLVCRLWCKKKLW-COOH |
| NV1G1109 | NV1D2899 | 67 | GPSCQKWMQTCDAERKCCEGLVCRLWCKKKLW-COOH |
| | NV1D2905 | 68 | GPYCQKWFQTCDAERKCCEGFVCRLWCKKKLW-COOH |
| | NV1D2906 | 69 | GPYCQKWFQTCDAERKCCEGLVCRLWCKKKLW-COOH |
| | NV1D2921 | 70 | GPACQKWFQTCDAERKCCEGFVCRLWCKKKLW-COOH |
| | NV1D2922 | 71 | GPACQKWFQTCDAERKCCEGLVCRLWCKKKLW-COOH |
| | NV1D2909 | 72 | GPQCQKWFQTCDAERKCCEGFVCRLWCKKKLW-COOH |
| | NV1D2910 | 73 | GPQCQKWFQTCDAERKCCEGLVCRLWCKKKLW-COOH |
| | NV1D2913 | 74 | GPRCQKWFQTCDAERKCCEGFVCRLWCKKKLW-COOH |
| | NV1D2914 | 75 | GPRCQKWFQTCDAERKCCEGLVCRLWCKKKLW-COOH |
| | NV1D2917 | 76 | GPSCQKWFQTCDAERKCCEGFVCRLWCKKKLW-COOH |
| | NV1D2918 | 77 | GPSCQKWFQTCDAERKCCEGLVCRLWCKKKLW-COOH |
| NV1G1153 | NV1D3034 | 78 | GPQCQKWMQTCDRERKCCEGFVCTLWCRKKLW-COOH |

**Table 3.**

| Protein name | Protoxin-II variant peptide name | SEQ ID NO: | Protein amino acid sequence |
|---|---|---|---|
| (-GP) NV1G1001 | (-GP) NV1D2773 | 109 | |
| (-GP) NV1G1001-NH-Me | (-GP) NV1D2773-NH2 | 110 | |
| NV1G1007-NH2 | NV1D2775-NH2 | 111 | |
| NV1G1107-NH2 | NV1D2890-NH2 | 112 | |
| NV1G1137 | NV1D2974 | 113 | |
| (-GP) N-Ac-NV1G1137-NH2 | (-GP) N-Ac-NV1D2974-NH2 | 114 | |
| (-GP) N-Ac-NV1G1137- | (-GP) N-Ac-NV1D2974 | 115 | |
| NV1G1153 | NV1D3034 | 116 | |
| NV1G1153-NH2 | NV1D3034-NH2 | 117 | |
| NV1G1153-NH-butyl | NV1D3034-NH-butyl | 118 | |
| NV1G1153-NH-methyl | NV1D3034-NH-methyl | 119 | |
| (-GP) N-Ac-NV1G1153 | (-GP) N-Ac-NV1D3034 | 120 | |
| (-GP) N-Ac-NV1G1153-NH2 | (-GP) N-Ac-NV1D3034-NH2 | 121 | |
| NV1G1818 | NV1D3368 | 122 | |
| NV1G1818-NH2 | NV1D3368-NH2 | 123 | |
| NV1G1147 | NV1D2969 | 124 | |
| NV1G1145 | NV1D2970 | 125 | |
| NV1G1143 | NV1D2971 | 126 | |
| NV1G1141 | NV1D2972 | 127 | |
| NV1G1139 | NV1D2973 | 128 | |
| NV1G1137 | NV1D2974 | 129 | |
| NV1G1137-NH2 | NV1D2974-NH2 | 130 | |
| NV1G1517 | NV1D3004 | 131 | |
| NV1G1515 | NV1D3005 | 132 | |
| NV1G1519 | NV1D3006 | 133 | |
| | | | |
| NV1G1513 | NV1D3007 | 134 | |
| NV1G1523 | NV1D3012 | 135 | |
| NV1G1525 | NV1D3013 | 136 | |
| NV1G1255 | NV1D3014 | 137 | |
| NV1G1187 | NV1D3015 | 138 | |
| NV1G1257 | NV1D3016 | 139 | |
| NV1G1221 | NV1D3017 | 140 | |
| NV1G1521 | NV1D3018 | 141 | |
| NV1G1531 | NV1D3019 | 142 | |
| NV1G1239 | NV1D3020 | 143 | |
| NV1G1583 | NV1D3030 | 144 | |
| NV1G1527 | NV1D3031 | 145 | |
| NV1G1511 | NV1D3032 | 146 | |
| NV1G1509 | NV1D3033 | 147 | |
| NV1G1231 | NV1D3035 | 148 | |
| NV1G1211 | NV1D3036 | 149 | |
| NV1G1267 | NV1D3044 | 150 | |
| NV1G1269 | NV1D3045 | 151 | |
| NV1G1215 | NV1D3048 | 152 | |
| NV1G1593 | NV1D3050 | 153 | |
| NV1G1263 | NV1D3051 | 154 | |
| NV1G1585 | NV1D3052 | 155 | |
| NV1G1623 | NV1D3056 | 156 | |
| NV1G1613 | NV1D3057 | 157 | |
| | | | |
| NV1G1259 | NV1D3058 | 158 | |
| NV1G1265 | NV1D3062 | 159 | |
| NV1G1273 | NV1D3109 | 160 | |
| NV1G1225 | NV1D3121 | 161 | |
| NV1G1886 | NV1D3249 | 162 | |
| NV1G1633 | NV1D3251 | 163 | |
| NV1G1631 | NV1D3252 | 164 | |
| NV1G1885 | NV1D3254 | 165 | |
| NV1G1884 | NV1D3256 | 166 | |
| NV1G1881 | NV1D3257 | 167 | |
| NV1G1879 | NV1D3259 | 168 | |
| NV1G1883 | NV1D3260 | 169 | |
| NV1G1880 | NV1D3261 | 170 | |
| NV1G1882 | NV1D3262 | 171 | |
| NV1G1776 | NV1D3339 | 172 | |
| NV1G1775 | NV1D3340 | 173 | |
| NV1G1768 | NV1D3341 | 174 | |
| NV1G1777 | NV1D3342 | 175 | |
| NV1G1770 | NV1D3344 | 176 | |
| NV1G1767 | NV1D3345 | 177 | |
| NV1G1769 | NV1D3346 | 178 | |
| NV1G1774 | NV1D3347 | 179 | |
| NV1G1771 | NV1D3348 | 180 | |
| NV1G1778 | NV1D3349 | 181 | |
| NV1G1773 | NV1D3350 | 182 | |
| NV1G1779 | NV1D3351 | 183 | |
| NV1G1772 | NV1D3352 | 184 | |
| NV1G1868 | NV1D3353 | 185 | |
| NV1G1824 | NV1D3354 | 186 | |
| NV1G1863 | NV1D3356 | 187 | |
| NV1G1826 | NV1D3357 | 188 | |
| NV1G1810 | NV1D3358 | 189 | |
| NV1G1836 | NV1D3359 | 190 | |
| NV1G1834 | NV1D3360 | 191 | |
| NV1G1829 | NV1D3361 | 192 | |
| NV1G1820 | NV1D3362 | 193 | |
| NV1G1828 | NV1D3363 | 194 | |
| NV1G1827 | NV1D3365 | 195 | |
| NV1G1857 | NV1D3366 | 196 | |
| NV1G1823 | NV1D3367 | 197 | |
| NV1G1818 | NV1D3368 | 198 | |
| NV1G1811 | NV1D3369 | 199 | |
| NV1G1853 | NV1D3370 | 200 | |
| NV1G1817 | NV1D3371 | 201 | |
| NV1G1814 | NV1D3372 | 202 | |
| NV1G1831 | NV1D3374 | 203 | |
| NV1G1819 | NV1D3375 | 204 | |
| NV1G1859 | NV1D3376 | 205 | |
| NV1G1825 | NV1D3377 | 206 | |
| NV1G1821 | NV1D3378 | 207 | |
| NV1G1835 | NV1D3379 | 208 | |
| NV1G1815 | NV1D3380 | 209 | |
| NV1G1833 | NV1D3381 | 210 | |
| NV1G1812 | NV1D3382 | 211 | |
| NV1G1782 | NV1D3383 | 212 | |
| NV1G1783 | NV1D3384 | 213 | |
| NV1G1785 | NV1D3385 | 214 | |
| NV1G1784 | NV1D3386 | 215 | |
| NV1G1780 | NV1D3387 | 216 | |
| NV1G1781 | NV1D3388 | 217 | |
| NV1G1786 | NV1D3389 | 218 | |
| NV1G1851 | NV1D3390 | 219 | |
| NV1G1852 | NV1D3391 | 220 | |
| NV1G1854 | NV1D3392 | 221 | |
| NV1G1860 | NV1D3393 | 222 | |
| NV1G1789 | NV1D3394 | 223 | |
| NV1G1787 | NV1D3396 | 224 | |
| NV1G1856 | NV1D3397 | 225 | |
| NV1G1855 | NV1D3398 | 226 | |
| NV1G1788 | NV1D3399 | 227 | |
| NV1G1849 | NV1D3400 | 228 | |
| NV1G1795 | NV1D3401 | 229 | |
| NV1G1803 | NV1D3403 | 230 | |
| NV1G1807 | NV1D3408 | 231 | |
| NV1G1806 | NV1D3409 | 232 | |
| NV1G1805 | NV1D3410 | 233 | |
| NV1G1809 | NV1D3413 | 234 | |
| NV1G1850 | NV1D3414 | 235 | |
| NV1G1793 | NV1D3419 | 236 | |
| NV1G1822 | NV1D3423 | 237 | |
| NV1G1813 | NV1D3424 | 238 | |
| NV1G1840 | NV1D3425 | 239 | |
| NV1G1848 | NV1D3426 | 240 | |
| NV1G1841 | NV1D3427 | 241 | |
| NV1G1844 | NV1D3428 | 242 | |
| NV1G1842 | NV1D3430 | 243 | |
| NV1G1846 | NV1D3431 | 244 | |
| NV1G1843 | NV1D3432 | 245 | |
| NV1G1892 | NV1D3439 | 246 | |
| NV1G1916 | NV1D3465 | 247 | |
| NV1G1922 | NV1D3466 | 248 | |
| NV1G1915 | NV1D3467 | 249 | |
| NV1G1924 | NV1D3470 | 250 | |
| NV1G1709 | NV1D3510 | 251 | |
| NV1G1681 | NV1D3511 | 252 | |
| NV1G1693 | NV1D3512 | 253 | |
| NV1G1705 | NV1D3513 | 254 | |
| NV1G1689 | NV1D3514 | 255 | |
| NV1G1711 | NV1D3515 | 256 | |
| NV1G1685 | NV1D3516 | 257 | |
| NV1G1697 | NV1D3517 | 258 | |
| NV1G1695 | NV1D3518 | 259 | |
| NV1G1701 | NV1D3519 | 260 | |
| NV1G1691 | NV1D3520 | 261 | |
| NV1G1679 | NV1D3521 | 262 | |
| NV1G1683 | NV1D3523 | 263 | |
| NV1G1707 | NV1D3524 | 264 | |
| NV1G1713 | NV1D3525 | 265 | |
| NV1G1687 | NV1D3526 | 266 | |
| NV1G1699 | NV1D3527 | 267 | |
| NV1G1675 | NV1D3528 | 268 | |
| NV1G1754 | NV1D3529 | 269 | |
| NV1G1748 | NV1D3530 | 270 | |
| NV1G1747 | NV1D3531 | 271 | |
| NV1G1752 | NV1D3532 | 272 | |
| NV1G1722 | NV1D3533 | 273 | |
| NV1G1744 | NV1D3534 | 274 | |
| NV1G1742 | NV1D3535 | 275 | |
| NV1G1723 | NV1D3536 | 276 | |
| NV1G1745 | NV1D3537 | 277 | |
| NV1G1757 | NV1D3538 | 278 | |
| NV1G1762 | NV1D3539 | 279 | |
| NV1G1763 | NV1D3540 | 280 | |
| NV1G1728 | NV1D3541 | 281 | |
| NV1G1730 | NV1D3542 | 282 | |
| NV1G1760 | NV1D3543 | 283 | |
| NV1G1727 | NV1D3544 | 284 | |
| NV1G1729 | NV1D3545 | 285 | |
| NV1G1867 | NV1D3546 | 286 | |
| NV1G1759 | NV1D3547 | 287 | |
| NV1G1758 | NV1D3548 | 288 | |
| NV1G1766 | NV1D3549 | 289 | |
| NV1G1761 | NV1D3550 | 290 | |
| NV1G1726 | NV1D3551 | 291 | |
| NV1G1721 | NV1D3552 | 292 | |
| NV1G1765 | NV1D3553 | 293 | |
| NV1G1764 | NV1D3554 | 294 | |
| NV1G1732 | NV1D3555 | 295 | |
| NV1G1862 | NV1D3556 | 296 | |
| NV1G1751 | NV1D3558 | 297 | |
| NV1G1866 | NV1D3559 | 298 | |
| NV1G1865 | NV1D3560 | 299 | |
| NV1G1716 | NV1D3561 | 300 | |
| NV1G1724 | NV1D3562 | 301 | |
| NV1G1717 | NV1D3563 | 302 | |
| NV1G1743 | NV1D3564 | 303 | |
| NV1G1720 | NV1D3565 | 304 | |
| NV1G1735 | NV1D3566 | 305 | |
| NV1G1734 | NV1D3568 | 306 | |
| NV1G1741 | NV1D3569 | 307 | |
| NV1G1719 | NV1D3570 | 308 | |
| NV1G1718 | NV1D3571 | 309 | |
| NV1G1725 | NV1D3572 | 310 | |
| NV1G1869 | NV1D3573 | 311 | |
| NV1G1755 | NV1D3574 | 312 | |
| NV1G1756 | NV1D3575 | 313 | |
| NV1G1746 | NV1D3576 | 314 | |
| NV1G1733 | NV1D3577 | 315 | |
| NV1G1738 | NV1D3578 | 316 | |
| NV1G1737 | NV1D3579 | 317 | |
| NV1G1740 | NV1D3580 | 318 | |
| NV1G1864 | NV1D3581 | 319 | |
| NV1G1739 | NV1D3582 | 320 | |
| NV1G1870 | NV1D3583 | 321 | |
| NV1G1715 | NV1D3584 | 322 | |
| NV1G1753 | NV1D3585 | 323 | |
| NV1G1750 | NV1D3586 | 324 | |
| NV1G1750-NH2 | NV1D3586-NH2 | 325 | |
| NV1G1749 | NV1D3587 | 326 | |
| NV1G1871 | NV1D3772 | 327 | |
| NV1G1839 | NV1D3774 | 328 | |
| NV1G1877 | NV1D3775 | 329 | |
| NV1G1872 | NV1D3777 | 330 | |
| NV1G1941 | NV1D3782 | 331 | |
| NV1G1990 | NV1D3788 | 332 | |
| NV1G1991 | NV1D3789 | 333 | |
| NV1G1989 | NV1D3791 | 334 | |
| NV1G1993 | NV1D3792 | 335 | |
| NV1G1967 | NV1D3793 | 336 | |
| NV1G1969 | NV1D3795 | 337 | |
| | | | |
| NV1G1974 | NV1D3796 | 338 | |
| NV1G1950 | NV1D3797 | 339 | |
| NV1G1948 | NV1D3798 | 340 | |
| NV1G2057 | NV1D3799 | 341 | |
| NV1G1954 | NV1D3800 | 342 | |
| NV1G1956 | NV1D3801 | 343 | |
| NV1G1961 | NV1D3802 | 344 | |
| NV1G1960 | NV1D3803 | 345 | |
| NV1G1977 | NV1D3804 | 346 | |
| NV1G1982 | NV1D3805 | 347 | |
| NV1G1984 | NV1D3806 | 348 | |
| NV1G1985 | NV1D3808 | 349 | |
| NV1G1983 | NV1D3809 | 350 | |
| NV1G1973 | NV1D3810 | 351 | |
| NV1G1976 | NV1D3811 | 352 | |
| NV1G1980 | NV1D3812 | 353 | |
| NV1G1952 | NV1D3813 | 354 | |
| NV1G1957 | NV1D3814 | 355 | |
| NV1G1981 | NV1D3815 | 356 | |
| NV1G1959 | NV1D3818 | 357 | |
| NV1G1986 | NV1D3819 | 358 | |
| NV1G1968 | NV1D3822 | 359 | |
| NV1G1945 | NV1D3823 | 360 | |
| NV1G1972 | NV1D3824 | 361 | |
| NV1G1946 | NV1D3825 | 362 | |
| NV1G1970 | NV1D3826 | 363 | |
| NV1G1949 | NV1D3828 | 364 | |
| NV1G1951 | NV1D3829 | 365 | |
| NV1G1971 | NV1D3830 | 366 | |
| NV1G1975 | NV1D3832 | 367 | |
| NV1G1978 | NV1D3833 | 368 | |
| NV1G1979 | NV1D3834 | 369 | |
| NV1G2043 | NV1D3835 | 370 | |
| NV1G1955 | NV1D3838 | 371 | |

Some isolated Protoxin-II variants inhibit human Nav1.7 activity with an IC₅₀ value of about 3x10⁻⁸ M or less.

Some isolated Protoxin-II variants inhibit human Nav1.7 activity with an IC₅₀ value of between about 3x10⁻⁸ M to about 1x10⁻⁹ M.

The disclosure also provides an isolated Protoxin-II variant comprising the amino acid sequence
GPQCX₁X₂WX₃QX₄CX₅X₆X₇X₈X₉CCX₁₀X₁₁FX₁₂CX₁₃LWCX₁₄KKLW (SEQ ID NO: 404), wherein
- X₁: is Q, R, K, A or S;
- X₂: is K, S, Q or R;
- X₃: is M or F;
- X₄: is T, S, R, K or Q;
- X₅: is D or T;
- X₆: is S, A or R;
- X₇: is E, R, N, K, T or Q;
- X₈: is R or K;
- X₉: is K, Q, S or A;
- X₁₀: is E, Q or D;
- X₁₁: is G or Q;
- X₁₂: is V or S;
- X₁₃: is R or T; and
- X₁₄: is K or R.

Exemplary Protoxin-II variants that inhibit human Nav1.7 activity with an IC₅₀ value of about 30x10⁻⁹ M or less are variants comprising the amino acid sequences of SEQ ID NOs: 56, 78, 111, 114, 117, 118, 119, 122, 123, 129, 130, 131, 132, 133, 134, 135, 136, 138, 139, 140, 141, 142, 145,146, 147, 149 ,150, 151, 152, 153, 154, 156, 158, 159, 165, 172, 173, 175, 177, 178, 183, 184, 185, 186, 189, 190, 193, 197, 199, 207, 210, 211, 216, 217, 224, 266, 273, 282 or 335.

In some embodiments, the isolated Protoxin-II variant selectively inhibits human Nav1.7. The Protoxin-II variants of the invention may be more selective towards Nav1.7 when compared to the recombinant Protoxin-II (SEQ ID NO: 2). In the QPatch electrophysiology assay, recombinant Protoxin-II has an IC₅₀ of about 2.2x10⁻⁹ M for Nav1.7 and an IC₅₀ of about 62x10⁻⁹ M for Nav1.6, and therefore the ratio of IC₅₀ for Nav1.6 to IC₅₀ for Nav1.7 about 28 fold. "Selectivity" or "selective" or "more selective" or "selectively blocks" or "selectively inhibits" when used herein refers to a Protoxin-II variant that has a ratio of IC₅₀ for Nav1.6 to IC₅₀ for Nav1.7 (IC₅₀(Nav1.6)/ IC₅₀(Nav1.7)) equal or over about 30. IC₅₀ for Nav1.6 may be assayed in a QPatch electrophysiology assay using cell lines stably expressing Nav1.6 using similar methods to those described for Nav1.7.

Residue positions in Protoxin-II that can be mutagenized to improve selectivity include residues 7 and 19, and optionally residues 1 and 11, and further optionally 12, 20, 22 and 26 (residue numbering according to SEQ ID NO: 1). Exemplary substitutions to improve selectivity are Y1Q, W7Q, S11R, S11A, E12T, M19F, V20S,, R22T, and K26R. Exemplary Protoxin-II variants with improved selectivity are variants of SEQ ID NOs: 56, 59, 65, 78, 111, 114, 117, 118, 119, 121, 122, 123, 129, 130, 133, 150, 190, 217, 281, 324, 325 or 326.

An isolated Protoxin-II variant may comprise the sequence GPX₁CQKWMQX₂CDX₃X₄RKCCX₅GFX₆CX₇LWCX₈KKLW (SEQ ID NO: 405); wherein
- X₁: is Y, Q, A, S or R;
- X₂: is T or S;
- X₃: is S, R or A;
- X₄: is E, T or N;
- X₅: is E or Q;
- X₆: is V or S;
- X₇: is R or T; and
- X₈: is K or R;
wherein the Protoxin-II variant inhibits human Nav1.7 activity with an IC₅₀ value of about 3x10⁻⁸ M or less, and selectively inhibits human Nav1.7.

An isolated Protoxin-II variant may comprise the sequence GPQCQKWMQX₁CDX₂X₃RKCCX₄GFX₅CX₆LWCX₈KKLW (SEQ ID NO: 406); wherein
- X₁: is T or S;
- X₂: is S, R or A;
- X₃: is E, T or N;
- X₄: is E or Q;
- X₅: is V or S;
- X₆: is R or T; and
- X₇: is K or R.

An isolated Protoxin-II variant may comprise the amino acid sequence that is 90%, 91%, 92%, 93%, 94%, 95%, 96%, 97%, 98% or 99% identical to the amino acid sequence of SEQ ID NO: 78 (GPQCQKWMQTCDRERKCCEGFVCTLWCRKKLW-COOH); wherein
the amino acid sequence has Q at position 1, Q at position 7 and F at position 19, when residue numbering is according to SEQ ID NO: 1;
the polypeptide inhibits human Nav1.7 activity with an IC₅₀ value of about 30x10⁻⁹ M or less, wherein the IC₅₀ value is measured using a FLIPR® Tetra membrane depolarization assay using fluorescence resonance energy transfer (FRET) in the presence of 25x10⁻⁶ M 3-veratroylveracevine in HEK293 cells stably expressing human Nav1.7; and
the polypeptide selectively inhibits Nav1.7.

In some embodiments, the isolated Protoxin-II variant has a free C-terminal carboxylic acid, amide, methylamide or butylamide group, which are generated via routine synthetic methods.

The disclosure also provides an isolated fusion protein comprising the Protoxin-II variant of SEQ ID NOs: 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 109, 110, 111, 112, 113, 114, 115, 116, 117, 118, 119, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154 ,155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225,226, 227, 228, 229, 230, 231, 232, 233, 234, 235, 236, 237, 238, 239, 240, 241, 242, 243, 244, 245, 246, 247, 248, 249, 250, 251, 252, 253, 254, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 35, 357, 358, 359, 360, 361, 362, 363, 364, 365, 366, 367, 368 367, 370 or 371. Such second polypeptides may be well known leader or secretory signal sequences, or synthetic sequences resulting for example from cloning steps, or tags such as hexahistidine tag (SEQ ID NO: 108). Such second polypeptide may be a half-life extending moiety. In one embodiment, the isolated fusion protein comprises the Protoxin-II variant of the invention conjugated to a half-life extending moiety.

Exemplary half-life extending moieties that can be used include well known human serum albumin, transthyretin (TTR), a thyroxine-binding globulin (TGB), albumin-binding domains, or an Fc or fragments thereof. Biologically suitable polymers or copolymers may also be used, for example ethylene glycol or polyethylene glycol (PEG) molecules, such as PEG5000 or PEG20000, dextran, polylysine, fatty acids and fatty acid esters of different chain lengths, for example laurate, myristate, stearate, arachidate, behenate, oleate, arachidonate, octanedioic acid, tetradecanedioic acid, octadecanedioic acid, docosanedioic acid, and the like, octane, or carbohydrates (dextran, cellulose, oligo- or polysaccharides). These moieties may be direct fusions with the Protoxin-II variant polypeptides and may be generated by standard cloning and expression techniques. Alternatively, well known chemical coupling methods may be used to attach the moieties to recombinantly produced Protoxin-II variants of the invention.

In another embodiment, the half-life extending moiety of the fusion protein of the invention is human serum albumin, albumin binding domain (ABD), or polyethylene glycol (PEG).

In another embodiment, the half-life extending moiety of is conjugated to the Protoxin-II variant via a linker. Suitable linkers are well known and include linkers having the sequence shown in SEQ ID NOs: 80 or 81.

Exemplary fusion proteins incorporating Protoxin-II variants of the invention are those having the polypeptide sequence of SEQ ID NOs: 83, 85, 87 or 103.

Protoxin-II variants of the invention incorporating additional moieties may be compared for functionality by several well-known assays. For example, pharmacokinetic properties of Protoxin-II variants coupled to PEG may be evaluated in well known *in vivo* models.

Additional substitutions to the Protoxin-II variants can be made as long as the resulting variant or the fusion protein retains similar characteristics when compared to the parent peptide. Exemplary modifications are for example conservative substitutions that will result in Protoxin-II variants with similar characteristics to those of the parent molecules. Conservative replacements are those that take place within a family of amino acids that are related in their side chains. Genetically encoded amino acids can be divided into four families: (1) acidic (aspartate, glutamate); (2) basic (lysine, arginine, histidine); (3) nonpolar (alanine, valine, leucine, isoleucine, proline, phenylalanine, methionine, tryptophan); and (4) uncharged polar (glycine, asparagine, glutamine, cysteine, serine, threonine, tyrosine). Phenylalanine, tryptophan, and tyrosine are sometimes classified jointly as aromatic amino acids. Alternatively, the amino acid repertoire can be grouped as (1) acidic (aspartate, glutamate); (2) basic (lysine, arginine histidine), (3) aliphatic (glycine, alanine, valine, leucine, isoleucine, serine, threonine), with serine and threonine optionally grouped separately as aliphatic-hydroxyl; (4) aromatic (phenylalanine, tyrosine, tryptophan); (5) amide (asparagine, glutamine); and (6) sulfur-containing (cysteine and methionine) (Stryer (ed.), Biochemistry, 2nd ed, WH Freeman and Co., 1981). Non-conservative substitutions can be made to the Protoxin-II variants that involve substitutions of amino acid residues between different classes of amino acids to improve properties of the Protoxin-II variants and Protoxin-II variant fusion proteins. Whether a change in the amino acid sequence of a polypeptide or fragment thereof results in a functional homolog can be readily determined by assessing the ability of the modified polypeptide or fragment to produce a response in a fashion similar to the unmodified polypeptide or fragment using the assays described herein. Peptides, polypeptides or proteins in which more than one replacement takes place can readily be tested in the same manner.

Another embodiment of the invention is an isolated synthetic polynucleotide comprising a polynucleotide encoding the Protoxin-II variant of the invention.

Certain exemplary synthetic polynucleotides are disclosed herein, however, other synthetic polynucleotides which, given the degeneracy of the genetic code or codon preferences in a given expression system, encode the Protoxin-II variants and Protoxin-II variant fusion proteins of the invention are also within the scope of the invention. Exemplary synthetic polynucleotides are for example polynucleotide sequences shown in SEQ ID NOs: 84, 86, 88 and 104, which encode the Protoxin-II variant fusion proteins of the invention. Those skilled in the art can readily identify the polynucleotide segments in the fusion proteins that encode the Protoxin-II variant itself. The synthetic polynucleotide sequences encoding the Protoxin-II variants or fusion proteins of the invention can be operably linked to one or more regulatory elements, such as a promoter and enhancer, that allow expression of the nucleotide sequence in the intended host cell. The synthetic polynucleotide may be a cDNA.

The polynucleotides of the invention may be produced by chemical synthesis such as solid phase polynucleotide synthesis on an automated polynucleotide synthesizer. Alternatively, the polynucleotides of the invention may be produced by other techniques such as PCR based duplication, vector based duplication, or restriction enzyme based DNA manipulation techniques. Techniques for producing or obtaining polynucleotides of known sequences are well known.

The polynucleotides of the invention may also comprise at least one non-coding sequence, such as transcribed but not translated sequences, termination signals, ribosome binding sites, mRNA stabilizing sequences, introns and polyadenylation signals. The polynucleotide sequences may also comprise additional sequences encoding additional amino acids. These additional polynucleotide sequences may, for example, encode a marker or well-known tag sequences such as a hexa-histidine (SEQ ID NO: 108) or a HA tag which facilitate the purification of fused polypeptides.

Another embodiment of the invention is a vector comprising the polynucleotide of the invention. Such vectors may be plasmid vectors, viral vectors, vectors for baculovirus expression, transposon based vectors or any other vector suitable for introduction of the polynucleotide of the invention into a given organism or genetic background by any means. For example, polynucleotides encoding the Protoxin-II variants or the Protoxin-II variant fusion proteins of the invention are inserted into an expression vector and may be operably linked to control sequences in the expression vector to ensure efficient expression, such as signal sequences, promoters (e.g. naturally associated or heterologous promoters), enhancer elements, and transcription termination sequences, and are chosen to be compatible with the host cell chosen to express the Protoxin-II variant or the Protoxin-II variant fusion protein of the invention. Once the vector has been incorporated into the appropriate host, the host is maintained under conditions suitable for high level expression of the proteins encoded by the incorporated polynucleotides.

Suitable expression vectors are typically replicable in the host organisms either as episomes or as an integral part of the host chromosomal DNA. Commonly, expression vectors contain selection markers such as ampicillin-resistance, hygromycin-resistance, tetracycline resistance, kanamycin resistance or neomycin resistance to permit detection of those cells transformed with the desired DNA sequences.

Suitable promoter and enhancer elements are known in the art. For expression in a bacterial cell, suitable promoters include, but are not limited to, lacl, lacZ, T3, T7, gpt, lambda P and trc. For expression in a eukaryotic cell, suitable promoters include, but are not limited to, light and/or heavy chain immunoglobulin gene promoter and enhancer elements; cytomegalovirus immediate early promoter; herpes simplex virus thymidine kinase promoter; early and late SV40 promoters; promoter present in long terminal repeats from a retrovirus; mouse metallothionein-I promoter; and various art-known tissue specific promoters. For expression in a yeast cell, a suitable promoter is a constitutive promoter such as an ADH1 PGK1, ENO or PYK1 promoter and the like, or a regulatable promoter such as a GAL1 or GAL10 promoter. Selection of the appropriate vector and promoter is well within the level of ordinary skill in the art.

Large numbers of suitable vectors and promoters are known to those of skill in the art; many are commercially available for generating recombinant constructs. The following vectors are provided by way of example. Bacterial: pBs, phagescript, PsiX174, pBluescript SK, pBs KS, pNH8a, pNH16a, pNH18a, pNH46a (Stratagene, La Jolla, Calif., USA); pTrc99A, pKK223-3, pKK233-3, pDR540, and pRIT5 (Pharmacia, Uppsala, Sweden). Eukaryotic: pWLneo, pSV2cat, pOG44, PXR1, pSG (Stratagene) pSVK3, pBPV, pMSG and pSVL (Pharmacia).

An exemplary vector for expression of the Protoxin-II variants or Protoxin-II variant fusion proteins is a vector having ampicillin-resistance selection marker, CMV promoter, CMV intron, signal peptide, neomycin resistance, f1 origin of replication, SV40 polyadenylation signal, and cDNA encoding the Protoxin-II variant or the Protoxin-II variant fusion protein of the invention.

Another embodiment of the invention is a host cell comprising the vector of the invention. The term "host cell" refers to a cell into which a vector has been introduced. It is understood that the term host cell is intended to refer not only to the particular subject cell but also to the progeny of such a cell. Because certain modifications may occur in succeeding generations due to either mutation or environmental influences, such progeny may not be identical to the parent cell, but are still included within the scope of the term "host cell" as used herein. Such host cells may be eukaryotic cells, prokaryotic cells, plant cells or archeal cells.

Escherichia coli, bacilli, such as Bacillus subtilis, and other enterobacteriaceae, such as Salmonella, Serratia, and various Pseudomonas species, are examples of prokaryotic host cells. Other microbes, such as yeast, are also useful for expression. Saccharomyces (e.g., S. cerevisiae) and Pichia are examples of suitable yeast host cells. Exemplary eukaryotic cells may be of mammalian, insect, avian or other animal origins. Mammalian eukaryotic cells include immortalized cell lines such as hybridomas or myeloma cell lines such as SP2/0 (American Type Culture Collection (ATCC), Manassas, VA, CRL-1581), NS0 (European Collection of Cell Cultures (ECACC), Salisbury, Wiltshire, UK, ECACC No. 85110503), FO (ATCC CRL-1646) and Ag653 (ATCC CRL-1580) murine cell lines. An exemplary human myeloma cell line is U266 (ATTC CRL-TIB-196). Other useful cell lines include those derived from Chinese Hamster Ovary (CHO) cells such as CHO-K1SV (Lonza Biologics, Walkersville, MD), CHO-K1 (ATCC CRL-61) or DG44.

Introduction of a polynucleotide, such as a vector, into a host cell can be effected by methods well known to those skilled in the art. Exemplary methods are calcium phosphate transfection, DEAE-Dextran mediated transfection, microinjection, cationic lipid-mediated transfection and electroporation.

Another embodiment of the invention is a method for producing the Protoxin-II variant of the invention comprising the steps of providing a host cell of the invention; culturing the host cell under conditions sufficient for the expression of at least one Protoxin-II variant of the invention; and recovering the Protoxin-II variant produced by the host cell.

Host cells can be cultured under any conditions suitable for maintaining or propagating a given type of host cell and sufficient for expressing a polypeptide. Culture conditions, media, and related methods sufficient for the expression of polypeptides are well known in the art. For example, many mammalian cell types can be aerobically cultured at 37°C using appropriately buffered DMEM media while bacterial, yeast and other cell types may be cultured at 37°C under appropriate atmospheric conditions in LB media.

In the methods of the invention, the expression of the Protoxin-II variant can be confirmed using a variety of well-known methods. For example, expression of a polypeptide can be confirmed using detection reagents, such as using SDS-PAGE or HPLC.

Another aspect of the disclosure is a method of modulating the activity of Nav1.7 in a biological tissue, the method comprising contacting the biological tissue expressing Nav1.7 with a Nav1.7-modulating amount of the Protoxin-II variant of the invention.

### USES OF PROTOXIN-II VARIANTS

Protoxin-II variants of the invention may be utilized in any therapy where it is desired to treat, reduce or alleviate symptoms of pain or other disorders of sensory or sympathetic neuron dysfunction.

Pain treated with the Protoxin-II variants of the invention may be any type of pain, such as chronic pain, acute pain, neuropathic pain, nociceptive pain, visceral pain, back pain, pain associated with inflammatory conditions, post-operative pain, thermal pain or pain associated with disease and degeneration.

Pain treated with the Protoxin-II variants of the invention may be Nav1.7-mediated pain.

Nav1.7-mediated pain as used herein refers to pain resulting at least partially from increased Nav1.7 channel activity.

The Protoxin-II variants of the invention may be used to treat an animal patient belonging to any classification. Examples of such animals include mammals such as humans, rodents, dogs, cats and farm animals.

The pain and/or Nav1.7-mediated pain may result from one or more causes, such as peripheral neuropathy, central neuropathy, nerve compression or entrapment syndromes such as carpal tunnel syndrome, tarsus tunnel syndrome, ulnar nerve entrapment, compression radiculopathy, lumbar spinal stenosis, sciatic nerve compression, spinal root compression, intercostal neuralgia, compression radiculopathy and radicular lower back pain, spinal root lesions, neuritis, autoimmune diseases, general inflammation, chronic inflammatory conditions, arthritis, rheumatic diseases, lupus, osteoarthritis, general gastrointestinal disorders, colitis, gastric ulceration, duodenal ulcers, inflammatory bowel disorders, irritable bowel syndrome, pain associated with diarrhea, inflammatory eye disorders, inflammatory or unstable bladder disorders, psoriasis, skin complaints with inflammatory components, sunburn, carditis, dermatitis, myositis, neuritis, collagen vascular diseases, inflammatory pain and associated hyperalgesia and allodynia, neuropathic pain and associated hyperalgesia and allodynia, multiple sclerosis, demyelinating diseases, diabetes, diabetic neuropathy pain, causalgia, pain resulting from amputation or abscess, phantom limb pain, fracture pain, bone injury, direct trauma, HIV infection, acquired immune deficiency syndrome ("AIDS"), small pox infection, herpes infection, exposure to toxins or other foreign particles or molecules, invasive cancer, cancer, chemotherapy, radiotherapy, hormonal therapy, burns, congenital defect, dental pain, gout pain, fibromyalgias, encephalitis, chronic alcoholism, hypothyroidism, uremia and vitamin deficiencies, trigeminal neuralgia, stroke, thalamic pain syndrome, general headache, migraine, cluster headache, tension headache, mixed- vascular and non-vascular syndromes, sympathetically maintained pain, deafferentation syndromes, asthma, epithelial tissue damage or dysfunction, disturbances of visceral motility at respiratory, genitourinary, gastrointestinal or vascular regions, wounds, burns, allergic skin reactions, pruritis, vasomotor or allergic rhinitis, or bronchial disorders, dysmenorrhoea, pain during labor and delivery, dyspepsia, gastroesophageal reflux, pancreatitis, and visceralgia.

Other disorders of sensory or sympathetic neuron dysfunction that may be alleviated by the Protoxin-II variants of the invention include itch, cough and asthma. In mice, global deletion of the SCN9A gene leads to complete insensitivity to histamine-induced itch (Gingras *et al.,* American Pain Society Meeting Abstract 2013 and U.S. Pat. Publ. No. 2012/0185956). This finding suggests that peptide Nav1.7 blockers may have utility in the treatment of itch, which may arise from various sources, such as dermatological or inflammatory disorders; or inflammatory disorders such as renal or hepatobiliary disorders, immunological disorders, medication reactions and unknown/idiopathic conditions, including dermatitis, psoriasis, eczema, insect sting or bite. Nav1.7 is also expressed in sensory nerves innervating the airways (Muroi et al., J Physiol. 2011 Dec 1;589(Pt 23):5663-76; Muroi et al., Am J Physiol Regul Integr Comp Physiol. 2013 Apr 10), suggesting that peptide Nav1.7 blockers may be beneficial in the treatment of cough e.g., acute or chronic cough, or cough caused by irritation from gastroesophageal reflux disease, and inflammatory diseases of the airways such as asthma and allergy-related immune responses, bronchospasm, chronic obstructive pulmonary disease, chronic bronchitis, emphysema, and hiccups (hiccoughs, singultus). Silencing Nav1.7 in vivo in nodose ganglia of guinea pigs using shRNA nearly abolished the cough reflex induced by mechanical probing (Muroi et al., Am J Physiol Regul Integr Comp Physiol. 2013 Apr 10).

The Protoxin-II variants of the invention can be used in a method of alleviating or treating itch, cough or asthma in a subject by administering a therapeutically effective amount of the Protoxin-II variant of the invention to a subject in need thereof for a time sufficient to alleviate the itch, cough or asthma.

The Protoxin-II variants of the invention can be used in a method of alleviating or treating Nav1.7-mediated itch, Nav1.7-mediated cough or Nav1.7-mediated asthma in a subject by administering a therapeutically effective amount of the Protoxin-II variant of the invention to a subject in need thereof for a time sufficient to alleviate the itch, cough or asthma.

Nav1.7-mediated itch as used herein refers to itch resulting at least partially from increased Nav1.7 channel activity.

Nav1.7-mediated cough as used herein refers to cough resulting at least partially from increased Nav1.7 channel activity.

Nav1.7-mediated asthma as used herein refers to asthma resulting at least partially from increased Nav1.7 channel activity.

Protoxin-II variants of the invention may be tested for their effect in reducing or alleviating pain and/or Nav1.7-mediated pain using animal models described herein, and models such as the rat spinal nerve ligation (SNL) model of neuropathic pain, carageenan induced allodynia model, the Freund's complete adjuvant (CFA)-induced allodynia model, the thermal injury model, the formalin model and the Bennett Model, and other models as described in U.S. Pat. Appl. No. 2011/0124711 and U.S. Pat. No. 7,998,980. Carageenan induced allodynia and CFA-induced allodynia are models of inflammatory pain. The Bennett model provides an animal model for chronic pain including post-operative pain, complex regional pain syndrome, and reflex sympathetic dystrophy.

Any of the foregoing animal models may be used to evaluate the efficacy of Protoxin-II variants of the invention inhibitor in treating pain and/or NAv1.7-mediated pain. The efficacy of the Protoxin-II variants of the invention may be compared to a no treatment or placebo control. Additionally or alternatively, efficacy may be evaluated in comparison to one or more known pain-relieving medicaments.

The present invention provides Protoxin-II variants for use in treating Nav1.7-mediated pain using the Protoxin-II variants of the invention. It has been discovered in the pending application by the inventors (U.S. Patent Application Number 61/781,276) that administration of Nav1.7 blocking peptides are efficacious in treating and/or alleviating pain in various animal models of pain, contrary to what was disclosed and suggested in the literature. While peptide inhibitors of Nav1.7 have been shown to be potent and/or selective towards Nav1.7 in *in vitro* cell culture models using overexpressed Nav1.7 or on isolated neurons in which the blood-nerve barrier is subverted through desheathing or hypertonic saline injection, they have so far proven non-efficacious in *in vivo* animal models of pain, where the lack of efficacy has been reported to result from the inability of the peptides to pass the blood-nerve barrier. Several publications describe lack of efficacy of Nav1.7 blocking peptides in animal models of pain or in isolated nerves. For example Hackel et al., Proc Natl Acad Sci 109:E2018-27, 2012, describes the inability of ProTx-II to inhibit action potential firing in isolated nerves unless the perineural barrier, which provides a diffusion barrier in this model, is compromised. ProTx-II was found non-efficacious in rodent models of acute and inflammatory pain; a likely explanation stated the inability of ProTx-II to cross the blood-nerve barrier (Schmalhofer et al., Mol Pharmacol 74:1476-1484, 2008). It has been proposed that Nav1.7 peptide toxin blockers have poor oral bioavailability and they are difficult to deliver to nerve endings, implying that their use as therapeutic agents remain limited (Dib-Hajj et al., Nature Rev Neuroscience 14, 49-62, 2013).

Nav1.7 is expressed in the peripheral nervous system e.g., in nociceptive dorsal root ganglions (DRG), most notably in nociceptive small-diameter DRG neurons, in particular in peripheral terminals in the skin, with little representation in the brain. Nav1.7 distribution (e.g. sensory ending) and physiology predispose it to a major role in transmitting painful stimuli.

One embodiment of the invention is a Protoxin-II variant for use in treating Nav1.7-mediated pain by administering a therapeutically effective amount of the Protoxin-II variant of the invention to a subject in need thereof for a time sufficient to treat the Nav1.7-mediated pain.

The Protoxin-II variants of the invention Nav1.7 may be utilized in any therapy where it is desired to treat Nav1.7-mediated pain or other disorders of sensory or sympathetic neuron dysfunction. "Treat" or "treatment" of pain is meant to include partially or completely to prevent, stop, inhibit, reduce, or delay the perception of pain.

In some embodiments, the Nav1.7-mediated pain is chronic pain, acute pain, neuropathic pain, nociceptive pain, visceral pain, back pain, post-operative pain, thermal pain, phantom limb pain, or pain associated with inflammatory conditions, primary erythemalgia (PE), paraoxysmal extreme pain disorder (PEPD), osteoarthritis, rheumatoid arthritis, lumbar discectomy, pancreatitis, fibromyalgia, painful diabetic neuropathy (PDN), post-herpetic neuropathy (PHN), trigeminal neuralgia (TN), spinal cord injuries or multiple sclerosis, or pain associated with disease and degeneration.

Neuropathic pain includes for example painful diabetic neuropathy (PDN), post-herpetic neuropathy (PHN) or trigeminal neuralgia (TN). Other causes of neuropathic pain include spinal cord injuries, multiple sclerosis, phantom limb pain, post-stroke pain and HIV-associated pain. Conditions such as chronic back pain, osteoarthritis and cancer may also result in the generation of neuropathic-related pain and thus are potentially suitable for treatment with the Protoxin-II variants of the invention.

In another embodiment, the Nav1.7-mediated pain is associated with primary erythemalgia (PE), paraoxysmal extreme pain disorder (PEPD), osteoarthritis, rheumatoid arthritis, lumbar discectomy, pancreatitis or fibromyalgia.

The Protoxin-II variants of the invention may be conjugated to a second polypeptide to form a fusion protein. Such fusion proteins are for example the well-known Fc fusions or fusions to human serum albumin to extend half-life of the peptide inhibitors. The conjugation may be a direct conjugation via a linker, such as a glycine-serine rich linker. Such linkers are well known in the art. The Protoxin-II variants of the invention incorporating additional moieties may be compared for their Nav1.7 blocking ability and efficacy in treatment or reducing pain using well known methods and those described herein.

Other disorders of sensory or sympathetic neuron dysfunction that can be treated with the Protoxin-II variants of the invention, including asthma, cough, heart-burn, itch, dermatitis, bladder instability, and Reynaud's disease.

### PHARMACEUTICAL COMPOSITIONS

The Protoxin-II variants of the invention may be formulated in a pharmaceutically acceptable vehicle or carrier. One embodiment of the invention is a pharmaceutical composition comprising the isolated Protoxin-II variant of the invention and a pharmaceutically acceptable excipient.

A suitable vehicle or carrier may be water for injection, physiological saline solution or artificial cerebrospinal fluid, possibly supplemented with other materials common in compositions for parenteral administration. Neutral buffered saline or saline mixed with serum albumin are further exemplary vehicles. These solutions are sterile and generally free of particulate matter, and may be sterilized by conventional, well-known sterilization techniques (e.g., filtration). The compositions may contain pharmaceutically acceptable excipients as required to approximate physiological conditions, such as pH adjusting and buffering agents, stabilizing, thickening, lubricating and coloring agents, etc. Suitable vehicles and their formulation and packaging are described, for example, in Remington: The Science and Practice of Pharmacy (21st ed., Troy, D. ed., Lippincott Williams & Wilkins, Baltimore, MD (2005) Chapters 40 and 41).

The Protoxin-II variants of the invention may be administered by peripheral administration. "Peripheral administration" or "administered peripherally" means introducing an agent into a subject outside of the central nervous system. Peripheral administration encompasses any route of administration other than direct administration to the spine or brain.

Peripheral administration can be local or systemic. Local administration may be used to concentrate the therapeutic to the site of action, such as local administration to joints, spinal cord, surgical wounds, sites of injury/trauma, peripheral nerve fibers, various organs (GI, urogenital, etc) or inflamed tissues. Systemic administration results in delivery of a pharmaceutical composition to essentially the entire peripheral nervous system of the subject and may also result in delivery to the central nervous system depending on the properties of the composition.

Routes of peripheral administration encompass, without limitation, topical administration, intravenous or other injection, and implanted mini-pumps or other extended release devices or formulations.

Pharmaceutical compositions of the invention include formulations involving the Protoxin-II variants of the invention in sustained- or controlled-delivery formulations. These formulations may be achieved through use of for example injectable microspheres, bio-erodible particles, microemulsions, nanoparticles, nanocapsules, macroemulsions, polymeric compounds (such as polyesters, polyamino acids, hydrogels, poly(lactic acid), polyglycolic acid or ethylene vinylacetate copolymers), beads or liposomes, hyaluronic acid or implantable drug delivery devices.

The Protoxin-II variants of the invention may be prepared for use for parenteral (subcutaneous, intramuscular or intravenous), intracerebral (intra-parenchymal), intracerebroventricular, intramuscular, intra-ocular, intraarterial, intraportal, or intralesional routes; by sustained release systems or by implantation devices, or any other administration, particularly in the form of liquid solutions or suspensions; for buccal or sublingual administration such as in the form of tablets or capsules; or intranasally such as in form of powders, nasal drops or aerosols or certain agents; transdermally in a form of a gel, ointment, lotion, cream or dusting powder, suspension or patch delivery system with chemical enhancers to either modify the skin structure or to increase the drug concentration in the transdermal patch, or with agents that enable the application of formulations containing proteins and peptides onto the skin (Int. Pat. Publ. No. WO98/53847), or applications of electric fields to create transient transport pathways such as electroporation, or to increase the mobility of charged drugs through the skin such as iontophoresis, or application of ultrasound such as sonophoresis (U.S. Pat. Nos. 4,309,989 and 4,767,402). The composition also may be administered locally via implantation of a membrane, sponge or another appropriate material onto which the desired molecule has been absorbed or encapsulated.

In certain embodiments, where an implantation device is used, the device may be implanted into any suitable tissue or organ, and delivery of the desired molecule may be via diffusion, timed-release bolus, or continuous administration.

The concentration of the Protoxin-II variants of the invention or other peptide inhibitors of Nav1.7 in such pharmaceutical formulation can vary widely, for example from about 0.1%, 0.2%, 0.3%, 0.4%, 0.5%, 0.6%, 0.7%, 0.8%, 0.9%, 1.0%, 1.1%, 1.2%, 1.3%, 1.4%, 1.5%, 1.6%, 1.7%, 1.8%, 1.9%, 2%, or between 2% to 5%, up to as much as 15%, 20%, 30%, 40%, 50%, 60% or 70% by weight and will be selected primarily based on fluid volumes, viscosities and other factors, according to the particular mode of administration selected. The Protoxin-II variants of the invention can be lyophilized for storage and reconstituted in a suitable vehicle prior to use. This technique has been shown to be effective with conventional protein preparations. Lyophilization and reconstitution techniques are well known in the art.

An exemplary pharmaceutical compositions of the present invention may comprise Tris buffer of about pH 7.0-8.5, or acetate buffer of about pH 4.0-5.5, and may further include sorbitol, sucrose, Tween-20 and/or a suitable substitute thereof.

The appropriate therapeutically effective dose may be determined readily by those skilled in the art. An effective dose refers to an amount or dosage sufficient to produce a desired result, i.e. to partially or completely prevent, stop, inhibit, reduce, or delay the perception of pain associated with any painful medical condition. The effective amount may vary depending on the specific vehicle and the Protoxin-II variants of the invention selected, and is also dependent on a variety of factors and conditions related to the subject to be treated and the severity of the pain. For example, factors such as age, weight and health of the subject to be administered with the pharmaceutical compositions of the invention as well as dose response curves and toxicity data obtained in preclinical animal work could be among those considered. A determined dose may, if necessary, be repeated at appropriate time intervals selected as appropriate by a physician or other person skilled in the relevant art (e.g. nurse, veterinarian, or veterinary technician) during the treatment period. The determination of an effective amount or a therapeutically effective amount for a given agent is well within the ability of those skilled in the art.

Thus, a pharmaceutical composition of the invention for intramuscular injection could be prepared to contain 1 ml sterile buffered water, and between about 1 ng to about 100 mg, about 50 ng to about 30 mg or about 5 mg to about 25 mg of a Protoxin-II variant of the invention. Similarly, a pharmaceutical composition of the invention for intravenous infusion could be made up to contain about 250 ml of sterile Ringer's solution, and about 1 mg to about 30 mg or about 5 mg to about 25 mg of the Protoxin-II variants of the invention. Actual methods for preparing parenterally administrable compositions are well known and are described in more detail in, for example, "Remington's Pharmaceutical Science", 15th ed., Mack Publishing Company, Easton, PA.

The present invention will now be described with reference to the following specific, non-limiting examples.

### Example 1: Design and generation of Protoxin-II variants

Protoxin-II single position limited amino acid scanning library substitution was designed to assess to what degree selectivity, peptide yield, and homogeneity can be improved.

Protoxin-II variants were designed as HRV3C protease cleavable HSA fusion proteins in the following format from N-to C- terminus: 6xHis-HSA-linker-HRV3C cleavable peptide-Protoxin-II variant ("6xHis" disclosed as SEQ ID NO: 108); linker being (GGGGSGGGGSGGGGSGGGGS; SEQ ID NO: 80, HSA having the sequence of SEQ ID NO: 106, HRV3C cleavable peptide having the sequence of SEQ ID NO: 82). Each Protoxin-II variant, after cleavage from HSA had a residual N-terminal GP from the cleavage site.

The variants were characterized in membrane depolarization assays using FLIPR® Tetra as described in Example 3 FLIPR® Tetra membrane depolarization assay, and in whole cell patch clamp experiments using the QPatch assay as described in Example 3.

Combinatorial libraries were designed to test for additive effects of select single position hits in an attempt to generate Nav1.7 antagonists with further improved potency and selectivity profile compared to the native peptide.

### Construction of the Expression Vectors

The designed Protoxin-II variant genes were generated using synthetic gene assembly technology as described in U.S. Pat. No. US6,521,427. The amino acid sequences of the designed peptide variants were back-translated to DNA sequences using human high-frequency codons. The DNA sequence of each variant gene, together with a portion of vector DNA including the DNA cloning sites, was synthesized as multiple oligonucleotides, some of which contained degenerate codons, and assembled into full-length DNA fragments. The assembled DNA fragments were amplified by PCR and PCR products were subsequently cloned as a pool. Pooled PCR products were digested with the appropriate restriction enzymes and cloned into the designed expression vector in such a manner as to fuse each toxin variant gene to the signal peptide and the fusion partner (6xHis-HSA-linker-HRV3C cleavable peptide ("6xHis" disclosed as SEQ ID NO: 108) contained in the vector. Standard molecular biology techniques were used to identify a positive clone for each designed variant. The plasmid DNA from these positive clones was purified and sequence confirmed before expressing the Protoxin-II peptide variant fusion proteins using standard methods.

### Protein Expression

HEK 293-F cells were maintained in 293 Freestyle™ media (Invitrogen Cat # 12338) and split when the cell concentration was between 1.5 and 2.0 x 10⁶ cells per ml. The cells were grown in suspension, shaking at 125 RPM in a humidified incubator set at 37°C and 8% CO₂. HEK 293F cells were transiently transfected using a DNA/lipid complex after they were diluted to 1.0 x 10⁶ cells per ml. To generate the complex, 1.25 µg DNA per ml of transfection was diluted in 1.0 ml of OptiPro media (Invitrogen Cat # 12309) and 1.25 ml of Freestyle™ Max transfection reagent (Invitrogen Cat # 16447) was diluted in 1.0 ml of OptiPro media. The DNA and Max transfection reagent were mixed together and incubated for 10 minutes at room temperature before adding to the cells. Transfected cells were placed in a humidified incubator set at 37°C and 8% CO₂ for 4 days shaking at 125 RPM. The supernatant was separated from the cells by centrifugation at 5,000 x g for 10 minutes and filtered through a 0.2µm filter (Corning; Cat #431153), then concentrated 10 and 50 fold using an Amicon Ultra Concentrator 10K (Cat #UFC901096), and centrifuging for approximately 10 minutes at 3,750 x g.

### Example 2: Purification of Protoxin-II variants

Protoxin-II variants were expressed as HSA fusion proteins as indicated in Example 1 and the Protoxin-II variant peptides were cleaved with HRV3C protease prior to purification. Two methodologies were tested for efficient purification of the Protoxin-II variants.

### Protein Purification

### Purification of Protoxin-II Variants by RP-HPLC

The secreted proteins were purified from the expression supernatants via IMAC using 1 ml HisTrap HP columns (GE Healthcare Cat# 17-5247-01). The chromatography method was run using an AKTA Xpress and protein was eluted from the column using a step gradient of Imidazole. Peak fractions were pooled and digested overnight with HRV 3C protease (1µg protease / 150µg fusion).

Cleaved peptide-fusion pools were further purified using a Dionex HPLC system with a reverse phase Phenomenex Luna 5 µm C18(2) column (Cat# 00B-4252-P0-AX). Samples were eluted from the column with a 0-68% Acetonitrile (0.05% TFA) linear gradient. Elution fractions were pooled, lyophilized overnight and reconstituted in HEPES buffered saline, pH 7.4 (10 mM HEPES, 137mM NaCl, 5.4 mM KCl, 5 mM glucose, 2mM CaCl₂, 1mM MgCl₂).

Table 4 shows yields of Protoxin-II variants purified by RP-HPLC. The average mg yield/L was 0.01615.

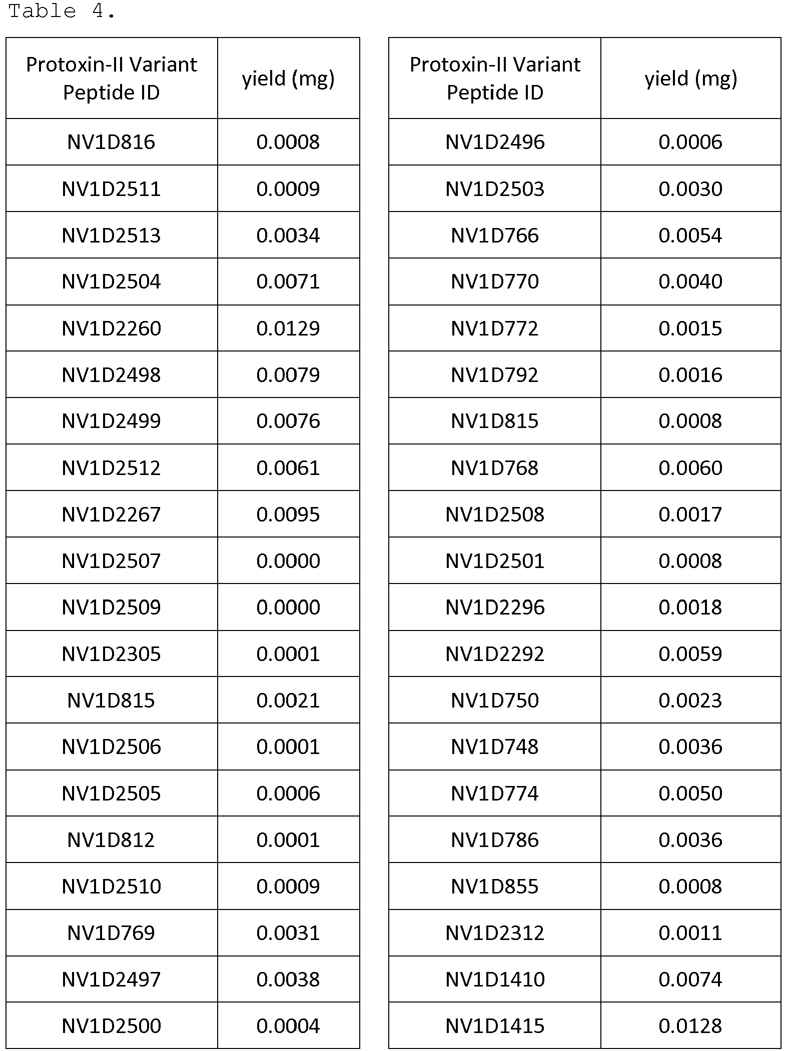

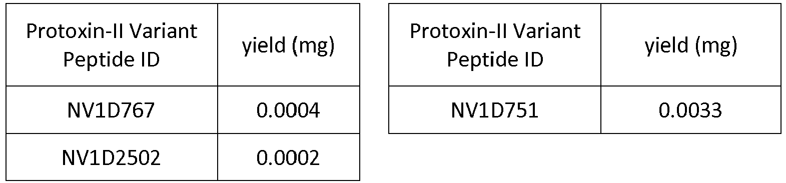

### Purification of Protoxin-II Variants by Solid Phase Extraction (SPE)

The secreted proteins were purified from the expression supernatants via IMAC using 1 ml HisTrap HP columns (GE Healthcare Cat# 17-5247-01). The chromatography method was run using an AKTA Xpress and protein was eluted from the column using a step gradient of Imidazole. Peak fractions were pooled and digested overnight with HRV3C protease (1µg protease / 150µg fusion). The cleaved sample was loaded into a 50 kDa molecular weight cut off centrifugal filter unit (Millipore UFC805096) and cleaved peptide collected in the filtrate fraction.

Peptide pools were loaded onto a 96-well solid phase extraction block (Agilent Bond Elut Plexa A3969030) for further purification, desalting, and concentration. Blocks were used in conjunction with a vacuum manifold (Whatman). Peptide samples were loaded and washed in 0.05% TFA in water and eluted with a step gradient of acetonitrile with 0.05% TFA in water. Elution fractions were then lyophilized overnight and reconstituted in HEPES buffered saline, pH 7.4 (10 mM HEPES, 137mM NaCl, 5.4 mM KCl, 5 mM glucose, 2mM CaCl₂, 1mM MgCl₂).

Peptides were reconstituted in supplemented HEPES buffered saline, pH 7.4 (10 mM HEPES, 137mM NaCl, 5.4 mM KCl, 5 mM glucose, 2mM CaCl₂, 1mM MgCl₂) and absorbance was measured at 280 nm. Concentration values were then calculated using each sample's extinction coefficient. 2 µg of each peptide were loaded onto an Invitrogen NuPAGE® Novex® Bis-Tris Gel 15 well gel and run in MES buffer non-reduced.

Samples were analyzed on Agilent 1100 HPLC using 4-80% acetonitrile in 0.05% TFA linear gradient with a Phenomenex Luna C18(2) analytical column (Cat#00A-4041-B0). Concentrations of all peptides were normalized and 10 µl of each were injected for a total of 1.3 µg per sample. Absorbance at 220 nm was monitored and chromatograms analyzed were using Chromeleon software.

Table 5 shows yields (mg) of Protoxin-II variants purified by SPE. The average mg yield/L was 0.05353.

The benefits of the SPE purification process are ease and throughput of purification since samples are processed in parallel in a 96-well block rather than serially on RP-HPLC, and improvement in yield. There was, on average, more than 3-fold higher yield (mg/L) for variants purified by SPE versus RP-HPLC.

**Table 5.**

| Protoxin-II Variant Peptide ID | yield (mg) | | Protoxin-II Variant Peptide ID | yield (mg) |
|---|---|---|---|---|
| NV1D12 | 0.0054 | | NV1D2734 | 0.0602 |
| NV1D2659 | 0.0234 | | NV1D2772 | 0.2050 |
| NV1D2664 | 0.0060 | | NV1D2775 | 0.2225 |
| NV1D2666 | 0.0225 | | NV1D2738 | 0.0512 |
| NV1D2708 | 0.0721 | | NV1D2740 | 0.0373 |
| NV1D2725 | 0.0144 | | NV1D2733 | 0.1913 |
| NV1D2739 | 0.0053 | | NV1D788 | 0.0000 |
| NV1D2765 | 0.0097 | | NV1D757 | 0.0021 |
| NV1D2748 | 0.0995 | | NV1D791 | 0.0007 |
| NV1D2771 | 0.0103 | | NV1D2310 | 0.0011 |
| NV1D2770 | 0.0121 | | NV1D2308 | 0.0014 |
| NV1D2778 | 0.0644 | | NV1D778 | 0.0019 |
| NV1D2782 | 0.0202 | | NV1D2294 | 0.0000 |
| NV1D2756 | 0.0466 | | NV1D856 | 0.0047 |
| NV1D2759 | 0.0218 | | NV1D2309 | 0.0023 |
| NV1D2712 | 0.0558 | | NV1D846 | 0.0020 |
| NV1D12 | 0.0127 | | NV1D2896 | 0.0504 |
| NV1D2673 | 0.0625 | | NV1D2913 | 0.0203 |
| NV1D2662 | 0.0433 | | NV1D2910 | 0.0253 |
| NV1D2669 | 0.2661 | | NV1D2893 | 0.0569 |
| NV1D2665 | 0.0389 | | NV1D2909 | 0.0195 |
| NV1D2731 | 0.2547 | | NV1D2917 | 0.0339 |
| NV1D2767 | 0.0238 | | NV1D2914 | 0.0201 |
| NV1D2730 | 0.2566 | | NV1D2922 | 0.0554 |
| NV1D2766 | 0.0198 | | NV1D2902 | 0.0061 |
| NV1D2667 | 0.0050 | | NV1D2889 | 0.0022 |
| NV1D2769 | 0.0142 | | NV1D2887 | 0.0025 |
| NV1D2719 | 0.0675 | | NV1D2878 | 0.0272 |
| NV1D2776 | 0.0633 | | NV1D2877 | 0.0129 |
| NV1D2663 | 0.0344 | | NV1D2851 | 0.0029 |
| NV1D2709 | 0.1841 | | NV1D2850 | 0.0026 |
| NV1D2720 | 0.0538 | | NV1D2820 | 0.0020 |
| NV1D12 | 0.0095 | | NV1D2819 | 0.0015 |
| NV1D2773 | 0.1921 | | NV1D2814 | 0.0163 |
| NV1D2810 | 0.0086 | | NV1D2918 | 0.0256 |
| NV1D2732 | 0.0262 | | NV1D2921 | 0.0533 |
| NV1D757 | 0.0026 | | NV1D2905 | 0.0126 |
| NV1D791 | 0.0206 | | NV1D2906 | 0.0189 |
| NV1D2310 | 0.0085 | | NV1D2881 | 0.0207 |
| NV1D2308 | 0.0179 | | NV1D2882 | 0.0223 |
| NV1D778 | 0.0094 | | NV1D2869 | 0.0038 |
| NV1D856 | 0.0247 | | NV1D2870 | 0.0187 |
| NV1D2309 | 0.0035 | | NV1D2867 | 0.0147 |
| NV1D846 | 0.0043 | | NV1D2888 | 0.0045 |
| NV1D2889 | 0.0107 | | NV1D2816 | 0.0133 |
| NV1D2887 | 0.0061 | | NV1D2885 | 0.0025 |
| NV1D2861 | 0.0469 | | NV1D2974 | 0.0418 |
| NV1D2729 | 0.1101 | | NV1D2972 | 0.1089 |
| NV1D2890 | 0.0088 | | NV1D2971 | 0.0407 |
| NV1D2899 | 0.0402 | | NV1D2970 | 0.0557 |
| NV1D2804 | 0.0044 | | NV1D2969 | 0.0799 |

### Example 3: Characterization of Protoxin-II variants

Select Protoxin-II variants were characterized in membrane depolarization and whole cell patch clamp assays to assess their potency and selectivity towards Nav1.7.

### FLIPR® Tetra membrane depolarization assay

The ability of the generated peptides to inhibit membrane depolarization induced by Nav1.7 agonist veratridine (3-Veratroylveracevine; Biomol, Catalog# NA125) was measured with a FRET (fluorescence resonance energy transfer) assay on FLIPR® Tetra using DISBAC2(3) (Invitrogen, K1018) as an electron acceptor and PTS18 (Trisodium 8-octadecyloxypyrene-1,3,6-trisulfonate) (Sigma) as a donor by exciting the donor at 390-420 nm and measuring FRET at 515-575 nm.

HEK293 cells stably expressing human Nav1.7 were cultured in DMEM/F-12 media (1:1), supplemented with 10% fetal bovine serum, 1% penicillin/streptomycin, 400 pg/mL geneticin and 100 µM NEAAs (all reagents from Invitrogen). 50 µL of harvested cells were plated at 25,000 cells/well into poly-lysine coated 384-well black clear bottom plates. The plates were incubated at room temperature (RT) for 15 min followed by an overnight incubation at 37°C. All incubations were done in the dark unless otherwise stated. The next day, the wells were washed 4 times with assay buffer (137 mM NaCl, 4mM KCl, 2mM MgCl₂, 2mM CaCl₂, 5 mM Glucose, 10 mM HEPES, pH 7.4), and resuspended in 25 µL of assay buffer. 2x stock (6 µM) of the PTS18 dye was prepared by suspending the dye in 10% pluronic F127 in DMSO at 1:1 (v/v ratio). 25 µL of the 2x PTS18 stock was added into the wells and the cells were stained for 30 min at RT, after which the dye was washed off with the assay buffer. Peptides were suspended at 3x their final concentration in the assay buffer containing 10 µM DISBAC2(3) and 400 µM VABSC-1 to suppress background fluorescence (Sigma, cat# 201987). 25 pL/well of the suspended peptides were added into each well, and incubated for 60 minutes at RT. Depolarization was induced by 25 µM final concentration of veratridine (by adding 25 µL/well of 75µM (3x) stock solution), and the reduction in the mean intensity of FRET dye fluorescence was measured 30-100 seconds after adding the agonist. A 1.3X dilution of each measured peptide occurred after adding veratridine by convention, the concentration at the beginning of the FLIPR® Tetra assay is reported.

Concentration-response curves of synthetic Protoxin-II (Peptide International) were constructed in each experimental series and were used as controls. Fluorescence counts for each well were converted to % response by normalizing the signal to the difference between negative control (response to agonist veratridine alone) and positive control (response to veratridine in the presence of 10 µM tetracaine) values. For measurements, "spatial uniformity correction" (all fluorescence traces are normalized to the average initial starting intensity) and "subtract bias value" (subtract the initial starting intensity from each trace) were turned on in FLIPR® Tetra. Each data point represented the response in an individual well. All individual data points were used in a non-linear least-squares fitting procedure to find the best fit to a Hill function using Origin (Microcal). IC₅₀ values were extracted from the resultant fitted curve. The mean responses of the positive (P) and negative (N) controls were used to calculate the % response in a well as follows: % response = 100^{∗}(N-R)/(N-P).

Assay plates were accepted if the potency of control antagonists for that day were within ±0.5 log units of their historical mean.

### QPatch Assay

HEK293 cells stably expressing human Nav1.5 (SEQ ID NO: 105), Nav1.7 (SEQ ID NO: 79) or Nav1.6 (SEQ ID NO: 407) were cultured in DMEM/F-12 media (1:1), supplemented with 10% fetal bovine serum, 1% penicillin/ streptomycin, 400 µg/mL Geneticin and 100 µM NEAAs (all reagents from Invitrogen). Cells were maintained at 37°C and in 5% CO2 and assayed upon reaching ∼50-90% confluency. CHO cells stably expressing human Nav1.6 in a tetracycline-inducible manner(SEQ ID NO: 407) were cultured in HAMs F12, supplemented with 10% fetal bovine serum, 1% penicillin/ streptomycin, 10 pg/mL Blasticidin and 400 µg/mL Zeocin. Cells were maintained at 37°C and in 5% CO2, and assayed upon reaching ∼50-90% confluency. Nav1.6 expression was induced with 1 µg/ml of tetracycline, 24-48h prior to an experiment.

Before testing in QPatch HT (Sophion), cells were first dissociated using 0.05% trypsin (5 min at 37°C), resuspended in CHO-S-SFM media (Life Technologies) and gently triturated to break up cell clumps. Cell density was adjusted to 1-2×10⁶/mL with the same media and cells were the transferred to a cell "hotel" in QPatch HT and used in experiments for several hours. For giga-ohm seal formation and whole-cell patch clamp recording, the extracellular solution contained 137 mM NaCl, 5.4 mM KCl, 1 mM MgCl₂, 2 mM CaCl₂, 5 mM glucose, and 10 mM HEPES, pH = 7.4 and osmolarity = 315 mOsm. The intracellular solution contained 135 mM CsF, 10 mM CsCl, 5 mM EGTA, 5 mM NaCl and 10 mM HEPES, pH = 7.3 and osmolarity = 290 mOsm. The voltage protocol used in the assay was as follows. From a holding potential of -75 mV (Nav1.7), -60 mV (Nav1.6), or -105 mV (Nav1.5) cells were first hyperpolarized to -120 mV for 2 sec and then depolarized to 0 mV for 5 ms before returning to the holding potential. This protocol was repeated once every 60 sec during liquid applications (see below). Cells were otherwise held at the holding potential when the above voltage protocol was not executed. Upon establishment of the whole-cell recording configuration, a total of five applications of the extracellular solution (all containing 0.1% bovine serum albumin (BSA) with or without test compound, except for the last application, which contained 1 µM TTX or 10 mM lidocaine as a positive control) were made on to cells being recorded. The first liquid application contained only the control buffer (5 µl). The voltage protocol was executed 10 times (for a total duration of 10 min) five sec after the application. The next three liquid applications (5 µl each) contained a test compound (same compound at the same concentration for all three applications) or control buffer (for control cells only). Five seconds after each of these applications, the voltage protocol was again executed 10 times (also once per min). The last application contained positive (composed of three 10µl sub-applications, each separated by 2 sec), five seconds after which the same voltage protocol was executed twice to obtain the baseline current. Currents were sampled at 25 kHz and filtered at 5 kHz with an 8-pole Bessle filter. The series resistance compensation level was set at 80%. For each cell, the peak current amplitude at 0 mV for each current trace in the first four liquid applications was first subtracted from that of the last trace in the presence of positive control and then normalized to that of the last trace in the first (control buffer) application as % inhibition. To control for current rundown, this (% inhibition) value for each cell in the presence of a test compound was further normalized to the average % inhibition value for control (typically 5-6) cells in the same experiment. The mean of the last two such values in the last compound application (i.e., the corrected % inhibition value for each concentration of a test compound) were taken as the % inhibition value for each cell at the particular compound concentration tested. The % inhibition values for all cells tested at each compound concentration were averaged and used in concentration response calculations. All experiments were performed at room temperature (∼22 °C). Data are expressed as mean±se. Wild type Protoxin-II was included in each experiment as a positive control. Data were accepted only if the potency of Protoxin-II was within ±0.5 log units of its historical mean.

IC₅₀ values for Nav1.7 for select Protoxin-II variants obtained using the FLIPR® Tetra are shown in Table 6.

**Table 6.**

| Protein ID | Protoxin-II Variant Peptide ID | Protoxin-II variant Peptide SEQ ID NO: | hNav1.7 |
|---|---|---|---|
| | | | TETRA |
| | | | IC₅₀ (nM) |
| NV1D12_5 | NV1D12 | 2 | 4.1±3.6 |
| NV1G1045 | NV1D791 | 11 | 4.8±0.4 |
| NV1D1332_1 | NV1D1332 | 12 | 6.7±0.5 |
| NV1D1336_1 | NV1D1336 | 14 | 10.5±1.2 |
| NV1D1337_1 | NV1D1337 | 15 | 10.3±1.0 |
| NV1G1049 | NV1D2308 | 16 | 4.5±0.4 |
| NV1 G953 | NV1D2670 | 17 | 22.2±3.3 |
| NV1 G951 | NV1D2674 | 18 | 4.0±0.2 |
| NV1 G963 | NV1D2671 | 20 | 31.5±6.4 |
| NV1G949 | NV1D2675 | 21 | 4.3±0.3 |
| NV1 G977 | NV1D2665 | 22 | 4.9±0.4 |
| NV1G957 | NV1D2668 | 23 | 17.5±2.6 |
| NV1 G965 | NV1D2672 | 24 | 4.5±0.3 |
| NV1 G973 | NV1D2662 | 25 | 4.0±0.4 |
| NV1 G975 | NV1D2669 | 26 | 18.4±5.7 |
| NV1 G971 | NV1D2673 | 27 | 4.3±0.5 |
| NV1 G995 | NV1D2663 | 28 | 4.2±0.4 |
| NV1 G961 | NV1D2676 | 29 | 26.5±2.9 |
| NV1G911 | NV1D2666 | 30 | 66.5±36.7 |
| NV1G1133 | NV1D2816 | 31 | 667±93.6 |
| NV1 G905 | NV1D2735 | 32 | 60.0±16.2 |
| NV1 G979 | NV1D2731 | 34 | 20.7±7.2 |
| NV1G1097 | NV1 D281 0 | 35 | 339±5750 |
| NV1G1099 | NV1D2732 | 36 | 126±26.9 |
| NV1G1011 | NV1D2740 | 37 | 3.6±9.9 |
| NV1G1105 | NV1D2729 | 39 | 8.0±0.9 |
| NV1G1013 | NV1D2733 | 40 | 7.5±2.9 |
| NV1G1095 | NV1D2814 | 41 | 754±51.3 |
| NV1 G983 | NV1D2730 | 43 | 25.5±4.3 |
| NV1G1003 | NV1D2734 | 44 | 13.4±0.8 |
| NV1G1009 | NV1D2738 | 45 | 2.6±0.2 |
| NV1G1129 | NV1D2867 | 49 | >1000 |
| NV1G1121 | NV1D2881 | 50 | 488±72.2 |
| NV1G1123 | NV1D2882 | 51 | 857±65.7 |
| NV1 G899 | NV1D2774 | 52 | 50.5±15.2 |
| NV1G1103 | NV1D2861 | 54 | >1000 |
| NV1G1127 | NV1D2870 | 55 | 784±84.8 |
| NV1G1007 | NV1D2775 | 56 | 25.4±2.0 |
| NV1G1067 | NV1D2893 | 57 | 75.5±10.5 |
| NV1G1005 | NV1D2772 | 59 | 15.6±1.8 |
| NV1G1061 | NV1D2896 | 60 | 80.3±7.1 |
| NV1G1085 | NV1D2877 | 61 | 441±73.3 |
| NV1G1083 | NV1D2878 | 62 | 680±40.7 |
| NV1G1079 | NV1D2889 | 64 | 12.1±1.5 |
| NV1G1001 | NV1D2773 | 65 | 18.8±1.5 |
| NV1G1107 | NV1D2890 | 66 | 25.8±4.2 |
| NV1G1109 | NV1D2899 | 67 | 33.3±6.7 |
| NV1G1117 | NV1D2905 | 68 | 713±87.3 |
| NV1G1119 | NV1D2906 | 69 | 940±86.7 |
| NV1G1115 | NV1D2921 | 70 | 586±71.7 |
| NV1G1075 | NV1D2922 | 71 | 204±45.7 |
| NV1G1069 | NV1D2909 | 72 | 97.1±10.1 |
| NV1G1065 | NV1D2910 | 73 | 441±41.7 |
| NV1G1063 | NV1D2913 | 74 | 79.7±9.3 |
| NV1G1073 | NV1D2914 | 75 | 135±7.8 |
| NV1G1071 | NV1D2917 | 76 | 197±48.3 |
| NV1G1113 | NV1D2918 | 77 | 983±98.7 |
| NV1G1153 | NV1D3034 | 78 | 10.3±2.1 |

Select Protoxin-II variants were tested for selectivity against human Nav1.5 using QPatch. IC₅₀ values for both Nav1.7 and Nav1.5 for select peptides obtained using QPatch are shown in Table 7.

**Table 7.**

| Protein ID | Protoxin-II Variant Peptide ID | Protoxin-II variant Peptide SEQ ID NO: | hNav1.7 | hNav1.5 |
|---|---|---|---|---|
| | | | QPatch | |
| | | | IC₅₀ (nM) | IC₅₀ (nM) |
| NV1D12_5 | NV1D12 | 2 | 2.2±1.3 | >1000 |
| NV1G899 | NV1D2774 | 52 | 18.7±13.6 | >3000 |
| NV1G1007 | NV1D2775 | 56 | 4.0±8.9 | >3000 |
| NV1G1005 | NV1D2772 | 59 | 6.2±3.2 | >3000 |
| NV1G1001 | NV1D2773 | 65 | 4.3±3.3 | >3000 |
| NV1G1153 | NV1D3034 | 78 | 4.3±4.3 | >1000 |

### Example 4. Generation and characterization of combinatorial Protoxin-II variants

Combinatorial libraries were designed to test for additive effects of select single position hits in an attempt to generate Nav1.7 antagonists with further improved potency and selectivity profile compared to the native peptide using several approaches.

A limited amino acid scan was conducted at all non-cysteine Protoxin-II positions using A, D, Q, R, K and S for diversification. In these experiments, Protoxin-II was expressed and tested as monovalent Fc fusion protein as described in Example 1. From this scan, substitutions Y1Q, W7Q, S11A, were identified that improved potency and/or selectivity of the resulting variants.

A full amino acid scan (excluding cys and trp) at positions M6 and M19 was also conducted. M19F substitution was identified from this scan that improved potency and/or selectivity of the resulting variants.

Protoxin-II/Huwentoxin-IV single position chimeras were designed bidirectionally. The purpose of this library was to obtain Protoxin-II variants that retained potency and selectivity profile of the wild type Protoxin-II and would achieve beneficial refolding properties associated with Huwentoxin-IV. Substitutions R22T and E12N were identified from this scan.

Peptide NV1G1153 was further engineered by diversifying position Y1 by a limited amino acid scan using R, K, T, A, D, E, Q and S, and by charge cluster engineering, where all sets of charged residues in the three-dimensional structure of the peptide (D10/E12, K4/E17, D10/E12/R13) were mutated.

N- and C-terminal extensions were introduced to select peptides, including NV1G1153 with the purpose of improving peptide distribution to the site of action and of improving half-life of the peptides without significantly increasing the molecular weight of the resulting peptide. The N- and C-terminal extensions that were used are shown in Table 8 and 9, respectively, and are described in Oi et. al., Neuroscience Letters 434, 266-272, 2008; Whitney et. al., Nature Biotechnology 2011 29:4, 352-356; Sockolosky et. al., (2012) 109:40, 16095-16100. Cell penetrating peptides HIV Tat and polyarginine were also used. Various linkers were used to couple the Protoxin-II variant to the N- and/or C-terminal extensions. The linkers used are shown in Table 10.

Protoxin-II variants from each campaign were tested for their potency and selectivity for Nav1.7 using methods described in Example 3. The amino acid sequences of the variants that inhibited Nav1.7 with an IC₅₀ value of 200 nM or less are shown in Table 3. Table 11 shows the amino acid substitutions in select variant when compared to the wild type Protoxin-II, and the IC₅₀ values for Nav1.7 inhibition in the FLIPR Tetra assay.

**Table 8.**

| N-terminal extension | |
|---|---|
| Amino acid sequence | SEQ ID NO: |
| GPAAAAA | 372 |
| GPAPAPA | 373 |
| GGGGGG | 374 |
| GPCCNCSSKWCRDHSRCC | 375 |
| GPSPGARAF | 376 |
| GPDGPWRKM | 377 |
| GPFGQKASS | 378 |
| GPCRTIGPSVC | 379 |
| GPSHSNTQTLAKAPEHTG | 380 |
| GPQRFVTGHFGGLYPANG | 381 |
| GPGWCGDPGATCGKLRLYCCSGFCDSYTKTCKDKSSA | 382 |
| APAPAPAPAP | 383 |
| GPYGRKKRRQRRR | 384 |
| GPRRRRRRRRRRR | 385 |

**Table 9.**

| C-terminal extensions | |
|---|---|
| Amino acid sequence | SEQ ID NO: |
| CRTIGPSVC | 386 |
| YGRKKRRQRRR | 387 |
| GGGGG | 374 |
| DGPWRKM | 388 |
| CCNCSSKWCRDHSRCC | 389 |
| RRRRRRRRRRR | 390 |
| SHSNTQTLAKAPEHTG | 391 |
| APAPA | 392 |
| AAAAA | 393 |
| FGQKASS | 394 |
| QRFVTGHFGGLYPANG | 395 |
| SPGARAF | 396 |
| GPGWCGDPGATCGKLRLYCCSGFCDAYTKTCKDKSSA | 397 |

**Table 10.**

| Linkers | |
|---|---|
| Amino acid sequence | SEQ ID NO: |
| GSAPAPAPAPAPGS | 398 |
| GSAPAPAPAPAPAPAPAPAPAPGS | 399 |
| | 400 |
| APAPA | 392 |
| | 401 |
| APAPAPAPAP | 383 |
| APAPAPAPAPAPAPAPAPAP | 402 |

**Table 11.**

| Protein name | Protoxin-II variant peptide name | Protein SEQ ID NO: | Substitutions | Nav1. 7 IC₅₀ (nM) | SE |
|---|---|---|---|---|---|
| NV1G1728 | NV1D3541 | 281 | Y1A,W7Q, S11R, E12N, M19F, R22T,K26R | 9.4 | 1.2 |
| NV1G1870 | NV1D3583 | 321 | Y1A,W7Q,S11A,E12R,M19F ,V20S | 13.1 | 1.57 |
| NV1G1752 | NV1D3532 | 272 | Y1A,W7Q,S11A,E12K,M19F ,R22T,K26R | 17.3 | 2 |
| NV1G1749 | NV1D3587 | 326 | Y1A,W7Q,S11A, E12N, M19F, V20S | 18.3 | 2.6 |
| NV1G1725 | NV1D3572 | 310 | Y1A,W7Q,S11A,E12R,M19F ,R22T | 19.8 | 2.2 |
| NV1G1745 | NV1D3537 | 277 | Y1A,W7Q,S11A,E12K,M19F ,V20S,R22T,K26R | 21.4 | 4.1 |
| NV1G1720 | NV1D3565 | 304 | Y1A,W7Q,S11A,E12R,M19F ,V20S,R22T | 23 | 2.8 |
| NV1G1761 | NV1D3550 | 290 | Y1A,W7Q,S11R,M19F,R22T, K26R | 25.8 | 2.7 |
| NV1G1746 | NV1D3576 | 314 | Y1A,W7Q,S11A,E12N,M19F ,R22T | 26.7 | 5.2 |
| NV1G979 | NV1D2731 | 34 | Y1A,W7Q,S11A | 20.7 | 7.2 |
| NV1G953 | NV1D2670 | 17 | Y1A,W7Q | 22.2 | 3.3 |
| NV1G1519 | NV1D3006 | 133 | Y1Q,W7Q,S11A,E12R, M19F | 4.03 | 1.05 |
| NV1G1007-NH2 | NV1D2775-NH2 | 111 | Y1Q,W7Q,S11A,M19F | 5.06 | 0.473 |
| NV1G1517 | NV1D3004 | 131 | Y1Q,W7Q,S11R,M19F | 6.23 | 1.56 |
| (-GP) N-Ac-NV1G1137-NH2 | (-GP) N-Ac-NV1D2974-NH2 | 114 | Y1Q,W7Q,S11A,M19F,V20S ,R22T | 6.43 | 1.06 |
| NV1G1776 | NV1D3339 | 172 | Y1Q, Q3R,W7Q,S11R,M19F,R22T ,K26R | 6.57 | 0.675 |
| NV1G1153-NH-methyl | NV1D3034-NH-methyl | 119 | Y1Q,W7Q,S11R,M19F,R22T ,K26R | 7.1 | 0.9 |
| (-GP) N-Ac-NV1G1153-NH2 | (-GP) N-Ac-NV1D3034-NH2 | 121 | Y1Q,W7Q,S11R,M19F,R22T ,K26R | 7.63 | 1.04 |
| NV1G1523 | NV1D3012 | 135 | Y1Q,W7Q,S11R,E12N,M19F | 7.74 | 0.904 |
| NV1G1515 | NV1D3005 | 132 | Y1Q,W7Q,S11A,E12N,M19F | 7.83 | 1.38 |
| NV1G1187 | NV1D3015 | 138 | Y1Q,W7Q,S11R,M19F,K26R | 8.86 | 2.28 |
| NV1G1521 | NV1D3018 | 141 | Y1Q,W7Q,S11A,E12N,M19F ,K26R | 9.79 | 2.91 |
| NV1G1267 | NV1D3044 | 150 | Y1Q,W7Q,S11R,E12N,M19F ,R22T,K26R | 9.8 | 0.849 |
| NV1G1153 | NV1D3034 | 78 | Y1Q,W7Q,S11R,M19F,R22T ,K26R | 10.3 | 2.14 |
| NV1G1836 | NV1D3359 | 190 | YI0,W7Q,T8S, S11R,M19F, R22T,K26R | 10.5 | 0.739 |
| NV1G1593 | NV1D3050 | 153 | Y1Q,W7Q,S11R,E12K,M19F | 10.8 | 1.3 |
| NV1G1215 | NV1D3048 | 152 | Y1Q,W7Q,S11A,E12K,M19F | 11.1 | 1.05 |
| NV1G1868 | NV1D3353 | 185 | Y10,W7Q,T8R,S11R,M19F, R22T,K26R | 11.2 | 1.25 |
| NV1G1525 | NV1D3013 | 136 | Y1Q,W7Q,S11R,E12R,M19F | 11.3 | 1.83 |
| NV1G1775 | NV1D3340 | 173 | Y1Q,Q3K,W7Q,S11R,M19F, R22T,K26R | 11.5 | 0.798 |
| NV1G1833 | NV1D3381 | 210 | Y1Q,W7Q,S11RK14Q, M19F, R22T,K26R | 12.2 | 1.56 |
| NV1G1153-NH2 | NV1D3034-NH2 | 117 | Y1Q,W7Q,S11R,M19F,R22T ,K26R | 12.2 | 1 |
| NV1G1777 | NV1D3342 | 175 | Y1Q,Q3A,W7Q,S11R,M19F, | 12.8 | 2.67 |
| | | | R22T,K26R | | |
| NV1G1259 | NV1D3058 | 158 | Y1Q,W7Q,S11A,E12K,M19F ,R22T,K26R | 12.9 | 1.29 |
| NV1G1511 | NV1D3032 | 146 | Y1Q,W7Q,S11R,E12N,M19F ,K26R | 13 | 203 |
| NV1G1527 | NV1D3031 | 145 | Y1Q,W7Q,S11R,E12R,M19F ,R22T | 13 | 1.36 |
| NV1G1265 | NV1D3062 | 159 | Y1Q,W7Q,S11R,E12K,M19F ,R22T,K26R | 13.2 | 1.43 |
| NV1G1781 | NV1D3388 | 217 | Y1Q,W7Q,S11RE17Q,M19F, R22T,K26R | 13.5 | 1.14 |
| NV1G1824 | NV1D3354 | 186 | Y1Q,W7Q,T8K,S11R,M19F, R22T,K26R | 13.9 | 1.12 |
| NV1G1772 | NV1D3352 | 184 | Y1Q,K4S,W7Q,S11R,M19F, R22T,K26R | 14.2 | 2.01 |
| NV1G1509 | NV1D3033 | 147 | Y1Q,W7Q,S11R,E12R,M19F ,K26R | 14.5 | 2.18 |
| NV1G1779 | NV1D3351 | 183 | Y1Q,K4Q,W7Q,S11R,M19F, R22T,K26R | 15.3 | 2.39 |
| NV1G1687 | NV1D3526 | 266 | Y1Q,W7Q,S11R,M19F,R22T ,K26R | 15.4 | |
| NV1G1269 | NV1D3045 | 151 | Y1Q,W7Q,S11R,E12R,M19F ,R22T,K26R | 15.6 | 1.39 |
| NV1G1623 | NV1D3056 | 156 | Y1Q,W7Q,S11R,E12K,M19F ,R22T | 16.2 | 2.99 |
| NV1G1859 | NV1D3376 | 205 | Y1Q,W7Q,S11R,K14R,M19F ,R22T,K26R | 16.3 | 2.53 |
| NV1G1153-NH-butyl | NV1D3034-NH-butyl | 118 | Y1Q,W7Q,S11R,M19F,R22T ,K26R | 16.6 | 1.4 |
| NV1G1211 | NV1D3036 | 149 | Y1Q,W7Q,S11A,E12R,M19F ,R22T,K26R | 17.2 | 1.55 |
| NV1G1885 | NV1D3254 | 165 | Y1Q,W7Q,S11A,M19F | 17.5 | 2.45 |
| NV1G1730 | NV1D3542 | 282 | Y1Q,W7Q,S11R,E12N,M19F ,V20S,R22T,K26R | 17.7 | 2.5 |
| NV1G1263 | NV1D3051 | 154 | Y1Q,W7Q,S11A,E12K,M19F ,R22T | 17.9 | 1.78 |
| NV1G1818 | NV1D3368 | 122 | Y1Q,W7Q,S11R,E12T, M19F, R22T,K26R | 17.9 | 1.89 |
| NV1G1153 (synthetic) | NV1D3034 | 116 | Y1Q,W7Q,S11R,M19F,R22T ,K26R | 18 | 2.5 |
| NV1G1823 | NV1D3367 | 197 | Y1Q,W7Q,S11R,E12Q,M19F ,R22T,K26R | 18.6 | 2.17 |
| NV1G1820 | NV1D3362 | 193 | Y1Q,W7Q,D10T,S11R,M19F ,R22T,K26R | 20.1 | 2.32 |
| NV1G1811 | NV1D3369 | 199 | Y1Q,W7Q,S11R,R13K,M19F ,R22T,K26R | 20.4 | 2.44 |
| NV1G1810 | NV1D3358 | 189 | Y1Q,W7Q,T8Q,S11R,M19F, R22T,K26R | 20.5 | 2.11 |
| NV1G1818-NH2 | NV1D3368-NH2 | 123 | Y1Q,W7Q,S11R,E12T,M19F ,R22T,K26R | 20.5 | 2.8 |
| NV1G1137 (synthetic) | NV1D2974 | 129 | Y1Q,W7Q,S11A,M19F,V20S ,R22T | 21.6 | 1.34 |
| NV1G1221 | NV1D3017 | 140 | Y1Q,W7Q,S11A,E12R,M19F ,R22T | 21.9 | 2.48 |
| NV1G1722 | NV1D3533 | 273 | Y1Q,W7Q,S11A,E12K,M19F ,V20S,R22T,K26R | 22.4 | 3.5 |
| NV1G1767 | NV1D3345 | 177 | Y1Q,Q3S,W7Q,S11R,M19F, R22T,K26R | 22.4 | 2.52 |
| NV1G1769 | NV1D3346 | 178 | Y1Q,K4R,W7Q,S11R,M19F, R22T,K26R | 23.2 | 3.39 |
| NV1G1780 | NV1D3387 | 216 | Y1Q,W7Q,S11R,E17D,M19F ,R22T,K26R | 23.7 | 2.85 |
| NV1G1886 | NV1D3249 | 162 | Y1Q,W7Q,S11A,M19F | 24.1 | 11.5 |
| NV1G1812 | NV1D3382 | 211 | Y1Q,W7Q,S11R,K14S,M19F ,R22T,K26R | 24.3 | 2.14 |
| NV1G1857 | NV1D3366 | 196 | Y1Q,W7Q,D10S,S11R,M19F ,R22T,K26R | 24.6 | 3.8 |
| NV1G1821 | NV1D3378 | 207 | Y1Q,W7Q,S11R,K14A,M19F ,R22T,K26R | 24.8 | 2.66 |
| NV1G1993 | NV1D3792 | 335 | Y1Q,W7Q,S11R,M19F,R22T ,K26R | 25.3 | 2.8 |
| NV1G1007 | NV1D2775 | 56 | Y1Q,W7Q,S11A,M19F | 25.4 | 2 |
| NV1G1787 | NV1D3396 | 224 | Y1Q,W7Q,S11R,G18Q,M19 F,R22T,K26R | 26.4 | 3.17 |
| NV1G1257 | NV1D3016 | 139 | Y1Q,W7Q,S11A,E12N,M19F ,R22T | 26.6 | 3.1 |
| NV1G1153 (synthetic) | NV1D3034 | 116 | Y1Q,W7Q,S11R,M19F,R22T ,K26R | 27.3 | 2.02 |
| NV1G1803 | NV1D3403 | 230 | Y1Q,W7Q,S11R,M19F,R22T ,K26R,K27A | 28.3 | 1.97 |
| (-GP) N-Ac-NV1G1137 | N-Ac-NV1D2974 | 115 | Y1Q,W7Q,S11A,M19F,V20S ,R22T | 28.6 | 2.23 |
| NV1G1531 | NV1D3019 | 142 | Y1Q,W7Q,S11A,E12R,M19F ,K26R | 28.7 | 4.78 |
| NV1G1513 | NV1D3007 | 134 | Y1Q,W7Q,S11A,M19F,K26R | 29.6 | 9.17 |
| NV1G1991 | NV1D3789 | 333 | Y1Q,W7Q,S11R,M19F,R22T ,K26R | 29.9 | 5.19 |
| NV1G1013 | NV1D2733 | 40 | Y1R,W7Q,M19F | 7.54 | 2.9 |
| NV1G1740 | NV1D3580 | 318 | Y1R,W7Q,S11A,E12R,M19F, V20S | 8.4 | 1.5 |
| NV1G1757 | NV1D3538 | 278 | Y1R,W7Q,S11R,E12N,M19F ,R22T,K26R | 11.6 | 1.4 |
| NV1G1741 | NV1D3569 | 307 | Y1R,W7Q,S11A,E12R,M19F, R22T | 11.9 | 0.8 |
| NV1G1715 | NV1D3584 | 322 | Y1R,W7Q,S11A,E12N,M19F ,V20S | 13.9 | 1.4 |
| NV1G1754 | NV1D3529 | 269 | Y1R,W7Q,S11A,E12K,M19F, R22T,K26R | 14.6 | 1.7 |
| NV1G1005 | NV1D2772 | 59 | Y1R,W7Q,S11A,M19F | 15.6 | 1.8 |
| NV1G1733 | NV1D3577 | 315 | Y1R,W7Q,S11A,M19F,V20S | 18.8 | 2.2 |
| NV1G1744 | NV1D3534 | 274 | Y1R,W7Q,S11A,E12K,M19F, V20S,R22T,K26R | 20.6 | 2.2 |
| NV1G1724 | NV1D3562 | 301 | Y1R,W7Q,S11A,E12R,M19F, V20S,R22T | 23.6 | 2.7 |
| NV1G1735 | NV1D3566 | 305 | Y1R,W7Q,S11A,M19F,R22T | 23.7 | 2.5 |
| NV1G1760 | NV1D3543 | 283 | Y1R,W7Q,S11R,E12N,M19F ,V20S,R22T,K26R | 23.8 | 1.9 |
| NV1G1759 | NV1D3547 | 287 | Y1R,W7Q,S11R,M19F,R22T, K26R | 26.5 | 2.1 |
| NV1G1751 | NV1D3558 | 297 | Y1R,W7Q,S11A,E12N,M19F ,V20S,R22T | 26.7 | 3.4 |
| NV1G1726 | NV1D3551 | 291 | Y1R,W7Q,S11R,M19F,V20S, R22T,K26R | 29.3 | 3.8 |
| NV1G1105 | NV1D2729 | 39 | Y1R,W7Q,S11A | 8 | 8.85E-01 |
| NV1G957 | NV1D2668 | 23 | Y1R,W7Q | 17.5 | 2.6 |
| (-GP) NV1G1001 | (-GP) NV1D2773 | 109 | Y1S,W7Q,S11A,M19F | 9.47 | 1.28 |
| (-GP) NV1G1001-NH-methyl | (-GP) NV1D2773-NH-methyl | 110 | Y1S,W7Q,S11A,M19F | 11.5 | 0.61 |
| NV1G1003 | NV1D2734 | 44 | Y1S,W7Q,M19F | 13.4 | 0.8 |
| NV1G1864 | NV1D3581 | 319 | Y1S,W7Q,S11A,E12R,M19F, V20S | 14.6 | 1.7 |
| NV1G1748 | NV1D3530 | 270 | Y1S,W7Q,S11A,E12K,M19F, R22T,K26R | 15.6 | 2.2 |
| NV1G1758 | NV1D3548 | 288 | Y1S,W7Q,S11R,M19F,R22T, K26R | 17.6 | 1.9 |
| NV1G1727 | NV1D3544 | 284 | Y1S,W7Q,S11R,E12N,M19F, V20S,R22T,K26R | 17.8 | 2.2 |
| NV1G1719 | NV1D3570 | 308 | Y1S,W7Q,S11A,E12R,M19F, R22T | 18.1 | 1.5 |
| NV1G1742 | NV1D3535 | 275 | Y1S,W7Q,S11A,E12K,M19F, V20S,R22T,K26R | 18.7 | 2.8 |
| NV1G1001 | NV1D2773 | 65 | Y1S,W7Q,S11A,M19F | 18.8 | 1.5 |
| NV1G1753 | NV1D3585 | 323 | Y1S,W7Q,S11A,E12N,M19F ,V20S | 19.4 | 2.1 |
| NV1G1762 | NV1D3539 | 279 | Y1S,W7Q,S11R,E12N,M19F, R22T,K26R | 19.4 | 1.8 |
| NV1G1755 | NV1D3574 | 312 | Y1S,W7Q,S11A,E12N,M19F ,R22T | 22.3 | 2.7 |
| NV1G1717 | NV1D3563 | 302 | Y1S,W7Q,S11A,E12R,M19F, V20S,R22T | 22.4 | 2.4 |
| NV1G1866 | NV1D3559 | 298 | Y1S,W7Q,S11A,E12N,M19F ,V20S,R22T | 26.5 | 5.02 |
| NV1G1721 | NV1D3552 | 292 | Y1S,W7Q,S11R,M19F,V20S, R22T,K26R | 28.1 | 3.7 |
| NV1G975 | NV1D2669 | 26 | Y1S,W7Q | 18.4 | 5.7 |
| NV1G983 | NV1D2730 | 43 | Y1S,W7Q,S11A | 25.5 | 4.3 |
| NV1G1750-NH2 | NV1D3586-NH2 | 325 | W7Q,S11A,E12N,M19F,V20 S | 4.23 | 0.33 |
| NV1G1747 | NV1D3531 | 271 | W7Q,S11A,E12K,M19F,R22 T,K26R | 13 | 2.1 |
| NV1G1763 | NV1D3540 | 280 | W7Q,S11R,E12N,M19F,R22 T,K26R | 16 | 1.5 |
| NV1G1739 | NV1D3582 | 320 | W7Q,S11A,E12R,M19F,V20 S | 17.8 | 2.2 |
| NV1G1750 | NV1D3586 | 324 | W7Q, S11A, E12N, M19F, V20S | 20.5 | 2.2 |
| NV1G1718 | NV1D3571 | 309 | W7Q,S11A,E12R,M19F,R22 T | 21 | 2.3 |
| NV1G1865 | NV1D3560 | 299 | W7Q,S11A,E12N,M19F,V20 S,R22T | 27.2 | 3.42 |
| NV1G1766 | NV1D3549 | 289 | W7Q,S11R,M19F,R22T,K26 R | 27.5 | 3.2 |
| NV1G961 | NV1D2676 | 29 | W7Q,S11A | 26.5 | 2.9 |
| NV1G951 | NV1D2674 | 18 | Y1A,S11A | 4.03 | 0.2 |
| NV1G1011 | NV1D2740 | 37 | Y1Q,S11A,M19F | 3.62 | 9.9 |
| NV1G977 | NV1D2665 | 22 | Y1Q,M19F | 4.9 | 0.4 |
| NV1G949 | NV1D2675 | 21 | Y1Q,S11A | 4.33 | 0.3 |
| NV1G973 | NV1D2662 | 25 | Y1R,M19F | 4.03 | 0.4 |
| NV1G965 | NV1D2672 | 24 | Y1R,S11A | 4.5 | 0.3 |
| NV1G1009 | NV1D2738 | 45 | Y1S,S11A,M19F | 2.57 | 0.2 |
| NV1G995 | NV1D2663 | 28 | Y1S,M19F | 4.19 | 0.4 |
| NV1G1107-NH2 | NV1D2890-NH2 | 112 | Y1S,M6F,S11A,M19L | 9.12 | 1.17 |
| NV1G971 | NV1D2673 | 27 | Y1S,S11A | 4.31 | 0.5 |
| NV1G1782 | NV1D3383 | 212 | Y1Q,W7Q,S11R,E17R,M19F ,R22T,K26R, | 30.3 | 4.06 |
| NV1G1990 | NV1D3788 | 332 | Y1Q,W7Q,S11R,M19F,R22T ,K26R, | 30.3 | 4.78 |
| (-GP) N-Ac-NV1G1153- | (-GP) N-Ac-NV1D3034 | 120 | Y1Q,W7Q,S11R,M19F,R22T ,K26R | 30.4 | 2.96 |
| NV1G1786 | NV1D3389 | 218 | Y1Q,W7Q,S11R,E17S,M19F ,R22T,K26R, | 30.8 | 4.48 |
| NV1G1147 | NV1D2969 | 124 | Y1S, W7Q, S11A, M19F, V20S | 31 | 6.15 |
| NV1G1764 | NV1D3554 | 294 | Y1A,W7Q,S11R,M19F,V20S ,R22T,K26R | 31.4 | 3.3 |
| NV1G963 | NV1D2671 | 20 | Y1Q,W7Q | 31.5 | 6.4 |
| NV1G1835 | NV1D3379 | 208 | Y1Q,K4D,W7Q,S11R,M19F, R22T,K26R | 31.6 | 2.88 |
| NV1G1231 | NV1D3035 | 148 | Y1Q,W7Q,S11A,E12N,M19 F,R22T,K26R | 32 | 4.9 |
| NV1G1743 | NV1D3564 | 303 | W7Q,S11A,E12R,M19F,V2 0S,R22T | 32.3 | 3.1 |
| NV1G1960 | NV1D3803 | 345 | Y1Q,W7Q,S11R,M19F,R22 T,K26R | 32.3 | 5.33 |
| NV1G1924 | NV1D3470 | 250 | Y1Q,W7Q,S11R,M19L,R22 T,K26R | 32.5 | 403 |
| NV1G1756 | NV1D3575 | 313 | W7Q,S11A,E12N,M19F,R22 T | 33.2 | 3.9 |
| NV1G1109 | NV1D2899 | 67 | Y1S,W7Q,S11A,M19L | 33.3 | 6.7 |
| NV1G1818 | NV1D3368 | 122 | Y1Q,W7Q,S11R,E12T,M19 F,R22T,K26R | 33.5 | 10.7 |
| NV1G1784 | NV1D3386 | 215 | Y1Q,W7Q,S11R,E17A,M19 F,R22T,K26R | 33.6 | 4.71 |
| NV1G1141 | NV1D2972 | 127 | Y1Q,W7Q,S11A,M19F,V20 S | 34.1 | 6.2 |
| NV1G1774 | NV1D3347 | 179 | Y1Q,K4T,W7Q,S11R,M19F, R22T,K26R | 34.2 | 5.99 |
| NV1G1881 | NV1D3257 | 167 | Y1Q,W7Q,S11A,M19F | 34.2 | 2.81 |
| NV1G1915 | NV1D3467 | 249 | Y1Q,W7Q,S11R,E17G,M19 F,R22T,K26R | 34.5 | 4 |
| NV1G1984 | NV1D3806 | 348 | Y1Q,W7Q,S11R,M19F,R22 T,K26R | 35.1 | 4.56 |
| NV1G1716 | NV1D3561 | 300 | Y1A,W7Q,S11A,E12N,M19F ,V20S,R22T, | 35.6 | 5 |
| NV1G1255 | NV1D3014 | 137 | Y1Q,W7Q,S11R,M19F,R22T | 36.1 | 5.37 |
| NV1G1959 | NV1D3818 | 357 | Y1Q,W7Q,S11R,M19F,R22 T,K26R | 36.3 | 204 |
| NV1G1825 | NV1D3377 | 206 | Y1Q,W7Q,S11R,K14T,M19 F,R22T,K26R | 36.4 | 4.83 |
| NV1G1723 | NV1D3536 | 276 | W7Q,S11A,E12K,M19F,V20 S,R22T,K26R | 37 | 5.4 |
| NV1G1732 | NV1D3555 | 295 | Y1R,W7Q,S11A,M19F, V20S, R22T, | 37.4 | 4.3 |
| NV1G1983 | NV1D3809 | 350 | Y1Q,W7Q,S11R,M19F,R22 T,K26R | 38.9 | 4.81 |
| NV1G1982 | NV1D3805 | 347 | Y1Q,W7Q,S11R,M19F,R22 T,K26R | 41.2 | 5.44 |
| NV1G1785 | NV1D3385 | 214 | Y1Q,W7Q,S11R,E17T,M19 F,R22T,K26R | 41.5 | 6.5 |
| NV1G1583 | NV1D3030 | 144 | Y1Q,W7Q,S11R,E12N,M19 F,R22T | 41.9 | 5.15 |
| NV1G1729 | NV1D3545 | 285 | W7Q,S11R,E12N,M19F,V20 S,R22T,K26R | 42.8 | 4.6 |
| NV1G1007 | NV1D2775 | 56 | Y1Q,W7Q,S11A,M19F | 42.9 | 6.7 |
| NV1G1734 | NV1D3568 | 306 | Q1A,W7Q,S11A,M19F,R22T | 44 | 8.3 |
| NV1G1683 | NV1D3523 | 263 | Y1Q,W7Q,S11R,M19F,R22 T,K26R | 44.7 | |
| NV1G1834 | NV1D3360 | 191 | Y1Q,W7Q,D10R,S11R,M19 F,R22T,K26R | 45.2 | 3.79 |
| NV1G1795 | NV1D3401 | 229 | Y1Q,W7Q,S11R,M19F,R22 T,K26R,K27R | 45.5 | 6.58 |
| NV1G1689 | NV1D3514 | 255 | Y1Q,W7Q,S11R,M19F,R22 T,K26R | 46.4 | |
| NV1G2043 | NV1D3835 | 370 | Y1Q,W7Q,S11R,M19F,R22 T,K26R | 46.4 | 4.09 |
| NV1G1783 | NV1D3384 | 213 | Y1Q,W7Q,S11R,E17K,M19 F,R22T,K26R | 46.8 | 7.39 |
| NV1G1239 | NV1D3020 | 143 | Y1Q,W7Q,S11A,M19F,R22 T,K26R | 47.2 | 7.84 |
| NV1G1788 | NV1D3399 | 227 | Y1Q,W7Q,S11R,M19F,V20 T,R22T,K26R | 47.3 | 6.36 |
| NV1G899 | NV1D2774 | 52 | Y1A,W7Q,S11A,M19F | 50.5 | 15.2 |
| NV1G2057 | NV1D3799 | 341 | Y1Q,W7Q,S11R,M19F,R22 T,K26R | 50.6 | 6.33 |
| NV1G1738 | NV1D3578 | 316 | W7Q,S11A,M19F,V20S, | 50.7 | 5.7 |
| NV1G1713 | NV1D3525 | 265 | Y1Q,W7Q,S11R,M19F,R22 T,K26R | 52.3 | |
| NV1G1765 | NV1D3553 | 293 | W7Q,S11R,M19F,V20S,R22 T,K26R | 52.4 | 10 |
| NV1G1916 | NV1D3465 | 247 | Y1Q,W5F,W7Q,S11R,M19F ,R22T,K26R | 52.8 | 10.3 |
| NV1G1977 | NV1D3804 | 346 | Y1Q,W7Q,S11R,M19F,R22 T,K26R | 53.6 | 6.27 |
| NV1G1879 | NV1D3259 | 168 | Y1Q,W7Q,S11A,M19F | 54.9 | 7.62 |
| NV1G1884 | NV1D3256 | 166 | Y1Q,W7Q,S11A,M19F | 55.7 | 10.5 |
| NV1G1986 | NV1D3819 | 358 | Y1Q,W7Q,S11R,M19F,R22 T,K26R | 56 | 6.57 |
| NV1G1633 | NV1D3251 | 163 | Y1Q,W7Q,S11A,M19F | 56.1 | 13.9 |
| NV1G1880 | NV1D3261 | 170 | Y1Q,W7Q,S11A,M19F | 57 | 6.25 |
| NV1G1985 | NV1D3808 | 349 | Y1Q,W7Q,S11R,M19F,R22 T,K26R | 57 | 6.74 |
| NV1G1849 | NV1D3400 | 228 | Y1Q,W7Q,S11R,M19F,V20 Q,R22T,K26R | 57.3 | 9.52 |
| NV1G1883 | NV1D3260 | 169 | Y1Q,W7Q,S11A,M19F | 57.6 | 6.91 |
| NV1G1145 | NV1D2970 | 125 | Y1S,W7Q,S11A,M19F,R22T | 58 | 18.8 |
| NV1G1697 | NV1D3517 | 258 | Y1Q,W7Q,S11R,M19F,R22 T,K26R | 58.5 | |
| NV1G1737 | NV1D3579 | 317 | Y1A,W7Q,S11A,M19F,V20S | 59.9 | 9.6 |
| NV1G1978 | NV1D3833 | 368 | Y1Q,W7Q,S11R,M19F,R22 T,K26R | 60.3 | 9.57 |
| NV1G1954 | NV1D3800 | 342 | Y1Q,W7Q,S11R,M19F,R22 T,K26R | 60.9 | 6.43 |
| NV1G1989 | NV1D3791 | 334 | Y1Q,W7Q,S11R,M19F,R22 T,K26R | 61.8 | 8.66 |
| NV1G1815 | NV1D3380 | 209 | Y1Q,K4E,W7Q,S11R,M19F, R22T,K26R | 64 | 10.5 |
| NV1G1967 | NV1D3793 | 336 | Y1Q,W7Q,S11R,M19F,R22 T,K26R | 64.6 | 8.19 |
| NV1G1869 | NV1D3573 | 311 | Y1R,W7Q,S11A,E12N,M19 F,R22T | 64.7 | 50.7 |
| NV1G1872 | NV1D3777 | 330 | Y1Q,W7Q,S11R,M19F,R22 T,K26R | 64.9 | 15.3 |
| NV1G1979 | NV1D3834 | 369 | Y1Q,W7Q,S11R,M19F,R22 T,K26R | 65.5 | 7.59 |
| NV1G1827 | NV1D3365 | 195 | Y1Q,W7Q,D10Q,S11R,M19 F,R22T,K26R | 66.1 | 10.1 |
| NV1G1768 | NV1D3341 | 174 | Y1Q,Q3T,W7Q,S11R,M19F, R22T,K26R | 66.2 | 9.32 |
| NV1G911 | NV1D2666 | 30 | W7Q,M19F | 66.5 | 36.7 |
| NV1G1856 | NV1D3397 | 225 | Y1Q,W7Q,S11R,G18S,M19 F,R22T,K26R | 66.7 | 7.31 |
| NV1G1973 | NV1D3810 | 351 | Y1Q,W7Q,S11R,M19F,R22 T,K26R | 66.9 | 7.04 |
| NV1G1855 | NV1D3398 | 226 | Y1Q,W7Q,S11R,M19F,V20 S,R22T,K26R | 67.3 | 11 |
| NV1G1961 | NV1D3802 | 344 | Y1Q,W7Q,S11R,M19F,R22 T,K26R | 68 | 8.23 |
| NV1G1846 | NV1D3431 | 244 | Y1Q,K4E,W7Q,S11R,E17K, M19F,R22T,K26R | 68.6 | 13.9 |
| NV1G1771 | NV1D3348 | 180 | Y1Q,K4A,W7Q,S11R,M19F, R22T,K26R | 70.6 | 15.9 |
| NV1G1691 | NV1D3520 | 261 | Y1Q,W7Q,S11R,M19F,R22 | 71.4 | |
| | | | T,K26R | | |
| NV1G1681 | NV1D3511 | 252 | Y1Q,W7Q,S11R,M19F,R22 T,K26R | 71.5 | |
| NV1G1968 | NV1D3822 | 359 | Y1Q,W7Q,S11R,M19F,R22 T,K26R | 74.2 | 11.1 |
| NV1G1813 | NV1D3424 | 238 | Y1Q,W7Q,D10K,S11R,E12K ,M19F,R22T,K26R | 75.2 | 12.2 |
| NV1G1067 | NV1D2893 | 57 | Y1Q,W7Q,S11A,M19L | 75.5 | 10.5 |
| NV1G1867 | NV1D3546 | 286 | Y1A,W7Q,S11R,E12N,M19 F,V20S,R22T,K26R | 76 | 17.6 |
| NV1G1143 | NV1D2971 | 126 | Y1S,W7Q,S11A,M19F,V20S ,R22T | 77.5 | 22.1 |
| NV1G1806 | NV1D3409 | 232 | Y1Q,W7Q,S11R,M19F,R22 T,K26R,K28T | 79.1 | 11.3 |
| NV1G1061 | NV1D2896 | 60 | Y1R,W7Q,S11A,M19L | 80.3 | 7.13 |
| NV1G1793 | NV1D3419 | 236 | Y1Q,W7Q,S11R,M19F,R22 T,K26R,W30D | 80.9 | 11.9 |
| NV1G1613 | NV1D3057 | 157 | Y1Q,W7Q,S11R,E12K,M19 F,K26R | 83.4 | 16.6 |
| NV1G1585 | NV1D3052 | 155 | Y1Q,W7Q,S11A, E12K,M19F,K26R | 84.8 | 28.8 |
| NV1G1707 | NV1D3524 | 264 | Y1Q,W7Q,S11R,M19F,R22 T,K26R | 84.9 | |
| NV1G1773 | NV1D3350 | 182 | Y1Q,K4E,W7Q,S11R,M19F, R22T,K26R | 85.6 | 14.4 |
| NV1G1949 | NV1D3828 | 364 | Y1Q,W7Q,S11R,M19F,R22 T,K26R | 87.5 | 11 |
| NV1G1976 | NV1D3811 | 352 | Y1Q,W7Q,S11R,M19F,R22 T,K26R | 87.7 | 15.7 |
| NV1G1956 | NV1D3801 | 343 | Y1Q,W7Q,S11R,M19F,R22 T,K26R | 88.1 | 11.4 |
| NV1G1975 | NV1D3832 | 367 | Y1Q,W7Q,S11R,M19F,R22 T,K26R | 88.4 | 12.3 |
| NV1G1839 | NV1D3774 | 328 | Y1Q,W7Q,S11R,M19F,R22 T,K26R | 88.6 | 19.6 |
| NV1G1971 | NV1D3830 | 366 | Y1Q,W7Q,S11R,M19F,R22 T,K26R | 88.6 | 9.88 |
| NV1G1882 | NV1D3262 | 171 | Y1Q,W7Q,S11A,M19F | 89.2 | 8.32 |
| NV1G1950 | NV1D3797 | 339 | Y1Q,W7Q,S11R,M19F,R22 T,K26R | 91.1 | 13.5 |
| NV1G1828 | NV1D3363 | 194 | Y1Q,W7Q,D10A,S11R,M19 F,R22T,K26R | 93.1 | 15.3 |
| NV1G1139 | NV1D2973 | 128 | Y1Q,W7Q,S11A,M19F,R22 T | 93.9 | 19.5 |
| NV1G1842 | NV1D3430 | 243 | Y1Q,K4D,W7Q,S11R,E17K, M19F,R22T,K26R | 93.9 | 14.1 |
| NV1G1948 | NV1D3798 | 340 | Y1Q,W7Q,S11R,M19F,R22 T,K26R | 94.5 | 17.8 |
| NV1G1807 | NV1D3408 | 231 | Y1Q,W7Q,S11R,M19F,R22 T,K26R,K28R | 94.8 | 17.8 |
| NV1G1137 | NV1D2974 | 129 | Y1Q,W7Q,S11A,M19F,V20 S,R22T | 95.7 | 16.2 |
| NV1G1843 | NV1D3432 | 245 | Y1Q,K4E,W7Q,S11R,E17R, M19F,R22T,K26R | 95.9 | 10.4 |
| NV1G1822 | NV1D3423 | 237 | Y1Q,W7Q,D10R,S11R,E12R ,M19F,R22T,K26R | 99.5 | 9.45 |
| NV1G1862 | NV1D3556 | 296 | W7Q,S11A,M19F,V20S,R22 T | 100 | 18.5 |
| NV1G1969 | NV1D3795 | 337 | Y1Q,W7Q,S11R,M19F,R22 T,K26R | 100 | 14.5 |
| NV1G1980 | NV1D3812 | 353 | Y1Q,W7Q,S11R,M19F,R22 T,K26R | 101 | 23.6 |
| NV1G1850 | NV1D3414 | 235 | Y1Q,W7Q,S11R,M19F,R22 T,K26R,K28S | 102 | 19.4 |
| NV1G1981 | NV1D3815 | 356 | Y1Q,W7Q,S11R,M19F,R22 T,K26R | 102 | 13.5 |
| NV1G1851 | NV1D3390 | 219 | Y1Q,W7Q,S11R,G18R,M19 F,R22T,K26R | 108 | 15.5 |
| NV1G1922 | NV1D3466 | 248 | Y1Q,W7Q,S11E,M19F,R22 T,K26R | 108 | 922 |
| NV1G1778 | NV1D3349 | 181 | Y1Q,K4D,W7Q,S11R,M19F, R22T,K26R | 109 | 16 |
| NV1G1972 | NV1D3824 | 361 | Y1Q,W7Q,S11R,M19F,R22 T,K26R | 110 | 16.1 |
| NV1G1974 | NV1D3796 | 338 | Y1Q,W7Q,S11R,M19F,R22 T,K26R | 110 | 19.6 |
| NV1G1826 | NV1D3357 | 188 | Y1Q,W7Q,T8E,S11R,M19F, R22T,K26R | 111 | 15.1 |
| NV1G1892 | NV1D3439 | 246 | Y1Q,W7Q,S11R,M19F,R22 T,K26R,W30G | 112 | 13.2 |
| NV1G1819 | NV1D3375 | 204 | Y1Q,W7Q,S11R,R13S,M19 F,R22T,K26R | 113 | 1270 |
| NV1G1805 | NV1D3410 | 233 | Y1Q,W7Q,S11R,M19F,R22 T,K26R,K28A | 114 | 21.5 |
| NV1G1831 | NV1D3374 | 203 | Y1Q,W7Q,S11R,R13Q,M19 F,R22T,K26R | 114 | 1600 |
| NV1G1693 | NV1D3512 | 253 | Y1Q,W7Q,S11R,M19F,R22 T,K26R | 115.6 | |
| NV1G1854 | NV1D3392 | 221 | Y1Q,W7Q,S11R,G18T,M19 F,R22T,K26R | 117 | 21.8 |
| NV1G1951 | NV1D3829 | 365 | Y1Q,W7Q,S11R,M19F,R22 T,K26R | 122 | 13.3 |
| NV1G1860 | NV1D3393 | 222 | Y1Q,W7Q,S11R,G18A,M19 F,R22T,K26R | 125 | 24.8 |
| NV1G1099 | NV1D2732 | 36 | Y1Q,W7Q,S11A | 126 | 26.9 |
| NV1G1705 | NV1D3513 | 254 | Y1Q,W7Q,S11R,M19F,R22 T,K26R | 131.2 | |
| NV1G1848 | NV1D3426 | 240 | Y1Q,W7Q,D10K,S11R,E12K ,R13D,M19F,R22T,K26R | 135 | 39.9 |
| NV1G1952 | NV1D3813 | 354 | Y1Q,W7Q,S11R,M19F,R22 T,K26R | 139 | 30.1 |
| NV1G1631 | NV1D3252 | 164 | Y1Q,W7Q,S11A,M19F | 145 | 53 |
| NV1G1817 | NV1D3371 | 201 | Y1Q,W7Q,S11R,R13A,M19 F,R22T,K26R | 151 | 33.7 |
| NV1G1789 | NV1D3394 | 223 | Y1Q,W7Q,S11R,G18D,M19 F,R22T,K26R | 155 | 41.4 |
| NV1G1852 | NV1D3391 | 220 | Y1Q,W7Q,S11R,G18K,M19 F,R22T,K26R | 157 | 23.1 |
| NV1G1709 | NV1D3510 | 251 | Y1Q,W7Q,S11R,M19F,R22 T,K26R | 159 | |
| NV1G1840 | NV1D3425 | 239 | Y1Q,W7Q,D10R,S11R, E12R, R13D,M19F,R22T,K26R | 161 | 27.9 |
| NV1G1809 | NV1D3413 | 234 | Y1Q,W7Q,S11R,M19F,R22 T,K26R,K28Q | 164 | 43.7 |
| NV1G1863 | NV1D3356 | 187 | Y1Q,W7Q,T8D,S11R,M19F, R22T,K26R | 167 | 32.2 |
| NV1G1699 | NV1D3527 | 267 | Y1Q,W7Q,S11R,M19F,R22 T,K26R | 169.1 | |
| NV1G1844 | NV1D3428 | 242 | Y1Q,W7Q,D10K,S11R,E12 K,R13E,M19F,R22T,K26R | 180 | 52.4 |
| NV1G1853 | NV1D3370 | 200 | Y1Q,W7Q,S11R,R13T,M19 F,R22T,K26R | 181 | 25.1 |
| NV1G1946 | NV1D3825 | 362 | Y1Q,W7Q,S11R,M19F,R22 T,K26R | 194 | 28.4 |

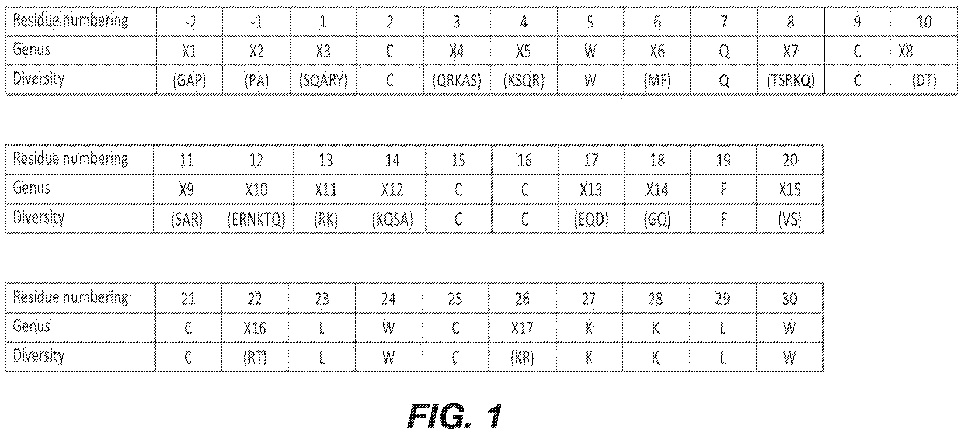

The wild-type Protoxin-II inhibits Navl.7 with an IC₅₀ value of about 4 nM in FLIPR assay as described in Example 3. Variants retaining significant Navl.7 potency were characterized further. Figure 1 shows the sequence genus of generated Protoxin-II variants that inhibit Navl.7 with an IC₅₀ value of 30 nM or less.

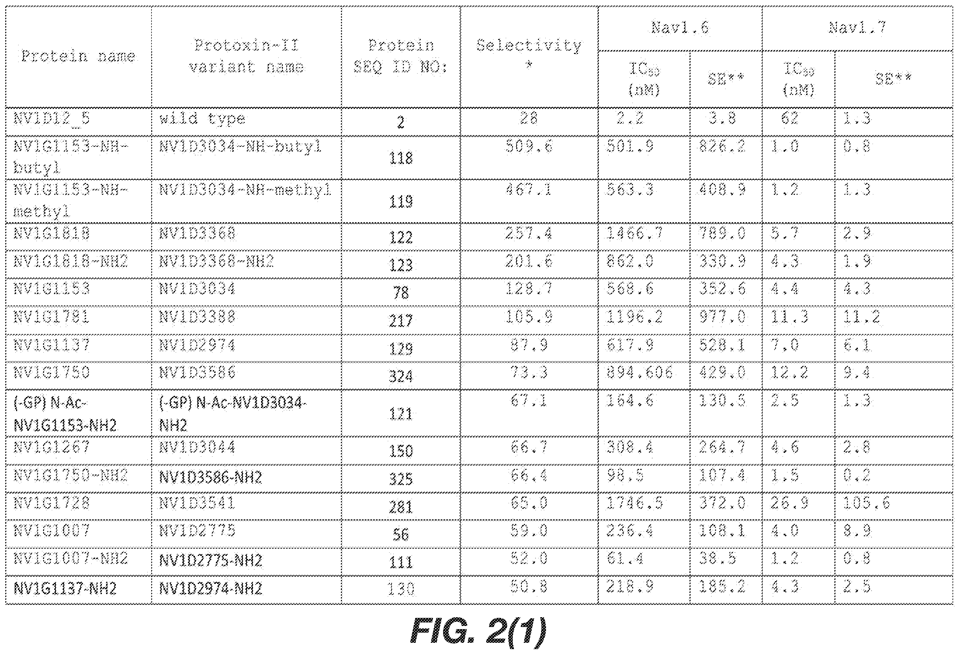

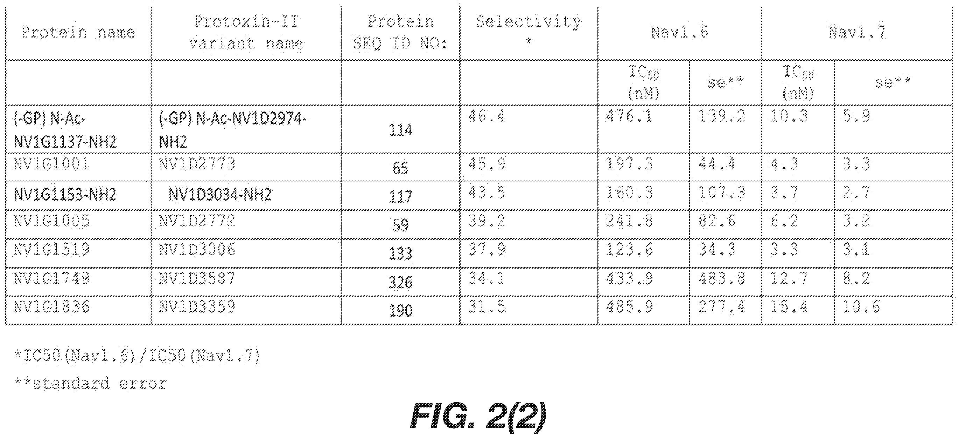

Select Protoxin-II variants were tested for their inhibition of Navl.7 and for their selectivity against human Navl.6 using QPatch. IC₅₀ values for both Navl.7 and Navl.6 for select peptides obtained using QPatch are shown in Figure 2. These peptides inhibited Navl.7 with an IC₅₀ of 30 nM or less, and were at least 30-fold selective over Navl.7 when compared to Navl.6.

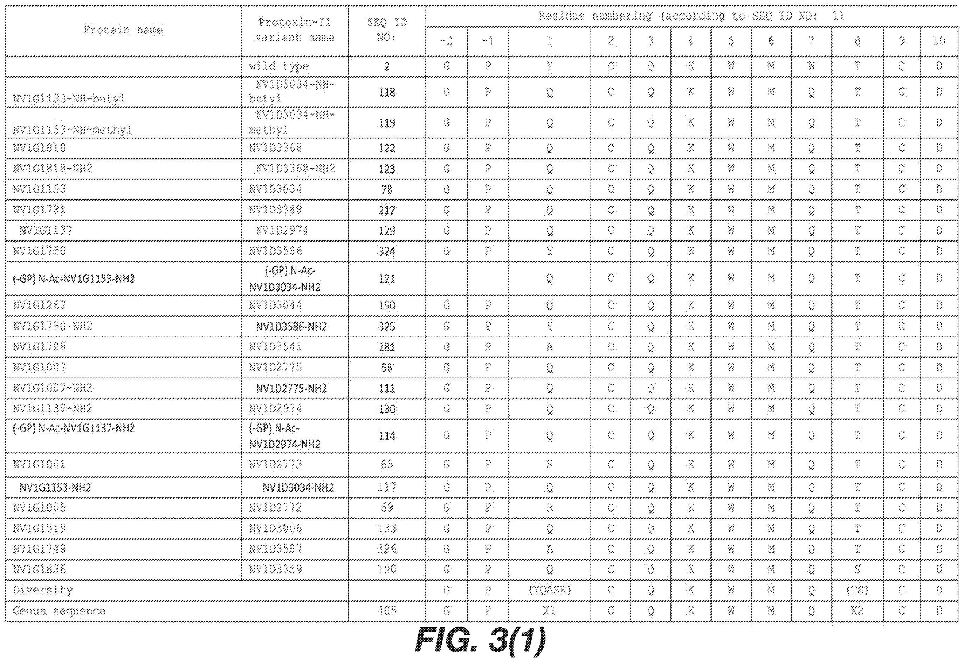

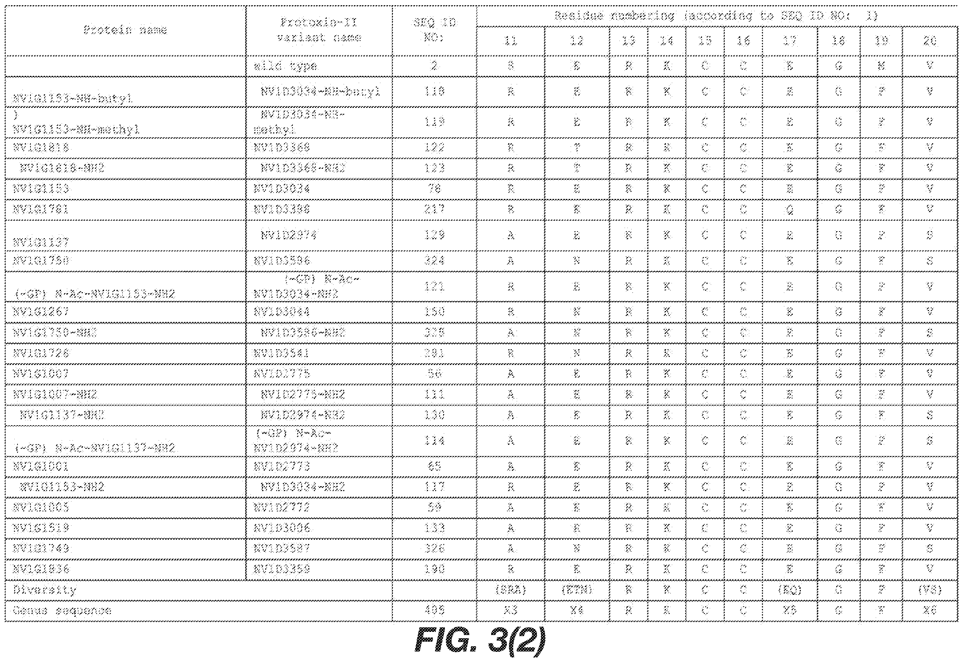

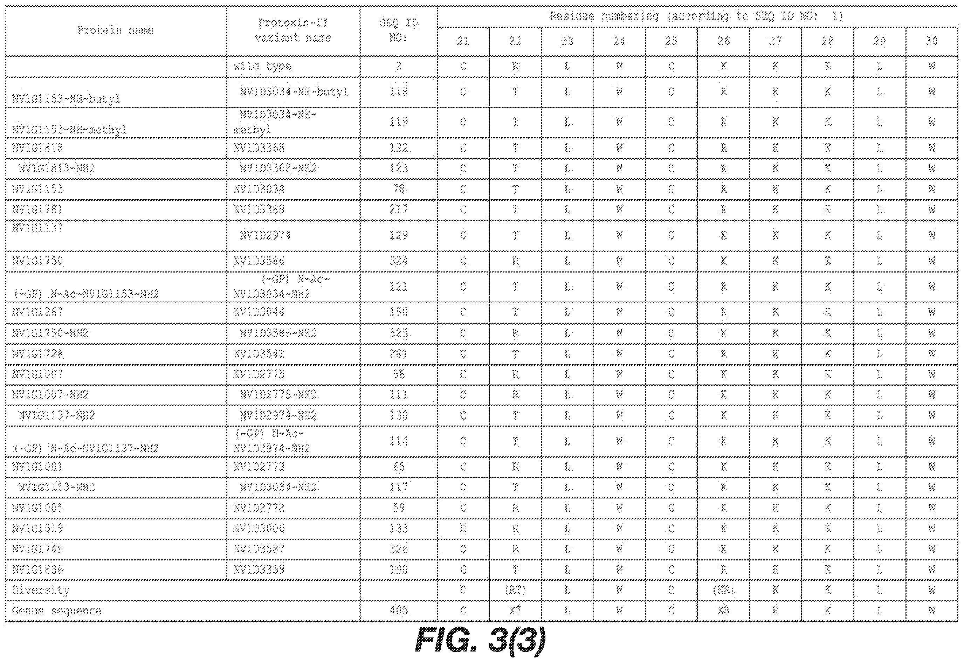

The amino acid sequences of the peptides shown in Figure 2 are shown in Figure 3. All these peptides had W7Q and M19F substitutions when compared to the wild type Protoxin-II.

The protoxin-II variants were expressed and purified as described in Example 1, or synthesized by standard solid phase synthesis methods. The yields of the recombinant or synthetic peptides were compared to the yields of the wild-type protoxin. Table 12 shows that the yields of the select protoxin-II variants were significantly higher than that of protoxin-II, indicating improved folding properties of the variants. The scale of the solid-phase synthesis was 0.5 mmol.

**Table 12.**

| Peptide | Solid phase synthesis | | | Recombinant expression |
|---|---|---|---|---|
| | Total yield | Yield from Crude | Yield From Linear | % active isomer |
| Protoxin-II | 52 mg | 2.7% | 7.3% | 54.0% |
| NV1D2775 | 84 mg | 4.5% | 18.7% | 89.1% |
| NV1D3034 | 149 mg | 8.0% | 21.0% | 85.2% |
| NV1D3368 | 83 mg | 4.0% | 24.0% | 93.8% |

### Example 5. Protoxin-II variants are efficient in in vivo models of pain

### Materials and methods

**Animals** Male C57Bl/6 mice (24-26g), ordered from Charles River and housed individually, were used for this study.

### Behavioral Tests

*Von Frey Test:* Mechanical (tactile) threshold was assessed by Von Frey Hairs following the Up-Down method (Dixon, 1980, Chaplan et al., 1994). 7 graded stimuli (von Frey filaments: 0.03, 0.07, 0.16, 0.4, 0.6, 1, 2 g; Stoelting, Wood Dale, IL) were used. Von Frey hairs were presented perpendicularly against the center plantar area (between toris) on a hindpaw. Sufficient force was applied to bend the filament slightly and held for 3 seconds. Per the Chaplan paper, a positive response can be either 1) a sharp withdrawal or 2) immediate flinching upon removal of the filament. See Chaplan et al for more details. Mice were acclimated to the wire mesh in the testing chamber for 30-60 minutes prior to testing.

*Hargreaves Test:* A modified Hargreaves box was used to measure thermal paw withdrawal latency (PWL) (Hargreaves et al., 1988, Pain, 32:77-88; Dirig et al.,1997, J Neurosci. Methods, 76:183-191). This box consists of a chamber with a raised glass floor maintained at a constant temperature (27°C). The thermal nociceptive stimulus originates from a projection bulb light beam below the glass surface. The light beam is aimed at the area between toris (center plantar). The "start" button will turn on the light and start the timer. Movements (such as a sudden withdrawal) of the stimulated paw will trigger the switch to turn off the light and stop the timer. The latency in seconds is displayed. If no movement occurs, the bulb will be turned off after 20 seconds (cutoff) to prevent tissue injury. The animals were allowed to habituate on the glass surface for 30-60 minutes before PWL measurement. Constant amperage was used throughout the study, which resulted in Pre-test paw withdrawal latencies between 8-12 seconds when averaged over 3 to 6 read-outs taken at least 5 minutes apart.

*MPE% Calculation:* Percent maximum possible effect (MPE%) = (T₁ - T₀) / (Tc - T₀) x 100%. T₀: threshold on day0 (post-CFA, pre-pump); T₁: threshold on dayl post pump implantation; Tc: cut-off of the test (20s for the Hargreaves test and 2g for the Von Frey test)
*Hotplate Test:* Animals were placed on a 10" x 10" metal plate surrounded by 4 Plexiglas walls (15 inches high). The plate was maintained at a temperature of either 50 or 55°C. The response latency (time when the animal first flinches or licks its hind paw, jumps, or vocalizes) was measured and the animal removed from the plate. Animals showing no response were removed from the plate after 40s (50°C) or 20s (55°C) to prevent any possible tissue damage. This trial was repeated 2-5 times every 15-60 minutes in a day.

### Inflammatory Pain Models

*CFA Model:* Animals were anesthetized with isoflurane (4% induction and 2% maintenance) and 20 µL of 100% Complete Freund's Adjuvant (CFA; Sigma-Aldrich; Saint Louis, MO) was injected into the center plantar area on one hind paw using a 27gauge needle attached to a 50µL Hamilton syringe. *Carrageenan model:* Animals were anesthetized with isoflurane (4% induction and 2% maintenance) and 25 µL of 2% λ-carrageenan (Sigma-Aldrich; Saint Louis, MO) dissolved in normal saline was injected into the center plantar area on hind paws using an insulin syringe (BD; Franklin Lakes, New Jersey).

### Implantation of Mini Pumps

Alzet micro-osmotic mini pumps (Durect Corporation Model 1003D and 2001D) were filled and primed per manufacturer's guide. Mice were anesthetized with isoflurane (5% induction; 2% maintenance). Their backs were shaved, wiped down with isopropyl alcohol and povidone iodine, and a small incision was made between the scapulae. Using a pair of forceps or hemostat, a small pocket was formed by spreading the subcutaneous connective tissues apart. The pump was inserted into the pocket with the flow moderator pointing away from the incision. The skin incision was then closed using 7mm staples and the animals were allowed to recover in their home cages.

### Data Analysis

Data are represented as mean ± s.e.m. Prism (Graphpad Software Inc., La Jolla, CA) was used for graphing and statistical analysis. For comparison of threshold values over time, a two-way ANOVA followed by Bonferroni's multiple comparison test was used with a significance level of p<0.05. Hotplate and data were analyzed by one-way ANOVA followed by Bonferroni's multiple comparison test.

### Results

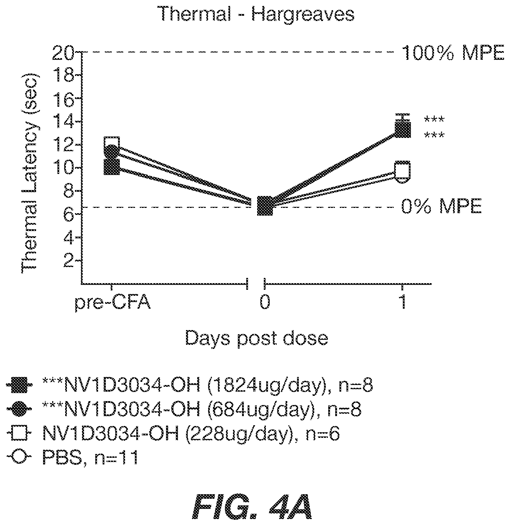

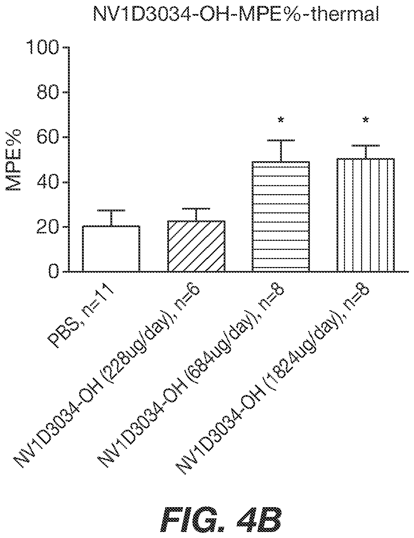

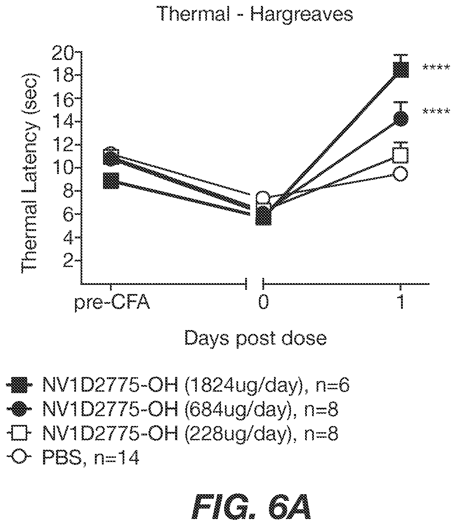

Efficacy of variants NV1D3034-OH (NV1D3034-COOH), NV1D3368-OH (NV1D3368-COOH) and NV1D2775-OH (NV1D2775-COOH) was studied in the CFA model, a commonly used model of inflammatory pain. The injection of CFA in the hindpaw induced paw edema (not shown) and hypersensitivity to thermal stimuli (thermal hyperalgesia), as indicated by the lowered thermal latency in the injected paw on day0 (Figure **6A**). Thermal hyperalgesia was completely reversed by NV1D3034-OH at 684 and 1824µg/day, when administered by a subcutaneous osmotic mini-pump (Figure **4A** and 4**B**).

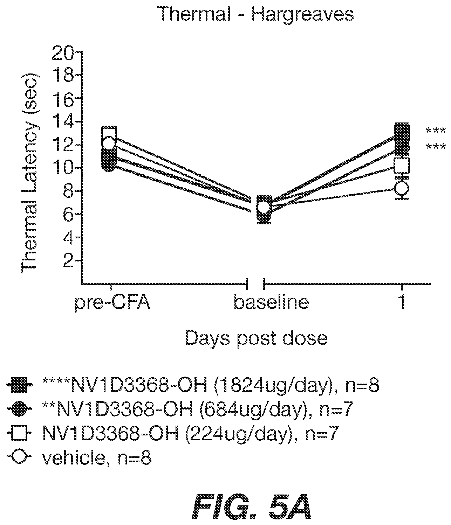

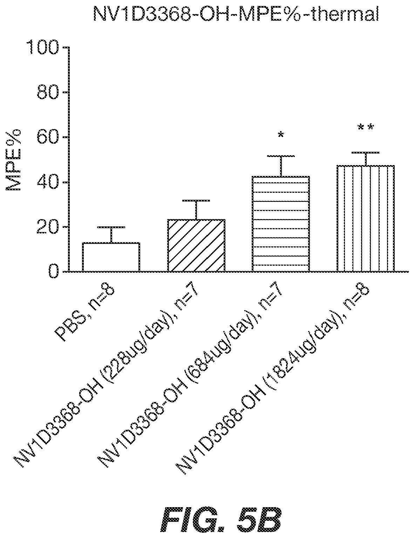

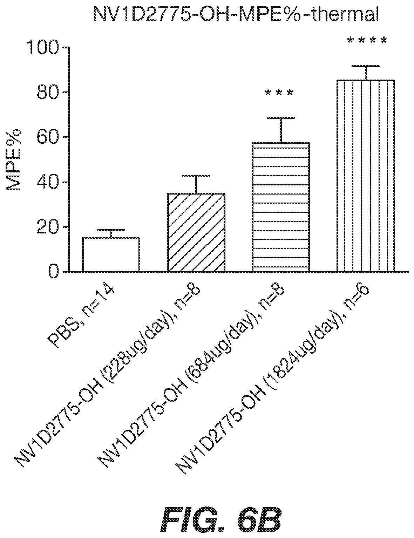

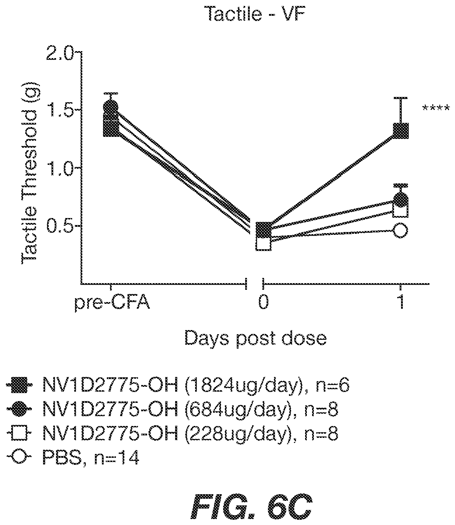

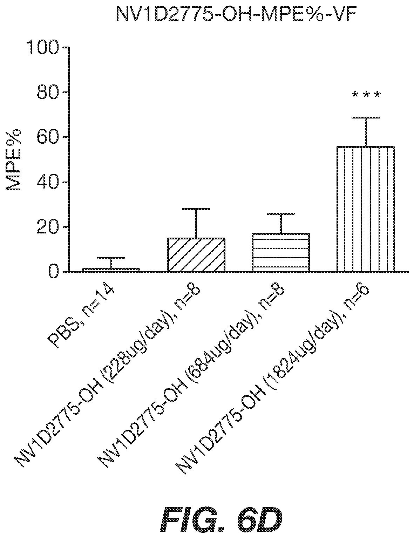

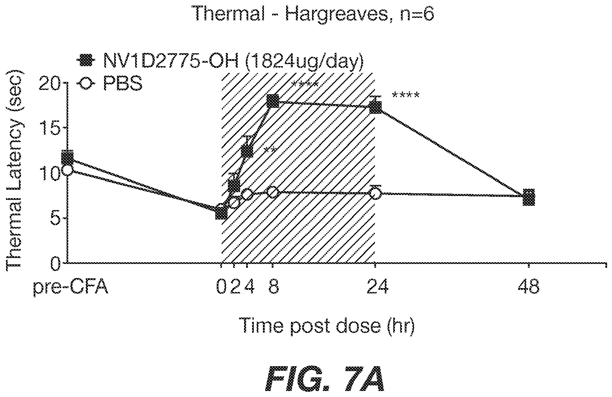

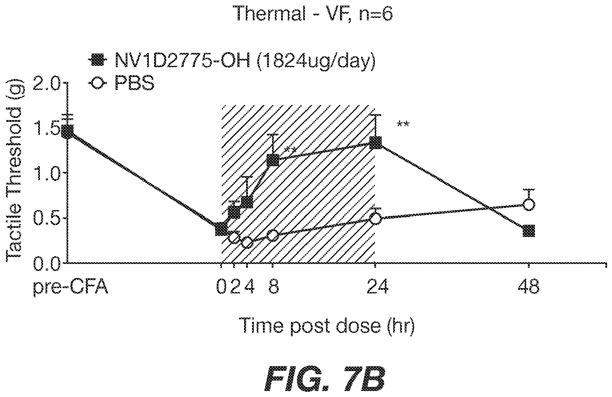

NV1D3368-OH fully reversed CFA-induced thermal hyperalgesia at 684 and 1824µg/day (Figure **5A** and 5**B**). NV1D2775-OH demonstrated strong efficacy in the CFA model. Thermal latencies reached values close to the cut-off following NV1D2775 administration (Figure **6A** and 6**B**, 1824µg/day), suggesting a strong analgesia effect on top of the anti-hyperalgesia effect. In addition, NV1D2775-OH reversed CFA-induced tactile allodynia (Figure **6C** and 6**D**, 1824µg/day). The anti-hyperalgesic effect of NV1D2775-OH was seen as early as 4hr post-pump implantation (Figure **7A**). The effect reached the maximum at 8hr in both the thermal and tactile tests (Figure **7A** and 7**B**), which was maintained at 24hr. Thermal latency and tactile threshold returned the control level by 48h post pump implantation (approximately 24h after the pumps were predicted to be empty) (Figure **7A** and 7**B**).

CFA-induced thermal hyperalgesia was readily reversed by two additional peptides, NV1D3368-amide (NV1D3368-NH₂) and NV1D3034-N-methylamide (NV1D3034-NHMe). Thermal from the experiments is summarized in Table 13.

**Table 13.**

| Peptide | Dose (µg/day/mouse) | | | |
|---|---|---|---|---|
| | Vehicle (PBS) | 228 | 684 | 1824 |
| NV1D3034-OH | 20±7 (11) | 22±6 (6) | 48±10* (8) | 50±6* (8) |
| NV1D3368-OH | 13±7 (8) | 23±8 (7) | 42±9* (7) | 47±6** (8) |
| NV1D2775-OH | 15±4 (20) | 35±8 (8) | 57±12*** (8) | 85±6**** (12) |
| NV1D3368-NH₂ | 15±13 (6) | 27±4 (4) | 46±9 (4) | 55±15 (6) |
| NV1D3034-NHMe | 5±25 (3) | | | 49±17 (6) |
| *P<0.05, **P<0.01, ***P<0.001 and ****P<0.0001 vs. PBS, one-way ANOVA followed by Bonferroni's multiple comparison. | | | | |

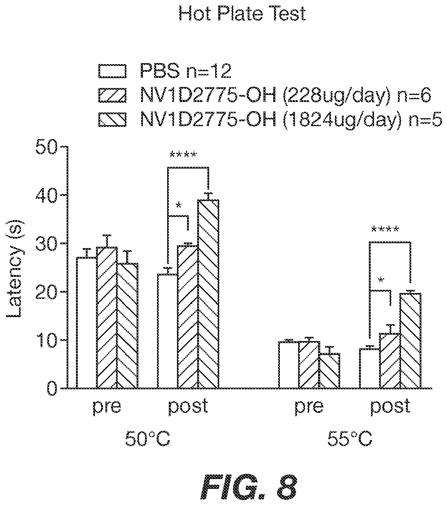

NV1D2775-OH also exhibited strong, dose-dependent efficacy in the hotplate test (Figure **8**). Latencies at 50 and 55°C reached values near cut-off following the administration of 1824µg/day. At 228µg/day, NV1D2775-OH produced a modest yet significant increase in the thermal latency, compared to the PBS control.

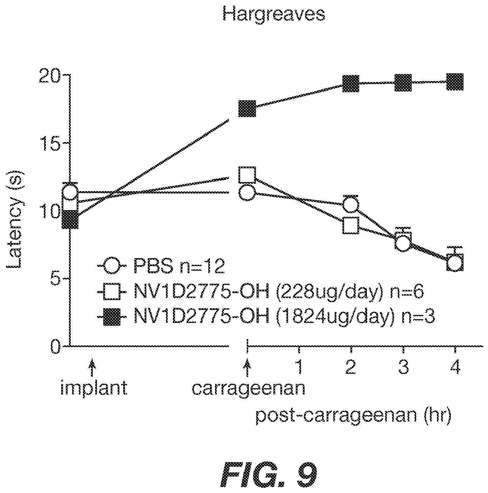

The efficacy of NV1D2775-OH was evaluated in another model of inflammatory pain, the carrageenan model. Animals were implanted with NV1D2775-OH or PBS pumps. Thermal withdrawal latencies were measured pre-and on dayl post-pump. λ-carrageenan was injected into the hindpaws and thermal latencies were measured again on 2, 3 and 4hr following carrageenan. NV1D2775-OH at 1824µg/day produced significant analgesia (Figure **9**). Injection of λ-carrageenan in the hindpaws induced inflammation (not shown) and lowered thermal paw withdrawal latency in the Hargreaves test over the 4hr test-period (Figure **9****,** PBS group). Animals pretreated with NV1D2775-OH at 1824µg/day were fully protected from carrageenan-induced hyperalgesia.

## Claims

1. An isolated Protoxin-II variant comprising an amino acid sequence selected from the group consisting of SEQ ID Nos:
40, 44, 56, 59, 65, 78, 109, 110, 111, 114, 115, 116, 117, 118, 119, 121, 122, 123, 129, 131, 132, 133, 134, 135, 136, 138, 139, 140, 141, 142, 145, 146, 147, 149, 150, 151, 152, 153, 154, 156, 158, 159, 172, 173, 175, 177, 178, 183, 184, 185, 186, 189, 190, 193, 196, 197, 199, 205, 207, 210, 211, 216, 217, 224, 230, 269, 270, 271, 272, 273, 274, 275, 277, 278, 279, 280, 281, 282, 283, 284, 287, 288, 289, 290, 291, 292, 297, 298, 299, 301, 302, 304, 305, 307, 308, 309, 310, 312, 314, 315, 318, 319, 320, 321, 322, 323, 324, 325, and 326,
wherein the variant inhibits human Nav1.7 activity with an IC₅₀ value of 30x10⁻⁹ M or less, wherein the IC₅₀ value is measured using a FLIPR Tetra membrane depolarisation assay, the assay comprising fluorescence resonance energy transfer (FRET) in the presence of 25x10⁻⁶ M 3-veratroylveracevine in HEK293 cells stably expressing human Nav1.7 and using bis-1,3(diethylthiobarbituric acid)trimethine oxonol as an electron acceptor and trisodium 8-octadecyloxypyrene-1,3,6-trisulfonate as a donor by exciting the donor at 390- 420 nm and measuring FRET at 515-575 nm.

2. The isolated Protoxin-II variant of claim 1, wherein the variant selectively inhibits human Navl.7.

3. The isolated Protoxin-II variant of claim 2, comprising an amino acid sequence selected from the group consisting of the sequence of SEQ ID NOs: 56, 78, 114, 129, 133, and 146.

4. The isolated Protoxin-II variant of any of the claims 1, 2 or 3, having a free C-terminal carboxylic acid, amide, methylamide or butylamide group.

5. A conjugate comprising the Protoxin-II variant of any of the claims 1-3 conjugated to a half-life extending moiety; wherein the conjugate is a fusion protein comprising the Protoxin-II variant and a human serum albumin (HSA), albumin binding domain (ABD), or a Fc polypeptide; or the conjugate comprises polyethylene glycol (PEG) chemically coupled to the Protoxin-II variant.

6. An isolated polynucleotide encoding the Protoxin-II variant of any one of claims 1-3.

7. A vector comprising the isolated polynucleotide of claim 6.

8. A host cell comprising the vector of claim 7.

9. A method of producing an isolated Protoxin-II variant, comprising culturing the host cell of claim 8 and recovering the Protoxin-II variant produced by the host cell.

10. A pharmaceutical composition comprising the isolated Protoxin-II variant of any one of claims 1-4 or the conjugate of claim 5 and a pharmaceutically acceptable excipient.

11. The Protoxin-II variant of any one of claims 1-4 or the pharmaceutical composition of claim 10 for use in treating Navl.7-mediated pain in a subject in need thereof; wherein optionally
(a) pain is chronic pain, acute pain, neuropathic pain, nociceptive pain, visceral pain, back pain, post-operative pain, thermal pain, phantom limb pain, or pain associated with inflammatory conditions, primary erythemalgia (PE), paroxysmal extreme pain disorder (PEPD), osteoarthritis, rheumatoid arthritis, lumbar discectomy, pancreatitis, fibromyalgia, painful diabetic neuropathy (PDN), post-herpetic neuropathy (PHN), trigeminal neuralgia (TN), spinal cord injuries or multiple sclerosis; or
(b) the Protoxin-II variant is administered peripherally; or
(c) the Protoxin-II variant is administered locally to a joint, spinal cord, surgical wound, sites of injury or trauma, peripheral nerve fibers, urogenital organs, or inflamed tissues; or
(d) the subject is a human.

## Patentansprüche

1. Isolierte Protoxin-II-Variante, umfassend eine Aminosäuresequenz, ausgewählt aus der Gruppe bestehend aus SEQ ID Nrn.:
40, 44, 56, 59, 65, 78, 109, 110, 111, 114, 115, 116, 117, 118, 119, 121, 122, 123, 129, 131, 132, 133, 134, 135, 136, 138, 139, 140, 141, 142, 145, 146, 147, 149, 150, 151, 152, 153, 154, 156, 158, 159, 172, 173, 175, 177, 178, 183, 184, 185, 186, 189, 190, 193, 196, 197, 199, 205, 207, 210, 211, 216, 217, 224, 230, 269, 270, 271, 272, 273, 274, 275, 277, 278, 279, 280, 281, 282, 283, 284, 287, 288, 289, 290, 291, 292, 297, 298, 299, 301, 302, 304, 305, 307, 308, 309, 310, 312, 314, 315, 318, 319, 320, 321, 322, 323, 324, 325 und 326,
wobei die Variante humane Nav1.7-Aktivität mit einem IC₅₀-Wert von 30x10⁻⁹ M oder weniger hemmt, wobei der IC₅₀-Wert unter Verwendung eines FLIPR Tetra Membran-Depolarisations-Assays gemessen wird, wobei der Assay Förster-Resonanz-Energie-Transfer (FRET) in Gegenwart von 25x10⁻⁶ M 3-Veratroylveracevin in HEK293-Zellen, die stabil humanen Nav1.7 exprimieren und unter Verwendung von Bis-1,3(diethylthiobarbitursäure)trimethinoxonol als einen Elektronenakzeptor und Trinatrium-8-octadecyloxypyren-1,3,6-trisulfonat als Donor durch Anregung des Donors bei 390-420 nm und Messung des FRET bei 515-575 nm umfasst.

2. Isolierte Protoxin-II-Variante nach Anspruch 1, wobei die Variante selektiv humanen Nav1.7 hemmt.

3. Isolierte Protoxin-II-Variante nach Anspruch 2, die eine Aminosäuresequenz umfasst, die aus der Gruppe bestehend aus der Sequenz unter SEQ ID Nrn.: 56, 78, 114 129, 133 und 146 ausgewählt wird.

4. Isolierte Protoxin-II-Variante nach einem der Ansprüche 1, 2 oder 3, die eine freie C-terminale Carbonsäure-, Amid-, Methylamid oder Butylamidgruppe aufweist.

5. Konjugat, das die Protoxin-II-Variante nach einem der Ansprüche 1-3 konjugiert mit einer Halbwertszeit verlängernden Einheit umfasst; wobei es sich bei dem Konjugat um ein Fusionsprotein handelt, das die Protoxin-II-Variante und ein Humanserumalbumin (HSA), eine Albumin bindende Domäne (ABD) oder ein Fc-Polypeptid umfasst; oder das Konjugat Polyethylenglykol (PEG), das chemisch an die Protoxin-II-Variante gekoppelt ist, umfasst.

6. Isoliertes Polynukleotid, das die Protoxin-II-Variante nach einem der Ansprüche 1-3 kodiert.

7. Vektor, der das isolierte Polynukleotid nach Anspruch 6 umfasst.

8. Wirtszelle, die den Vektor nach Anspruch 7 umfasst.

9. Verfahren zum Herstellen einer isolierten Protoxin-II-Variante, das das Kultivieren der Wirtszelle nach Anspruch 8 und das Gewinnen der Protoxin-II-Variante, die durch die Wirtszelle hergestellt wird, umfasst.

10. Pharmazeutische Zusammensetzung, die die isolierte Protoxin-II-Variante nach einem der Ansprüche 1-4 oder das Konjugat nach Anspruch 5 und einen pharmazeutisch verträglichen Hilfsstoff umfasst.

11. Protoxin-II-Variante nach einem der Ansprüche 1-4 oder pharmazeutische Zusammensetzung nach Anspruch 10 zur Verwendung bei der Behandlung von Nav1.7-vermittelten Schmerzen bei einem Individuum, der dies benötigt; wobei gegebenenfalls
(a) es sich bei den Schmerzen um chronische Schmerzen, akute Schmerzen, neuropathische Schmerzen, nozizeptive Schmerzen, viszerale Schmerzen, Rückenschmerzen, postoperative Schmerzen, thermische Schmerzen, Phantomschmerzen der Gliedmaßen oder Schmerzen im Zusammenhang mit entzündlichen Zuständen, primärer Erythermalgie (PE), paroxysmaler extremer Schmerzstörung (PEPD), Osteoarthritis, rheumatoider Arthritis, lumbaler Diskektomie, Pankreatitis, Fibromyalgie, schmerzhafter diabetischer Neuropathie (PDN), postherpetischer Neuropathie (PHN), Trigeminusneuralgie (TN), Rückenmarksverletzungen oder Multipler Sklerose handelt; oder
(b) die Protoxin-II-Variante peripher verabreicht wird; oder
(c) die Protoxin-II-Variante lokal an ein Gelenk, Rückenmark, eine chirurgische Wunde, Stellen von Verletzung oder Trauma, periphere Nervenfasern, urogenitale Organe oder entzündete Gewebe verabreicht wird; oder
(d) das Individuum ein Mensch ist.

## Revendications

1. Variant de Protoxine-II isolé comprenant une séquence d'acides aminés choisie dans le groupe consistant en SEQ ID NO :
40, 44, 56, 59, 65, 78, 109, 110, 111, 114, 115, 116, 117, 118, 119, 121, 122, 123, 129, 131, 132, 133, 134, 135, 136, 138, 139, 140, 141, 142, 145, 146, 147, 149, 150, 151, 152, 153, 154, 156, 158, 159, 172, 173, 175, 177, 178, 183, 184, 185, 186, 189, 190, 193, 196, 197, 199, 205, 207, 210, 211, 216, 217, 224, 230, 269, 270, 271, 272, 273, 274, 275, 277, 278, 279, 280, 281, 282, 283, 284, 287, 288, 289, 290, 291, 292, 297, 298, 299, 301, 302, 304, 305, 307, 308, 309, 310, 312, 314, 315, 318, 319, 320, 321, 322, 323, 324, 325, et 326,
dans lequel le variant inhibe l'activité du Nav1.7 humain avec une CI₅₀ de 30x10⁻⁹ M ou moins, dans lequel la CI₅₀ est mesurée par utilisation d'un essai de dépolarisation tétramembranaire FLIPR, l'essai comprenant un transfert d'énergie par résonance en fluorescence (FRET) en présence de 25x10⁻⁶ M de 3-vératroylvéracévine dans des cellules HEK293 exprimant stablement le Nav1.7 humain et utilisant du bis(acide 1,3-diéthylthiobarbiturique)triméthine oxonol en tant qu'accepteur d'électrons et du 8-octadécyloxypyrène-1,3,6-trisulfonate trisodique en tant que donneur par excitation du donneur à 390-420 nm, et mesure de FRET à 515-575 nm.

2. Variant de Protoxine-II isolé selon la revendication 1, dans lequel le variant inhibe sélectivement le Nav1.7 humain.

3. Variant de Protoxine-II isolé selon la revendication 2, comprenant une séquence d'acides aminés choisie dans le groupe consistant en la séquence de SEQ ID NO : 56, 78, 114, 129, 133 et 146.

4. Variant de Protoxine-II isolé selon l'une quelconque des revendications 1, 2 ou 3, ayant un groupe acide carboxylique, amide, méthylamide ou butylamide C-terminal libre.

5. Conjugué comprenant le variant de Protoxine-II selon l'une quelconque des revendications 1 à 3 conjugué à un fragment d'extension de demi-vie ; dans lequel le conjugué est une protéine de fusion comprenant le variant de Protoxine-II et une sérumalbumine bovine (HSA), un domaine de liaison à l'albumine (ABD), ou un polypeptide Fc ; ou le conjugué comprend du polyéthylèneglycol (PEG) chimiquement couplé au variant de Protoxine-II.

6. Polynucléotide isolé codant pour le variant de Protoxine-II selon l'une quelconque des revendications 1 à 3.

7. Vecteur comprenant le polynucléotide isolé selon la revendication 6.

8. Cellule hôte comprenant le vecteur selon la revendication 7.

9. Procédé de production d'un variant de Protoxine-II isolé, comprenant la culture de la cellule hôte selon la revendication 8 et la récupération du variant de Protoxine-II produit par la cellule hôte.

10. Composition pharmaceutique comprenant le variant de Protoxine-II isolé selon l'une quelconque des revendications 1 à 4 ou le conjugué selon la revendication 5 et un excipient pharmaceutiquement acceptable.

11. Variant de Protoxine-II selon l'une quelconque des revendications 1 à 4 ou composition pharmaceutique selon la revendication 10 pour une utilisation dans le traitement d'une douleur médiée par le Nav1.7 chez un sujet qui en a besoin ; dans lequel, éventuellement
(a) la douleur est une douleur chronique, une douleur aiguë, une douleur neuropathique, une douleur nociceptive, une douleur viscérale, une douleur dorsale, une douleur post-opératoire, une douleur thermique, une douleur du membre fantôme ou une douleur associée à des états inflammatoires, une érythermalgie primaire (EP), un syndrome de douleur extrême paroxystique (PEPD), une ostéoarthrite, une polyarthrite rhumatoïde, une discectomie lombaire, une pancréatite, une fibromyalgie, une neuropathie diabétique douloureuse (NDD), une neuropathie post-herpétique (NPH), une névralgie trigéminale (NT), des lésions de la moelle épinière ou la sclérose en plaques ; ou
(b) le variant de Protoxine-II est administré par voie périphérique ; ou
(c) le variant de Protoxine-II est administré localement par une articulation, la moelle épinière, une plaie chirurgicale, des sites de liaison ou de traumatisme, des fibres nerveuses périphériques, des organes urogénitaux ou des tissus enflammés ; ou
(d) le sujet est un humain.


### SEQUENCE LISTING

<110> Janssen Biotech, Inc.
<120> PROTOXIN-II VARIANTS AND METHODS OF USE
<130> P068172EP
<140> EP14790422.1
   <141> 2013-10-03
<150> PCT/US2014/058972
   <151> 2013-10-03
<150> 61/886,100
   <151> 2013-10-03
<160> 407
<170> PatentIn version 3.5
<210> 1
   <211> 30
   <212> PRT
   <213> Thrixopelma pruriens
<220>
   <221> MOD_RES
   <222> (30)..(30)
   <223> -COOH
<400> 1
   Tyr Cys Gln Lys Trp Met Trp Thr Cys Asp Ser Glu Arg Lys Cys Cys
   1               5                   10                  15
   Glu Gly Met Val Cys Arg Leu Trp Cys Lys Lys Lys Leu Trp
               20                  25                  30
<210> 2
   <211> 32
   <212> PRT
   <213> Artificial Sequence
<220>
   <223> Description of Artificial Sequence: Synthetic NV1D12 polypeptide
<220>
   <221> MOD_RES
   <222> (32)..(32)
   <223> -COOH
<400> 2
   Gly Pro Tyr Cys Gln Lys Trp Met Trp Thr Cys Asp Ser Glu Arg Lys
   1               5                   10                  15
   Cys Cys Glu Gly Met Val Cys Arg Leu Trp Cys Lys Lys Lys Leu Trp
               20                  25                  30
<210> 3
   <211> 32
   <212> PRT
   <213> Artificial Sequence
<220>
   <223> Description of Artificial Sequence: Synthetic NV1D748 polypeptide
<220>
   <221> MOD_RES
   <222> (32)..(32)
   <223> -COOH
<400> 3
   Gly Pro Ala Cys Gln Lys Trp Met Trp Thr Cys Asp Ser Glu Arg Lys
   1               5                   10                  15
   Cys Cys Glu Gly Met Val Cys Arg Leu Trp Cys Lys Lys Lys Leu Trp
               20                  25                  30
<210> 4
   <211> 32
   <212> PRT
   <213> Artificial Sequence
<220>
   <223> Description of Artificial Sequence: Synthetic NV1D751 polypeptide
<220>
   <221> MOD_RES
   <222> (32)..(32)
   <223> -COOH
<400> 4
   Gly Pro Gln Cys Gln Lys Trp Met Trp Thr Cys Asp Ser Glu Arg Lys
   1               5                   10                  15
   Cys Cys Glu Gly Met Val Cys Arg Leu Trp Cys Lys Lys Lys Leu Trp
               20                  25                  30
<210> 5
   <211> 32
   <212> PRT
   <213> Artificial Sequence
<220>
   <223> Description of Artificial Sequence: Synthetic NV1D2292 polypeptide
<220>
   <221> MOD_RES
   <222> (32)..(32)
   <223> -COOH
<400> 5
   Gly Pro Arg Cys Gln Lys Trp Met Trp Thr Cys Asp Ser Glu Arg Lys
   1               5                   10                  15
   Cys Cys Glu Gly Met Val Cys Arg Leu Trp Cys Lys Lys Lys Leu Trp
               20                  25                  30
<210> 6
   <211> 32
   <212> PRT
   <213> Artificial Sequence
<220>
   <223> Description of Artificial Sequence: Synthetic NV1D750 polypeptide
<220>
   <221> MOD_RES
   <222> (32)..(32)
   <223> -COOH
<400> 6
   Gly Pro Ser Cys Gln Lys Trp Met Trp Thr Cys Asp Ser Glu Arg Lys
   1               5                   10                  15
   Cys Cys Glu Gly Met Val Cys Arg Leu Trp Cys Lys Lys Lys Leu Trp
               20                  25                  30
<210> 7
   <211> 32
   <212> PRT
   <213> Artificial Sequence
<220>
   <223> Description of Artificial Sequence: Synthetic NV1D1328 polypeptide
<220>
   <221> MOD_RES
   <222> (32)..(32)
   <223> -COOH
<400> 7
   Gly Pro Tyr Cys Gln Lys Trp Phe Trp Thr Cys Asp Ser Glu Arg Lys
   1 5 10 15
   Cys Cys Glu Gly Met Val Cys Arg Leu Trp Cys Lys Lys Lys Leu Trp
   20 25 30
<210> 8
   <211> 32
   <212> PRT
   <213> Artificial Sequence
<220>
   <223> Description of Artificial Sequence: Synthetic NV1D774 polypeptide
<220>
   <221> MOD_RES
   <222> (32)..(32)
   <223> -COOH
<400> 8
   Gly Pro Tyr Cys Gln Lys Trp Met Gln Thr Cys Asp Ser Glu Arg Lys
   1 5 10 15
   Cys Cys Glu Gly Met Val Cys Arg Leu Trp Cys Lys Lys Lys Leu Trp
   20 25 30
<210> 9
   <211> 32
   <212> PRT
   <213> Artificial Sequence
<220>
   <223> Description of Artificial Sequence: Synthetic NV1D786 polypeptide
<220>
   <221> MOD_RES
   <222> (32)..(32)
   <223> -COOH
<400> 9
   Gly Pro Tyr Cys Gln Lys Trp Met Trp Thr Cys Asp Ala Glu Arg Lys
   1 5 10 15
   Cys Cys Glu Gly Met Val Cys Arg Leu Trp Cys Lys Lys Lys Leu Trp
   20 25 30
<210> 10
   <211> 32
   <212> PRT
   <213> Artificial Sequence
<220>
   <223> Description of Artificial Sequence: Synthetic NV1D2300 polypeptide
<220>
   <221> MOD_RES
   <222> (32)..(32)
   <223> -COOH
<400> 10
   Gly Pro Tyr Cys Gln Lys Trp Met Trp Thr Cys Asp Arg Glu Arg Lys
   1               5                   10                  15
   Cys Cys Glu Gly Met Val Cys Arg Leu Trp Cys Lys Lys Lys Leu Trp
               20                  25                  30
<210> 11
   <211> 32
   <212> PRT
   <213> Artificial Sequence
<220>
   <223> Description of Artificial Sequence: Synthetic NV1D791 polypeptide
<220>
   <221> MOD_RES
   <222> (32)..(32)
   <223> -COOH
<400> 11
   Gly Pro Tyr Cys Gln Lys Trp Met Trp Thr Cys Asp Ser Lys Arg Lys
   1               5                   10                  15
   Cys Cys Glu Gly Met Val Cys Arg Leu Trp Cys Lys Lys Lys Leu Trp
               20                  25                  30
<210> 12
   <211> 32
   <212> PRT
   <213> Artificial Sequence
<220>
   <223> Description of Artificial Sequence: Synthetic NV1D1332 polypeptide
<220>
   <221> MOD_RES
   <222> (32)..(32)
   <223> -COOH
<400> 12
   Gly Pro Tyr Cys Gln Lys Trp Met Trp Thr Cys Asp Ser Asn Arg Lys
   1               5                   10                  15
   Cys Cys Glu Gly Met Val Cys Arg Leu Trp Cys Lys Lys Lys Leu Trp
               20                  25                  30
<210> 13
   <211> 32
   <212> PRT
   <213> Artificial Sequence
<220>
   <223> Description of Artificial Sequence: Synthetic NV1D2512 polypeptide
<220>
   <221> MOD_RES
   <222> (32)..(32)
   <223> -COOH
<400> 13
   Gly Pro Tyr Cys Gln Lys Trp Met Trp Thr Cys Asp Ser Glu Arg Lys
   1               5                   10                  15
   Cys Cys Glu Gly Phe Val Cys Arg Leu Trp Cys Lys Lys Lys Leu Trp
               20                  25                  30
<210> 14
   <211> 32
   <212> PRT
   <213> Artificial Sequence
<220>
   <223> Description of Artificial Sequence: Synthetic NV1D1336 polypeptide
<220>
   <221> MOD_RES
   <222> (32)..(32)
   <223> -COOH
<400> 14
   Gly Pro Tyr Cys Gln Lys Trp Met Trp Thr Cys Asp Ser Glu Arg Lys
   1               5                   10                  15
   Cys Cys Glu Gly Leu Val Cys Arg Leu Trp Cys Lys Lys Lys Leu Trp
               20                  25                  30
<210> 15
   <211> 32
   <212> PRT
   <213> Artificial Sequence
<220>
   <223> Description of Artificial Sequence: Synthetic NV1D1337 polypeptide
<220>
   <221> MOD_RES
   <222> (32)..(32)
   <223> -COOH
<400> 15
   Gly Pro Tyr Cys Gln Lys Trp Met Trp Thr Cys Asp Ser Glu Arg Lys
   1 5 10 15
   Cys Cys Glu Gly Met Val Cys Thr Leu Trp Cys Lys Lys Lys Leu Trp
   20 25 30
<210> 16
   <211> 32
   <212> PRT
   <213> Artificial Sequence
<220>
   <223> Description of Artificial Sequence: Synthetic NV1D2308 polypeptide
<220>
   <221> MOD_RES
   <222> (32)..(32)
   <223> -COOH
<400> 16
   Gly Pro Tyr Cys Gln Lys Trp Met Trp Thr Cys Asp Ser Glu Arg Lys
   1 5 10 15
   Cys Cys Glu Gly Met Val Cys Arg Leu Trp Cys Arg Lys Lys Leu Trp
   20 25 30
<210> 17
   <211> 32
   <212> PRT
   <213> Artificial Sequence
<220>
   <223> Description of Artificial Sequence: Synthetic NV1D2670 polypeptide
<220>
   <221> MOD_RES
   <222> (32)..(32)
   <223> -COOH
<400> 17
   Gly Pro Ala Cys Gln Lys Trp Met Gln Thr Cys Asp Ser Glu Arg Lys
   1 5 10 15
   Cys Cys Glu Gly Met Val Cys Arg Leu Trp Cys Lys Lys Lys Leu Trp
   20 25 30
<210> 18
   <211> 32
   <212> PRT
   <213> Artificial Sequence
<220>
   <223> Description of Artificial Sequence: Synthetic NV1D2674 polypeptide
<220>
   <221> MOD_RES
   <222> (32)..(32)
   <223> -COOH
<400> 18
   Gly Pro Ala Cys Gln Lys Trp Met Trp Thr Cys Asp Ala Glu Arg Lys
   1               5                   10                  15
   Cys Cys Glu Gly Met Val Cys Arg Leu Trp Cys Lys Lys Lys Leu Trp
               20                  25                  30
<210> 19
   <211> 32
   <212> PRT
   <213> Artificial Sequence
<220>
   <223> Description of Artificial Sequence: Synthetic NV1D2664 polypeptide
<220>
   <221> MOD_RES
   <222> (32)..(32)
   <223> -COOH
<400> 19
   Gly Pro Ala Cys Gln Lys Trp Met Trp Thr Cys Asp Ser Glu Arg Lys
   1               5                   10                  15
   Cys Cys Glu Gly Phe Val Cys Arg Leu Trp Cys Lys Lys Lys Leu Trp
               20                  25                  30
<210> 20
   <211> 32
   <212> PRT
   <213> Artificial Sequence
<220>
   <223> Description of Artificial Sequence: Synthetic NV1D2671 polypeptide
<220>
   <221> MOD_RES
   <222> (32)..(32)
   <223> -COOH
<400> 20
   Gly Pro Gln Cys Gln Lys Trp Met Gln Thr Cys Asp Ser Glu Arg Lys
   1               5                   10                  15
   Cys Cys Glu Gly Met Val Cys Arg Leu Trp Cys Lys Lys Lys Leu Trp
   20 25 30
<210> 21
   <211> 32
   <212> PRT
   <213> Artificial Sequence
<220>
   <223> Description of Artificial Sequence: Synthetic NV1D2675 polypeptide
<220>
   <221> MOD_RES
   <222> (32)..(32)
   <223> -COOH
<400> 21
   Gly Pro Gln Cys Gln Lys Trp Met Trp Thr Cys Asp Ala Glu Arg Lys
   1 5 10 15
   Cys Cys Glu Gly Met Val Cys Arg Leu Trp Cys Lys Lys Lys Leu Trp
   20 25 30
<210> 22
   <211> 32
   <212> PRT
   <213> Artificial Sequence
<220>
   <223> Description of Artificial Sequence: Synthetic NV1D2665 polypeptide
<220>
   <221> MOD_RES
   <222> (32)..(32)
   <223> -COOH
<400> 22
   Gly Pro Gln Cys Gln Lys Trp Met Trp Thr Cys Asp Ser Glu Arg Lys
   1 5 10 15
   Cys Cys Glu Gly Phe Val Cys Arg Leu Trp Cys Lys Lys Lys Leu Trp
   20 25 30
<210> 23
   <211> 32
   <212> PRT
   <213> Artificial Sequence
<220>
   <223> Description of Artificial Sequence: Synthetic NV1D2668 polypeptide
<220>
   <221> MOD_RES
   <222> (32)..(32)
   <223> -COOH
<400> 23
   Gly Pro Arg Cys Gln Lys Trp Met Gln Thr Cys Asp Ser Glu Arg Lys
   1 5 10 15
   Cys Cys Glu Gly Met Val Cys Arg Leu Trp Cys Lys Lys Lys Leu Trp
   20 25 30
<210> 24
   <211> 32
   <212> PRT
   <213> Artificial Sequence
<220>
   <223> Description of Artificial Sequence: Synthetic NV1D2672 polypeptide
<220>
   <221> MOD_RES
   <222> (32)..(32)
   <223> -COOH
<400> 24
   Gly Pro Arg Cys Gln Lys Trp Met Trp Thr Cys Asp Ala Glu Arg Lys
   1 5 10 15
   Cys Cys Glu Gly Met Val Cys Arg Leu Trp Cys Lys Lys Lys Leu Trp
   20 25 30
<210> 25
   <211> 32
   <212> PRT
   <213> Artificial Sequence
<220>
   <223> Description of Artificial Sequence: Synthetic NV1D2662 polypeptide
<220>
   <221> MOD_RES
   <222> (32)..(32)
   <223> -COOH
<400> 25
   Gly Pro Arg Cys Gln Lys Trp Met Trp Thr Cys Asp Ser Glu Arg Lys
   1 5 10 15
   Cys Cys Glu Gly Phe Val Cys Arg Leu Trp Cys Lys Lys Lys Leu Trp
   20 25 30
<210> 26
   <211> 32
   <212> PRT
   <213> Artificial Sequence
<220>
   <223> Description of Artificial Sequence: Synthetic NV1D2669 polypeptide
<220>
   <221> MOD_RES
   <222> (32)..(32)
   <223> -COOH
<400> 26
   Gly Pro Ser Cys Gln Lys Trp Met Gln Thr Cys Asp Ser Glu Arg Lys
   1               5                   10                  15
   Cys Cys Glu Gly Met Val Cys Arg Leu Trp Cys Lys Lys Lys Leu Trp
               20                  25                  30
<210> 27
   <211> 32
   <212> PRT
   <213> Artificial Sequence
<220>
   <223> Description of Artificial Sequence: Synthetic NV1D2673 polypeptide
<220>
   <221> MOD_RES
   <222> (32)..(32)
   <223> -COOH
<400> 27
   Gly Pro Ser Cys Gln Lys Trp Met Trp Thr Cys Asp Ala Glu Arg Lys
   1               5                   10                  15
   Cys Cys Glu Gly Met Val Cys Arg Leu Trp Cys Lys Lys Lys Leu Trp
               20                  25                  30
<210> 28
   <211> 32
   <212> PRT
   <213> Artificial Sequence
<220>
   <223> Description of Artificial Sequence: Synthetic NV1D2663 polypeptide
<220>
   <221> MOD_RES
   <222> (32)..(32)
   <223> -COOH
<400> 28
   Gly Pro Ser Cys Gln Lys Trp Met Trp Thr Cys Asp Ser Glu Arg Lys
   1 5 10 15
   Cys Cys Glu Gly Phe Val Cys Arg Leu Trp Cys Lys Lys Lys Leu Trp
   20 25 30
<210> 29
   <211> 32
   <212> PRT
   <213> Artificial Sequence
<220>
   <223> Description of Artificial Sequence: Synthetic NV1D2676 polypeptide
<220>
   <221> MOD_RES
   <222> (32)..(32)
   <223> -COOH
<400> 29
   Gly Pro Tyr Cys Gln Lys Trp Met Gln Thr Cys Asp Ala Glu Arg Lys
   1 5 10 15
   Cys Cys Glu Gly Met Val Cys Arg Leu Trp Cys Lys Lys Lys Leu Trp
   20 25 30
<210> 30
   <211> 32
   <212> PRT
   <213> Artificial Sequence
<220>
   <223> Description of Artificial Sequence: Synthetic NV1D2666 polypeptide
<220>
   <221> MOD_RES
   <222> (32)..(32)
   <223> -COOH
<400> 30
   Gly Pro Tyr Cys Gln Lys Trp Met Gln Thr Cys Asp Ser Glu Arg Lys
   1 5 10 15
   Cys Cys Glu Gly Phe Val Cys Arg Leu Trp Cys Lys Lys Lys Leu Trp
   20 25 30
<210> 31
   <211> 32
   <212> PRT
   <213> Artificial Sequence
<220>
   <223> Description of Artificial Sequence: Synthetic NV1D2816 polypeptide
<220>
   <221> MOD_RES
   <222> (32)..(32)
   <223> -COOH
<400> 31
   Gly Pro Ala Cys Gln Lys Trp Phe Gln Thr Cys Asp Ser Glu Arg Lys
   1               5                   10                  15
   Cys Cys Glu Gly Met Val Cys Arg Leu Trp Cys Lys Lys Lys Leu Trp
               20                  25                  30
<210> 32
   <211> 32
   <212> PRT
   <213> Artificial Sequence
<220>
   <223> Description of Artificial Sequence: Synthetic NV1D2735 polypeptide
<220>
   <221> MOD_RES
   <222> (32)..(32)
   <223> -COOH
<400> 32
   Gly Pro Ala Cys Gln Lys Trp Met Gln Thr Cys Asp Ser Glu Arg Lys
   1               5                   10                  15
   Cys Cys Glu Gly Phe Val Cys Arg Leu Trp Cys Lys Lys Lys Leu Trp
               20                  25                  30
<210> 33
   <211> 32
   <212> PRT
   <213> Artificial Sequence
<220>
   <223> Description of Artificial Sequence: Synthetic NV1D2739 polypeptide
<220>
   <221> MOD_RES
   <222> (32)..(32)
   <223> -COOH
<400> 33
   Gly Pro Ala Cys Gln Lys Trp Met Trp Thr Cys Asp Ala Glu Arg Lys
   1               5                   10                  15
   Cys Cys Glu Gly Phe Val Cys Arg Leu Trp Cys Lys Lys Lys Leu Trp
               20                  25                  30
<210> 34
   <211> 32
   <212> PRT
   <213> Artificial Sequence
<220>
   <223> Description of Artificial Sequence: Synthetic NV1D2731 polypeptide
<220>
   <221> MOD_RES
   <222> (32)..(32)
   <223> -COOH
<400> 34
   Gly Pro Ala Cys Gln Lys Trp Met Gln Thr Cys Asp Ala Glu Arg Lys
   1               5                   10                  15
   Cys Cys Glu Gly Met Val Cys Arg Leu Trp Cys Lys Lys Lys Leu Trp
               20                  25                  30
<210> 35
   <211> 32
   <212> PRT
   <213> Artificial Sequence
<220>
   <223> Description of Artificial Sequence: Synthetic NV1D2810 polypeptide
<220>
   <221> MOD_RES
   <222> (32)..(32)
   <223> -COOH
<400> 35
   Gly Pro Gln Cys Gln Lys Trp Phe Gln Thr Cys Asp Ser Glu Arg Lys
   1               5                   10                  15
   Cys Cys Glu Gly Met Val Cys Arg Leu Trp Cys Lys Lys Lys Leu Trp
               20                  25                  30
<210> 36
   <211> 32
   <212> PRT
   <213> Artificial Sequence
<220>
   <223> Description of Artificial Sequence: Synthetic NV1D2732 polypeptide
<220>
   <221> MOD_RES
   <222> (32)..(32)
   <223> -COOH
<400> 36
   Gly Pro Gln Cys Gln Lys Trp Met Gln Thr Cys Asp Ala Glu Arg Lys
   1               5                   10                  15
   Cys Cys Glu Gly Met Val Cys Arg Leu Trp Cys Lys Lys Lys Leu Trp
               20                  25                  30
<210> 37
   <211> 32
   <212> PRT
   <213> Artificial Sequence
<220>
   <223> Description of Artificial Sequence: Synthetic NV1D2740 polypeptide
<220>
   <221> MOD_RES
   <222> (32)..(32)
   <223> -COOH
<400> 37
   Gly Pro Gln Cys Gln Lys Trp Met Trp Thr Cys Asp Ala Glu Arg Lys
   1               5                   10                  15
   Cys Cys Glu Gly Phe Val Cys Arg Leu Trp Cys Lys Lys Lys Leu Trp
               20                  25                  30
<210> 38
   <211> 32
   <212> PRT
   <213> Artificial Sequence
<220>
   <223> Description of Artificial Sequence: Synthetic NV1D2819 polypeptide
<220>
   <221> MOD_RES
   <222> (32)..(32)
   <223> -COOH
<400> 38
   Gly Pro Arg Cys Gln Lys Trp Phe Trp Thr Cys Asp Ala Glu Arg Lys
   1               5                   10                  15
   Cys Cys Glu Gly Met Val Cys Arg Leu Trp Cys Lys Lys Lys Leu Trp
               20                  25                  30
<210> 39
   <211> 32
   <212> PRT
   <213> Artificial Sequence
<220>
   <223> Description of Artificial Sequence: Synthetic NV1D2729 polypeptide
<220>
   <221> MOD_RES
   <222> (32)..(32)
   <223> -COOH
<400> 39
   Gly Pro Arg Cys Gln Lys Trp Met Gln Thr Cys Asp Ala Glu Arg Lys
   1               5                   10                  15
   Cys Cys Glu Gly Met Val Cys Arg Leu Trp Cys Lys Lys Lys Leu Trp
               20                  25                  30
<210> 40
   <211> 32
   <212> PRT
   <213> Artificial Sequence
<220>
   <223> Description of Artificial Sequence: Synthetic NV1D2733 polypeptide
<220>
   <221> MOD_RES
   <222> (32)..(32)
   <223> -COOH
<400> 40
   Gly Pro Arg Cys Gln Lys Trp Met Gln Thr Cys Asp Ser Glu Arg Lys
   1               5                   10                  15
   Cys Cys Glu Gly Phe Val Cys Arg Leu Trp Cys Lys Lys Lys Leu Trp
               20                  25                  30
<210> 41
   <211> 32
   <212> PRT
   <213> Artificial Sequence
<220>
   <223> Description of Artificial Sequence: Synthetic NV1D2814 polypeptide
<220>
   <221> MOD_RES
   <222> (32)..(32)
   <223> -COOH
<400> 41
   Gly Pro Ser Cys Gln Lys Trp Phe Gln Thr Cys Asp Ser Glu Arg Lys
   1 5 10 15
   Cys Cys Glu Gly Met Val Cys Arg Leu Trp Cys Lys Lys Lys Leu Trp
   20 25 30
<210> 42
   <211> 32
   <212> PRT
   <213> Artificial Sequence
<220>
   <223> Description of Artificial Sequence: Synthetic NV1D2820 polypeptide
<220>
   <221> MOD_RES
   <222> (32)..(32)
   <223> -COOH
<400> 42
   Gly Pro Ser Cys Gln Lys Trp Phe Trp Thr Cys Asp Ala Glu Arg Lys
   1 5 10 15
   Cys Cys Glu Gly Met Val Cys Arg Leu Trp Cys Lys Lys Lys Leu Trp
   20 25 30
<210> 43
   <211> 32
   <212> PRT
   <213> Artificial Sequence
<220>
   <223> Description of Artificial Sequence: Synthetic NV1D2730 polypeptide
<220>
   <221> MOD_RES
   <222> (32)..(32)
   <223> -COOH
<400> 43
   Gly Pro Ser Cys Gln Lys Trp Met Gln Thr Cys Asp Ala Glu Arg Lys
   1 5 10 15
   Cys Cys Glu Gly Met Val Cys Arg Leu Trp Cys Lys Lys Lys Leu Trp
   20 25 30
<210> 44
   <211> 32
   <212> PRT
   <213> Artificial Sequence
<220>
   <223> Description of Artificial Sequence: Synthetic NV1D2734 polypeptide
<220>
   <221> MOD_RES
   <222> (32)..(32)
   <223> -COOH
<400> 44
   Gly Pro Ser Cys Gln Lys Trp Met Gln Thr Cys Asp Ser Glu Arg Lys
   1               5                   10                  15
   Cys Cys Glu Gly Phe Val Cys Arg Leu Trp Cys Lys Lys Lys Leu Trp
               20                  25                  30
<210> 45
   <211> 32
   <212> PRT
   <213> Artificial Sequence
<220>
   <223> Description of Artificial Sequence: Synthetic NV1D2738 polypeptide
<220>
   <221> MOD_RES
   <222> (32)..(32)
   <223> -COOH
<400> 45
   Gly Pro Ser Cys Gln Lys Trp Met Trp Thr Cys Asp Ala Glu Arg Lys
   1               5                   10                  15
   Cys Cys Glu Gly Phe Val Cys Arg Leu Trp Cys Lys Lys Lys Leu Trp
               20                  25                  30
<210> 46
   <211> 32
   <212> PRT
   <213> Artificial Sequence
<220>
   <223> Description of Artificial Sequence: Synthetic NV1D2851 polypeptide
<220>
   <221> MOD_RES
   <222> (32)..(32)
   <223> -COOH
<400> 46
   Gly Pro Tyr Cys Gln Lys Trp Phe Lys Thr Cys Asp Ala Glu Arg Lys
   1               5                   10                  15
   Cys Cys Glu Gly Met Val Cys Arg Leu Trp Cys Lys Lys Lys Leu Trp
               20                  25                  30
<210> 47
   <211> 32
   <212> PRT
   <213> Artificial Sequence
<220>
   <223> Description of Artificial Sequence: Synthetic NV1D2850 polypeptide
<220>
   <221> MOD_RES
   <222> (32)..(32)
   <223> -COOH
<400> 47
   Gly Pro Tyr Cys Gln Lys Trp Phe Gln Thr Cys Asp Ala Glu Arg Lys
   1               5                   10                  15
   Cys Cys Glu Gly Met Val Cys Arg Leu Trp Cys Lys Lys Lys Leu Trp
               20                  25                  30
<210> 48
   <211> 32
   <212> PRT
   <213> Artificial Sequence
<220>
   <223> Description of Artificial Sequence: Synthetic NV1D2667 polypeptide
<220>
   <221> MOD_RES
   <222> (32)..(32)
   <223> -COOH
<400> 48
   Gly Pro Tyr Cys Gln Lys Trp Met Trp Thr Cys Asp Ala Glu Arg Lys
   1               5                   10                  15
   Cys Cys Glu Gly Phe Val Cys Arg Leu Trp Cys Lys Lys Lys Leu Trp
               20                  25                  30
<210> 49
   <211> 32
   <212> PRT
   <213> Artificial Sequence
<220>
   <223> Description of Artificial Sequence: Synthetic NV1D2867 polypeptide
<220>
   <221> MOD_RES
   <222> (32)..(32)
   <223> -COOH
<400> 49
   Gly Pro Ala Cys Gln Lys Trp Phe Gln Thr Cys Asp Ala Glu Arg Lys
   1               5                   10                  15
   Cys Cys Glu Gly Met Val Cys Arg Leu Trp Cys Lys Lys Lys Leu Trp
               20                  25                  30
<210> 50
   <211> 32
   <212> PRT
   <213> Artificial Sequence
<220>
   <223> Description of Artificial Sequence: Synthetic NV1D2881 polypeptide
<220>
   <221> MOD_RES
   <222> (32)..(32)
   <223> -COOH
<400> 50
   Gly Pro Ala Cys Gln Lys Trp Phe Gln Thr Cys Asp Ser Glu Arg Lys
   1               5                   10                  15
   Cys Cys Glu Gly Phe Val Cys Arg Leu Trp Cys Lys Lys Lys Leu Trp
               20                  25                  30
<210> 51
   <211> 32
   <212> PRT
   <213> Artificial Sequence
<220>
   <223> Description of Artificial Sequence: Synthetic NV1D2882 polypeptide
<220>
   <221> MOD_RES
   <222> (32)..(32)
   <223> -COOH
<400> 51
   Gly Pro Ala Cys Gln Lys Trp Phe Gln Thr Cys Asp Ser Glu Arg Lys
   1               5                   10                  15
   Cys Cys Glu Gly Leu Val Cys Arg Leu Trp Cys Lys Lys Lys Leu Trp
               20                  25                  30
<210> 52
   <211> 32
   <212> PRT
   <213> Artificial Sequence
<220>
   <223> Description of Artificial Sequence: Synthetic NV1D2774 polypeptide
<220>
   <221> MOD_RES
   <222> (32)..(32)
   <223> -COOH
<400> 52
   Gly Pro Ala Cys Gln Lys Trp Met Gln Thr Cys Asp Ala Glu Arg Lys
   1               5                   10                  15
   Cys Cys Glu Gly Phe Val Cys Arg Leu Trp Cys Lys Lys Lys Leu Trp
               20                  25                  30
<210> 53
   <211> 32
   <212> PRT
   <213> Artificial Sequence
<220>
   <223> Description of Artificial Sequence: Synthetic NV1D2902 polypeptide
<220>
   <221> MOD_RES
   <222> (32)..(32)
   <223> -COOH
<400> 53
   Gly Pro Ala Cys Gln Lys Trp Met Gln Thr Cys Asp Ala Glu Arg Lys
   1               5                   10                  15
   Cys Cys Glu Gly Leu Val Cys Arg Leu Trp Cys Lys Lys Lys Leu Trp
               20                  25                  30
<210> 54
   <211> 32
   <212> PRT
   <213> Artificial Sequence
<220>
   <223> Description of Artificial Sequence: Synthetic NV1D2861 polypeptide
<220>
   <221> MOD_RES
   <222> (32)..(32)
   <223> -COOH
<400> 54
   Gly Pro Gln Cys Gln Lys Trp Phe Gln Thr Cys Asp Ala Glu Arg Lys
   1               5                   10                  15
   Cys Cys Glu Gly Met Val Cys Arg Leu Trp Cys Lys Lys Lys Leu Trp
               20                  25                  30
<210> 55
   <211> 32
   <212> PRT
   <213> Artificial Sequence
<220>
   <223> Description of Artificial Sequence: Synthetic NV1D2870 polypeptide
<220>
   <221> MOD_RES
   <222> (32)..(32)
   <223> -COOH
<400> 55
   Gly Pro Gln Cys Gln Lys Trp Phe Gln Thr Cys Asp Ser Glu Arg Lys
   1               5                   10                  15
   Cys Cys Glu Gly Leu Val Cys Arg Leu Trp Cys Lys Lys Lys Leu Trp
               20                  25                  30
<210> 56
   <211> 32
   <212> PRT
   <213> Artificial Sequence
<220>
   <223> Description of Artificial Sequence: Synthetic NV1D2775 polypeptide
<220>
   <221> MOD_RES
   <222> (32)..(32)
   <223> -COOH
<400> 56
   Gly Pro Gln Cys Gln Lys Trp Met Gln Thr Cys Asp Ala Glu Arg Lys
   1               5                   10                  15
   Cys Cys Glu Gly Phe Val Cys Arg Leu Trp Cys Lys Lys Lys Leu Trp
               20                  25                  30
<210> 57
   <211> 32
   <212> PRT
   <213> Artificial Sequence
<220>
   <223> Description of Artificial Sequence: Synthetic NV1D2893 polypeptide
<220>
   <221> MOD_RES
   <222> (32)..(32)
   <223> -COOH
<400> 57
   Gly Pro Gln Cys Gln Lys Trp Met Gln Thr Cys Asp Ala Glu Arg Lys
   1 5 10 15
   Cys Cys Glu Gly Leu Val Cys Arg Leu Trp Cys Lys Lys Lys Leu Trp
   20 25 30
<210> 58
   <211> 32
   <212> PRT
   <213> Artificial Sequence
<220>
   <223> Description of Artificial Sequence: Synthetic NV1D2887 polypeptide
<220>
   <221> MOD_RES
   <222> (32)..(32)
   <223> -COOH
<400> 58
   Gly Pro Arg Cys Gln Lys Trp Phe Trp Thr Cys Asp Ala Glu Arg Lys
   1 5 10 15
   Cys Cys Glu Gly Phe Val Cys Arg Leu Trp Cys Lys Lys Lys Leu Trp
   20 25 30
<210> 59
   <211> 32
   <212> PRT
   <213> Artificial Sequence
<220>
   <223> Description of Artificial Sequence: Synthetic NV1D2772 polypeptide
<220>
   <221> MOD_RES
   <222> (32)..(32)
   <223> -COOH
<400> 59
   Gly Pro Arg Cys Gln Lys Trp Met Gln Thr Cys Asp Ala Glu Arg Lys
   1 5 10 15
   Cys Cys Glu Gly Phe Val Cys Arg Leu Trp Cys Lys Lys Lys Leu Trp
   20 25 30
<210> 60
   <211> 32
   <212> PRT
   <213> Artificial Sequence
<220>
   <223> Description of Artificial Sequence: Synthetic NV1D2896 polypeptide
<220>
   <221> MOD_RES
   <222> (32)..(32)
   <223> -COOH
<400> 60
   Gly Pro Arg Cys Gln Lys Trp Met Gln Thr Cys Asp Ala Glu Arg Lys
   1               5                   10                  15
   Cys Cys Glu Gly Leu Val Cys Arg Leu Trp Cys Lys Lys Lys Leu Trp
               20                  25                  30
<210> 61
   <211> 32
   <212> PRT
   <213> Artificial Sequence
<220>
   <223> Description of Artificial Sequence: Synthetic NV1D2877 polypeptide
<220>
   <221> MOD_RES
   <222> (32)..(32)
   <223> -COOH
<400> 61
   Gly Pro Ser Cys Gln Lys Trp Phe Gln Thr Cys Asp Ser Glu Arg Lys
   1               5                   10                  15
   Cys Cys Glu Gly Phe Val Cys Arg Leu Trp Cys Lys Lys Lys Leu Trp
               20                  25                  30
<210> 62
   <211> 32
   <212> PRT
   <213> Artificial Sequence
<220>
   <223> Description of Artificial Sequence: Synthetic NV1D2878 polypeptide
<220>
   <221> MOD_RES
   <222> (32)..(32)
   <223> -COOH
<400> 62
   Gly Pro Ser Cys Gln Lys Trp Phe Gln Thr Cys Asp Ser Glu Arg Lys
   1               5                   10                  15
   Cys Cys Glu Gly Leu Val Cys Arg Leu Trp Cys Lys Lys Lys Leu Trp
               20                  25                  30
<210> 63
   <211> 32
   <212> PRT
   <213> Artificial Sequence
<220>
   <223> Description of Artificial Sequence: Synthetic NV1D2889 polypeptide
<220>
   <221> MOD_RES
   <222> (32)..(32)
   <223> -COOH
<400> 63
   Gly Pro Ser Cys Gln Lys Trp Phe Trp Thr Cys Asp Ala Glu Arg Lys
   1               5                   10                  15
   Cys Cys Glu Gly Phe Val Cys Arg Leu Trp Cys Lys Lys Lys Leu Trp
               20                  25                  30
<210> 64
   <211> 32
   <212> PRT
   <213> Artificial Sequence
<220>
   <223> Description of Artificial Sequence: Synthetic NV1D2889 polypeptide
<220>
   <221> MOD_RES
   <222> (32)..(32)
   <223> -COOH
<400> 64
   Gly Pro Ser Cys Gln Lys Trp Phe Trp Thr Cys Asp Ala Glu Arg Lys
   1               5                   10                  15
   Cys Cys Glu Gly Phe Val Cys Arg Leu Trp Cys Lys Lys Lys Leu Trp
               20                  25                  30
<210> 65
   <211> 32
   <212> PRT
   <213> Artificial Sequence
<220>
   <223> Description of Artificial Sequence: Synthetic NV1D2773 polypeptide
<220>
   <221> MOD_RES
   <222> (32)..(32)
   <223> -COOH
<400> 65
   Gly Pro Ser Cys Gln Lys Trp Met Gln Thr Cys Asp Ala Glu Arg Lys
   1 5 10 15
   Cys Cys Glu Gly Phe Val Cys Arg Leu Trp Cys Lys Lys Lys Leu Trp
   20 25 30
<210> 66
   <211> 32
   <212> PRT
   <213> Artificial Sequence
<220>
   <223> Description of Artificial Sequence: Synthetic NV1D2890 polypeptide
<220>
   <221> MOD_RES
   <222> (32)..(32)
   <223> -COOH
<400> 66
   Gly Pro Ser Cys Gln Lys Trp Phe Trp Thr Cys Asp Ala Glu Arg Lys
   1 5 10 15
   Cys Cys Glu Gly Leu Val Cys Arg Leu Trp Cys Lys Lys Lys Leu Trp
   20 25 30
<210> 67
   <211> 32
   <212> PRT
   <213> Artificial Sequence
<220>
   <223> Description of Artificial Sequence: Synthetic NV1D2899 polypeptide
<220>
   <221> MOD_RES
   <222> (32)..(32)
   <223> -COOH
<400> 67
   Gly Pro Ser Cys Gln Lys Trp Met Gln Thr Cys Asp Ala Glu Arg Lys
   1 5 10 15
   Cys Cys Glu Gly Leu Val Cys Arg Leu Trp Cys Lys Lys Lys Leu Trp
   20 25 30
<210> 68
   <211> 32
   <212> PRT
   <213> Artificial Sequence
<220>
   <223> Description of Artificial Sequence: Synthetic NV1D2905 polypeptide
<220>
   <221> MOD_RES
   <222> (32)..(32)
   <223> -COOH
<400> 68
   Gly Pro Tyr Cys Gln Lys Trp Phe Gln Thr Cys Asp Ala Glu Arg Lys
   1               5                   10                  15
   Cys Cys Glu Gly Phe Val Cys Arg Leu Trp Cys Lys Lys Lys Leu Trp
               20                  25                  30
<210> 69
   <211> 32
   <212> PRT
   <213> Artificial Sequence
<220>
   <223> Description of Artificial Sequence: Synthetic NV1D2906 polypeptide
<220>
   <221> MOD_RES
   <222> (32)..(32)
   <223> -COOH
<400> 69
   Gly Pro Tyr Cys Gln Lys Trp Phe Gln Thr Cys Asp Ala Glu Arg Lys
   1               5                   10                  15
   Cys Cys Glu Gly Leu Val Cys Arg Leu Trp Cys Lys Lys Lys Leu Trp
               20                  25                  30
<210> 70
   <211> 32
   <212> PRT
   <213> Artificial Sequence
<220>
   <223> Description of Artificial Sequence: Synthetic NV1D2921 polypeptide
<220>
   <221> MOD_RES
   <222> (32)..(32)
   <223> -COOH
<400> 70
   Gly Pro Ala Cys Gln Lys Trp Phe Gln Thr Cys Asp Ala Glu Arg Lys
   1               5                   10                  15
   Cys Cys Glu Gly Phe Val Cys Arg Leu Trp Cys Lys Lys Lys Leu Trp
               20                  25                  30
<210> 71
   <211> 32
   <212> PRT
   <213> Artificial Sequence
<220>
   <223> Description of Artificial Sequence: Synthetic NV1D2922 polypeptide
<220>
   <221> MOD_RES
   <222> (32)..(32)
   <223> -COOH
<400> 71
   Gly Pro Ala Cys Gln Lys Trp Phe Gln Thr Cys Asp Ala Glu Arg Lys
   1               5                   10                  15
   Cys Cys Glu Gly Leu Val Cys Arg Leu Trp Cys Lys Lys Lys Leu Trp
               20                  25                  30
<210> 72
   <211> 32
   <212> PRT
   <213> Artificial Sequence
<220>
   <223> Description of Artificial Sequence: Synthetic NV1D2909 polypeptide
<220>
   <221> MOD_RES
   <222> (32)..(32)
   <223> -COOH
<400> 72
   Gly Pro Gln Cys Gln Lys Trp Phe Gln Thr Cys Asp Ala Glu Arg Lys
   1               5                   10                  15
   Cys Cys Glu Gly Phe Val Cys Arg Leu Trp Cys Lys Lys Lys Leu Trp
               20                  25                  30
<210> 73
   <211> 32
   <212> PRT
   <213> Artificial Sequence
<220>
   <223> Description of Artificial Sequence: Synthetic NV1D2910 polypeptide
<220>
   <221> MOD_RES
   <222> (32)..(32)
   <223> -COOH
<400> 73
   Gly Pro Gln Cys Gln Lys Trp Phe Gln Thr Cys Asp Ala Glu Arg Lys
   1 5 10 15
   Cys Cys Glu Gly Leu Val Cys Arg Leu Trp Cys Lys Lys Lys Leu Trp
   20 25 30
<210> 74
   <211> 32
   <212> PRT
   <213> Artificial Sequence
<220>
   <223> Description of Artificial Sequence: Synthetic NV1D2913 polypeptide
<220>
   <221> MOD_RES
   <222> (32)..(32)
   <223> -COOH
<400> 74
   Gly Pro Arg Cys Gln Lys Trp Phe Gln Thr Cys Asp Ala Glu Arg Lys
   1 5 10 15
   Cys Cys Glu Gly Phe Val Cys Arg Leu Trp Cys Lys Lys Lys Leu Trp
   20 25 30
<210> 75
   <211> 32
   <212> PRT
   <213> Artificial Sequence
<220>
   <223> Description of Artificial Sequence: Synthetic NV1D2914 polypeptide
<220>
   <221> MOD_RES
   <222> (32)..(32)
   <223> -COOH
<400> 75
   Gly Pro Arg Cys Gln Lys Trp Phe Gln Thr Cys Asp Ala Glu Arg Lys
   1 5 10 15
   Cys Cys Glu Gly Leu Val Cys Arg Leu Trp Cys Lys Lys Lys Leu Trp
   20 25 30
<210> 76
   <211> 32
   <212> PRT
   <213> Artificial Sequence
<220>
   <223> Description of Artificial Sequence: Synthetic NV1D2917 polypeptide
<220>
   <221> MOD_RES
   <222> (32)..(32)
   <223> -COOH
<400> 76
   Gly Pro Ser Cys Gln Lys Trp Phe Gln Thr Cys Asp Ala Glu Arg Lys
   1               5                   10                  15
   Cys Cys Glu Gly Phe Val Cys Arg Leu Trp Cys Lys Lys Lys Leu Trp
               20                  25                  30
<210> 77
   <211> 32
   <212> PRT
   <213> Artificial Sequence
<220>
   <223> Description of Artificial Sequence: Synthetic NV1D2918 polypeptide
<220>
   <221> MOD_RES
   <222> (32)..(32)
   <223> -COOH
<400> 77
   Gly Pro Ser Cys Gln Lys Trp Phe Gln Thr Cys Asp Ala Glu Arg Lys
   1               5                   10                  15
   Cys Cys Glu Gly Leu Val Cys Arg Leu Trp Cys Lys Lys Lys Leu Trp
               20                  25                  30
<210> 78
   <211> 32
   <212> PRT
   <213> Artificial Sequence
<220>
   <223> Description of Artificial Sequence: Synthetic NV1D3034 polypeptide
<220>
   <221> MOD_RES
   <222> (32)..(32)
   <223> -COOH
<400> 78
   Gly Pro Gln Cys Gln Lys Trp Met Gln Thr Cys Asp Arg Glu Arg Lys
   1 5 10 15
   Cys Cys Glu Gly Phe Val Cys Thr Leu Trp Cys Arg Lys Lys Leu Trp
   20 25 30
<210> 79
   <211> 1977
   <212> PRT
   <213> Homo Sapiens
<400> 79
   Met Ala Met Leu Pro Pro Pro Gly Pro Gln Ser Phe Val His Phe Thr
   1               5                   10                  15
   Lys Gln Ser Leu Ala Leu Ile Glu Gln Arg Ile Ala Glu Arg Lys Ser
               20                  25                  30
   Lys Glu Pro Lys Glu Glu Lys Lys Asp Asp Asp Glu Glu Ala Pro Lys
           35                  40                  45
   Pro Ser Ser Asp Leu Glu Ala Gly Lys Gln Leu Pro Phe Ile Tyr Gly
       50                  55                  60
   Asp Ile Pro Pro Gly Met Val Ser Glu Pro Leu Glu Asp Leu Asp Pro
   65                  70                  75                  80
   Tyr Tyr Ala Asp Lys Lys Thr Phe Ile Val Leu Asn Lys Gly Lys Thr
                   85                  90                  95
   Ile Phe Arg Phe Asn Ala Thr Pro Ala Leu Tyr Met Leu Ser Pro Phe
               100                 105                 110
   Ser Pro Leu Arg Arg Ile Ser Ile Lys Ile Leu Val His Ser Leu Phe
           115                 120                 125
   Ser Met Leu Ile Met Cys Thr Ile Leu Thr Asn Cys Ile Phe Met Thr
       130                 135                 140
   Met Asn Asn Pro Pro Asp Trp Thr Lys Asn Val Glu Tyr Thr Phe Thr
   145                 150                 155                 160
   Gly Ile Tyr Thr Phe Glu Ser Leu Val Lys Ile Leu Ala Arg Gly Phe
                   165                 170                 175
   Cys Val Gly Glu Phe Thr Phe Leu Arg Asp Pro Trp Asn Trp Leu Asp
               180                 185                 190
   Phe Val Val Ile Val Phe Ala Tyr Leu Thr Glu Phe Val Asn Leu Gly
               195                 200                 205
   Asn Val Ser Ala Leu Arg Thr Phe Arg Val Leu Arg Ala Leu Lys Thr
       210                 215                 220
   Ile Ser Val Ile Pro Gly Leu Lys Thr Ile Val Gly Ala Leu Ile Gln
   225                 230                 235                 240
   Ser Val Lys Lys Leu Ser Asp Val Met Ile Leu Thr Val Phe Cys Leu
                   245                 250                 255
   Ser Val Phe Ala Leu Ile Gly Leu Gln Leu Phe Met Gly Asn Leu Lys
               260                 265                 270
   His Lys Cys Phe Arg Asn Ser Leu Glu Asn Asn Glu Thr Leu Glu Ser
           275                 280                 285
   Ile Met Asn Thr Leu Glu Ser Glu Glu Asp Phe Arg Lys Tyr Phe Tyr
       290                 295                 300
   Tyr Leu Glu Gly Ser Lys Asp Ala Leu Leu Cys Gly Phe Ser Thr Asp
   305                 310                 315                 320
   Ser Gly Gln Cys Pro Glu Gly Tyr Thr Cys Val Lys Ile Gly Arg Asn
                   325                 330                 335
   Pro Asp Tyr Gly Tyr Thr Ser Phe Asp Thr Phe Ser Trp Ala Phe Leu
               340                 345                 350
   Ala Leu Phe Arg Leu Met Thr Gln Asp Tyr Trp Glu Asn Leu Tyr Gln
           355                 360                 365
   Gln Thr Leu Arg Ala Ala Gly Lys Thr Tyr Met Ile Phe Phe Val Val
       370                 375                 380
   Val Ile Phe Leu Gly Ser Phe Tyr Leu Ile Asn Leu Ile Leu Ala Val
   385                 390                 395                 400
   Val Ala Met Ala Tyr Glu Glu Gln Asn Gln Ala Asn Ile Glu Glu Ala
                   405                 410                 415
   Lys Gln Lys Glu Leu Glu Phe Gln Gln Met Leu Asp Arg Leu Lys Lys
               420                 425                 430
   Glu Gln Glu Glu Ala Glu Ala Ile Ala Ala Ala Ala Ala Glu Tyr Thr
           435                 440                 445
   Ser Ile Arg Arg Ser Arg Ile Met Gly Leu Ser Glu Ser Ser Ser Glu
       450                 455                 460
   Thr Ser Lys Leu Ser Ser Lys Ser Ala Lys Glu Arg Arg Asn Arg Arg
   465                 470                 475                 480
   Lys Lys Lys Asn Gln Lys Lys Leu Ser Ser Gly Glu Glu Lys Gly Asp
                   485                 490                 495
   Ala Glu Lys Leu Ser Lys Ser Glu Ser Glu Asp Ser Ile Arg Arg Lys
               500                 505                 510
   Ser Phe His Leu Gly Val Glu Gly His Arg Arg Ala His Glu Lys Arg
           515                 520                 525
   Leu Ser Thr Pro Asn Gln Ser Pro Leu Ser Ile Arg Gly Ser Leu Phe
       530                 535                 540
   Ser Ala Arg Arg Ser Ser Arg Thr Ser Leu Phe Ser Phe Lys Gly Arg
   545                 550                 555                 560
   Gly Arg Asp Ile Gly Ser Glu Thr Glu Phe Ala Asp Asp Glu His Ser
                   565                 570                 575
   Ile Phe Gly Asp Asn Glu Ser Arg Arg Gly Ser Leu Phe Val Pro His
               580                 585                 590
   Arg Pro Gln Glu Arg Arg Ser Ser Asn Ile Ser Gln Ala Ser Arg Ser
           595                 600                 605
   Pro Pro Met Leu Pro Val Asn Gly Lys Met His Ser Ala Val Asp Cys
       610                 615                 620
   Asn Gly Val Val Ser Leu Val Asp Gly Arg Ser Ala Leu Met Leu Pro
   625                 630                 635                 640
   Asn Gly Gln Leu Leu Pro Glu Gly Thr Thr Asn Gln Ile His Lys Lys
                   645                 650                 655
   Arg Arg Cys Ser Ser Tyr Leu Leu Ser Glu Asp Met Leu Asn Asp Pro
               660                 665                 670
   Asn Leu Arg Gln Arg Ala Met Ser Arg Ala Ser Ile Leu Thr Asn Thr
           675                 680                 685
   Val Glu Glu Leu Glu Glu Ser Arg Gln Lys Cys Pro Pro Trp Trp Tyr
       690                 695                 700
   Arg Phe Ala His Lys Phe Leu Ile Trp Asn Cys Ser Pro Tyr Trp Ile
   705                 710                 715                 720
   Lys Phe Lys Lys Cys Ile Tyr Phe Ile Val Met Asp Pro Phe Val Asp
                   725                 730                 735
   Leu Ala Ile Thr Ile Cys Ile Val Leu Asn Thr Leu Phe Met Ala Met
               740                 745                 750
   Glu His His Pro Met Thr Glu Glu Phe Lys Asn Val Leu Ala Ile Gly
           755                 760                 765
   Asn Leu Val Phe Thr Gly Ile Phe Ala Ala Glu Met Val Leu Lys Leu
       770                 775                 780
   Ile Ala Met Asp Pro Tyr Glu Tyr Phe Gln Val Gly Trp Asn Ile Phe
   785                 790                 795                 800
   Asp Ser Leu Ile Val Thr Leu Ser Leu Val Glu Leu Phe Leu Ala Asp
                   805                 810                 815
   Val Glu Gly Leu Ser Val Leu Arg Ser Phe Arg Leu Leu Arg Val Phe
               820                 825                 830
   Lys Leu Ala Lys Ser Trp Pro Thr Leu Asn Met Leu Ile Lys Ile Ile
           835                 840                 845
   Gly Asn Ser Val Gly Ala Leu Gly Asn Leu Thr Leu Val Leu Ala Ile
       850                 855                 860
   Ile Val Phe Ile Phe Ala Val Val Gly Met Gln Leu Phe Gly Lys Ser
   865                 870                 875                 880
   Tyr Lys Glu Cys Val Cys Lys Ile Asn Asp Asp Cys Thr Leu Pro Arg
                   885                 890                 895
   Trp His Met Asn Asp Phe Phe His Ser Phe Leu Ile Val Phe Arg Val
               900                 905                 910
   Leu Cys Gly Glu Trp Ile Glu Thr Met Trp Asp Cys Met Glu Val Ala
           915                 920                 925
   Gly Gln Ala Met Cys Leu Ile Val Tyr Met Met Val Met Val Ile Gly
       930                 935                 940
   Asn Leu Val Val Leu Asn Leu Phe Leu Ala Leu Leu Leu Ser Ser Phe
   945                 950                 955                 960
   Ser Ser Asp Asn Leu Thr Ala Ile Glu Glu Asp Pro Asp Ala Asn Asn
                   965                 970                 975
   Leu Gln Ile Ala Val Thr Arg Ile Lys Lys Gly Ile Asn Tyr Val Lys
               980                 985                 990
   Gln Thr Leu Arg Glu Phe Ile Leu  Lys Ala Phe Ser Lys  Lys Pro Lys
           995                 1000                 1005
   Ile Ser  Arg Glu Ile Arg Gln  Ala Glu Asp Leu Asn  Thr Lys Lys
       1010                 1015                 1020
   Glu Asn  Tyr Ile Ser Asn His  Thr Leu Ala Glu Met  Ser Lys Gly
       1025                 1030                 1035
   His Asn  Phe Leu Lys Glu Lys  Asp Lys Ile Ser Gly  Phe Gly Ser
       1040                 1045                 1050
   Ser Val  Asp Lys His Leu Met  Glu Asp Ser Asp Gly  Gln Ser Phe
       1055                 1060                 1065
   Ile His  Asn Pro Ser Leu Thr  Val Thr Val Pro Ile  Ala Pro Gly
       1070                 1075                 1080
   Glu Ser  Asp Leu Glu Asn Met  Asn Ala Glu Glu Leu  Ser Ser Asp
       1085                 1090                 1095
   Ser Asp  Ser Glu Tyr Ser Lys  Val Arg Leu Asn Arg  Ser Ser Ser
       1100                 1105                 1110
   Ser Glu  Cys Ser Thr Val Asp  Asn Pro Leu Pro Gly  Glu Gly Glu
       1115                 1120                 1125
   Glu Ala  Glu Ala Glu Pro Met  Asn Ser Asp Glu Pro  Glu Ala Cys
       1130                 1135                 1140
   Phe Thr  Asp Gly Cys Val Arg  Arg Phe Ser Cys Cys  Gln Val Asn
       1145                 1150                 1155
   Ile Glu  Ser Gly Lys Gly Lys  Ile Trp Trp Asn Ile  Arg Lys Thr
       1160                 1165                 1170
   Cys Tyr  Lys Ile Val Glu His  Ser Trp Phe Glu Ser  Phe Ile Val
       1175                 1180                 1185
   Leu Met  Ile Leu Leu Ser Ser  Gly Ala Leu Ala Phe  Glu Asp Ile
   1190 1195 1200
   Tyr Ile Glu Arg Lys Lys Thr Ile Lys Ile Ile Leu Glu Tyr Ala
   1205 1210 1215
   Asp Lys Ile Phe Thr Tyr Ile Phe Ile Leu Glu Met Leu Leu Lys
   1220 1225 1230
   Trp Ile Ala Tyr Gly Tyr Lys Thr Tyr Phe Thr Asn Ala Trp Cys
   1235 1240 1245
   Trp Leu Asp Phe Leu Ile Val Asp Val Ser Leu Val Thr Leu Val
   1250 1255 1260
   Ala Asn Thr Leu Gly Tyr Ser Asp Leu Gly Pro Ile Lys Ser Leu
   1265 1270 1275
   Arg Thr Leu Arg Ala Leu Arg Pro Leu Arg Ala Leu Ser Arg Phe
   1280 1285 1290
   Glu Gly Met Arg Val Val Val Asn Ala Leu Ile Gly Ala Ile Pro
   1295 1300 1305
   Ser Ile Met Asn Val Leu Leu Val Cys Leu Ile Phe Trp Leu Ile
   1310 1315 1320
   Phe Ser Ile Met Gly Val Asn Leu Phe Ala Gly Lys Phe Tyr Glu
   1325 1330 1335
   Cys Ile Asn Thr Thr Asp Gly Ser Arg Phe Pro Ala Ser Gln Val
   1340 1345 1350
   Pro Asn Arg Ser Glu Cys Phe Ala Leu Met Asn Val Ser Gln Asn
   1355 1360 1365
   Val Arg Trp Lys Asn Leu Lys Val Asn Phe Asp Asn Val Gly Leu
   1370 1375 1380
   Gly Tyr Leu Ser Leu Leu Gln Val Ala Thr Phe Lys Gly Trp Thr
   1385 1390 1395
   Ile Ile Met Tyr Ala Ala Val Asp Ser Val Asn Val Asp Lys Gln
   1400 1405 1410
   Pro Lys Tyr Glu Tyr Ser Leu Tyr Met Tyr Ile Tyr Phe Val Val
   1415 1420 1425
   Phe Ile  Ile Phe Gly Ser Phe  Phe Thr Leu Asn Leu  Phe Ile Gly
       1430                 1435                 1440
   Val Ile  Ile Asp Asn Phe Asn  Gln Gln Lys Lys Lys  Leu Gly Gly
       1445                 1450                 1455
   Gln Asp  Ile Phe Met Thr Glu  Glu Gln Lys Lys Tyr  Tyr Asn Ala
       1460                 1465                 1470
   Met Lys  Lys Leu Gly Ser Lys  Lys Pro Gln Lys Pro  Ile Pro Arg
       1475                 1480                 1485
   Pro Gly  Asn Lys Ile Gln Gly  Cys Ile Phe Asp Leu  Val Thr Asn
       1490                 1495                 1500
   Gln Ala  Phe Asp Ile Ser Ile  Met Val Leu Ile Cys  Leu Asn Met
       1505                 1510                 1515
   Val Thr  Met Met Val Glu Lys  Glu Gly Gln Ser Gln  His Met Thr
       1520                 1525                 1530
   Glu Val  Leu Tyr Trp Ile Asn  Val Val Phe Ile Ile  Leu Phe Thr
       1535                 1540                 1545
   Gly Glu  Cys Val Leu Lys Leu  Ile Ser Leu Arg His  Tyr Tyr Phe
       1550                 1555                 1560
   Thr Val  Gly Trp Asn Ile Phe  Asp Phe Val Val Val  Ile Ile Ser
       1565                 1570                 1575
   Ile Val  Gly Met Phe Leu Ala  Asp Leu Ile Glu Thr  Tyr Phe Val
       1580                 1585                 1590
   Ser Pro  Thr Leu Phe Arg Val  Ile Arg Leu Ala Arg  Ile Gly Arg
       1595                 1600                 1605
   Ile Leu  Arg Leu Val Lys Gly  Ala Lys Gly Ile Arg  Thr Leu Leu
       1610                 1615                 1620
   Phe Ala  Leu Met Met Ser Leu  Pro Ala Leu Phe Asn  Ile Gly Leu
       1625                 1630                 1635
   Leu Leu  Phe Leu Val Met Phe  Ile Tyr Ala Ile Phe  Gly Met Ser
       1640                 1645                 1650
   Asn Phe  Ala Tyr Val Lys Lys  Glu Asp Gly Ile Asn  Asp Met Phe
       1655                 1660                 1665
   Asn Phe  Glu Thr Phe Gly Asn  Ser Met Ile Cys Leu  Phe Gln Ile
       1670                 1675                 1680
   Thr Thr  Ser Ala Gly Trp Asp  Gly Leu Leu Ala Pro  Ile Leu Asn
       1685                 1690                 1695
   Ser Lys  Pro Pro Asp Cys Asp  Pro Lys Lys Val His  Pro Gly Ser
       1700                 1705                 1710
   Ser Val  Glu Gly Asp Cys Gly  Asn Pro Ser Val Gly  Ile Phe Tyr
       1715                 1720                 1725
   Phe Val  Ser Tyr Ile Ile Ile  Ser Phe Leu Val Val  Val Asn Met
       1730                 1735                 1740
   Tyr Ile  Ala Val Ile Leu Glu  Asn Phe Ser Val Ala  Thr Glu Glu
       1745                 1750                 1755
   Ser Thr  Glu Pro Leu Ser Glu  Asp Asp Phe Glu Met  Phe Tyr Glu
       1760                 1765                 1770
   Val Trp  Glu Lys Phe Asp Pro  Asp Ala Thr Gln Phe  Ile Glu Phe
       1775                 1780                 1785
   Ser Lys  Leu Ser Asp Phe Ala  Ala Ala Leu Asp Pro  Pro Leu Leu
       1790                 1795                 1800
   Ile Ala  Lys Pro Asn Lys Val  Gln Leu Ile Ala Met  Asp Leu Pro
       1805                 1810                 1815
   Met Val  Ser Gly Asp Arg Ile  His Cys Leu Asp Ile  Leu Phe Ala
       1820                 1825                 1830
   Phe Thr  Lys Arg Val Leu Gly  Glu Ser Gly Glu Met  Asp Ser Leu
       1835                 1840                 1845
   Arg Ser  Gln Met Glu Glu Arg  Phe Met Ser Ala Asn  Pro Ser Lys
       1850                 1855                 1860
   Val Ser  Tyr Glu Pro Ile Thr  Thr Thr Leu Lys Arg  Lys Gln Glu
       1865                 1870                 1875
   Asp Val  Ser Ala Thr Val Ile  Gln Arg Ala Tyr Arg  Arg Tyr Arg
       1880                 1885                 1890
   Leu Arg  Gln Asn Val Lys Asn  Ile Ser Ser Ile Tyr  Ile Lys Asp
       1895                 1900                 1905
   Gly Asp  Arg Asp Asp Asp Leu  Leu Asn Lys Lys Asp  Met Ala Phe
       1910                 1915                 1920
   Asp Asn  Val Asn Glu Asn Ser  Ser Pro Glu Lys Thr  Asp Ala Thr
       1925                 1930                 1935
   Ser Ser  Thr Thr Ser Pro Pro  Ser Tyr Asp Ser Val  Thr Lys Pro
       1940                 1945                 1950
   Asp Lys  Glu Lys Tyr Glu Gln  Asp Arg Thr Glu Lys  Glu Asp Lys
       1955                 1960                 1965
   Gly Lys  Asp Ser Lys Glu Ser  Lys Lys
       1970                 1975
<210> 80
   <211> 20
   <212> PRT
   <213> Artificial Sequence
<220>
   <223> Description of Artificial Sequence: Synthetic Linker peptide
<400> 80
   Gly Gly Gly Gly Ser Gly Gly Gly Gly Ser Gly Gly Gly Gly Ser Gly
   1               5                   10                  15
   Gly Gly Gly Ser
               20
<210> 81
   <211> 30
   <212> PRT
   <213> Artificial Sequence
<220>
   <223> Description of Artificial Sequence: Synthetic Linker polypeptide
<400> 81
   Gly Gly Gly Gly Ser Gly Gly Gly Gly Ser Gly Gly Gly Gly Ser Gly
   1               5                   10                  15
   Gly Gly Gly Ser Gly Gly Gly Gly Ser Gly Gly Gly Gly Ser
               20                  25                  30
<210> 82
   <211> 8
   <212> PRT
   <213> Artificial Sequence
<220>
   <223> Description of Artificial Sequence: Synthetic HRV3C cleavage site peptide
<400> 82
   Leu Glu Val Leu Phe Gln Gly Pro
   1               5
<210> 83
   <211> 672
   <212> PRT
   <213> Artificial Sequence
<220>
   <223> Description of Artificial Sequence: Synthetic NV1G1007 (NV1D2775) Y1Q, W7Q, S11A, M19F polypeptide
<400> 83
   Met Ala Trp Val Trp Thr Leu Leu Phe Leu Met Ala Ala Ala Gln Ser
   1               5                   10                  15
   Ile Gln Ala Gly Ser His His His His His His Asp Ala His Lys Ser
               20                  25                  30
   Glu Val Ala His Arg Phe Lys Asp Leu Gly Glu Glu Asn Phe Lys Ala
           35                  40                  45
   Leu Val Leu Ile Ala Phe Ala Gln Tyr Leu Gln Gln Cys Pro Phe Glu
       50                  55                  60
   Asp His Val Lys Leu Val Asn Glu Val Thr Glu Phe Ala Lys Thr Cys
   65                  70                  75                  80
   Val Ala Asp Glu Ser Ala Glu Asn Cys Asp Lys Ser Leu His Thr Leu
                   85                  90                  95
   Phe Gly Asp Lys Leu Cys Thr Val Ala Thr Leu Arg Glu Thr Tyr Gly
               100                 105                 110
   Glu Met Ala Asp Cys Cys Ala Lys Gln Glu Pro Glu Arg Asn Glu Cys
           115                 120                 125
   Phe Leu Gln His Lys Asp Asp Asn Pro Asn Leu Pro Arg Leu Val Arg
       130                 135                 140
   Pro Glu Val Asp Val Met Cys Thr Ala Phe His Asp Asn Glu Glu Thr
   145                 150                 155                 160
   Phe Leu Lys Lys Tyr Leu Tyr Glu Ile Ala Arg Arg His Pro Tyr Phe
                   165                 170                 175
   Tyr Ala Pro Glu Leu Leu Phe Phe Ala Lys Arg Tyr Lys Ala Ala Phe
               180                 185                 190
   Thr Glu Cys Cys Gln Ala Ala Asp Lys Ala Ala Cys Leu Leu Pro Lys
           195                 200                 205
   Leu Asp Glu Leu Arg Asp Glu Gly Lys Ala Ser Ser Ala Lys Gln Arg
       210                 215                 220
   Leu Lys Cys Ala Ser Leu Gln Lys Phe Gly Glu Arg Ala Phe Lys Ala
   225                 230                 235                     240
   Trp Ala Val Ala Arg Leu Ser Gln Arg Phe Pro Lys Ala Glu Phe Ala
                   245                 250                 255
   Glu Val Ser Lys Leu Val Thr Asp Leu Thr Lys Val His Thr Glu Cys
               260                 265                 270
   Cys His Gly Asp Leu Leu Glu Cys Ala Asp Asp Arg Ala Asp Leu Ala
           275                 280                 285
   Lys Tyr Ile Cys Glu Asn Gln Asp Ser Ile Ser Ser Lys Leu Lys Glu
       290                 295                 300
   Cys Cys Glu Lys Pro Leu Leu Glu Lys Ser His Cys Ile Ala Glu Val
   305                 310                 315                     320
   Glu Asn Asp Glu Met Pro Ala Asp Leu Pro Ser Leu Ala Ala Asp Phe
                   325                 330                 335
   Val Glu Ser Lys Asp Val Cys Lys Asn Tyr Ala Glu Ala Lys Asp Val
               340                 345                 350
   Phe Leu Gly Met Phe Leu Tyr Glu Tyr Ala Arg Arg His Pro Asp Tyr
           355                 360                 365
   Ser Val Val Leu Leu Leu Arg Leu Ala Lys Thr Tyr Glu Thr Thr Leu
       370                 375                 380
   Glu Lys Cys Cys Ala Ala Ala Asp Pro His Glu Cys Tyr Ala Lys Val
   385                 390                 395                     400
   Phe Asp Glu Phe Lys Pro Leu Val Glu Glu Pro Gln Asn Leu Ile Lys
                   405                 410                 415
   Gln Asn Cys Glu Leu Phe Glu Gln Leu Gly Glu Tyr Lys Phe Gln Asn
   420 425 430
   Ala Leu Leu Val Arg Tyr Thr Lys Lys Val Pro Gln Val Ser Thr Pro
   435 440 445
   Thr Leu Val Glu Val Ser Arg Asn Leu Gly Lys Val Gly Ser Lys Cys
   450 455 460
   Cys Lys His Pro Glu Ala Lys Arg Met Pro Cys Ala Glu Asp Tyr Leu
   465 470 475 480
   Ser Val Val Leu Asn Gln Leu Cys Val Leu His Glu Lys Thr Pro Val
   485 490 495
   Ser Asp Arg Val Thr Lys Cys Cys Thr Glu Ser Leu Val Asn Arg Arg
   500 505 510
   Pro Cys Phe Ser Ala Leu Glu Val Asp Glu Thr Tyr Val Pro Lys Glu
   515 520 525
   Phe Asn Ala Glu Thr Phe Thr Phe His Ala Asp Ile Cys Thr Leu Ser
   530 535 540
   Glu Lys Glu Arg Gln Ile Lys Lys Gln Thr Ala Leu Val Glu Leu Val
   545 550 555 560
   Lys His Lys Pro Lys Ala Thr Lys Glu Gln Leu Lys Ala Val Met Asp
   565 570 575
   Asp Phe Ala Ala Phe Val Glu Lys Cys Cys Lys Ala Asp Asp Lys Glu
   580 585 590
   Thr Cys Phe Ala Glu Glu Gly Lys Lys Leu Val Ala Ala Ser Gln Ala
   595 600 605
   Ala Leu Gly Leu Gly Ser Gly Gly Gly Gly Ser Gly Gly Gly Gly Ser
   610 615 620
   Gly Gly Gly Gly Ser Gly Gly Gly Gly Ser Leu Glu Val Leu Phe Gln
   625 630 635 640
   Gly Pro Gln Cys Gln Lys Trp Met Gln Thr Cys Asp Ala Glu Arg Lys
   645 650 655
   Cys Cys Glu Gly Phe Val Cys Arg Leu Trp Cys Lys Lys Lys Leu Trp
   660 665 670
<210> 84
   <211> 2019
   <212> DNA
   <213> Artificial Sequence
<220>
   <223> Description of Artificial Sequence: Synthetic NV1G1007 (NV1D2775) Y1Q, W7Q, S11A, M19F polynucleotide
<400> 84
   atggcttggg tgtggacctt gctattcctg atggcggccg cccaaagtat acaggccggg 60
   agccaccacc accaccacca cgacgcccac aagagcgagg tggcccaccg gttcaaggac 120
   ctgggcgagg agaacttcaa ggccctggtg ctgatcgcct tcgcccagta cctgcagcag 180
   tgccccttcg aggaccacgt gaagctggtg aacgaggtga ccgagttcgc caagacctgc 240
   gtggccgacg agagcgccga gaactgcgac aagagcctgc acaccctgtt cggcgacaag 300
   ctgtgcaccg tggccaccct gcgggagacc tacggcgaga tggccgactg ctgcgccaag 360
   caggagcccg agcggaacga gtgcttcctg cagcacaagg acgacaaccc caacctgccc 420
   cggctggtgc ggcccgaggt ggacgtgatg tgcaccgcct tccacgacaa cgaggagacc 480
   ttcctgaaga agtacctgta cgagatcgcc cggcggcacc cctacttcta cgcccccgag 540
   ctgctgttct tcgccaagcg gtacaaggcc gccttcaccg agtgctgcca ggccgccgac 600
   aaggccgcct gcctgctgcc caagctggac gagctgcggg acgagggcaa ggccagcagc 660
   gccaagcagc ggctgaagtg cgccagcctg cagaagttcg gcgagcgggc cttcaaggcc 720
   tgggccgtgg cccggctgag ccagcggttc cccaaggccg agttcgccga ggtgagcaag 780
   ctggtgaccg acctgaccaa ggtgcacacc gagtgctgcc acggcgacct gctggagtgc 840
   gccgacgacc gggccgacct ggccaagtac atctgcgaga accaggacag catcagcagc 900
   aagctgaagg agtgctgcga gaagcccctg ctggagaaga gccactgcat cgccgaggtg 960
   gagaacgacg agatgcccgc cgacctgccc agcctggccg ccgacttcgt ggagagcaag 1020
   gacgtgtgca agaactacgc cgaggccaag gacgtgttcc tgggcatgtt cctgtacgag 1080
   tacgcccggc ggcaccccga ctacagcgtg gtgctgctgc tgcggctggc caagacctac 1140
   gagaccaccc tggagaagtg ctgcgccgcc gccgaccccc acgagtgcta cgccaaggtg 1200
   ttcgacgagt tcaagcccct ggtggaggag ccccagaacc tgatcaagca gaactgcgag 1260
   ctgttcgagc agctgggcga gtacaagttc cagaacgccc tgctggtgcg gtacaccaag 1320
   aaggtgcccc aggtgagcac ccccaccctg gtggaggtga gccggaacct gggcaaggtg 1380
   ggcagcaagt gctgcaagca ccccgaggcc aagcggatgc cctgcgccga ggactacctg 1440
   agcgtggtgc tgaaccagct gtgcgtgctg cacgagaaga cccccgtgag cgaccgggtg 1500
   accaagtgct gcaccgagag cctggtgaac cggcggccct gcttcagcgc cctggaggtg 1560
   gacgagacct acgtgcccaa ggagttcaac gccgagacct tcaccttcca cgccgacatc 1620
   tgcaccctga gcgagaagga gcggcagatc aagaagcaga ccgccctggt ggagctggtg     1680
   aagcacaagc ccaaggccac caaggagcag ctgaaggccg tgatggacga cttcgccgcc     1740
   ttcgtggaga agtgctgcaa ggccgacgac aaggagacct gcttcgccga ggagggcaag     1800
   aagctggtgg ccgccagcca ggccgccctg ggcctgggca gcggcggcgg cggcagcggc     1860
   ggcggcggat ctggtggagg tggcagtgga ggagggggat ccctcgaggt cctctttcag     1920
   ggaccacagt gccagaagtg gatgcagaca tgcgacgccg agcgcaagtg ctgcgaaggc     1980
   ttcgtgtgtc gcctgtggtg taaaaagaag ttgtggtga                            2019
<210> 85
   <211> 672
   <212> PRT
   <213> Artificial Sequence
<220>
   <223> Description of Artificial Sequence: Synthetic NV1G1005 (NV1D2772) Y1R, W7Q, S11A, M19F polypeptide
<400> 85
   Met Ala Trp Val Trp Thr Leu Leu Phe Leu Met Ala Ala Ala Gln Ser
   1               5                   10                  15
   Ile Gln Ala Gly Ser His His His His His His Asp Ala His Lys Ser
               20                  25                  30
   Glu Val Ala His Arg Phe Lys Asp Leu Gly Glu Glu Asn Phe Lys Ala
           35                  40                  45
   Leu Val Leu Ile Ala Phe Ala Gln Tyr Leu Gln Gln Cys Pro Phe Glu
       50                  55                  60
   Asp His Val Lys Leu Val Asn Glu Val Thr Glu Phe Ala Lys Thr Cys
   65                  70                  75                  80
   Val Ala Asp Glu Ser Ala Glu Asn Cys Asp Lys Ser Leu His Thr Leu
                   85                  90                  95
   Phe Gly Asp Lys Leu Cys Thr Val Ala Thr Leu Arg Glu Thr Tyr Gly
               100                 105                 110
   Glu Met Ala Asp Cys Cys Ala Lys Gln Glu Pro Glu Arg Asn Glu Cys
           115                 120                 125
   Phe Leu Gln His Lys Asp Asp Asn Pro Asn Leu Pro Arg Leu Val Arg
       130                 135                 140
   Pro Glu Val Asp Val Met Cys Thr Ala Phe His Asp Asn Glu Glu Thr
   145                 150                 155                 160
   Phe Leu Lys Lys Tyr Leu Tyr Glu Ile Ala Arg Arg His Pro Tyr Phe
                   165                 170                 175
   Tyr Ala Pro Glu Leu Leu Phe Phe Ala Lys Arg Tyr Lys Ala Ala Phe
               180                 185                 190
   Thr Glu Cys Cys Gln Ala Ala Asp Lys Ala Ala Cys Leu Leu Pro Lys
           195                 200                 205
   Leu Asp Glu Leu Arg Asp Glu Gly Lys Ala Ser Ser Ala Lys Gln Arg
       210                 215                 220
   Leu Lys Cys Ala Ser Leu Gln Lys Phe Gly Glu Arg Ala Phe Lys Ala
   225                 230                 235                 240
   Trp Ala Val Ala Arg Leu Ser Gln Arg Phe Pro Lys Ala Glu Phe Ala
                   245                 250                 255
   Glu Val Ser Lys Leu Val Thr Asp Leu Thr Lys Val His Thr Glu Cys
               260                 265                 270
   Cys His Gly Asp Leu Leu Glu Cys Ala Asp Asp Arg Ala Asp Leu Ala
           275                 280                 285
   Lys Tyr Ile Cys Glu Asn Gln Asp Ser Ile Ser Ser Lys Leu Lys Glu
       290                 295                 300
   Cys Cys Glu Lys Pro Leu Leu Glu Lys Ser His Cys Ile Ala Glu Val
   305                 310                 315                 320
   Glu Asn Asp Glu Met Pro Ala Asp Leu Pro Ser Leu Ala Ala Asp Phe
                   325                 330                 335
   Val Glu Ser Lys Asp Val Cys Lys Asn Tyr Ala Glu Ala Lys Asp Val
               340                 345                 350
   Phe Leu Gly Met Phe Leu Tyr Glu Tyr Ala Arg Arg His Pro Asp Tyr
           355                 360                 365
   Ser Val Val Leu Leu Leu Arg Leu Ala Lys Thr Tyr Glu Thr Thr Leu
       370                 375                 380
   Glu Lys Cys Cys Ala Ala Ala Asp Pro His Glu Cys Tyr Ala Lys Val
   385                 390                 395                 400
   Phe Asp Glu Phe Lys Pro Leu Val Glu Glu Pro Gln Asn Leu Ile Lys
                   405                 410                 415
   Gln Asn Cys Glu Leu Phe Glu Gln Leu Gly Glu Tyr Lys Phe Gln Asn
               420                 425                 430
   Ala Leu Leu Val Arg Tyr Thr Lys Lys Val Pro Gln Val Ser Thr Pro
           435                 440                 445
   Thr Leu Val Glu Val Ser Arg Asn Leu Gly Lys Val Gly Ser Lys Cys
       450                 455                 460
   Cys Lys His Pro Glu Ala Lys Arg Met Pro Cys Ala Glu Asp Tyr Leu
   465                 470                 475                 480
   Ser Val Val Leu Asn Gln Leu Cys Val Leu His Glu Lys Thr Pro Val
                   485                 490                 495
   Ser Asp Arg Val Thr Lys Cys Cys Thr Glu Ser Leu Val Asn Arg Arg
               500                 505                 510
   Pro Cys Phe Ser Ala Leu Glu Val Asp Glu Thr Tyr Val Pro Lys Glu
           515                 520                 525
   Phe Asn Ala Glu Thr Phe Thr Phe His Ala Asp Ile Cys Thr Leu Ser
       530                 535                 540
   Glu Lys Glu Arg Gln Ile Lys Lys Gln Thr Ala Leu Val Glu Leu Val
   545                 550                 555                 560
   Lys His Lys Pro Lys Ala Thr Lys Glu Gln Leu Lys Ala Val Met Asp
                   565                 570                 575
   Asp Phe Ala Ala Phe Val Glu Lys Cys Cys Lys Ala Asp Asp Lys Glu
               580                 585                 590
   Thr Cys Phe Ala Glu Glu Gly Lys Lys Leu Val Ala Ala Ser Gln Ala
           595                 600                 605
   Ala Leu Gly Leu Gly Ser Gly Gly Gly Gly Ser Gly Gly Gly Gly Ser
       610                 615                 620
   Gly Gly Gly Gly Ser Gly Gly Gly Gly Ser Leu Glu Val Leu Phe Gln
   625                 630                 635                 640
   Gly Pro Arg Cys Gln Lys Trp Met Gln Thr Cys Asp Ala Glu Arg Lys
                   645                 650                 655
   Cys Cys Glu Gly Phe Val Cys Arg Leu Trp Cys Lys Lys Lys Leu Trp
           660                 665                 670
<210> 86
   <211> 2019
   <212> DNA
   <213> Artificial Sequence
<220>
   <223> Description of Artificial Sequence: Synthetic NV1G1005 (NV1D2772) Y1R, W7Q, S11A, M19F polynucleotide
<400> 86
   atggcttggg tgtggacctt gctattcctg atggcggccg cccaaagtat acaggccggg      60
   agccaccacc accaccacca cgacgcccac aagagcgagg tggcccaccg gttcaaggac     120
   ctgggcgagg agaacttcaa ggccctggtg ctgatcgcct tcgcccagta cctgcagcag     180
   tgccccttcg aggaccacgt gaagctggtg aacgaggtga ccgagttcgc caagacctgc      240
   gtggccgacg agagcgccga gaactgcgac aagagcctgc acaccctgtt cggcgacaag     300
   ctgtgcaccg tggccaccct gcgggagacc tacggcgaga tggccgactg ctgcgccaag     360
   caggagcccg agcggaacga gtgcttcctg cagcacaagg acgacaaccc caacctgccc     420
   cggctggtgc ggcccgaggt ggacgtgatg tgcaccgcct tccacgacaa cgaggagacc     480
   ttcctgaaga agtacctgta cgagatcgcc cggcggcacc cctacttcta cgcccccgag     540
   ctgctgttct tcgccaagcg gtacaaggcc gccttcaccg agtgctgcca ggccgccgac     600
   aaggccgcct gcctgctgcc caagctggac gagctgcggg acgagggcaa ggccagcagc     660
   gccaagcagc ggctgaagtg cgccagcctg cagaagttcg gcgagcgggc cttcaaggcc     720
   tgggccgtgg cccggctgag ccagcggttc cccaaggccg agttcgccga ggtgagcaag     780
   ctggtgaccg acctgaccaa ggtgcacacc gagtgctgcc acggcgacct gctggagtgc     840
   gccgacgacc gggccgacct ggccaagtac atctgcgaga accaggacag catcagcagc     900
   aagctgaagg agtgctgcga gaagcccctg ctggagaaga gccactgcat cgccgaggtg     960
   gagaacgacg agatgcccgc cgacctgccc agcctggccg ccgacttcgt ggagagcaag    1020
   gacgtgtgca agaactacgc cgaggccaag gacgtgttcc tgggcatgtt cctgtacgag    1080
   tacgcccggc ggcaccccga ctacagcgtg gtgctgctgc tgcggctggc caagacctac    1140
   gagaccaccc tggagaagtg ctgcgccgcc gccgaccccc acgagtgcta cgccaaggtg    1200
   ttcgacgagt tcaagcccct ggtggaggag ccccagaacc tgatcaagca gaactgcgag    1260
   ctgttcgagc agctgggcga gtacaagttc cagaacgccc tgctggtgcg gtacaccaag    1320
   aaggtgcccc aggtgagcac ccccaccctg gtggaggtga gccggaacct gggcaaggtg    1380
   ggcagcaagt gctgcaagca ccccgaggcc aagcggatgc cctgcgccga ggactacctg    1440
   agcgtggtgc tgaaccagct gtgcgtgctg cacgagaaga cccccgtgag cgaccgggtg 1500
   accaagtgct gcaccgagag cctggtgaac cggcggccct gcttcagcgc cctggaggtg 1560
   gacgagacct acgtgcccaa ggagttcaac gccgagacct tcaccttcca cgccgacatc 1620
   tgcaccctga gcgagaagga gcggcagatc aagaagcaga ccgccctggt ggagctggtg 1680
   aagcacaagc ccaaggccac caaggagcag ctgaaggccg tgatggacga cttcgccgcc 1740
   ttcgtggaga agtgctgcaa ggccgacgac aaggagacct gcttcgccga ggagggcaag 1800
   aagctggtgg ccgccagcca ggccgccctg ggcctgggca gcggcggcgg cggcagcggc 1860
   ggcggcggat ctggtggagg tggcagtgga ggagggggat ccctcgaggt cctctttcag 1920
   ggaccacggt gccagaagtg gatgcagaca tgcgacgccg agcgcaagtg ctgcgaaggc 1980
   ttcgtgtgtc gcctgtggtg taaaaagaag ttgtggtga 2019
<210> 87
   <211> 672
   <212> PRT
   <213> Artificial Sequence
<220>
   <223> Description of Artificial Sequence: Synthetic NV1G1001 (NV1D2773) Y1S, W7Q, S11A, M19F polypeptide
<400> 87
   Met Ala Trp Val Trp Thr Leu Leu Phe Leu Met Ala Ala Ala Gln Ser
   1 5 10 15
   Ile Gln Ala Gly Ser His His His His His His Asp Ala His Lys Ser
   20 25 30
   Glu Val Ala His Arg Phe Lys Asp Leu Gly Glu Glu Asn Phe Lys Ala
   35 40 45
   Leu Val Leu Ile Ala Phe Ala Gln Tyr Leu Gln Gln Cys Pro Phe Glu
   50 55 60
   Asp His Val Lys Leu Val Asn Glu Val Thr Glu Phe Ala Lys Thr Cys
   65 70 75 80
   Val Ala Asp Glu Ser Ala Glu Asn Cys Asp Lys Ser Leu His Thr Leu
   85 90 95
   Phe Gly Asp Lys Leu Cys Thr Val Ala Thr Leu Arg Glu Thr Tyr Gly
   100 105 110
   Glu Met Ala Asp Cys Cys Ala Lys Gln Glu Pro Glu Arg Asn Glu Cys
   115 120 125
   Phe Leu Gln His Lys Asp Asp Asn Pro Asn Leu Pro Arg Leu Val Arg
       130                 135                 140
   Pro Glu Val Asp Val Met Cys Thr Ala Phe His Asp Asn Glu Glu Thr
   145                 150                 155                 160
   Phe Leu Lys Lys Tyr Leu Tyr Glu Ile Ala Arg Arg His Pro Tyr Phe
                   165                 170                 175
   Tyr Ala Pro Glu Leu Leu Phe Phe Ala Lys Arg Tyr Lys Ala Ala Phe
               180                 185                 190
   Thr Glu Cys Cys Gln Ala Ala Asp Lys Ala Ala Cys Leu Leu Pro Lys
           195                 200                 205
   Leu Asp Glu Leu Arg Asp Glu Gly Lys Ala Ser Ser Ala Lys Gln Arg
       210                 215                 220
   Leu Lys Cys Ala Ser Leu Gln Lys Phe Gly Glu Arg Ala Phe Lys Ala
   225                 230                 235                 240
   Trp Ala Val Ala Arg Leu Ser Gln Arg Phe Pro Lys Ala Glu Phe Ala
                   245                 250                 255
   Glu Val Ser Lys Leu Val Thr Asp Leu Thr Lys Val His Thr Glu Cys
               260                 265                 270
   Cys His Gly Asp Leu Leu Glu Cys Ala Asp Asp Arg Ala Asp Leu Ala
           275                 280                 285
   Lys Tyr Ile Cys Glu Asn Gln Asp Ser Ile Ser Ser Lys Leu Lys Glu
       290                 295                 300
   Cys Cys Glu Lys Pro Leu Leu Glu Lys Ser His Cys Ile Ala Glu Val
   305                 310                 315                 320
   Glu Asn Asp Glu Met Pro Ala Asp Leu Pro Ser Leu Ala Ala Asp Phe
                   325                 330                 335
   Val Glu Ser Lys Asp Val Cys Lys Asn Tyr Ala Glu Ala Lys Asp Val
               340                 345                 350
   Phe Leu Gly Met Phe Leu Tyr Glu Tyr Ala Arg Arg His Pro Asp Tyr
           355                 360                 365
   Ser Val Val Leu Leu Leu Arg Leu Ala Lys Thr Tyr Glu Thr Thr Leu
       370                 375                 380
   Glu Lys Cys Cys Ala Ala Ala Asp Pro His Glu Cys Tyr Ala Lys Val
   385                 390                 395                 400
   Phe Asp Glu Phe Lys Pro Leu Val Glu Glu Pro Gln Asn Leu Ile Lys
                   405                 410                 415
   Gln Asn Cys Glu Leu Phe Glu Gln Leu Gly Glu Tyr Lys Phe Gln Asn
               420                 425                 430
   Ala Leu Leu Val Arg Tyr Thr Lys Lys Val Pro Gln Val Ser Thr Pro
           435                 440                 445
   Thr Leu Val Glu Val Ser Arg Asn Leu Gly Lys Val Gly Ser Lys Cys
       450                 455                 460
   Cys Lys His Pro Glu Ala Lys Arg Met Pro Cys Ala Glu Asp Tyr Leu
   465                 470                 475                 480
   Ser Val Val Leu Asn Gln Leu Cys Val Leu His Glu Lys Thr Pro Val
                   485                 490                 495
   Ser Asp Arg Val Thr Lys Cys Cys Thr Glu Ser Leu Val Asn Arg Arg
               500                 505                 510
   Pro Cys Phe Ser Ala Leu Glu Val Asp Glu Thr Tyr Val Pro Lys Glu
           515                 520                 525
   Phe Asn Ala Glu Thr Phe Thr Phe His Ala Asp Ile Cys Thr Leu Ser
       530                 535                 540
   Glu Lys Glu Arg Gln Ile Lys Lys Gln Thr Ala Leu Val Glu Leu Val
   545                 550                 555                 560
   Lys His Lys Pro Lys Ala Thr Lys Glu Gln Leu Lys Ala Val Met Asp
                   565                 570                 575
   Asp Phe Ala Ala Phe Val Glu Lys Cys Cys Lys Ala Asp Asp Lys Glu
               580                 585                 590
   Thr Cys Phe Ala Glu Glu Gly Lys Lys Leu Val Ala Ala Ser Gln Ala
           595                 600                 605
   Ala Leu Gly Leu Gly Ser Gly Gly Gly Gly Ser Gly Gly Gly Gly Ser
       610                 615                 620
   Gly Gly Gly Gly Ser Gly Gly Gly Gly Ser Leu Glu Val Leu Phe Gln
   625 630 635 640
   Gly Pro Ser Cys Gln Lys Trp Met Gln Thr Cys Asp Ala Glu Arg Lys
   645 650 655
   Cys Cys Glu Gly Phe Val Cys Arg Leu Trp Cys Lys Lys Lys Leu Trp
   660 665 670
<210> 88
   <211> 2019
   <212> DNA
   <213> Artificial Sequence
<220>
   <223> Description of Artificial Sequence: Synthetic NV1G1001 (NV1D2773) Y1S, W7Q, S11A, M19F polynucleotide
<400> 88
   atggcttggg tgtggacctt gctattcctg atggcggccg cccaaagtat acaggccggg 60
   agccaccacc accaccacca cgacgcccac aagagcgagg tggcccaccg gttcaaggac 120
   ctgggcgagg agaacttcaa ggccctggtg ctgatcgcct tcgcccagta cctgcagcag 180
   tgccccttcg aggaccacgt gaagctggtg aacgaggtga ccgagttcgc caagacctgc 240
   gtggccgacg agagcgccga gaactgcgac aagagcctgc acaccctgtt cggcgacaag 300
   ctgtgcaccg tggccaccct gcgggagacc tacggcgaga tggccgactg ctgcgccaag 360
   caggagcccg agcggaacga gtgcttcctg cagcacaagg acgacaaccc caacctgccc 420
   cggctggtgc ggcccgaggt ggacgtgatg tgcaccgcct tccacgacaa cgaggagacc 480
   ttcctgaaga agtacctgta cgagatcgcc cggcggcacc cctacttcta cgcccccgag 540
   ctgctgttct tcgccaagcg gtacaaggcc gccttcaccg agtgctgcca ggccgccgac 600
   aaggccgcct gcctgctgcc caagctggac gagctgcggg acgagggcaa ggccagcagc 660
   gccaagcagc ggctgaagtg cgccagcctg cagaagttcg gcgagcgggc cttcaaggcc 720
   tgggccgtgg cccggctgag ccagcggttc cccaaggccg agttcgccga ggtgagcaag 780
   ctggtgaccg acctgaccaa ggtgcacacc gagtgctgcc acggcgacct gctggagtgc 840
   gccgacgacc gggccgacct ggccaagtac atctgcgaga accaggacag catcagcagc 900
   aagctgaagg agtgctgcga gaagcccctg ctggagaaga gccactgcat cgccgaggtg 960
   gagaacgacg agatgcccgc cgacctgccc agcctggccg ccgacttcgt ggagagcaag 1020
   gacgtgtgca agaactacgc cgaggccaag gacgtgttcc tgggcatgtt cctgtacgag 1080
   tacgcccggc ggcaccccga ctacagcgtg gtgctgctgc tgcggctggc caagacctac 1140
   gagaccaccc tggagaagtg ctgcgccgcc gccgaccccc acgagtgcta cgccaaggtg 1200
   ttcgacgagt tcaagcccct ggtggaggag ccccagaacc tgatcaagca gaactgcgag 1260
   ctgttcgagc agctgggcga gtacaagttc cagaacgccc tgctggtgcg gtacaccaag 1320
   aaggtgcccc aggtgagcac ccccaccctg gtggaggtga gccggaacct gggcaaggtg 1380
   ggcagcaagt gctgcaagca ccccgaggcc aagcggatgc cctgcgccga ggactacctg 1440
   agcgtggtgc tgaaccagct gtgcgtgctg cacgagaaga cccccgtgag cgaccgggtg 1500
   accaagtgct gcaccgagag cctggtgaac cggcggccct gcttcagcgc cctggaggtg 1560
   gacgagacct acgtgcccaa ggagttcaac gccgagacct tcaccttcca cgccgacatc 1620
   tgcaccctga gcgagaagga gcggcagatc aagaagcaga ccgccctggt ggagctggtg 1680
   aagcacaagc ccaaggccac caaggagcag ctgaaggccg tgatggacga cttcgccgcc 1740
   ttcgtggaga agtgctgcaa ggccgacgac aaggagacct gcttcgccga ggagggcaag 1800
   aagctggtgg ccgccagcca ggccgccctg ggcctgggca gcggcggcgg cggcagcggc 1860
   ggcggcggat ctggtggagg tggcagtgga ggagggggat ccctcgaggt cctctttcag 1920
   ggaccaagct gccagaagtg gatgcagaca tgcgacgccg agcgcaagtg ctgcgaaggc 1980
   ttcgtgtgtc gcctgtggtg taaaaagaag ttgtggtga 2019
<210> 89
   <211> 672
   <212> PRT
   <213> Artificial Sequence
<220>
   <223> Description of Artificial Sequence: Synthetic NV1G1079 (NV1D2889) Y1S,M6F,S11A,M19F polypeptide
<400> 89
   Met Ala Trp Val Trp Thr Leu Leu Phe Leu Met Ala Ala Ala Gln Ser
   1               5                   10                  15
   Ile Gln Ala Gly Ser His His His His His His Asp Ala His Lys Ser
               20                  25                  30
   Glu Val Ala His Arg Phe Lys Asp Leu Gly Glu Glu Asn Phe Lys Ala
           35                  40                  45
   Leu Val Leu Ile Ala Phe Ala Gln Tyr Leu Gln Gln Cys Pro Phe Glu
       50                  55                  60
   Asp His Val Lys Leu Val Asn Glu Val Thr Glu Phe Ala Lys Thr Cys
   65                  70                  75                  80
   Val Ala Asp Glu Ser Ala Glu Asn Cys Asp Lys Ser Leu His Thr Leu
                   85                  90                  95
   Phe Gly Asp Lys Leu Cys Thr Val Ala Thr Leu Arg Glu Thr Tyr Gly
               100                 105                 110
   Glu Met Ala Asp Cys Cys Ala Lys Gln Glu Pro Glu Arg Asn Glu Cys
           115                 120                 125
   Phe Leu Gln His Lys Asp Asp Asn Pro Asn Leu Pro Arg Leu Val Arg
       130                 135                 140
   Pro Glu Val Asp Val Met Cys Thr Ala Phe His Asp Asn Glu Glu Thr
   145                 150                 155                 160
   Phe Leu Lys Lys Tyr Leu Tyr Glu Ile Ala Arg Arg His Pro Tyr Phe
                   165                 170                 175
   Tyr Ala Pro Glu Leu Leu Phe Phe Ala Lys Arg Tyr Lys Ala Ala Phe
               180                 185                 190
   Thr Glu Cys Cys Gln Ala Ala Asp Lys Ala Ala Cys Leu Leu Pro Lys
           195                 200                 205
   Leu Asp Glu Leu Arg Asp Glu Gly Lys Ala Ser Ser Ala Lys Gln Arg
       210                 215                 220
   Leu Lys Cys Ala Ser Leu Gln Lys Phe Gly Glu Arg Ala Phe Lys Ala
   225                 230                 235                 240
   Trp Ala Val Ala Arg Leu Ser Gln Arg Phe Pro Lys Ala Glu Phe Ala
                   245                 250                 255
   Glu Val Ser Lys Leu Val Thr Asp Leu Thr Lys Val His Thr Glu Cys
               260                 265                 270
   Cys His Gly Asp Leu Leu Glu Cys Ala Asp Asp Arg Ala Asp Leu Ala
           275                 280                 285
   Lys Tyr Ile Cys Glu Asn Gln Asp Ser Ile Ser Ser Lys Leu Lys Glu
       290                 295                 300
   Cys Cys Glu Lys Pro Leu Leu Glu Lys Ser His Cys Ile Ala Glu Val
   305                 310                 315                 320
   Glu Asn Asp Glu Met Pro Ala Asp Leu Pro Ser Leu Ala Ala Asp Phe
                   325                 330                 335
   Val Glu Ser Lys Asp Val Cys Lys Asn Tyr Ala Glu Ala Lys Asp Val
               340                 345                 350
   Phe Leu Gly Met Phe Leu Tyr Glu Tyr Ala Arg Arg His Pro Asp Tyr
           355                 360                 365
   Ser Val Val Leu Leu Leu Arg Leu Ala Lys Thr Tyr Glu Thr Thr Leu
       370                 375                 380
   Glu Lys Cys Cys Ala Ala Ala Asp Pro His Glu Cys Tyr Ala Lys Val
   385                 390                 395                 400
   Phe Asp Glu Phe Lys Pro Leu Val Glu Glu Pro Gln Asn Leu Ile Lys
                   405                 410                 415
   Gln Asn Cys Glu Leu Phe Glu Gln Leu Gly Glu Tyr Lys Phe Gln Asn
               420                 425                 430
   Ala Leu Leu Val Arg Tyr Thr Lys Lys Val Pro Gln Val Ser Thr Pro
           435                 440                 445
   Thr Leu Val Glu Val Ser Arg Asn Leu Gly Lys Val Gly Ser Lys Cys
       450                 455                 460
   Cys Lys His Pro Glu Ala Lys Arg Met Pro Cys Ala Glu Asp Tyr Leu
   465                 470                 475                 480
   Ser Val Val Leu Asn Gln Leu Cys Val Leu His Glu Lys Thr Pro Val
                   485                 490                 495
   Ser Asp Arg Val Thr Lys Cys Cys Thr Glu Ser Leu Val Asn Arg Arg
               500                 505                 510
   Pro Cys Phe Ser Ala Leu Glu Val Asp Glu Thr Tyr Val Pro Lys Glu
           515                 520                 525
   Phe Asn Ala Glu Thr Phe Thr Phe His Ala Asp Ile Cys Thr Leu Ser
       530                 535                 540
   Glu Lys Glu Arg Gln Ile Lys Lys Gln Thr Ala Leu Val Glu Leu Val
   545                 550                 555                 560
   Lys His Lys Pro Lys Ala Thr Lys Glu Gln Leu Lys Ala Val Met Asp
                   565                 570                 575
   Asp Phe Ala Ala Phe Val Glu Lys Cys Cys Lys Ala Asp Asp Lys Glu
               580                 585                 590
   Thr Cys Phe Ala Glu Glu Gly Lys Lys Leu Val Ala Ala Ser Gln Ala
           595                 600                 605
   Ala Leu Gly Leu Gly Ser Gly Gly Gly Gly Ser Gly Gly Gly Gly Ser
       610                 615                 620
   Gly Gly Gly Gly Ser Gly Gly Gly Gly Ser Leu Glu Val Leu Phe Gln
   625                 630                 635                 640
   Gly Pro Ser Cys Gln Lys Trp Phe Trp Thr Cys Asp Ala Glu Arg Lys
                   645                 650                 655
   Cys Cys Glu Gly Phe Val Cys Arg Leu Trp Cys Lys Lys Lys Leu Trp
               660                 665                 670
<210> 90
   <211> 2019
   <212> DNA
   <213> Artificial Sequence
<220>
   <223> Description of Artificial Sequence: Synthetic NV1G1079 (NV1D2889) Y1S,M6F,S11A,M19F polynucleotide
<400> 90
   atggcttggg tgtggacctt gctattcctg atggcggccg cccaaagtat acaggccggg      60
   agccaccacc accaccacca cgacgcccac aagagcgagg tggcccaccg gttcaaggac     120
   ctgggcgagg agaacttcaa ggccctggtg ctgatcgcct tcgcccagta cctgcagcag     180
   tgccccttcg aggaccacgt gaagctggtg aacgaggtga ccgagttcgc caagacctgc     240
   gtggccgacg agagcgccga gaactgcgac aagagcctgc acaccctgtt cggcgacaag     300
   ctgtgcaccg tggccaccct gcgggagacc tacggcgaga tggccgactg ctgcgccaag     360
   caggagcccg agcggaacga gtgcttcctg cagcacaagg acgacaaccc caacctgccc     420
   cggctggtgc ggcccgaggt ggacgtgatg tgcaccgcct tccacgacaa cgaggagacc     480
   ttcctgaaga agtacctgta cgagatcgcc cggcggcacc cctacttcta cgcccccgag     540
   ctgctgttct tcgccaagcg gtacaaggcc gccttcaccg agtgctgcca ggccgccgac     600
   aaggccgcct gcctgctgcc caagctggac gagctgcggg acgagggcaa ggccagcagc     660
   gccaagcagc ggctgaagtg cgccagcctg cagaagttcg gcgagcgggc cttcaaggcc     720
   tgggccgtgg cccggctgag ccagcggttc cccaaggccg agttcgccga ggtgagcaag     780
   ctggtgaccg acctgaccaa ggtgcacacc gagtgctgcc acggcgacct gctggagtgc     840
   gccgacgacc gggccgacct ggccaagtac atctgcgaga accaggacag catcagcagc     900
   aagctgaagg agtgctgcga gaagcccctg ctggagaaga gccactgcat cgccgaggtg     960
   gagaacgacg agatgcccgc cgacctgccc agcctggccg ccgacttcgt ggagagcaag    1020
   gacgtgtgca agaactacgc cgaggccaag gacgtgttcc tgggcatgtt cctgtacgag    1080
   tacgcccggc ggcaccccga ctacagcgtg gtgctgctgc tgcggctggc caagacctac     1140
   gagaccaccc tggagaagtg ctgcgccgcc gccgaccccc acgagtgcta cgccaaggtg     1200
   ttcgacgagt tcaagcccct ggtggaggag ccccagaacc tgatcaagca gaactgcgag     1260
   ctgttcgagc agctgggcga gtacaagttc cagaacgccc tgctggtgcg gtacaccaag     1320
   aaggtgcccc aggtgagcac ccccaccctg gtggaggtga gccggaacct gggcaaggtg     1380
   ggcagcaagt gctgcaagca ccccgaggcc aagcggatgc cctgcgccga ggactacctg     1440
   agcgtggtgc tgaaccagct gtgcgtgctg cacgagaaga cccccgtgag cgaccgggtg     1500
   accaagtgct gcaccgagag cctggtgaac cggcggccct gcttcagcgc cctggaggtg     1560
   gacgagacct acgtgcccaa ggagttcaac gccgagacct tcaccttcca cgccgacatc     1620
   tgcaccctga gcgagaagga gcggcagatc aagaagcaga ccgccctggt ggagctggtg     1680
   aagcacaagc ccaaggccac caaggagcag ctgaaggccg tgatggacga cttcgccgcc     1740
   ttcgtggaga agtgctgcaa ggccgacgac aaggagacct gcttcgccga ggagggcaag     1800
   aagctggtgg ccgccagcca ggccgccctg ggcctgggca gcggcggcgg cggcagcggc     1860
   ggcggcggat ctggtggagg tggcagtgga ggagggggat ccctcgaggt cctctttcag     1920
   ggaccaagct gccagaagtg gttctggaca tgcgacgccg agcgcaagtg ctgcgaaggc     1980
   ttcgtgtgtc gcctgtggtg taaaaagaag ttgtggtga                            2019
<210> 91
   <211> 672
   <212> PRT
   <213> Artificial Sequence
<220>
   <223> Description of Artificial Sequence: Synthetic NV1G1107 (NV1D2890) Y1S,M6F,S11A,M19L polypeptide
<400> 91
   Met Ala Trp Val Trp Thr Leu Leu Phe Leu Met Ala Ala Ala Gln Ser
   1               5                   10                  15
   Ile Gln Ala Gly Ser His His His His His His Asp Ala His Lys Ser
               20                  25                  30
   Glu Val Ala His Arg Phe Lys Asp Leu Gly Glu Glu Asn Phe Lys Ala
           35                  40                  45
   Leu Val Leu Ile Ala Phe Ala Gln Tyr Leu Gln Gln Cys Pro Phe Glu
       50                  55                  60
   Asp His Val Lys Leu Val Asn Glu Val Thr Glu Phe Ala Lys Thr Cys
   65                  70                  75                  80
   Val Ala Asp Glu Ser Ala Glu Asn Cys Asp Lys Ser Leu His Thr Leu
                   85                  90                  95
   Phe Gly Asp Lys Leu Cys Thr Val Ala Thr Leu Arg Glu Thr Tyr Gly
               100                 105                 110
   Glu Met Ala Asp Cys Cys Ala Lys Gln Glu Pro Glu Arg Asn Glu Cys
           115                 120                 125
   Phe Leu Gln His Lys Asp Asp Asn Pro Asn Leu Pro Arg Leu Val Arg
       130                 135                 140
   Pro Glu Val Asp Val Met Cys Thr Ala Phe His Asp Asn Glu Glu Thr
   145                 150                 155                 160
   Phe Leu Lys Lys Tyr Leu Tyr Glu Ile Ala Arg Arg His Pro Tyr Phe
                   165                 170                 175
   Tyr Ala Pro Glu Leu Leu Phe Phe Ala Lys Arg Tyr Lys Ala Ala Phe
               180                 185                 190
   Thr Glu Cys Cys Gln Ala Ala Asp Lys Ala Ala Cys Leu Leu Pro Lys
           195                 200                 205
   Leu Asp Glu Leu Arg Asp Glu Gly Lys Ala Ser Ser Ala Lys Gln Arg
       210                 215                 220
   Leu Lys Cys Ala Ser Leu Gln Lys Phe Gly Glu Arg Ala Phe Lys Ala
   225                 230                 235                 240
   Trp Ala Val Ala Arg Leu Ser Gln Arg Phe Pro Lys Ala Glu Phe Ala
                   245                 250                 255
   Glu Val Ser Lys Leu Val Thr Asp Leu Thr Lys Val His Thr Glu Cys
               260                 265                 270
   Cys His Gly Asp Leu Leu Glu Cys Ala Asp Asp Arg Ala Asp Leu Ala
           275                 280                 285
   Lys Tyr Ile Cys Glu Asn Gln Asp Ser Ile Ser Ser Lys Leu Lys Glu
       290                 295                 300
   Cys Cys Glu Lys Pro Leu Leu Glu Lys Ser His Cys Ile Ala Glu Val
   305                 310                 315                 320
   Glu Asn Asp Glu Met Pro Ala Asp Leu Pro Ser Leu Ala Ala Asp Phe
                   325                 330                 335
   Val Glu Ser Lys Asp Val Cys Lys Asn Tyr Ala Glu Ala Lys Asp Val
               340                 345                 350
   Phe Leu Gly Met Phe Leu Tyr Glu Tyr Ala Arg Arg His Pro Asp Tyr
           355                 360                 365
   Ser Val Val Leu Leu Leu Arg Leu Ala Lys Thr Tyr Glu Thr Thr Leu
       370                 375                 380
   Glu Lys Cys Cys Ala Ala Ala Asp Pro His Glu Cys Tyr Ala Lys Val
   385                 390                 395                 400
   Phe Asp Glu Phe Lys Pro Leu Val Glu Glu Pro Gln Asn Leu Ile Lys
                   405                 410                 415
   Gln Asn Cys Glu Leu Phe Glu Gln Leu Gly Glu Tyr Lys Phe Gln Asn
               420                 425                 430
   Ala Leu Leu Val Arg Tyr Thr Lys Lys Val Pro Gln Val Ser Thr Pro
           435                 440                 445
   Thr Leu Val Glu Val Ser Arg Asn Leu Gly Lys Val Gly Ser Lys Cys
       450                 455                 460
   Cys Lys His Pro Glu Ala Lys Arg Met Pro Cys Ala Glu Asp Tyr Leu
   465                 470                 475                 480
   Ser Val Val Leu Asn Gln Leu Cys Val Leu His Glu Lys Thr Pro Val
                   485                 490                 495
   Ser Asp Arg Val Thr Lys Cys Cys Thr Glu Ser Leu Val Asn Arg Arg
               500                 505                 510
   Pro Cys Phe Ser Ala Leu Glu Val Asp Glu Thr Tyr Val Pro Lys Glu
           515                 520                 525
   Phe Asn Ala Glu Thr Phe Thr Phe His Ala Asp Ile Cys Thr Leu Ser
       530                 535                 540
   Glu Lys Glu Arg Gln Ile Lys Lys Gln Thr Ala Leu Val Glu Leu Val
   545                 550                 555                 560
   Lys His Lys Pro Lys Ala Thr Lys Glu Gln Leu Lys Ala Val Met Asp
                   565                 570                 575
   Asp Phe Ala Ala Phe Val Glu Lys Cys Cys Lys Ala Asp Asp Lys Glu
               580                 585                 590
   Thr Cys Phe Ala Glu Glu Gly Lys Lys Leu Val Ala Ala Ser Gln Ala
           595                 600                 605
   Ala Leu Gly Leu Gly Ser Gly Gly Gly Gly Ser Gly Gly Gly Gly Ser
       610                 615                 620
   Gly Gly Gly Gly Ser Gly Gly Gly Gly Ser Leu Glu Val Leu Phe Gln
   625                 630                 635                 640
   Gly Pro Ser Cys Gln Lys Trp Phe Trp Thr Cys Asp Ala Glu Arg Lys
                   645                 650                 655
   Cys Cys Glu Gly Leu Val Cys Arg Leu Trp Cys Lys Lys Lys Leu Trp
               660                 665                 670
<210> 92
   <211> 2019
   <212> DNA
   <213> Artificial Sequence
<220>
   <223> Description of Artificial Sequence: Synthetic NV1G1107 (NV1D2890) Y1S,M6F,S11A,M19L polynucleotide
<400> 92
   atggcttggg tgtggacctt gctattcctg atggcggccg cccaaagtat acaggccggg 60
   agccaccacc accaccacca cgacgcccac aagagcgagg tggcccaccg gttcaaggac 120
   ctgggcgagg agaacttcaa ggccctggtg ctgatcgcct tcgcccagta cctgcagcag 180
   tgccccttcg aggaccacgt gaagctggtg aacgaggtga ccgagttcgc caagacctgc 240
   gtggccgacg agagcgccga gaactgcgac aagagcctgc acaccctgtt cggcgacaag 300
   ctgtgcaccg tggccaccct gcgggagacc tacggcgaga tggccgactg ctgcgccaag 360
   caggagcccg agcggaacga gtgcttcctg cagcacaagg acgacaaccc caacctgccc 420
   cggctggtgc ggcccgaggt ggacgtgatg tgcaccgcct tccacgacaa cgaggagacc 480
   ttcctgaaga agtacctgta cgagatcgcc cggcggcacc cctacttcta cgcccccgag 540
   ctgctgttct tcgccaagcg gtacaaggcc gccttcaccg agtgctgcca ggccgccgac 600
   aaggccgcct gcctgctgcc caagctggac gagctgcggg acgagggcaa ggccagcagc 660
   gccaagcagc ggctgaagtg cgccagcctg cagaagttcg gcgagcgggc cttcaaggcc 720
   tgggccgtgg cccggctgag ccagcggttc cccaaggccg agttcgccga ggtgagcaag 780
   ctggtgaccg acctgaccaa ggtgcacacc gagtgctgcc acggcgacct gctggagtgc 840
   gccgacgacc gggccgacct ggccaagtac atctgcgaga accaggacag catcagcagc 900
   aagctgaagg agtgctgcga gaagcccctg ctggagaaga gccactgcat cgccgaggtg      960
   gagaacgacg agatgcccgc cgacctgccc agcctggccg ccgacttcgt ggagagcaag     1020
   gacgtgtgca agaactacgc cgaggccaag gacgtgttcc tgggcatgtt cctgtacgag     1080
   tacgcccggc ggcaccccga ctacagcgtg gtgctgctgc tgcggctggc caagacctac     1140
   gagaccaccc tggagaagtg ctgcgccgcc gccgaccccc acgagtgcta cgccaaggtg     1200
   ttcgacgagt tcaagcccct ggtggaggag ccccagaacc tgatcaagca gaactgcgag     1260
   ctgttcgagc agctgggcga gtacaagttc cagaacgccc tgctggtgcg gtacaccaag     1320
   aaggtgcccc aggtgagcac ccccaccctg gtggaggtga gccggaacct gggcaaggtg     1380
   ggcagcaagt gctgcaagca ccccgaggcc aagcggatgc cctgcgccga ggactacctg     1440
   agcgtggtgc tgaaccagct gtgcgtgctg cacgagaaga cccccgtgag cgaccgggtg     1500
   accaagtgct gcaccgagag cctggtgaac cggcggccct gcttcagcgc cctggaggtg     1560
   gacgagacct acgtgcccaa ggagttcaac gccgagacct tcaccttcca cgccgacatc     1620
   tgcaccctga gcgagaagga gcggcagatc aagaagcaga ccgccctggt ggagctggtg     1680
   aagcacaagc ccaaggccac caaggagcag ctgaaggccg tgatggacga cttcgccgcc     1740
   ttcgtggaga agtgctgcaa ggccgacgac aaggagacct gcttcgccga ggagggcaag     1800
   aagctggtgg ccgccagcca ggccgccctg ggcctgggca gcggcggcgg cggcagcggc     1860
   ggcggcggat ctggtggagg tggcagtgga ggagggggat ccctcgaggt cctctttcag     1920
   ggaccaagct gccagaagtg gttctggacc tgcgacgccg agcggaagtg ctgcgagggc     1980
   ctggtgtgcc ggctgtggtg caagaagaag ctgtggtga                            2019
<210> 93
   <211> 672
   <212> PRT
   <213> Artificial Sequence
<220>
   <223> Description of Artificial Sequence: Synthetic NV1G1063 (NV1D2913) Y1R,M6F,W7Q,S11A,M19F polypeptide
<400> 93
   Met Ala Trp Val Trp Thr Leu Leu Phe Leu Met Ala Ala Ala Gln Ser
   1               5                   10                  15
   Ile Gln Ala Gly Ser His His His His His His Asp Ala His Lys Ser
               20                  25                  30
   Glu Val Ala His Arg Phe Lys Asp Leu Gly Glu Glu Asn Phe Lys Ala
           35                  40                  45
   Leu Val Leu Ile Ala Phe Ala Gln Tyr Leu Gln Gln Cys Pro Phe Glu
       50                  55                  60
   Asp His Val Lys Leu Val Asn Glu Val Thr Glu Phe Ala Lys Thr Cys
   65                  70                  75                  80
   Val Ala Asp Glu Ser Ala Glu Asn Cys Asp Lys Ser Leu His Thr Leu
                   85                  90                  95
   Phe Gly Asp Lys Leu Cys Thr Val Ala Thr Leu Arg Glu Thr Tyr Gly
               100                 105                 110
   Glu Met Ala Asp Cys Cys Ala Lys Gln Glu Pro Glu Arg Asn Glu Cys
           115                 120                 125
   Phe Leu Gln His Lys Asp Asp Asn Pro Asn Leu Pro Arg Leu Val Arg
       130                 135                 140
   Pro Glu Val Asp Val Met Cys Thr Ala Phe His Asp Asn Glu Glu Thr
   145                 150                 155                 160
   Phe Leu Lys Lys Tyr Leu Tyr Glu Ile Ala Arg Arg His Pro Tyr Phe
                   165                 170                 175
   Tyr Ala Pro Glu Leu Leu Phe Phe Ala Lys Arg Tyr Lys Ala Ala Phe
               180                 185                 190
   Thr Glu Cys Cys Gln Ala Ala Asp Lys Ala Ala Cys Leu Leu Pro Lys
           195                 200                 205
   Leu Asp Glu Leu Arg Asp Glu Gly Lys Ala Ser Ser Ala Lys Gln Arg
       210                 215                 220
   Leu Lys Cys Ala Ser Leu Gln Lys Phe Gly Glu Arg Ala Phe Lys Ala
   225                 230                 235                 240
   Trp Ala Val Ala Arg Leu Ser Gln Arg Phe Pro Lys Ala Glu Phe Ala
                   245                 250                 255
   Glu Val Ser Lys Leu Val Thr Asp Leu Thr Lys Val His Thr Glu Cys
               260                 265                 270
   Cys His Gly Asp Leu Leu Glu Cys Ala Asp Asp Arg Ala Asp Leu Ala
           275                 280                 285
   Lys Tyr Ile Cys Glu Asn Gln Asp Ser Ile Ser Ser Lys Leu Lys Glu
       290                 295                 300
   Cys Cys Glu Lys Pro Leu Leu Glu Lys Ser His Cys Ile Ala Glu Val
   305                 310                 315                 320
   Glu Asn Asp Glu Met Pro Ala Asp Leu Pro Ser Leu Ala Ala Asp Phe
                   325                 330                 335
   Val Glu Ser Lys Asp Val Cys Lys Asn Tyr Ala Glu Ala Lys Asp Val
               340                 345                 350
   Phe Leu Gly Met Phe Leu Tyr Glu Tyr Ala Arg Arg His Pro Asp Tyr
           355                 360                 365
   Ser Val Val Leu Leu Leu Arg Leu Ala Lys Thr Tyr Glu Thr Thr Leu
       370                 375                 380
   Glu Lys Cys Cys Ala Ala Ala Asp Pro His Glu Cys Tyr Ala Lys Val
   385                 390                 395                 400
   Phe Asp Glu Phe Lys Pro Leu Val Glu Glu Pro Gln Asn Leu Ile Lys
                   405                 410                 415
   Gln Asn Cys Glu Leu Phe Glu Gln Leu Gly Glu Tyr Lys Phe Gln Asn
               420                 425                 430
   Ala Leu Leu Val Arg Tyr Thr Lys Lys Val Pro Gln Val Ser Thr Pro
           435                 440                 445
   Thr Leu Val Glu Val Ser Arg Asn Leu Gly Lys Val Gly Ser Lys Cys
       450                 455                 460
   Cys Lys His Pro Glu Ala Lys Arg Met Pro Cys Ala Glu Asp Tyr Leu
   465                 470                 475                 480
   Ser Val Val Leu Asn Gln Leu Cys Val Leu His Glu Lys Thr Pro Val
                   485                 490                 495
   Ser Asp Arg Val Thr Lys Cys Cys Thr Glu Ser Leu Val Asn Arg Arg
               500                 505                 510
   Pro Cys Phe Ser Ala Leu Glu Val Asp Glu Thr Tyr Val Pro Lys Glu
           515                 520                 525
   Phe Asn Ala Glu Thr Phe Thr Phe His Ala Asp Ile Cys Thr Leu Ser
       530                 535                 540
   Glu Lys Glu Arg Gln Ile Lys Lys Gln Thr Ala Leu Val Glu Leu Val
   545                 550                 555                 560
   Lys His Lys Pro Lys Ala Thr Lys Glu Gln Leu Lys Ala Val Met Asp
   565 570 575
   Asp Phe Ala Ala Phe Val Glu Lys Cys Cys Lys Ala Asp Asp Lys Glu
   580 585 590
   Thr Cys Phe Ala Glu Glu Gly Lys Lys Leu Val Ala Ala Ser Gln Ala
   595 600 605
   Ala Leu Gly Leu Gly Ser Gly Gly Gly Gly Ser Gly Gly Gly Gly Ser
   610 615 620
   Gly Gly Gly Gly Ser Gly Gly Gly Gly Ser Leu Glu Val Leu Phe Gln
   625 630 635 640
   Gly Pro Arg Cys Gln Lys Trp Phe Gln Thr Cys Asp Ala Glu Arg Lys
   645 650 655
   Cys Cys Glu Gly Phe Val Cys Arg Leu Trp Cys Lys Lys Lys Leu Trp
   660 665 670
<210> 94
   <211> 2019
   <212> DNA
   <213> Artificial Sequence
<220>
   <223> Description of Artificial Sequence: Synthetic NV1G1063 (NV1D2913) Y1R,M6F,W7Q,S11A,M19F polynucleotide
<400> 94
   atggcttggg tgtggacctt gctattcctg atggcggccg cccaaagtat acaggccggg 60
   agccaccacc accaccacca cgacgcccac aagagcgagg tggcccaccg gttcaaggac 120
   ctgggcgagg agaacttcaa ggccctggtg ctgatcgcct tcgcccagta cctgcagcag 180
   tgccccttcg aggaccacgt gaagctggtg aacgaggtga ccgagttcgc caagacctgc 240
   gtggccgacg agagcgccga gaactgcgac aagagcctgc acaccctgtt cggcgacaag 300
   ctgtgcaccg tggccaccct gcgggagacc tacggcgaga tggccgactg ctgcgccaag 360
   caggagcccg agcggaacga gtgcttcctg cagcacaagg acgacaaccc caacctgccc 420
   cggctggtgc ggcccgaggt ggacgtgatg tgcaccgcct tccacgacaa cgaggagacc 480
   ttcctgaaga agtacctgta cgagatcgcc cggcggcacc cctacttcta cgcccccgag 540
   ctgctgttct tcgccaagcg gtacaaggcc gccttcaccg agtgctgcca ggccgccgac 600
   aaggccgcct gcctgctgcc caagctggac gagctgcggg acgagggcaa ggccagcagc 660
   gccaagcagc ggctgaagtg cgccagcctg cagaagttcg gcgagcgggc cttcaaggcc 720
   tgggccgtgg cccggctgag ccagcggttc cccaaggccg agttcgccga ggtgagcaag      780
   ctggtgaccg acctgaccaa ggtgcacacc gagtgctgcc acggcgacct gctggagtgc      840
   gccgacgacc gggccgacct ggccaagtac atctgcgaga accaggacag catcagcagc      900
   aagctgaagg agtgctgcga gaagcccctg ctggagaaga gccactgcat cgccgaggtg      960
   gagaacgacg agatgcccgc cgacctgccc agcctggccg ccgacttcgt ggagagcaag     1020
   gacgtgtgca agaactacgc cgaggccaag gacgtgttcc tgggcatgtt cctgtacgag     1080
   tacgcccggc ggcaccccga ctacagcgtg gtgctgctgc tgcggctggc caagacctac     1140
   gagaccaccc tggagaagtg ctgcgccgcc gccgaccccc acgagtgcta cgccaaggtg     1200
   ttcgacgagt tcaagcccct ggtggaggag ccccagaacc tgatcaagca gaactgcgag     1260
   ctgttcgagc agctgggcga gtacaagttc cagaacgccc tgctggtgcg gtacaccaag     1320
   aaggtgcccc aggtgagcac ccccaccctg gtggaggtga gccggaacct gggcaaggtg     1380
   ggcagcaagt gctgcaagca ccccgaggcc aagcggatgc cctgcgccga ggactacctg     1440
   agcgtggtgc tgaaccagct gtgcgtgctg cacgagaaga cccccgtgag cgaccgggtg     1500
   accaagtgct gcaccgagag cctggtgaac cggcggccct gcttcagcgc cctggaggtg     1560
   gacgagacct acgtgcccaa ggagttcaac gccgagacct tcaccttcca cgccgacatc     1620
   tgcaccctga gcgagaagga gcggcagatc aagaagcaga ccgccctggt ggagctggtg     1680
   aagcacaagc ccaaggccac caaggagcag ctgaaggccg tgatggacga cttcgccgcc     1740
   ttcgtggaga agtgctgcaa ggccgacgac aaggagacct gcttcgccga ggagggcaag     1800
   aagctggtgg ccgccagcca ggccgccctg ggcctgggca gcggcggcgg cggcagcggc     1860
   ggcggcggat ctggtggagg tggcagtgga ggagggggat ccctcgaggt cctctttcag     1920
   ggaccacggt gccagaagtg gttccagaca tgcgacgccg agcgcaagtg ctgcgaaggc     1980
   ttcgtgtgtc gcctgtggtg taaaaagaag ttgtggtga                            2019
<210> 95
   <211> 672
   <212> PRT
   <213> Artificial Sequence
<220>
   <223> Description of Artificial Sequence: Synthetic NV1G1097 (NV1D2810), Y1Q,M6F,W7Q polypeptide
<400> 95
   Met Ala Trp Val Trp Thr Leu Leu Phe Leu Met Ala Ala Ala Gln Ser
   1               5                   10                  15
   Ile Gln Ala Gly Ser His His His His His His Asp Ala His Lys Ser
               20                  25                  30
   Glu Val Ala His Arg Phe Lys Asp Leu Gly Glu Glu Asn Phe Lys Ala
   35 40 45
   Leu Val Leu Ile Ala Phe Ala Gln Tyr Leu Gln Gln Cys Pro Phe Glu
   50 55 60
   Asp His Val Lys Leu Val Asn Glu Val Thr Glu Phe Ala Lys Thr Cys
   65 70 75 80
   Val Ala Asp Glu Ser Ala Glu Asn Cys Asp Lys Ser Leu His Thr Leu
   85 90 95
   Phe Gly Asp Lys Leu Cys Thr Val Ala Thr Leu Arg Glu Thr Tyr Gly
   100 105 110
   Glu Met Ala Asp Cys Cys Ala Lys Gln Glu Pro Glu Arg Asn Glu Cys
   115 120 125
   Phe Leu Gln His Lys Asp Asp Asn Pro Asn Leu Pro Arg Leu Val Arg
   130 135 140
   Pro Glu Val Asp Val Met Cys Thr Ala Phe His Asp Asn Glu Glu Thr
   145 150 155 160
   Phe Leu Lys Lys Tyr Leu Tyr Glu Ile Ala Arg Arg His Pro Tyr Phe
   165 170 175
   Tyr Ala Pro Glu Leu Leu Phe Phe Ala Lys Arg Tyr Lys Ala Ala Phe
   180 185 190
   Thr Glu Cys Cys Gln Ala Ala Asp Lys Ala Ala Cys Leu Leu Pro Lys
   195 200 205
   Leu Asp Glu Leu Arg Asp Glu Gly Lys Ala Ser Ser Ala Lys Gln Arg
   210 215 220
   Leu Lys Cys Ala Ser Leu Gln Lys Phe Gly Glu Arg Ala Phe Lys Ala
   225 230 235 240
   Trp Ala Val Ala Arg Leu Ser Gln Arg Phe Pro Lys Ala Glu Phe Ala
   245 250 255
   Glu Val Ser Lys Leu Val Thr Asp Leu Thr Lys Val His Thr Glu Cys
   260 265 270
   Cys His Gly Asp Leu Leu Glu Cys Ala Asp Asp Arg Ala Asp Leu Ala
   275 280 285
   Lys Tyr Ile Cys Glu Asn Gln Asp Ser Ile Ser Ser Lys Leu Lys Glu
   290 295 300
   Cys Cys Glu Lys Pro Leu Leu Glu Lys Ser His Cys Ile Ala Glu Val
   305 310 315 320
   Glu Asn Asp Glu Met Pro Ala Asp Leu Pro Ser Leu Ala Ala Asp Phe
   325 330 335
   Val Glu Ser Lys Asp Val Cys Lys Asn Tyr Ala Glu Ala Lys Asp Val
   340 345 350
   Phe Leu Gly Met Phe Leu Tyr Glu Tyr Ala Arg Arg His Pro Asp Tyr
   355 360 365
   Ser Val Val Leu Leu Leu Arg Leu Ala Lys Thr Tyr Glu Thr Thr Leu
   370 375 380
   Glu Lys Cys Cys Ala Ala Ala Asp Pro His Glu Cys Tyr Ala Lys Val
   385 390 395 400
   Phe Asp Glu Phe Lys Pro Leu Val Glu Glu Pro Gln Asn Leu Ile Lys
   405 410 415
   Gln Asn Cys Glu Leu Phe Glu Gln Leu Gly Glu Tyr Lys Phe Gln Asn
   420 425 430
   Ala Leu Leu Val Arg Tyr Thr Lys Lys Val Pro Gln Val Ser Thr Pro
   435 440 445
   Thr Leu Val Glu Val Ser Arg Asn Leu Gly Lys Val Gly Ser Lys Cys
   450 455 460
   Cys Lys His Pro Glu Ala Lys Arg Met Pro Cys Ala Glu Asp Tyr Leu
   465 470 475 480
   Ser Val Val Leu Asn Gln Leu Cys Val Leu His Glu Lys Thr Pro Val
   485 490 495
   Ser Asp Arg Val Thr Lys Cys Cys Thr Glu Ser Leu Val Asn Arg Arg
   500 505 510
   Pro Cys Phe Ser Ala Leu Glu Val Asp Glu Thr Tyr Val Pro Lys Glu
   515 520 525
   Phe Asn Ala Glu Thr Phe Thr Phe His Ala Asp Ile Cys Thr Leu Ser
       530                 535                 540
   Glu Lys Glu Arg Gln Ile Lys Lys Gln Thr Ala Leu Val Glu Leu Val
   545                 550                 555                 560
   Lys His Lys Pro Lys Ala Thr Lys Glu Gln Leu Lys Ala Val Met Asp
                   565                 570                 575
   Asp Phe Ala Ala Phe Val Glu Lys Cys Cys Lys Ala Asp Asp Lys Glu
               580                 585                 590
   Thr Cys Phe Ala Glu Glu Gly Lys Lys Leu Val Ala Ala Ser Gln Ala
           595                 600                 605
   Ala Leu Gly Leu Gly Ser Gly Gly Gly Gly Ser Gly Gly Gly Gly Ser
       610                 615                 620
   Gly Gly Gly Gly Ser Gly Gly Gly Gly Ser Leu Glu Val Leu Phe Gln
   625                 630                 635                 640
   Gly Pro Gln Cys Gln Lys Trp Phe Gln Thr Cys Asp Ser Glu Arg Lys
                   645                 650                 655
   Cys Cys Glu Gly Met Val Cys Arg Leu Trp Cys Lys Lys Lys Leu Trp
               660                 665                 670
<210> 96
   <211> 1959
   <212> DNA
   <213> Artificial Sequence
<220>
   <223> Description of Artificial Sequence: Synthetic NV1G1097 (NV1D2810), Y1Q,M6F,W7Q polynucleotide
<400> 96
   gggagccacc accaccacca ccacgacgcc cacaagagcg aggtggccca ccggttcaag      60
   gacctgggcg aggagaactt caaggccctg gtgctgatcg ccttcgccca gtacctgcag     120
   cagtgcccct tcgaggacca cgtgaagctg gtgaacgagg tgaccgagtt cgccaagacc     180
   tgcgtggccg acgagagcgc cgagaactgc gacaagagcc tgcacaccct gttcggcgac     240
   aagctgtgca ccgtggccac cctgcgggag acctacggcg agatggccga ctgctgcgcc     300
   aagcaggagc ccgagcggaa cgagtgcttc ctgcagcaca aggacgacaa ccccaacctg     360
   ccccggctgg tgcggcccga ggtggacgtg atgtgcaccg ccttccacga caacgaggag     420
   accttcctga agaagtacct gtacgagatc gcccggcggc acccctactt ctacgccccc     480
   gagctgctgt tcttcgccaa gcggtacaag gccgccttca ccgagtgctg ccaggccgcc     540
   gacaaggccg cctgcctgct gcccaagctg gacgagctgc gggacgaggg caaggccagc 600
   agcgccaagc agcggctgaa gtgcgccagc ctgcagaagt tcggcgagcg ggccttcaag 660
   gcctgggccg tggcccggct gagccagcgg ttccccaagg ccgagttcgc cgaggtgagc 720
   aagctggtga ccgacctgac caaggtgcac accgagtgct gccacggcga cctgctggag 780
   tgcgccgacg accgggccga cctggccaag tacatctgcg agaaccagga cagcatcagc 840
   agcaagctga aggagtgctg cgagaagccc ctgctggaga agagccactg catcgccgag 900
   gtggagaacg acgagatgcc cgccgacctg cccagcctgg ccgccgactt cgtggagagc 960
   aaggacgtgt gcaagaacta cgccgaggcc aaggacgtgt tcctgggcat gttcctgtac 1020
   gagtacgccc ggcggcaccc cgactacagc gtggtgctgc tgctgcggct ggccaagacc 1080
   tacgagacca ccctggagaa gtgctgcgcc gccgccgacc cccacgagtg ctacgccaag 1140
   gtgttcgacg agttcaagcc cctggtggag gagccccaga acctgatcaa gcagaactgc 1200
   gagctgttcg agcagctggg cgagtacaag ttccagaacg ccctgctggt gcggtacacc 1260
   aagaaggtgc cccaggtgag cacccccacc ctggtggagg tgagccggaa cctgggcaag 1320
   gtgggcagca agtgctgcaa gcaccccgag gccaagcgga tgccctgcgc cgaggactac 1380
   ctgagcgtgg tgctgaacca gctgtgcgtg ctgcacgaga agacccccgt gagcgaccgg 1440
   gtgaccaagt gctgcaccga gagcctggtg aaccggcggc cctgcttcag cgccctggag 1500
   gtggacgaga cctacgtgcc caaggagttc aacgccgaga ccttcacctt ccacgccgac 1560
   atctgcaccc tgagcgagaa ggagcggcag atcaagaagc agaccgccct ggtggagctg 1620
   gtgaagcaca agcccaaggc caccaaggag cagctgaagg ccgtgatgga cgacttcgcc 1680
   gccttcgtgg agaagtgctg caaggccgac gacaaggaga cctgcttcgc cgaggagggc 1740
   aagaagctgg tggccgccag ccaggccgcc ctgggcctgg gcagcggcgg cggcggcagc 1800
   ggcggcggcg gatctggtgg aggtggcagt ggaggagggg gatccctcga ggtcctcttt 1860
   cagggaccac agtgccagaa gtggttccag acatgcgaca gcgagcgcaa gtgctgcgaa 1920
   ggcatggtgt gtcgcctgtg gtgtaaaaag aagttgtgg 1959
<210> 97
   <211> 672
   <212> PRT
   <213> Artificial Sequence
<220>
   <223> Description of Artificial Sequence: Synthetic NV1G1123 (NV1D2882), Y1A,M6F,W7Q,M19L polypeptide
<400> 97
   Met Ala Trp Val Trp Thr Leu Leu Phe Leu Met Ala Ala Ala Gln Ser
   1               5                   10                  15
   Ile Gln Ala Gly Ser His His His His His His Asp Ala His Lys Ser
               20                  25                  30
   Glu Val Ala His Arg Phe Lys Asp Leu Gly Glu Glu Asn Phe Lys Ala
           35                  40                  45
   Leu Val Leu Ile Ala Phe Ala Gln Tyr Leu Gln Gln Cys Pro Phe Glu
       50                  55                  60
   Asp His Val Lys Leu Val Asn Glu Val Thr Glu Phe Ala Lys Thr Cys
   65                  70                  75                  80
   Val Ala Asp Glu Ser Ala Glu Asn Cys Asp Lys Ser Leu His Thr Leu
                   85                  90                  95
   Phe Gly Asp Lys Leu Cys Thr Val Ala Thr Leu Arg Glu Thr Tyr Gly
               100                 105                 110
   Glu Met Ala Asp Cys Cys Ala Lys Gln Glu Pro Glu Arg Asn Glu Cys
           115                 120                 125
   Phe Leu Gln His Lys Asp Asp Asn Pro Asn Leu Pro Arg Leu Val Arg
       130                 135                 140
   Pro Glu Val Asp Val Met Cys Thr Ala Phe His Asp Asn Glu Glu Thr
   145                 150                 155                 160
   Phe Leu Lys Lys Tyr Leu Tyr Glu Ile Ala Arg Arg His Pro Tyr Phe
                   165                 170                 175
   Tyr Ala Pro Glu Leu Leu Phe Phe Ala Lys Arg Tyr Lys Ala Ala Phe
               180                 185                 190
   Thr Glu Cys Cys Gln Ala Ala Asp Lys Ala Ala Cys Leu Leu Pro Lys
           195                 200                 205
   Leu Asp Glu Leu Arg Asp Glu Gly Lys Ala Ser Ser Ala Lys Gln Arg
       210                 215                 220
   Leu Lys Cys Ala Ser Leu Gln Lys Phe Gly Glu Arg Ala Phe Lys Ala
   225                 230                 235                 240
   Trp Ala Val Ala Arg Leu Ser Gln Arg Phe Pro Lys Ala Glu Phe Ala
                   245                 250                 255
   Glu Val Ser Lys Leu Val Thr Asp Leu Thr Lys Val His Thr Glu Cys
               260                 265                 270
   Cys His Gly Asp Leu Leu Glu Cys Ala Asp Asp Arg Ala Asp Leu Ala
           275                 280                 285
   Lys Tyr Ile Cys Glu Asn Gln Asp Ser Ile Ser Ser Lys Leu Lys Glu
       290                 295                 300
   Cys Cys Glu Lys Pro Leu Leu Glu Lys Ser His Cys Ile Ala Glu Val
   305                 310                 315                 320
   Glu Asn Asp Glu Met Pro Ala Asp Leu Pro Ser Leu Ala Ala Asp Phe
                   325                 330                 335
   Val Glu Ser Lys Asp Val Cys Lys Asn Tyr Ala Glu Ala Lys Asp Val
               340                 345                 350
   Phe Leu Gly Met Phe Leu Tyr Glu Tyr Ala Arg Arg His Pro Asp Tyr
           355                 360                 365
   Ser Val Val Leu Leu Leu Arg Leu Ala Lys Thr Tyr Glu Thr Thr Leu
       370                 375                 380
   Glu Lys Cys Cys Ala Ala Ala Asp Pro His Glu Cys Tyr Ala Lys Val
   385                 390                 395                 400
   Phe Asp Glu Phe Lys Pro Leu Val Glu Glu Pro Gln Asn Leu Ile Lys
                   405                 410                 415
   Gln Asn Cys Glu Leu Phe Glu Gln Leu Gly Glu Tyr Lys Phe Gln Asn
               420                 425                 430
   Ala Leu Leu Val Arg Tyr Thr Lys Lys Val Pro Gln Val Ser Thr Pro
           435                 440                 445
   Thr Leu Val Glu Val Ser Arg Asn Leu Gly Lys Val Gly Ser Lys Cys
       450                 455                 460
   Cys Lys His Pro Glu Ala Lys Arg Met Pro Cys Ala Glu Asp Tyr Leu
   465                 470                 475                 480
   Ser Val Val Leu Asn Gln Leu Cys Val Leu His Glu Lys Thr Pro Val
                   485                 490                 495
   Ser Asp Arg Val Thr Lys Cys Cys Thr Glu Ser Leu Val Asn Arg Arg
               500                 505                 510
   Pro Cys Phe Ser Ala Leu Glu Val Asp Glu Thr Tyr Val Pro Lys Glu
   515 520 525
   Phe Asn Ala Glu Thr Phe Thr Phe His Ala Asp Ile Cys Thr Leu Ser
   530 535 540
   Glu Lys Glu Arg Gln Ile Lys Lys Gln Thr Ala Leu Val Glu Leu Val
   545 550 555 560
   Lys His Lys Pro Lys Ala Thr Lys Glu Gln Leu Lys Ala Val Met Asp
   565 570 575
   Asp Phe Ala Ala Phe Val Glu Lys Cys Cys Lys Ala Asp Asp Lys Glu
   580 585 590
   Thr Cys Phe Ala Glu Glu Gly Lys Lys Leu Val Ala Ala Ser Gln Ala
   595 600 605
   Ala Leu Gly Leu Gly Ser Gly Gly Gly Gly Ser Gly Gly Gly Gly Ser
   610 615 620
   Gly Gly Gly Gly Ser Gly Gly Gly Gly Ser Leu Glu Val Leu Phe Gln
   625 630 635 640
   Gly Pro Ala Cys Gln Lys Trp Phe Gln Thr Cys Asp Ser Glu Arg Lys
   645 650 655
   Cys Cys Glu Gly Leu Val Cys Arg Leu Trp Cys Lys Lys Lys Leu Trp
   660 665 670
<210> 98
   <211> 2019
   <212> DNA
   <213> Artificial Sequence
<220>
   <223> Description of Artificial Sequence: Synthetic NV1G1123 (NV1D2882), Y1A,M6F,W7Q,M19L polynucleotide
<400> 98
   atggcttggg tgtggacctt gctattcctg atggcggccg cccaaagtat acaggccggg       60
   agccaccacc accaccacca cgacgcccac aagagcgagg tggcccaccg gttcaaggac      120
   ctgggcgagg agaacttcaa ggccctggtg ctgatcgcct tcgcccagta cctgcagcag      180
   tgccccttcg aggaccacgt gaagctggtg aacgaggtga ccgagttcgc caagacctgc      240
   gtggccgacg agagcgccga gaactgcgac aagagcctgc acaccctgtt cggcgacaag      300
   ctgtgcaccg tggccaccct gcgggagacc tacggcgaga tggccgactg ctgcgccaag      360
   caggagcccg agcggaacga gtgcttcctg cagcacaagg acgacaaccc caacctgccc      420
   cggctggtgc ggcccgaggt ggacgtgatg tgcaccgcct tccacgacaa cgaggagacc 480
   ttcctgaaga agtacctgta cgagatcgcc cggcggcacc cctacttcta cgcccccgag 540
   ctgctgttct tcgccaagcg gtacaaggcc gccttcaccg agtgctgcca ggccgccgac 600
   aaggccgcct gcctgctgcc caagctggac gagctgcggg acgagggcaa ggccagcagc 660
   gccaagcagc ggctgaagtg cgccagcctg cagaagttcg gcgagcgggc cttcaaggcc 720
   tgggccgtgg cccggctgag ccagcggttc cccaaggccg agttcgccga ggtgagcaag 780
   ctggtgaccg acctgaccaa ggtgcacacc gagtgctgcc acggcgacct gctggagtgc 840
   gccgacgacc gggccgacct ggccaagtac atctgcgaga accaggacag catcagcagc 900
   aagctgaagg agtgctgcga gaagcccctg ctggagaaga gccactgcat cgccgaggtg 960
   gagaacgacg agatgcccgc cgacctgccc agcctggccg ccgacttcgt ggagagcaag 1020
   gacgtgtgca agaactacgc cgaggccaag gacgtgttcc tgggcatgtt cctgtacgag 1080
   tacgcccggc ggcaccccga ctacagcgtg gtgctgctgc tgcggctggc caagacctac 1140
   gagaccaccc tggagaagtg ctgcgccgcc gccgaccccc acgagtgcta cgccaaggtg 1200
   ttcgacgagt tcaagcccct ggtggaggag ccccagaacc tgatcaagca gaactgcgag 1260
   ctgttcgagc agctgggcga gtacaagttc cagaacgccc tgctggtgcg gtacaccaag 1320
   aaggtgcccc aggtgagcac ccccaccctg gtggaggtga gccggaacct gggcaaggtg 1380
   ggcagcaagt gctgcaagca ccccgaggcc aagcggatgc cctgcgccga ggactacctg 1440
   agcgtggtgc tgaaccagct gtgcgtgctg cacgagaaga cccccgtgag cgaccgggtg 1500
   accaagtgct gcaccgagag cctggtgaac cggcggccct gcttcagcgc cctggaggtg 1560
   gacgagacct acgtgcccaa ggagttcaac gccgagacct tcaccttcca cgccgacatc 1620
   tgcaccctga gcgagaagga gcggcagatc aagaagcaga ccgccctggt ggagctggtg 1680
   aagcacaagc ccaaggccac caaggagcag ctgaaggccg tgatggacga cttcgccgcc 1740
   ttcgtggaga agtgctgcaa ggccgacgac aaggagacct gcttcgccga ggagggcaag 1800
   aagctggtgg ccgccagcca ggccgccctg ggcctgggca gcggcggcgg cggcagcggc 1860
   ggcggcggat ctggtggagg tggcagtgga ggagggggat ccctcgaggt cctctttcag 1920
   ggaccagcct gccagaagtg gttccagacc tgcgacagcg agcggaagtg ctgcgagggc 1980
   ctggtgtgcc ggctgtggtg caagaagaag ctgtggtga 2019
<210> 99
   <211> 672
   <212> PRT
   <213> Artificial Sequence
<220>
   <223> Description of Artificial Sequence: Synthetic NV1G1127 (NV1D2870), Y1Q,M6F,W7Q,M19L polypeptide
<400> 99
   Met Ala Trp Val Trp Thr Leu Leu Phe Leu Met Ala Ala Ala Gln Ser
   1               5                   10                  15
   Ile Gln Ala Gly Ser His His His His His His Asp Ala His Lys Ser
               20                  25                  30
   Glu Val Ala His Arg Phe Lys Asp Leu Gly Glu Glu Asn Phe Lys Ala
           35                  40                  45
   Leu Val Leu Ile Ala Phe Ala Gln Tyr Leu Gln Gln Cys Pro Phe Glu
       50                  55                  60
   Asp His Val Lys Leu Val Asn Glu Val Thr Glu Phe Ala Lys Thr Cys
   65                  70                  75                  80
   Val Ala Asp Glu Ser Ala Glu Asn Cys Asp Lys Ser Leu His Thr Leu
                   85                  90                  95
   Phe Gly Asp Lys Leu Cys Thr Val Ala Thr Leu Arg Glu Thr Tyr Gly
               100                 105                 110
   Glu Met Ala Asp Cys Cys Ala Lys Gln Glu Pro Glu Arg Asn Glu Cys
           115                 120                 125
   Phe Leu Gln His Lys Asp Asp Asn Pro Asn Leu Pro Arg Leu Val Arg
       130                 135                 140
   Pro Glu Val Asp Val Met Cys Thr Ala Phe His Asp Asn Glu Glu Thr
   145                 150                 155                 160
   Phe Leu Lys Lys Tyr Leu Tyr Glu Ile Ala Arg Arg His Pro Tyr Phe
                   165                 170                 175
   Tyr Ala Pro Glu Leu Leu Phe Phe Ala Lys Arg Tyr Lys Ala Ala Phe
               180                 185                 190
   Thr Glu Cys Cys Gln Ala Ala Asp Lys Ala Ala Cys Leu Leu Pro Lys
           195                 200                 205
   Leu Asp Glu Leu Arg Asp Glu Gly Lys Ala Ser Ser Ala Lys Gln Arg
       210                 215                 220
   Leu Lys Cys Ala Ser Leu Gln Lys Phe Gly Glu Arg Ala Phe Lys Ala
   225                 230                 235                 240
   Trp Ala Val Ala Arg Leu Ser Gln Arg Phe Pro Lys Ala Glu Phe Ala
                   245                 250                 255
   Glu Val Ser Lys Leu Val Thr Asp Leu Thr Lys Val His Thr Glu Cys
               260                 265                 270
   Cys His Gly Asp Leu Leu Glu Cys Ala Asp Asp Arg Ala Asp Leu Ala
           275                 280                 285
   Lys Tyr Ile Cys Glu Asn Gln Asp Ser Ile Ser Ser Lys Leu Lys Glu
       290                 295                 300
   Cys Cys Glu Lys Pro Leu Leu Glu Lys Ser His Cys Ile Ala Glu Val
   305                 310                 315                 320
   Glu Asn Asp Glu Met Pro Ala Asp Leu Pro Ser Leu Ala Ala Asp Phe
                   325                 330                 335
   Val Glu Ser Lys Asp Val Cys Lys Asn Tyr Ala Glu Ala Lys Asp Val
               340                 345                 350
   Phe Leu Gly Met Phe Leu Tyr Glu Tyr Ala Arg Arg His Pro Asp Tyr
           355                 360                 365
   Ser Val Val Leu Leu Leu Arg Leu Ala Lys Thr Tyr Glu Thr Thr Leu
       370                 375                 380
   Glu Lys Cys Cys Ala Ala Ala Asp Pro His Glu Cys Tyr Ala Lys Val
   385                 390                 395                 400
   Phe Asp Glu Phe Lys Pro Leu Val Glu Glu Pro Gln Asn Leu Ile Lys
                   405                 410                 415
   Gln Asn Cys Glu Leu Phe Glu Gln Leu Gly Glu Tyr Lys Phe Gln Asn
               420                 425                 430
   Ala Leu Leu Val Arg Tyr Thr Lys Lys Val Pro Gln Val Ser Thr Pro
           435                 440                 445
   Thr Leu Val Glu Val Ser Arg Asn Leu Gly Lys Val Gly Ser Lys Cys
       450                 455                 460
   Cys Lys His Pro Glu Ala Lys Arg Met Pro Cys Ala Glu Asp Tyr Leu
   465                 470                 475                 480
   Ser Val Val Leu Asn Gln Leu Cys Val Leu His Glu Lys Thr Pro Val
                   485                 490                 495
   Ser Asp Arg Val Thr Lys Cys Cys Thr Glu Ser Leu Val Asn Arg Arg
               500                 505                 510
   Pro Cys Phe Ser Ala Leu Glu Val Asp Glu Thr Tyr Val Pro Lys Glu
           515                 520                 525
   Phe Asn Ala Glu Thr Phe Thr Phe His Ala Asp Ile Cys Thr Leu Ser
       530                 535                 540
   Glu Lys Glu Arg Gln Ile Lys Lys Gln Thr Ala Leu Val Glu Leu Val
   545                 550                 555                 560
   Lys His Lys Pro Lys Ala Thr Lys Glu Gln Leu Lys Ala Val Met Asp
                   565                 570                 575
   Asp Phe Ala Ala Phe Val Glu Lys Cys Cys Lys Ala Asp Asp Lys Glu
               580                 585                 590
   Thr Cys Phe Ala Glu Glu Gly Lys Lys Leu Val Ala Ala Ser Gln Ala
           595                 600                 605
   Ala Leu Gly Leu Gly Ser Gly Gly Gly Gly Ser Gly Gly Gly Gly Ser
       610                 615                 620
   Gly Gly Gly Gly Ser Gly Gly Gly Gly Ser Leu Glu Val Leu Phe Gln
   625                 630                 635                 640
   Gly Pro Gln Cys Gln Lys Trp Phe Gln Thr Cys Asp Ser Glu Arg Lys
                   645                 650                 655
   Cys Cys Glu Gly Leu Val Cys Arg Leu Trp Cys Lys Lys Lys Leu Trp
               660                 665                 670
<210> 100
   <211> 2019
   <212> DNA
   <213> Artificial Sequence
<220>
   <223> Description of Artificial Sequence: Synthetic NV1G1127 (NV1D2870), Y1Q,M6F,W7Q,M19L polynucleotide
<400> 100
   atggcttggg tgtggacctt gctattcctg atggcggccg cccaaagtat acaggccggg       60
   agccaccacc accaccacca cgacgcccac aagagcgagg tggcccaccg gttcaaggac      120
   ctgggcgagg agaacttcaa ggccctggtg ctgatcgcct tcgcccagta cctgcagcag      180
   tgccccttcg aggaccacgt gaagctggtg aacgaggtga ccgagttcgc caagacctgc      240
   gtggccgacg agagcgccga gaactgcgac aagagcctgc acaccctgtt cggcgacaag 300
   ctgtgcaccg tggccaccct gcgggagacc tacggcgaga tggccgactg ctgcgccaag 360
   caggagcccg agcggaacga gtgcttcctg cagcacaagg acgacaaccc caacctgccc 420
   cggctggtgc ggcccgaggt ggacgtgatg tgcaccgcct tccacgacaa cgaggagacc 480
   ttcctgaaga agtacctgta cgagatcgcc cggcggcacc cctacttcta cgcccccgag 540
   ctgctgttct tcgccaagcg gtacaaggcc gccttcaccg agtgctgcca ggccgccgac 600
   aaggccgcct gcctgctgcc caagctggac gagctgcggg acgagggcaa ggccagcagc 660
   gccaagcagc ggctgaagtg cgccagcctg cagaagttcg gcgagcgggc cttcaaggcc 720
   tgggccgtgg cccggctgag ccagcggttc cccaaggccg agttcgccga ggtgagcaag 780
   ctggtgaccg acctgaccaa ggtgcacacc gagtgctgcc acggcgacct gctggagtgc 840
   gccgacgacc gggccgacct ggccaagtac atctgcgaga accaggacag catcagcagc 900
   aagctgaagg agtgctgcga gaagcccctg ctggagaaga gccactgcat cgccgaggtg 960
   gagaacgacg agatgcccgc cgacctgccc agcctggccg ccgacttcgt ggagagcaag 1020
   gacgtgtgca agaactacgc cgaggccaag gacgtgttcc tgggcatgtt cctgtacgag 1080
   tacgcccggc ggcaccccga ctacagcgtg gtgctgctgc tgcggctggc caagacctac 1140
   gagaccaccc tggagaagtg ctgcgccgcc gccgaccccc acgagtgcta cgccaaggtg 1200
   ttcgacgagt tcaagcccct ggtggaggag ccccagaacc tgatcaagca gaactgcgag 1260
   ctgttcgagc agctgggcga gtacaagttc cagaacgccc tgctggtgcg gtacaccaag 1320
   aaggtgcccc aggtgagcac ccccaccctg gtggaggtga gccggaacct gggcaaggtg 1380
   ggcagcaagt gctgcaagca ccccgaggcc aagcggatgc cctgcgccga ggactacctg 1440
   agcgtggtgc tgaaccagct gtgcgtgctg cacgagaaga cccccgtgag cgaccgggtg 1500
   accaagtgct gcaccgagag cctggtgaac cggcggccct gcttcagcgc cctggaggtg 1560
   gacgagacct acgtgcccaa ggagttcaac gccgagacct tcaccttcca cgccgacatc 1620
   tgcaccctga gcgagaagga gcggcagatc aagaagcaga ccgccctggt ggagctggtg 1680
   aagcacaagc ccaaggccac caaggagcag ctgaaggccg tgatggacga cttcgccgcc 1740
   ttcgtggaga agtgctgcaa ggccgacgac aaggagacct gcttcgccga ggagggcaag 1800
   aagctggtgg ccgccagcca ggccgccctg ggcctgggca gcggcggcgg cggcagcggc 1860
   ggcggcggat ctggtggagg tggcagtgga ggagggggat ccctcgaggt cctctttcag 1920
   ggaccacagt gccagaagtg gttccagacc tgcgacagcg agcggaagtg ctgcgagggc 1980
   ctggtgtgcc ggctgtggtg caagaagaag ctgtggtga 2019
<210> 101
   <211> 672
   <212> PRT
   <213> Artificial Sequence
<220>
   <223> Description of Artificial Sequence: Synthetic NV1G1083 (NV1D2878), Y1S,M6F,W7Q,M19L polypeptide
<400> 101
   Met Ala Trp Val Trp Thr Leu Leu Phe Leu Met Ala Ala Ala Gln Ser
   1               5                   10                  15
   Ile Gln Ala Gly Ser His His His His His His Asp Ala His Lys Ser
               20                  25                  30
   Glu Val Ala His Arg Phe Lys Asp Leu Gly Glu Glu Asn Phe Lys Ala
           35                  40                  45
   Leu Val Leu Ile Ala Phe Ala Gln Tyr Leu Gln Gln Cys Pro Phe Glu
       50                  55                  60
   Asp His Val Lys Leu Val Asn Glu Val Thr Glu Phe Ala Lys Thr Cys
   65                  70                  75                  80
   Val Ala Asp Glu Ser Ala Glu Asn Cys Asp Lys Ser Leu His Thr Leu
                   85                  90                  95
   Phe Gly Asp Lys Leu Cys Thr Val Ala Thr Leu Arg Glu Thr Tyr Gly
               100                 105                 110
   Glu Met Ala Asp Cys Cys Ala Lys Gln Glu Pro Glu Arg Asn Glu Cys
           115                 120                 125
   Phe Leu Gln His Lys Asp Asp Asn Pro Asn Leu Pro Arg Leu Val Arg
       130                 135                 140
   Pro Glu Val Asp Val Met Cys Thr Ala Phe His Asp Asn Glu Glu Thr
   145                 150                 155                 160
   Phe Leu Lys Lys Tyr Leu Tyr Glu Ile Ala Arg Arg His Pro Tyr Phe
                   165                 170                 175
   Tyr Ala Pro Glu Leu Leu Phe Phe Ala Lys Arg Tyr Lys Ala Ala Phe
               180                 185                 190
   Thr Glu Cys Cys Gln Ala Ala Asp Lys Ala Ala Cys Leu Leu Pro Lys
           195                 200                 205
   Leu Asp Glu Leu Arg Asp Glu Gly Lys Ala Ser Ser Ala Lys Gln Arg
   210 215 220
   Leu Lys Cys Ala Ser Leu Gln Lys Phe Gly Glu Arg Ala Phe Lys Ala
   225 230 235 240
   Trp Ala Val Ala Arg Leu Ser Gln Arg Phe Pro Lys Ala Glu Phe Ala
   245 250 255
   Glu Val Ser Lys Leu Val Thr Asp Leu Thr Lys Val His Thr Glu Cys
   260 265 270
   Cys His Gly Asp Leu Leu Glu Cys Ala Asp Asp Arg Ala Asp Leu Ala
   275 280 285
   Lys Tyr Ile Cys Glu Asn Gln Asp Ser Ile Ser Ser Lys Leu Lys Glu
   290 295 300
   Cys Cys Glu Lys Pro Leu Leu Glu Lys Ser His Cys Ile Ala Glu Val
   305 310 315 320
   Glu Asn Asp Glu Met Pro Ala Asp Leu Pro Ser Leu Ala Ala Asp Phe
   325 330 335
   Val Glu Ser Lys Asp Val Cys Lys Asn Tyr Ala Glu Ala Lys Asp Val
   340 345 350
   Phe Leu Gly Met Phe Leu Tyr Glu Tyr Ala Arg Arg His Pro Asp Tyr
   355 360 365
   Ser Val Val Leu Leu Leu Arg Leu Ala Lys Thr Tyr Glu Thr Thr Leu
   370 375 380
   Glu Lys Cys Cys Ala Ala Ala Asp Pro His Glu Cys Tyr Ala Lys Val
   385 390 395 400
   Phe Asp Glu Phe Lys Pro Leu Val Glu Glu Pro Gln Asn Leu Ile Lys
   405 410 415
   Gln Asn Cys Glu Leu Phe Glu Gln Leu Gly Glu Tyr Lys Phe Gln Asn
   420 425 430
   Ala Leu Leu Val Arg Tyr Thr Lys Lys Val Pro Gln Val Ser Thr Pro
   435 440 445
   Thr Leu Val Glu Val Ser Arg Asn Leu Gly Lys Val Gly Ser Lys Cys
   450 455 460
   Cys Lys His Pro Glu Ala Lys Arg Met Pro Cys Ala Glu Asp Tyr Leu
   465 470 475 480
   Ser Val Val Leu Asn Gln Leu Cys Val Leu His Glu Lys Thr Pro Val
   485 490 495
   Ser Asp Arg Val Thr Lys Cys Cys Thr Glu Ser Leu Val Asn Arg Arg
   500 505 510
   Pro Cys Phe Ser Ala Leu Glu Val Asp Glu Thr Tyr Val Pro Lys Glu
   515 520 525
   Phe Asn Ala Glu Thr Phe Thr Phe His Ala Asp Ile Cys Thr Leu Ser
   530 535 540
   Glu Lys Glu Arg Gln Ile Lys Lys Gln Thr Ala Leu Val Glu Leu Val
   545 550 555 560
   Lys His Lys Pro Lys Ala Thr Lys Glu Gln Leu Lys Ala Val Met Asp
   565 570 575
   Asp Phe Ala Ala Phe Val Glu Lys Cys Cys Lys Ala Asp Asp Lys Glu
   580 585 590
   Thr Cys Phe Ala Glu Glu Gly Lys Lys Leu Val Ala Ala Ser Gln Ala
   595 600 605
   Ala Leu Gly Leu Gly Ser Gly Gly Gly Gly Ser Gly Gly Gly Gly Ser
   610 615 620
   Gly Gly Gly Gly Ser Gly Gly Gly Gly Ser Leu Glu Val Leu Phe Gln
   625 630 635 640
   Gly Pro Ser Cys Gln Lys Trp Phe Gln Thr Cys Asp Ser Glu Arg Lys
   645 650 655
   Cys Cys Glu Gly Leu Val Cys Arg Leu Trp Cys Lys Lys Lys Leu Trp
   660 665 670
<210> 102
   <211> 2019
   <212> DNA
   <213> Artificial Sequence
<220>
   <223> Description of Artificial Sequence: Synthetic NV1G1083 (NV1D2878), Y1S,M6F,W7Q,M19L polynucleotide
<400> 102
   atggcttggg tgtggacctt gctattcctg atggcggccg cccaaagtat acaggccggg 60
   agccaccacc accaccacca cgacgcccac aagagcgagg tggcccaccg gttcaaggac    120
   ctgggcgagg agaacttcaa ggccctggtg ctgatcgcct tcgcccagta cctgcagcag    180
   tgccccttcg aggaccacgt gaagctggtg aacgaggtga ccgagttcgc caagacctgc    240
   gtggccgacg agagcgccga gaactgcgac aagagcctgc acaccctgtt cggcgacaag    300
   ctgtgcaccg tggccaccct gcgggagacc tacggcgaga tggccgactg ctgcgccaag    360
   caggagcccg agcggaacga gtgcttcctg cagcacaagg acgacaaccc caacctgccc    420
   cggctggtgc ggcccgaggt ggacgtgatg tgcaccgcct tccacgacaa cgaggagacc    480
   ttcctgaaga agtacctgta cgagatcgcc cggcggcacc cctacttcta cgcccccgag    540
   ctgctgttct tcgccaagcg gtacaaggcc gccttcaccg agtgctgcca ggccgccgac    600
   aaggccgcct gcctgctgcc caagctggac gagctgcggg acgagggcaa ggccagcagc    660
   gccaagcagc ggctgaagtg cgccagcctg cagaagttcg gcgagcgggc cttcaaggcc    720
   tgggccgtgg cccggctgag ccagcggttc cccaaggccg agttcgccga ggtgagcaag    780
   ctggtgaccg acctgaccaa ggtgcacacc gagtgctgcc acggcgacct gctggagtgc    840
   gccgacgacc gggccgacct ggccaagtac atctgcgaga accaggacag catcagcagc    900
   aagctgaagg agtgctgcga gaagcccctg ctggagaaga gccactgcat cgccgaggtg    960
   gagaacgacg agatgcccgc cgacctgccc agcctggccg ccgacttcgt ggagagcaag   1020
   gacgtgtgca agaactacgc cgaggccaag gacgtgttcc tgggcatgtt cctgtacgag   1080
   tacgcccggc ggcaccccga ctacagcgtg gtgctgctgc tgcggctggc caagacctac   1140
   gagaccaccc tggagaagtg ctgcgccgcc gccgaccccc acgagtgcta cgccaaggtg   1200
   ttcgacgagt tcaagcccct ggtggaggag ccccagaacc tgatcaagca gaactgcgag   1260
   ctgttcgagc agctgggcga gtacaagttc cagaacgccc tgctggtgcg gtacaccaag   1320
   aaggtgcccc aggtgagcac ccccaccctg gtggaggtga gccggaacct gggcaaggtg   1380
   ggcagcaagt gctgcaagca ccccgaggcc aagcggatgc cctgcgccga ggactacctg   1440
   agcgtggtgc tgaaccagct gtgcgtgctg cacgagaaga cccccgtgag cgaccgggtg   1500
   accaagtgct gcaccgagag cctggtgaac cggcggccct gcttcagcgc cctggaggtg   1560
   gacgagacct acgtgcccaa ggagttcaac gccgagacct tcaccttcca cgccgacatc   1620
   tgcaccctga gcgagaagga gcggcagatc aagaagcaga ccgccctggt ggagctggtg   1680
   aagcacaagc ccaaggccac caaggagcag ctgaaggccg tgatggacga cttcgccgcc   1740
   ttcgtggaga agtgctgcaa ggccgacgac aaggagacct gcttcgccga ggagggcaag   1800
   aagctggtgg ccgccagcca ggccgccctg ggcctgggca gcggcggcgg cggcagcggc   1860
   ggcggcggat ctggtggagg tggcagtgga ggagggggat ccctcgaggt cctctttcag   1920
   ggaccaagct gccagaagtg gttccagaca tgcgacagcg agcgcaagtg ctgcgaaggc 1980
   ttagtgtgtc gcctgtggtg taaaaagaag ttgtggtga 2019
<210> 103
   <211> 672
   <212> PRT
   <213> Artificial Sequence
<220>
   <223> Description of Artificial Sequence: Synthetic NV1G1153 (NV1D3034) fusion protein
<400> 103
   Met Ala Trp Val Trp Thr Leu Leu Phe Leu Met Ala Ala Ala Gln Ser
   1               5                   10                  15
   Ile Gln Ala Gly Ser His His His His His His Asp Ala His Lys Ser
               20                  25                  30
   Glu Val Ala His Arg Phe Lys Asp Leu Gly Glu Glu Asn Phe Lys Ala
           35                  40                  45
   Leu Val Leu Ile Ala Phe Ala Gln Tyr Leu Gln Gln Cys Pro Phe Glu
       50                  55                  60
   Asp His Val Lys Leu Val Asn Glu Val Thr Glu Phe Ala Lys Thr Cys
   65                  70                  75                  80
   Val Ala Asp Glu Ser Ala Glu Asn Cys Asp Lys Ser Leu His Thr Leu
                   85                  90                  95
   Phe Gly Asp Lys Leu Cys Thr Val Ala Thr Leu Arg Glu Thr Tyr Gly
               100                 105                 110
   Glu Met Ala Asp Cys Cys Ala Lys Gln Glu Pro Glu Arg Asn Glu Cys
           115                 120                 125
   Phe Leu Gln His Lys Asp Asp Asn Pro Asn Leu Pro Arg Leu Val Arg
       130                 135                 140
   Pro Glu Val Asp Val Met Cys Thr Ala Phe His Asp Asn Glu Glu Thr
   145                 150                 155                 160
   Phe Leu Lys Lys Tyr Leu Tyr Glu Ile Ala Arg Arg His Pro Tyr Phe
                   165                 170                 175
   Tyr Ala Pro Glu Leu Leu Phe Phe Ala Lys Arg Tyr Lys Ala Ala Phe
               180                 185                 190
   Thr Glu Cys Cys Gln Ala Ala Asp Lys Ala Ala Cys Leu Leu Pro Lys
           195                 200                 205
   Leu Asp Glu Leu Arg Asp Glu Gly Lys Ala Ser Ser Ala Lys Gln Arg
       210                 215                 220
   Leu Lys Cys Ala Ser Leu Gln Lys Phe Gly Glu Arg Ala Phe Lys Ala
   225                 230                 235                 240
   Trp Ala Val Ala Arg Leu Ser Gln Arg Phe Pro Lys Ala Glu Phe Ala
                   245                 250                 255
   Glu Val Ser Lys Leu Val Thr Asp Leu Thr Lys Val His Thr Glu Cys
               260                 265                 270
   Cys His Gly Asp Leu Leu Glu Cys Ala Asp Asp Arg Ala Asp Leu Ala
           275                 280                 285
   Lys Tyr Ile Cys Glu Asn Gln Asp Ser Ile Ser Ser Lys Leu Lys Glu
       290                 295                 300
   Cys Cys Glu Lys Pro Leu Leu Glu Lys Ser His Cys Ile Ala Glu Val
   305                 310                 315                 320
   Glu Asn Asp Glu Met Pro Ala Asp Leu Pro Ser Leu Ala Ala Asp Phe
                   325                 330                 335
   Val Glu Ser Lys Asp Val Cys Lys Asn Tyr Ala Glu Ala Lys Asp Val
               340                 345                 350
   Phe Leu Gly Met Phe Leu Tyr Glu Tyr Ala Arg Arg His Pro Asp Tyr
           355                 360                 365
   Ser Val Val Leu Leu Leu Arg Leu Ala Lys Thr Tyr Glu Thr Thr Leu
       370                 375                 380
   Glu Lys Cys Cys Ala Ala Ala Asp Pro His Glu Cys Tyr Ala Lys Val
   385                 390                 395                 400
   Phe Asp Glu Phe Lys Pro Leu Val Glu Glu Pro Gln Asn Leu Ile Lys
                   405                 410                 415
   Gln Asn Cys Glu Leu Phe Glu Gln Leu Gly Glu Tyr Lys Phe Gln Asn
               420                 425                 430
   Ala Leu Leu Val Arg Tyr Thr Lys Lys Val Pro Gln Val Ser Thr Pro
           435                 440                 445
   Thr Leu Val Glu Val Ser Arg Asn Leu Gly Lys Val Gly Ser Lys Cys
   450 455 460
   Cys Lys His Pro Glu Ala Lys Arg Met Pro Cys Ala Glu Asp Tyr Leu
   465 470 475 480
   Ser Val Val Leu Asn Gln Leu Cys Val Leu His Glu Lys Thr Pro Val
   485 490 495
   Ser Asp Arg Val Thr Lys Cys Cys Thr Glu Ser Leu Val Asn Arg Arg
   500 505 510
   Pro Cys Phe Ser Ala Leu Glu Val Asp Glu Thr Tyr Val Pro Lys Glu
   515 520 525
   Phe Asn Ala Glu Thr Phe Thr Phe His Ala Asp Ile Cys Thr Leu Ser
   530 535 540
   Glu Lys Glu Arg Gln Ile Lys Lys Gln Thr Ala Leu Val Glu Leu Val
   545 550 555 560
   Lys His Lys Pro Lys Ala Thr Lys Glu Gln Leu Lys Ala Val Met Asp
   565 570 575
   Asp Phe Ala Ala Phe Val Glu Lys Cys Cys Lys Ala Asp Asp Lys Glu
   580 585 590
   Thr Cys Phe Ala Glu Glu Gly Lys Lys Leu Val Ala Ala Ser Gln Ala
   595 600 605
   Ala Leu Gly Leu Gly Ser Gly Gly Gly Gly Ser Gly Gly Gly Gly Ser
   610 615 620
   Gly Gly Gly Gly Ser Gly Gly Gly Gly Ser Leu Glu Val Leu Phe Gln
   625 630 635 640
   Gly Pro Gln Cys Gln Lys Trp Met Gln Thr Cys Asp Arg Glu Arg Lys
   645 650 655
   Cys Cys Glu Gly Phe Val Cys Thr Leu Trp Cys Arg Lys Lys Leu Trp
   660 665 670
<210> 104
   <211> 2019
   <212> DNA
   <213> Artificial Sequence
<220>
   <223> Description of Artificial Sequence: Synthetic NV1G1153 (NV1D3034) fusion polynucleotide
<400> 104
   atggcttggg tgtggacctt gctattcctg atggcggccg cccaaagtat acaggccggg      60
   agccaccacc accaccacca cgacgcccac aagagcgagg tggcccaccg gttcaaggac     120
   ctgggcgagg agaacttcaa ggccctggtg ctgatcgcct tcgcccagta cctgcagcag     180
   tgccccttcg aggaccacgt gaagctggtg aacgaggtga ccgagttcgc caagacctgc     240
   gtggccgacg agagcgccga gaactgcgac aagagcctgc acaccctgtt cggcgacaag     300
   ctgtgcaccg tggccaccct gcgggagacc tacggcgaga tggccgactg ctgcgccaag     360
   caggagcccg agcggaacga gtgcttcctg cagcacaagg acgacaaccc caacctgccc     420
   cggctggtgc ggcccgaggt ggacgtgatg tgcaccgcct tccacgacaa cgaggagacc     480
   ttcctgaaga agtacctgta cgagatcgcc cggcggcacc cctacttcta cgcccccgag     540
   ctgctgttct tcgccaagcg gtacaaggcc gccttcaccg agtgctgcca ggccgccgac     600
   aaggccgcct gcctgctgcc caagctggac gagctgcggg acgagggcaa ggccagcagc     660
   gccaagcagc ggctgaagtg cgccagcctg cagaagttcg gcgagcgggc cttcaaggcc     720
   tgggccgtgg cccggctgag ccagcggttc cccaaggccg agttcgccga ggtgagcaag     780
   ctggtgaccg acctgaccaa ggtgcacacc gagtgctgcc acggcgacct gctggagtgc     840
   gccgacgacc gggccgacct ggccaagtac atctgcgaga accaggacag catcagcagc     900
   aagctgaagg agtgctgcga gaagcccctg ctggagaaga gccactgcat cgccgaggtg     960
   gagaacgacg agatgcccgc cgacctgccc agcctggccg ccgacttcgt ggagagcaag    1020
   gacgtgtgca agaactacgc cgaggccaag gacgtgttcc tgggcatgtt cctgtacgag    1080
   tacgcccggc ggcaccccga ctacagcgtg gtgctgctgc tgcggctggc caagacctac    1140
   gagaccaccc tggagaagtg ctgcgccgcc gccgaccccc acgagtgcta cgccaaggtg    1200
   ttcgacgagt tcaagcccct ggtggaggag ccccagaacc tgatcaagca gaactgcgag    1260
   ctgttcgagc agctgggcga gtacaagttc cagaacgccc tgctggtgcg gtacaccaag    1320
   aaggtgcccc aggtgagcac ccccaccctg gtggaggtga gccggaacct gggcaaggtg    1380
   ggcagcaagt gctgcaagca ccccgaggcc aagcggatgc cctgcgccga ggactacctg    1440
   agcgtggtgc tgaaccagct gtgcgtgctg cacgagaaga cccccgtgag cgaccgggtg    1500
   accaagtgct gcaccgagag cctggtgaac cggcggccct gcttcagcgc cctggaggtg    1560
   gacgagacct acgtgcccaa ggagttcaac gccgagacct tcaccttcca cgccgacatc    1620
   tgcaccctga gcgagaagga gcggcagatc aagaagcaga ccgccctggt ggagctggtg    1680
   aagcacaagc ccaaggccac caaggagcag ctgaaggccg tgatggacga cttcgccgcc    1740
   ttcgtggaga agtgctgcaa ggccgacgac aaggagacct gcttcgccga ggagggcaag 1800
   aagctggtgg ccgccagcca ggccgccctg ggcctgggca gcggcggcgg cggcagcggc 1860
   ggcggcggat ctggtggagg tggcagtgga ggagggggat ccctcgaggt cctctttcag 1920
   ggaccacagt gccagaagtg gatgcagacc tgcgaccggg agcggaagtg ctgcgagggc 1980
   ttcgtgtgca ccctgtggtg ccggaagaag ctgtggtga 2019
<210> 105
   <211> 2016
   <212> PRT
   <213> Homo sapiens
<400> 105
<210> 106
   <211> 612
   <212> PRT
   <213> Homo sapiens
<400> 106
   Met Ala Trp Val Trp Thr Leu Leu Phe Leu Met Ala Ala Ala Gln Ser
   1 5 10 15
   Ile Gln Ala Gly Ser His His His His His His Asp Ala His Lys Ser
   20 25 30
   Glu Val Ala His Arg Phe Lys Asp Leu Gly Glu Glu Asn Phe Lys Ala
   35 40 45
   Leu Val Leu Ile Ala Phe Ala Gln Tyr Leu Gln Gln Cys Pro Phe Glu
   50 55 60
   Asp His Val Lys Leu Val Asn Glu Val Thr Glu Phe Ala Lys Thr Cys
   65 70 75 80
   Val Ala Asp Glu Ser Ala Glu Asn Cys Asp Lys Ser Leu His Thr Leu
                   85                  90                  95
   Phe Gly Asp Lys Leu Cys Thr Val Ala Thr Leu Arg Glu Thr Tyr Gly
               100                 105                 110
   Glu Met Ala Asp Cys Cys Ala Lys Gln Glu Pro Glu Arg Asn Glu Cys
           115                 120                 125
   Phe Leu Gln His Lys Asp Asp Asn Pro Asn Leu Pro Arg Leu Val Arg
       130                 135                 140
   Pro Glu Val Asp Val Met Cys Thr Ala Phe His Asp Asn Glu Glu Thr
   145                 150                 155                 160
   Phe Leu Lys Lys Tyr Leu Tyr Glu Ile Ala Arg Arg His Pro Tyr Phe
                   165                 170                 175
   Tyr Ala Pro Glu Leu Leu Phe Phe Ala Lys Arg Tyr Lys Ala Ala Phe
               180                 185                 190
   Thr Glu Cys Cys Gln Ala Ala Asp Lys Ala Ala Cys Leu Leu Pro Lys
           195                 200                 205
   Leu Asp Glu Leu Arg Asp Glu Gly Lys Ala Ser Ser Ala Lys Gln Arg
       210                 215                 220
   Leu Lys Cys Ala Ser Leu Gln Lys Phe Gly Glu Arg Ala Phe Lys Ala
   225                 230                 235                 240
   Trp Ala Val Ala Arg Leu Ser Gln Arg Phe Pro Lys Ala Glu Phe Ala
                   245                 250                 255
   Glu Val Ser Lys Leu Val Thr Asp Leu Thr Lys Val His Thr Glu Cys
               260                 265                 270
   Cys His Gly Asp Leu Leu Glu Cys Ala Asp Asp Arg Ala Asp Leu Ala
           275                 280                 285
   Lys Tyr Ile Cys Glu Asn Gln Asp Ser Ile Ser Ser Lys Leu Lys Glu
       290                 295                 300
   Cys Cys Glu Lys Pro Leu Leu Glu Lys Ser His Cys Ile Ala Glu Val
   305                 310                 315                 320
   Glu Asn Asp Glu Met Pro Ala Asp Leu Pro Ser Leu Ala Ala Asp Phe
   325 330 335
   Val Glu Ser Lys Asp Val Cys Lys Asn Tyr Ala Glu Ala Lys Asp Val
   340 345 350
   Phe Leu Gly Met Phe Leu Tyr Glu Tyr Ala Arg Arg His Pro Asp Tyr
   355 360 365
   Ser Val Val Leu Leu Leu Arg Leu Ala Lys Thr Tyr Glu Thr Thr Leu
   370 375 380
   Glu Lys Cys Cys Ala Ala Ala Asp Pro His Glu Cys Tyr Ala Lys Val
   385 390 395 400
   Phe Asp Glu Phe Lys Pro Leu Val Glu Glu Pro Gln Asn Leu Ile Lys
   405 410 415
   Gln Asn Cys Glu Leu Phe Glu Gln Leu Gly Glu Tyr Lys Phe Gln Asn
   420 425 430
   Ala Leu Leu Val Arg Tyr Thr Lys Lys Val Pro Gln Val Ser Thr Pro
   435 440 445
   Thr Leu Val Glu Val Ser Arg Asn Leu Gly Lys Val Gly Ser Lys Cys
   450 455 460
   Cys Lys His Pro Glu Ala Lys Arg Met Pro Cys Ala Glu Asp Tyr Leu
   465 470 475 480
   Ser Val Val Leu Asn Gln Leu Cys Val Leu His Glu Lys Thr Pro Val
   485 490 495
   Ser Asp Arg Val Thr Lys Cys Cys Thr Glu Ser Leu Val Asn Arg Arg
   500 505 510
   Pro Cys Phe Ser Ala Leu Glu Val Asp Glu Thr Tyr Val Pro Lys Glu
   515 520 525
   Phe Asn Ala Glu Thr Phe Thr Phe His Ala Asp Ile Cys Thr Leu Ser
   530 535 540
   Glu Lys Glu Arg Gln Ile Lys Lys Gln Thr Ala Leu Val Glu Leu Val
   545 550 555 560
   Lys His Lys Pro Lys Ala Thr Lys Glu Gln Leu Lys Ala Val Met Asp
   565 570 575
   Asp Phe Ala Ala Phe Val Glu Lys Cys Cys Lys Ala Asp Asp Lys Glu
   580 585 590
   Thr Cys Phe Ala Glu Glu Gly Lys Lys Leu Val Ala Ala Ser Gln Ala
   595 600 605
   Ala Leu Gly Leu
   610
<210> 107
   <211> 93
   <212> DNA
   <213> Thrixopelma pruriens
<400> 107
   tactgccaga agtggatgtg gacatgcgac agcgagcgca agtgctgcga aggcatggtg 60
   tgtcgcctgt ggtgtaaaaa gaagttgtgg tga 93
<210> 108
   <211> 6
   <212> PRT
   <213> Artificial Sequence
<220>
   <223> Description of Artificial Sequence: Synthetic 6xHis tag
<400> 108
   His His His His His His
   1 5
<210> 109
   <211> 30
   <212> PRT
   <213> Artificial sequence
<220>
   <223> variant (-GP) NV1D2773
<220>
   <221> MOD_RES
   <222> (30)..(30)
   <223> -COOH
<400> 109
   Ser Cys Gln Lys Trp Met Gln Thr Cys Asp Ala Glu Arg Lys Cys Cys
   1 5 10 15
   Glu Gly Phe Val Cys Arg Leu Trp Cys Lys Lys Lys Leu Trp
   20 25 30
<210> 110
   <211> 30
   <212> PRT
   <213> Artificial sequence
<220>
   <223> Variant (-GP) NV1D2773-NH2
<220>
   <221> MOD_RES
   <222> (30)..(30)
   <223> -NH2
<400> 110
   Ser Cys Gln Lys Trp Met Gln Thr Cys Asp Ala Glu Arg Lys Cys Cys
   1               5                   10                  15
   Glu Gly Phe Val Cys Arg Leu Trp Cys Lys Lys Lys Leu Trp
               20                  25                  30
<210> 111
   <211> 32
   <212> PRT
   <213> Artificial sequence
<220>
   <223> Variant NV1D2775-NH2
<220>
   <221> MOD_RES
   <222> (32)..(32)
   <223> -NH2
<400> 111
   Gly Pro Gln Cys Gln Lys Trp Met Gln Thr Cys Asp Ala Glu Arg Lys
   1               5                   10                  15
   Cys Cys Glu Gly Phe Val Cys Arg Leu Trp Cys Lys Lys Lys Leu Trp
               20                  25                  30
<210> 112
   <211> 30
   <212> PRT
   <213> Artificial sequence
<220>
   <223> Variant NV1D2890-NH2
<220>
   <221> MOD_RES
   <222> (30)..(30)
   <223> -NH2
<400> 112
   Ser Cys Gln Lys Trp Phe Trp Thr Cys Asp Ala Glu Arg Lys Cys Cys
   1 5 10 15
   Glu Gly Leu Val Cys Arg Leu Trp Cys Lys Lys Lys Leu Trp
   20 25 30
<210> 113
   <211> 32
   <212> PRT
   <213> Artificial sequence
<220>
   <223> Variant NV1D2974
<220>
   <221> MOD_RES
   <222> (32)..(32)
   <223> -COOH
<400> 113
   Gly Pro Gln Cys Gln Lys Trp Met Gln Thr Cys Asp Ala Glu Arg Lys
   1 5 10 15
   Cys Cys Glu Gly Phe Ser Cys Thr Leu Trp Cys Lys Lys Lys Leu Trp
   20 25 30
<210> 114
   <211> 30
   <212> PRT
   <213> Artificial sequence
<220>
   <223> Variant (-GP) N-Ac-NV1D2974-NH2
<220>
   <221> MOD_RES
   <222> (1)..(1)
   <223> ACETYLATION
<220>
   <221> MOD_RES
   <222> (30)..(30)
   <223> -NH2
<400> 114
   Gln Cys Gln Lys Trp Met Gln Thr Cys Asp Ala Glu Arg Lys Cys Cys
   1 5 10 15
   Glu Gly Phe Ser Cys Thr Leu Trp Cys Lys Lys Lys Leu Trp
   20 25 30
<210> 115
   <211> 30
   <212> PRT
   <213> Artificial sequence
<220>
   <223> Variant (-GP) N-Ac-NV1D2974
<220>
   <221> MOD_RES
   <222> (1)..(1)
   <223> ACETYLATION
<220>
   <221> MOD_RES
   <222> (30)..(30)
   <223> -NH2
<400> 115
   Gln Cys Gln Lys Trp Met Gln Thr Cys Asp Ala Glu Arg Lys Cys Cys
   1               5                   10                  15
   Glu Gly Phe Ser Cys Thr Leu Trp Cys Lys Lys Lys Leu Trp
               20                  25                  30
<210> 116
   <211> 32
   <212> PRT
   <213> Artificial sequence
<220>
   <223> Variant NV1D3034
<220>
   <221> MOD_RES
   <222> (32)..(32)
   <223> -COOH
<400> 116
   Gly Pro Gln Cys Gln Lys Trp Met Gln Thr Cys Asp Arg Glu Arg Lys
   1               5                   10                  15
   Cys Cys Glu Gly Phe Val Cys Thr Leu Trp Cys Arg Lys Lys Leu Trp
               20                  25                  30
<210> 117
   <211> 32
   <212> PRT
   <213> Artificial sequence
<220>
   <223> Variant NV1D3034-NH2
<220>
   <221> MOD_RES
   <222> (32)..(32)
   <223> -NH2
<400> 117
   Gly Pro Gln Cys Gln Lys Trp Met Gln Thr Cys Asp Arg Glu Arg Lys
   1               5                   10                  15
   Cys Cys Glu Gly Phe Val Cys Thr Leu Trp Cys Arg Lys Lys Leu Trp
               20                  25                  30
<210> 118
   <211> 32
   <212> PRT
   <213> Artificial sequence
<220>
   <223> NV1D3034-NH-butyl
<220>
   <221> MOD_RES
   <222> (32)..(32)
   <223> NH-CH2-CH2-CH2-CH3
<400> 118
   Gly Pro Gln Cys Gln Lys Trp Met Gln Thr Cys Asp Arg Glu Arg Lys
   1               5                   10                  15
   Cys Cys Glu Gly Phe Val Cys Thr Leu Trp Cys Arg Lys Lys Leu Trp
               20                  25                  30
<210> 119
   <211> 32
   <212> PRT
   <213> Artificial sequence
<220>
   <223> Variant NV1D3034-NH-methyl
<220>
   <221> MOD_RES
   <222> (32)..(32)
   <223> -NH-CH3
<400> 119
   Gly Pro Gln Cys Gln Lys Trp Met Gln Thr Cys Asp Arg Glu Arg Lys
   1               5                   10                  15
   Cys Cys Glu Gly Phe Val Cys Thr Leu Trp Cys Arg Lys Lys Leu Trp
               20                  25                  30
<210> 120
   <211> 30
   <212> PRT
   <213> Artificial sequence
<220>
   <223> Variant (-GP) N-Ac-NV1D3034
<220>
   <221> MOD_RES
   <222> (1)..(1)
   <223> ACETYLATION
<220>
   <221> MOD_RES
   <222> (30)..(30)
   <223> -COOH
<400> 120
   Gln Cys Gln Lys Trp Met Gln Thr Cys Asp Arg Glu Arg Lys Cys Cys
   1               5                   10                  15
   Glu Gly Phe Val Cys Thr Leu Trp Cys Arg Lys Lys Leu Trp
               20                  25                  30
<210> 121
   <211> 30
   <212> PRT
   <213> Artificial sequence
<220>
   <223> Variant (-GP) N-Ac-NV1D3034-NH2
<220>
   <221> MOD_RES
   <222> (1)..(1)
   <223> ACETYLATION
<220>
   <221> MOD_RES
   <222> (30)..(30)
   <223> -NH2
<400> 121
   Gln Cys Gln Lys Trp Met Gln Thr Cys Asp Arg Glu Arg Lys Cys Cys
   1               5                   10                  15
   Glu Gly Phe Val Cys Thr Leu Trp Cys Arg Lys Lys Leu Trp
               20                  25                  30
<210> 122
   <211> 32
   <212> PRT
   <213> Artificial sequence
<220>
   <223> Variant NV1D3368
<220>
   <221> MOD_RES
   <222> (32)..(32)
   <223> -COOH
<400> 122
   Gly Pro Gln Cys Gln Lys Trp Met Gln Thr Cys Asp Arg Thr Arg Lys
   1               5                   10                  15
   Cys Cys Glu Gly Phe Val Cys Thr Leu Trp Cys Arg Lys Lys Leu Trp
               20                  25                  30
<210> 123
   <211> 32
   <212> PRT
   <213> Artificial sequence
<220>
   <223> Variant NV1D3368-NH2
<220>
   <221> MOD_RES
   <222> (32)..(32)
   <223> -NH2
<400> 123
   Gly Pro Gln Cys Gln Lys Trp Met Gln Thr Cys Asp Arg Thr Arg Lys
   1               5                   10                  15
   Cys Cys Glu Gly Phe Val Cys Thr Leu Trp Cys Arg Lys Lys Leu Trp
               20                  25                  30
<210> 124
   <211> 32
   <212> PRT
   <213> Artificial sequence
<220>
   <223> Variant NV1D2969
<220>
   <221> MOD_RES
   <222> (32)..(32)
   <223> -COOH
<400> 124
   Gly Pro Ser Cys Gln Lys Trp Met Gln Thr Cys Asp Ala Glu Arg Lys
   1 5 10 15
   Cys Cys Glu Gly Phe Ser Cys Arg Leu Trp Cys Lys Lys Lys Leu Trp
   20 25 30
<210> 125
   <211> 32
   <212> PRT
   <213> Artificial sequence
<220>
   <223> Variant NV1D2970
<220>
   <221> MOD_RES
   <222> (32)..(32)
   <223> -COOH
<400> 125
   Gly Pro Ser Cys Gln Lys Trp Met Gln Thr Cys Asp Ala Glu Arg Lys
   1 5 10 15
   Cys Cys Glu Gly Phe Val Cys Thr Leu Trp Cys Lys Lys Lys Leu Trp
   20 25 30
<210> 126
   <211> 32
   <212> PRT
   <213> Artificial sequence
<220>
   <223> Variant NV1D2971
<220>
   <221> MOD_RES
   <222> (32)..(32)
   <223> -COOH
<400> 126
   Gly Pro Ser Cys Gln Lys Trp Met Gln Thr Cys Asp Ala Glu Arg Lys
   1 5 10 15
   Cys Cys Glu Gly Phe Ser Cys Thr Leu Trp Cys Lys Lys Lys Leu Trp
   20 25 30
<210> 127
   <211> 32
   <212> PRT
   <213> Artificial sequence
<220>
   <223> Variant NV1D2972
<220>
   <221> MOD_RES
   <222> (32)..(32)
   <223> -COOH
<400> 127
   Gly Pro Gln Cys Gln Lys Trp Met Gln Thr Cys Asp Ala Glu Arg Lys
   1               5                   10                  15
   Cys Cys Glu Gly Phe Ser Cys Arg Leu Trp Cys Lys Lys Lys Leu Trp
               20                  25                  30
<210> 128
   <211> 32
   <212> PRT
   <213> Artificial sequence
<220>
   <223> Variant NV1D2973
<220>
   <221> MOD_RES
   <222> (32)..(32)
   <223> -COOH
<400> 128
   Gly Pro Gln Cys Gln Lys Trp Met Gln Thr Cys Asp Ala Glu Arg Lys
   1               5                   10                  15
   Cys Cys Glu Gly Phe Val Cys Thr Leu Trp Cys Lys Lys Lys Leu Trp
               20                  25                  30
<210> 129
   <211> 32
   <212> PRT
   <213> Artificial sequence
<220>
   <223> Variant NV1D2974
<220>
   <221> MOD_RES
   <222> (32)..(32)
   <223> -COOH
<400> 129
   Gly Pro Gln Cys Gln Lys Trp Met Gln Thr Cys Asp Ala Glu Arg Lys
   1               5                   10                  15
   Cys Cys Glu Gly Phe Ser Cys Thr Leu Trp Cys Lys Lys Lys Leu Trp
               20                  25                  30
<210> 130
   <211> 32
   <212> PRT
   <213> Artificial sequence
<220>
   <223> Variant NV1D2974-NH2
<220>
   <221> MOD_RES
   <222> (32)..(32)
   <223> -NH2
<400> 130
   Gly Pro Gln Cys Gln Lys Trp Met Gln Thr Cys Asp Ala Glu Arg Lys
   1               5                   10                  15
   Cys Cys Glu Gly Phe Ser Cys Thr Leu Trp Cys Lys Lys Lys Leu Trp
               20                  25                  30
<210> 131
   <211> 32
   <212> PRT
   <213> Artificial sequence
<220>
   <223> Variant NV1D3004
<220>
   <221> MOD_RES
   <222> (32)..(32)
   <223> -COOH
<400> 131
   Gly Pro Gln Cys Gln Lys Trp Met Gln Thr Cys Asp Arg Glu Arg Lys
   1               5                   10                  15
   Cys Cys Glu Gly Phe Val Cys Arg Leu Trp Cys Lys Lys Lys Leu Trp
               20                  25                  30
<210> 132
   <211> 32
   <212> PRT
   <213> Artificial sequence
<220>
   <223> Variant NV1D3005
<220>
   <221> MOD_RES
   <222> (32)..(32)
   <223> -COOH
<400> 132
   Gly Pro Gln Cys Gln Lys Trp Met Gln Thr Cys Asp Ala Asn Arg Lys
   1 5 10 15
   Cys Cys Glu Gly Phe Val Cys Arg Leu Trp Cys Lys Lys Lys Leu Trp
   20 25 30
<210> 133
   <211> 32
   <212> PRT
   <213> Artificial sequence
<220>
   <223> Variant NV1D3006
<220>
   <221> MOD_RES
   <222> (32)..(32)
   <223> -COOH
<400> 133
   Gly Pro Gln Cys Gln Lys Trp Met Gln Thr Cys Asp Ala Arg Arg Lys
   1 5 10 15
   Cys Cys Glu Gly Phe Val Cys Arg Leu Trp Cys Lys Lys Lys Leu Trp
   20 25 30
<210> 134
   <211> 32
   <212> PRT
   <213> Artificial sequence
<220>
   <223> Variant NV1D3007
<220>
   <221> MOD_RES
   <222> (32)..(32)
   <223> -COOH
<400> 134
   Gly Pro Gln Cys Gln Lys Trp Met Gln Thr Cys Asp Ala Glu Arg Lys
   1 5 10 15
   Cys Cys Glu Gly Phe Val Cys Arg Leu Trp Cys Arg Lys Lys Leu Trp
   20 25 30
<210> 135
   <211> 32
   <212> PRT
   <213> Artificial sequence
<220>
   <223> Variant NV1D3012
<220>
   <221> MOD_RES
   <222> (32)..(32)
   <223> -COOH
<400> 135
   Gly Pro Gln Cys Gln Lys Trp Met Gln Thr Cys Asp Arg Asn Arg Lys
   1               5                   10                  15
   Cys Cys Glu Gly Phe Val Cys Arg Leu Trp Cys Lys Lys Lys Leu Trp
               20                  25                  30
<210> 136
   <211> 32
   <212> PRT
   <213> Artificial sequence
<220>
   <223> Variant NV1D3013
<220>
   <221> MOD_RES
   <222> (32)..(32)
   <223> -COOH
<400> 136
   Gly Pro Gln Cys Gln Lys Trp Met Gln Thr Cys Asp Arg Arg Arg Lys
   1               5                   10                  15
   Cys Cys Glu Gly Phe Val Cys Arg Leu Trp Cys Lys Lys Lys Leu Trp
               20                  25                  30
<210> 137
   <211> 32
   <212> PRT
   <213> Artificial sequence
<220>
   <223> Variant NV1D3014
<220>
   <221> MOD_RES
   <222> (32)..(32)
   <223> -COOH
<400> 137
   Gly Pro Gln Cys Gln Lys Trp Met Gln Thr Cys Asp Arg Glu Arg Lys
   1 5 10 15
   Cys Cys Glu Gly Phe Val Cys Thr Leu Trp Cys Lys Lys Lys Leu Trp
   20 25 30
<210> 138
   <211> 32
   <212> PRT
   <213> Artificial sequence
<220>
   <223> Variant NV1D3015
<220>
   <221> MOD_RES
   <222> (32)..(32)
   <223> -COOH
<400> 138
   Gly Pro Gln Cys Gln Lys Trp Met Gln Thr Cys Asp Arg Glu Arg Lys
   1 5 10 15
   Cys Cys Glu Gly Phe Val Cys Arg Leu Trp Cys Arg Lys Lys Leu Trp
   20 25 30
<210> 139
   <211> 32
   <212> PRT
   <213> Artificial sequence
<220>
   <223> Variant NV1D3016
<220>
   <221> MOD_RES
   <222> (32)..(32)
   <223> -COOH
<400> 139
   Gly Pro Gln Cys Gln Lys Trp Met Gln Thr Cys Asp Ala Asn Arg Lys
   1 5 10 15
   Cys Cys Glu Gly Phe Val Cys Thr Leu Trp Cys Lys Lys Lys Leu Trp
   20 25 30
<210> 140
   <211> 32
   <212> PRT
   <213> Artificial sequence
<220>
   <223> Variant NV1D3017
<220>
   <221> MOD_RES
   <222> (32)..(32)
   <223> -COOH
<400> 140
   Gly Pro Gln Cys Gln Lys Trp Met Gln Thr Cys Asp Ala Arg Arg Lys
   1               5                   10                  15
   Cys Cys Glu Gly Phe Val Cys Thr Leu Trp Cys Lys Lys Lys Leu Trp
               20                  25                  30
<210> 141
   <211> 32
   <212> PRT
   <213> Artificial sequence
<220>
   <223> Variant NV1D3018
<220>
   <221> MOD_RES
   <222> (32)..(32)
   <223> -COOH
<400> 141
   Gly Pro Gln Cys Gln Lys Trp Met Gln Thr Cys Asp Ala Asn Arg Lys
   1               5                   10                  15
   Cys Cys Glu Gly Phe Val Cys Arg Leu Trp Cys Arg Lys Lys Leu Trp
               20                  25                  30
<210> 142
   <211> 32
   <212> PRT
   <213> Artificial sequence
<220>
   <223> Variant NV1D3019
<220>
   <221> MOD_RES
   <222> (32)..(32)
   <223> -COOH
<400> 142
   Gly Pro Gln Cys Gln Lys Trp Met Gln Thr Cys Asp Ala Arg Arg Lys
   1               5                   10                  15
   Cys Cys Glu Gly Phe Val Cys Arg Leu Trp Cys Arg Lys Lys Leu Trp
               20                  25                  30
<210> 143
   <211> 32
   <212> PRT
   <213> Artificial sequence
<220>
   <223> Variant NV1D3020
<220>
   <221> MOD_RES
   <222> (32)..(32)
   <223> -COOH
<400> 143
   Gly Pro Gln Cys Gln Lys Trp Met Gln Thr Cys Asp Ala Glu Arg Lys
   1               5                   10                  15
   Cys Cys Glu Gly Phe Val Cys Thr Leu Trp Cys Arg Lys Lys Leu Trp
               20                  25                  30
<210> 144
   <211> 32
   <212> PRT
   <213> Artificial sequence
<220>
   <223> Variant NV1D3030
<220>
   <221> MOD_RES
   <222> (32)..(32)
   <223> -COOH
<400> 144
   Gly Pro Gln Cys Gln Lys Trp Met Gln Thr Cys Asp Arg Asn Arg Lys
   1               5                   10                  15
   Cys Cys Glu Gly Phe Val Cys Thr Leu Trp Cys Lys Lys Lys Leu Trp
               20                  25                  30
<210> 145
   <211> 32
   <212> PRT
   <213> Artificial sequence
<220>
   <223> Variant NV1D3031
<220>
   <221> MOD_RES
   <222> (32)..(32)
   <223> -COOH
<400> 145
   Gly Pro Gln Cys Gln Lys Trp Met Gln Thr Cys Asp Arg Arg Arg Lys
   1 5 10 15
   Cys Cys Glu Gly Phe Val Cys Thr Leu Trp Cys Lys Lys Lys Leu Trp
   20 25 30
<210> 146
   <211> 32
   <212> PRT
   <213> Artificial sequence
<220>
   <223> Variant NV1D3032
<220>
   <221> MOD_RES
   <222> (32)..(32)
   <223> -COOH
<400> 146
   Gly Pro Gln Cys Gln Lys Trp Met Gln Thr Cys Asp Arg Asn Arg Lys
   1 5 10 15
   Cys Cys Glu Gly Phe Val Cys Arg Leu Trp Cys Arg Lys Lys Leu Trp
   20 25 30
<210> 147
   <211> 32
   <212> PRT
   <213> Artificial sequence
<220>
   <223> Variant NV1D3033
<220>
   <221> MOD_RES
   <222> (32)..(32)
   <223> -COOH
<400> 147
   Gly Pro Gln Cys Gln Lys Trp Met Gln Thr Cys Asp Arg Arg Arg Lys
   1 5 10 15
   Cys Cys Glu Gly Phe Val Cys Arg Leu Trp Cys Arg Lys Lys Leu Trp
   20 25 30
<210> 148
   <211> 32
   <212> PRT
   <213> Artificial sequence
<220>
   <223> Variant NV1D3035
<220>
   <221> MOD_RES
   <222> (32)..(32)
   <223> -COOH
<400> 148
   Gly Pro Gln Cys Gln Lys Trp Met Gln Thr Cys Asp Ala Asn Arg Lys
   1               5                   10                  15
   Cys Cys Glu Gly Phe Val Cys Thr Leu Trp Cys Arg Lys Lys Leu Trp
               20                  25                  30
<210> 149
   <211> 32
   <212> PRT
   <213> Artificial sequence
<220>
   <223> Variant NV1D3036
<220>
   <221> MOD_RES
   <222> (32)..(32)
   <223> -COOH
<400> 149
   Gly Pro Gln Cys Gln Lys Trp Met Gln Thr Cys Asp Ala Arg Arg Lys
   1               5                   10                  15
   Cys Cys Glu Gly Phe Val Cys Thr Leu Trp Cys Arg Lys Lys Leu Trp
               20                  25                  30
<210> 150
   <211> 32
   <212> PRT
   <213> Artificial sequence
<220>
   <223> Variant NV1D3044
<220>
   <221> MOD_RES
   <222> (32)..(32)
   <223> -COOH
<400> 150
   Gly Pro Gln Cys Gln Lys Trp Met Gln Thr Cys Asp Arg Asn Arg Lys
   1               5                   10                  15
   Cys Cys Glu Gly Phe Val Cys Thr Leu Trp Cys Arg Lys Lys Leu Trp
               20                  25                  30
<210> 151
   <211> 32
   <212> PRT
   <213> Artificial sequence
<220>
   <223> Variant NV1D3045
<220>
   <221> MOD_RES
   <222> (32)..(32)
   <223> -COOH
<400> 151
   Gly Pro Gln Cys Gln Lys Trp Met Gln Thr Cys Asp Arg Arg Arg Lys
   1               5                   10                  15
   Cys Cys Glu Gly Phe Val Cys Thr Leu Trp Cys Arg Lys Lys Leu Trp
               20                  25                  30
<210> 152
   <211> 32
   <212> PRT
   <213> Artificial sequence
<220>
   <223> Variant NV1D3048
<220>
   <221> MOD_RES
   <222> (32)..(32)
   <223> -COOH
<400> 152
   Gly Pro Gln Cys Gln Lys Trp Met Gln Thr Cys Asp Ala Lys Arg Lys
   1               5                   10                  15
   Cys Cys Glu Gly Phe Val Cys Arg Leu Trp Cys Lys Lys Lys Leu Trp
               20                  25                  30
<210> 153
   <211> 32
   <212> PRT
   <213> Artificial sequence
<220>
   <223> Variant NV1D3050
<220>
   <221> MOD_RES
   <222> (32)..(32)
   <223> -COOH
<400> 153
   Gly Pro Gln Cys Gln Lys Trp Met Gln Thr Cys Asp Arg Lys Arg Lys
   1               5                   10                  15
   Cys Cys Glu Gly Phe Val Cys Arg Leu Trp Cys Lys Lys Lys Leu Trp
               20                  25                  30
<210> 154
   <211> 32
   <212> PRT
   <213> Artificial sequence
<220>
   <223> Variant NV1D3051
<220>
   <221> MOD_RES
   <222> (32)..(32)
   <223> -COOH
<400> 154
   Gly Pro Gln Cys Gln Lys Trp Met Gln Thr Cys Asp Ala Lys Arg Lys
   1               5                   10                  15
   Cys Cys Glu Gly Phe Val Cys Thr Leu Trp Cys Lys Lys Lys Leu Trp
               20                  25                  30
<210> 155
   <211> 32
   <212> PRT
   <213> Artificial sequence
<220>
   <223> Variant NV1D3052
<220>
   <221> MOD_RES
   <222> (32)..(32)
   <223> -COOH
<400> 155
   Gly Pro Gln Cys Gln Lys Trp Met Gln Thr Cys Asp Ala Lys Arg Lys
   1               5                   10                  15
   Cys Cys Glu Gly Phe Val Cys Arg Leu Trp Cys Arg Lys Lys Leu Trp
               20                  25                  30
<210> 156
   <211> 32
   <212> PRT
   <213> Artificial sequence
<220>
   <223> Variant NV1D3056
<220>
   <221> MOD_RES
   <222> (32)..(32)
   <223> -COOH
<400> 156
   Gly Pro Gln Cys Gln Lys Trp Met Gln Thr Cys Asp Arg Lys Arg Lys
   1               5                   10                  15
   Cys Cys Glu Gly Phe Val Cys Thr Leu Trp Cys Lys Lys Lys Leu Trp
               20                  25                  30
<210> 157
   <211> 32
   <212> PRT
   <213> Artificial sequence
<220>
   <223> Variant NV1D3057
<220>
   <221> MOD_RES
   <222> (32)..(32)
   <223> -COOH
<400> 157
   Gly Pro Gln Cys Gln Lys Trp Met Gln Thr Cys Asp Arg Lys Arg Lys
   1               5                   10                  15
   Cys Cys Glu Gly Phe Val Cys Arg Leu Trp Cys Arg Lys Lys Leu Trp
               20                  25                  30
<210> 158
   <211> 32
   <212> PRT
   <213> Artificial sequence
<220>
   <223> Variant NV1D3058
<220>
   <221> MOD_RES
   <222> (32)..(32)
   <223> -COOH
<400> 158
   Gly Pro Gln Cys Gln Lys Trp Met Gln Thr Cys Asp Ala Lys Arg Lys
   1 5 10 15
   Cys Cys Glu Gly Phe Val Cys Thr Leu Trp Cys Arg Lys Lys Leu Trp
   20 25 30
<210> 159
   <211> 32
   <212> PRT
   <213> Artificial sequence
<220>
   <223> Variant NV1D3062
<220>
   <221> MOD_RES
   <222> (32)..(32)
   <223> -COOH
<400> 159
   Gly Pro Gln Cys Gln Lys Trp Met Gln Thr Cys Asp Arg Lys Arg Lys
   1 5 10 15
   Cys Cys Glu Gly Phe Val Cys Thr Leu Trp Cys Arg Lys Lys Leu Trp
   20 25 30
<210> 160
   <211> 32
   <212> PRT
   <213> Artificial sequence
<220>
   <223> Variant NV1D3109
<220>
   <221> MOD_RES
   <222> (32)..(32)
   <223> -COOH
<400> 160
   Gly Pro Gln Cys Gln Lys Trp Met Trp Thr Cys Asp Ala Arg Arg Lys
   1 5 10 15
   Cys Cys Glu Gly Phe Val Cys Thr Leu Trp Cys Arg Lys Lys Leu Trp
   20 25 30
<210> 161
   <211> 32
   <212> PRT
   <213> Artificial sequence
<220>
   <223> Variant NV1D3121
<220>
   <221> MOD_RES
   <222> (32)..(32)
   <223> -COOH
<400> 161
   Gly Pro Gln Cys Gln Lys Trp Met Trp Thr Cys Asp Arg Lys Arg Lys
   1               5                   10                  15
   Cys Cys Glu Gly Phe Val Cys Thr Leu Trp Cys Arg Lys Lys Leu Trp
               20                  25                  30
<210> 162
   <211> 37
   <212> PRT
   <213> Artificial sequence
<220>
   <223> Variant NV1D3249
<220>
   <221> MOD_RES
   <222> (37)..(37)
   <223> -COOH
<400> 162
   Gly Pro Ala Ala Ala Ala Ala Gln Cys Gln Lys Trp Met Gln Thr Cys
   1               5                   10                  15
   Asp Ala Glu Arg Lys Cys Cys Glu Gly Phe Val Cys Arg Leu Trp Cys
               20                  25                  30
   Lys Lys Lys Leu Trp
           35
<210> 163
   <211> 37
   <212> PRT
   <213> Artificial sequence
<220>
   <223> Variant NV1D3251
<220>
   <221> MOD_RES
   <222> (37)..(37)
   <223> -COOH
<400> 163
   Gly Pro Ala Pro Ala Pro Ala Gln Cys Gln Lys Trp Met Gln Thr Cys
   1               5                   10                  15
   Asp Ala Glu Arg Lys Cys Cys Glu Gly Phe Val Cys Arg Leu Trp Cys
               20                  25                  30
   Lys Lys Lys Leu Trp
           35
<210> 164
   <211> 37
   <212> PRT
   <213> Artificial sequence
<220>
   <223> Variant NV1D3252
<220>
   <221> MOD_RES
   <222> (37)..(37)
   <223> -COOH
<400> 164
   Gly Pro Gln Cys Gln Lys Trp Met Gln Thr Cys Asp Ala Glu Arg Lys
   1               5                   10                  15
   Cys Cys Glu Gly Phe Val Cys Arg Leu Trp Cys Lys Lys Lys Leu Trp
               20                  25                  30
   Ala Pro Ala Pro Ala
           35
<210> 165
   <211> 37
   <212> PRT
   <213> Artificial sequence
<220>
   <223> Variant NV1D3254
<220>
   <221> MOD_RES
   <222> (37)..(37)
   <223> -COOH
<400> 165
   Gly Pro Gln Cys Gln Lys Trp Met Gln Thr Cys Asp Ala Glu Arg Lys
   1               5                   10                  15
   Cys Cys Glu Gly Phe Val Cys Arg Leu Trp Cys Lys Lys Lys Leu Trp
               20                  25                  30
   Gly Gly Gly Gly Gly
           35
<210> 166
   <211> 64
   <212> PRT
   <213> Artificial sequence
<220>
   <223> Variant NV1D3256
<220>
   <221> MOD_RES
   <222> (64)..(64)
   <223> -COOH
<400> 166
   Gly Pro Cys Cys Asn Cys Ser Ser Lys Trp Cys Arg Asp His Ser Arg
   1               5                   10                  15
   Cys Cys Gly Arg Gly Ser Ala Pro Ala Pro Ala Pro Ala Pro Ala Pro
               20                  25                  30
   Gly Ser Gln Cys Gln Lys Trp Met Gln Thr Cys Asp Ala Glu Arg Lys
           35                  40                  45
   Cys Cys Glu Gly Phe Val Cys Arg Leu Trp Cys Lys Lys Lys Leu Trp
       50                  55                  60
<210> 167
   <211> 62
   <212> PRT
   <213> Artificial sequence
<220>
   <223> Variant NV1D3257
<220>
   <221> MOD_RES
   <222> (62)..(62)
   <223> -COOH
<400> 167
   Gly Pro Gln Cys Gln Lys Trp Met Gln Thr Cys Asp Ala Glu Arg Lys
   1 5 10 15
   Cys Cys Glu Gly Phe Val Cys Arg Leu Trp Cys Lys Lys Lys Leu Trp
   20 25 30
   Gly Ser Ala Pro Ala Pro Ala Pro Ala Pro Ala Pro Gly Ser Cys Cys
   35 40 45
   Asn Cys Ser Ser Lys Trp Cys Arg Asp His Ser Arg Cys Cys
   50 55 60
<210> 168
   <211> 74
   <212> PRT
   <213> Artificial sequence
<220>
   <223> Variant NV1D3259
<220>
   <221> MOD_RES
   <222> (74)..(74)
   <223> -COOH
<400> 168
   Gly Pro Gln Cys Gln Lys Trp Met Gln Thr Cys Asp Ala Glu Arg Lys
   1 5 10 15
   Cys Cys Glu Gly Phe Val Cys Arg Leu Trp Cys Lys Lys Lys Leu Trp
   20 25 30
   Gly Ser Ala Pro Ala Pro Ala Pro Ala Pro Ala Pro Ala Pro Ala Pro
   35 40 45
   Ala Pro Ala Pro Ala Pro Gly Ser Cys Cys Asn Cys Ser Ser Lys Trp
   50 55 60
   Cys Arg Asp His Ser Arg Cys Cys Gly Arg
   65 70
<210> 169
   <211> 74
   <212> PRT
   <213> Artificial sequence
<220>
   <223> Variant NV1D3260
<220>
   <221> MOD_RES
   <222> (74)..(74)
   <223> -COOH
<400> 169
   Gly Pro Cys Cys Asn Cys Ser Ser Lys Trp Cys Arg Asp His Ser Arg
   1               5                   10                  15
   Cys Cys Gly Arg Gly Ser Ala Pro Ala Pro Ala Pro Ala Pro Ala Pro
               20                  25                  30
   Ala Pro Ala Pro Ala Pro Ala Pro Ala Pro Gly Ser Gln Cys Gln Lys
           35                  40                  45
   Trp Met Gln Thr Cys Asp Ala Glu Arg Lys Cys Cys Glu Gly Phe Val
       50                  55                  60
   Cys Arg Leu Trp Cys Lys Lys Lys Leu Trp
   65                  70
<210> 170
   <211> 72
   <212> PRT
   <213> Artificial sequence
<220>
   <223> Variant NV1D3261
<220>
   <221> MOD_RES
   <222> (72)..(72)
   <223> -COOH
<400> 170
   Gly Pro Gln Cys Gln Lys Trp Met Gln Thr Cys Asp Ala Glu Arg Lys
   1               5                   10                  15
   Cys Cys Glu Gly Phe Val Cys Arg Leu Trp Cys Lys Lys Lys Leu Trp
               20                  25                  30
   Gly Ser Ala Pro Ala Pro Ala Pro Ala Pro Ala Pro Ala Pro Ala Pro
           35                  40                  45
   Ala Pro Ala Pro Ala Pro Gly Ser Cys Cys Asn Cys Ser Ser Lys Trp
       50                  55                  60
   Cys Arg Asp His Ser Arg Cys Cys
   65                  70
<210> 171
   <211> 72
   <212> PRT
   <213> Artificial sequence
<220>
   <223> Variant NV1D3262
<220>
   <221> MOD_RES
   <222> (72)..(72)
   <223> -COOH
<400> 171
   Gly Pro Cys Cys Asn Cys Ser Ser Lys Trp Cys Arg Asp His Ser Arg
   1               5                   10                  15
   Cys Cys Gly Ser Ala Pro Ala Pro Ala Pro Ala Pro Ala Pro Ala Pro
               20                  25                  30
   Ala Pro Ala Pro Ala Pro Ala Pro Gly Ser Gln Cys Gln Lys Trp Met
           35                  40                  45
   Gln Thr Cys Asp Ala Glu Arg Lys Cys Cys Glu Gly Phe Val Cys Arg
       50                  55                  60
   Leu Trp Cys Lys Lys Lys Leu Trp
   65                  70
<210> 172
   <211> 32
   <212> PRT
   <213> Artificial sequence
<220>
   <223> Variant NV1D3339
<220>
   <221> MOD_RES
   <222> (32)..(32)
   <223> -COOH
<400> 172
   Gly Pro Gln Cys Arg Lys Trp Met Gln Thr Cys Asp Arg Glu Arg Lys
   1               5                   10                  15
   Cys Cys Glu Gly Phe Val Cys Thr Leu Trp Cys Arg Lys Lys Leu Trp
               20                  25                  30
<210> 173
   <211> 32
   <212> PRT
   <213> Artificial sequence
<220>
   <223> Variant NV1D3340
<220>
   <221> MOD_RES
   <222> (32)..(32)
   <223> -COOH
<400> 173
   Gly Pro Gln Cys Lys Lys Trp Met Gln Thr Cys Asp Arg Glu Arg Lys
   1               5                   10                  15
   Cys Cys Glu Gly Phe Val Cys Thr Leu Trp Cys Arg Lys Lys Leu Trp
               20                  25                  30
<210> 174
   <211> 32
   <212> PRT
   <213> Artificial sequence
<220>
   <223> Variant NV1D3341
<220>
   <221> MOD_RES
   <222> (32)..(32)
   <223> -COOH
<400> 174
   Gly Pro Gln Cys Thr Lys Trp Met Gln Thr Cys Asp Arg Glu Arg Lys
   1               5                   10                  15
   Cys Cys Glu Gly Phe Val Cys Thr Leu Trp Cys Arg Lys Lys Leu Trp
               20                  25                  30
<210> 175
   <211> 32
   <212> PRT
   <213> Artificial sequence
<220>
   <223> Variant NV1D3342
<220>
   <221> MOD_RES
   <222> (32)..(32)
   <223> -COOH
<400> 175
   Gly Pro Gln Cys Ala Lys Trp Met Gln Thr Cys Asp Arg Glu Arg Lys
   1               5                   10                  15
   Cys Cys Glu Gly Phe Val Cys Thr Leu Trp Cys Arg Lys Lys Leu Trp
               20                  25                  30
<210> 176
   <211> 32
   <212> PRT
   <213> Artificial sequence
<220>
   <223> Variant NV1D3344
<220>
   <221> MOD_RES
   <222> (32)..(32)
   <223> -COOH
<400> 176
   Gly Pro Gln Cys Glu Lys Trp Met Gln Thr Cys Asp Arg Glu Arg Lys
   1               5                   10                  15
   Cys Cys Glu Gly Phe Val Cys Thr Leu Trp Cys Arg Lys Lys Leu Trp
               20                  25                  30
<210> 177
   <211> 32
   <212> PRT
   <213> Artificial sequence
<220>
   <223> Variant NV1D3345
<220>
   <221> MOD_RES
   <222> (32)..(32)
   <223> -COOH
<400> 177
   Gly Pro Gln Cys Ser Lys Trp Met Gln Thr Cys Asp Arg Glu Arg Lys
   1               5                   10                  15
   Cys Cys Glu Gly Phe Val Cys Thr Leu Trp Cys Arg Lys Lys Leu Trp
               20                  25                  30
<210> 178
   <211> 32
   <212> PRT
   <213> Artificial sequence
<220>
   <223> Variant NV1D3346
<220>
   <221> MOD_RES
   <222> (32)..(32)
   <223> -COOH
<400> 178
   Gly Pro Gln Cys Gln Arg Trp Met Gln Thr Cys Asp Arg Glu Arg Lys
   1               5                   10                  15
   Cys Cys Glu Gly Phe Val Cys Thr Leu Trp Cys Arg Lys Lys Leu Trp
               20                  25                  30
<210> 179
   <211> 32
   <212> PRT
   <213> Artificial sequence
<220>
   <223> Variant NV1D3347
<220>
   <221> MOD_RES
   <222> (32)..(32)
   <223> -COOH
<400> 179
   Gly Pro Gln Cys Gln Thr Trp Met Gln Thr Cys Asp Arg Glu Arg Lys
   1               5                   10                  15
   Cys Cys Glu Gly Phe Val Cys Thr Leu Trp Cys Arg Lys Lys Leu Trp
               20                  25                  30
<210> 180
   <211> 32
   <212> PRT
   <213> Artificial sequence
<220>
   <223> Variant NV1D3348
<220>
   <221> MOD_RES
   <222> (32)..(32)
   <223> -COOH
<400> 180
   Gly Pro Gln Cys Gln Ala Trp Met Gln Thr Cys Asp Arg Glu Arg Lys
   1               5                   10                  15
   Cys Cys Glu Gly Phe Val Cys Thr Leu Trp Cys Arg Lys Lys Leu Trp
               20                  25                  30
<210> 181
   <211> 32
   <212> PRT
   <213> Artificial sequence
<220>
   <223> Variant NV1D3349
<220>
   <221> MOD_RES
   <222> (32)..(32)
   <223> -COOH
<400> 181
   Gly Pro Gln Cys Gln Asp Trp Met Gln Thr Cys Asp Arg Glu Arg Lys
   1               5                   10                  15
   Cys Cys Glu Gly Phe Val Cys Thr Leu Trp Cys Arg Lys Lys Leu Trp
               20                  25                  30
<211> 32
   <212> PRT
   <213> Artificial sequence
<220>
   <223> Variant NV1D3350
<220>
   <221> MOD_RES
   <222> (32)..(32)
   <223> -COOH
<400> 182
<210> 183
   <211> 32
   <212> PRT
   <213> Artificial sequence
<220>
   <223> Variant NV1D3351
<220>
   <221> MOD_RES
   <222> (32)..(32)
   <223> -COOH
<400> 183
   Gly Pro Gln Cys Gln Gln Trp Met Gln Thr Cys Asp Arg Glu Arg Lys
   1               5                   10                  15
   Cys Cys Glu Gly Phe Val Cys Thr Leu Trp Cys Arg Lys Lys Leu Trp
               20                  25                  30
<210> 184
   <211> 32
   <212> PRT
   <213> Artificial sequence
<220>
   <223> Variant NV1D3352
<220>
   <221> MOD_RES
   <222> (32)..(32)
   <223> -COOH
<400> 184
   Gly Pro Gln Cys Gln Ser Trp Met Gln Thr Cys Asp Arg Glu Arg Lys
   1               5                   10                  15
   Cys Cys Glu Gly Phe Val Cys Thr Leu Trp Cys Arg Lys Lys Leu Trp
               20                  25                  30
<210> 185
   <211> 32
   <212> PRT
   <213> Artificial sequence
<220>
   <223> Variant NV1D3353
<220>
   <221> MOD_RES
   <222> (32)..(32)
   <223> -COOH
<400> 185
   Gly Pro Gln Cys Gln Lys Trp Met Gln Arg Cys Asp Arg Glu Arg Lys
   1               5                   10                  15
   Cys Cys Glu Gly Phe Val Cys Thr Leu Trp Cys Arg Lys Lys Leu Trp
               20                  25                  30
<210> 186
   <211> 32
   <212> PRT
   <213> Artificial sequence
<220>
   <223> Variant NV1D3354
<220>
   <221> MOD_RES
   <222> (32)..(32)
   <223> -COOH
<400> 186
   Gly Pro Gln Cys Gln Lys Trp Met Gln Lys Cys Asp Arg Glu Arg Lys
   1               5                   10                  15
   Cys Cys Glu Gly Phe Val Cys Thr Leu Trp Cys Arg Lys Lys Leu Trp
               20                  25                  30
<210> 187
   <211> 32
   <212> PRT
   <213> Artificial sequence
<220>
   <223> Variant NV1D3356
<220>
   <221> MOD_RES
   <222> (32)..(32)
   <223> -COOH
<400> 187
   Gly Pro Gln Cys Gln Lys Trp Met Gln Asp Cys Asp Arg Glu Arg Lys
   1               5                   10                  15
   Cys Cys Glu Gly Phe Val Cys Thr Leu Trp Cys Arg Lys Lys Leu Trp
               20                  25                  30
<210> 188
   <211> 32
   <212> PRT
   <213> Artificial sequence
<220>
   <223> Variant NV1D3357
<220>
   <221> MOD_RES
   <222> (32)..(32)
   <223> -COOH
<400> 188
   Gly Pro Gln Cys Gln Lys Trp Met Gln Glu Cys Asp Arg Glu Arg Lys
   1 5 10 15
   Cys Cys Glu Gly Phe Val Cys Thr Leu Trp Cys Arg Lys Lys Leu Trp
   20 25 30
<210> 189
   <211> 32
   <212> PRT
   <213> Artificial sequence
<220>
   <223> Variant NV1D3358
<220>
   <221> MOD_RES
   <222> (32)..(32)
   <223> -COOH
<400> 189
   Gly Pro Gln Cys Gln Lys Trp Met Gln Gln Cys Asp Arg Glu Arg Lys
   1 5 10 15
   Cys Cys Glu Gly Phe Val Cys Thr Leu Trp Cys Arg Lys Lys Leu Trp
   20 25 30
<210> 190
   <211> 32
   <212> PRT
   <213> Artificial sequence
<220>
   <223> Variant NV1D3359
<220>
   <221> MOD_RES
   <222> (32)..(32)
   <223> -COOH
<400> 190
   Gly Pro Gln Cys Gln Lys Trp Met Gln Ser Cys Asp Arg Glu Arg Lys
   1 5 10 15
   Cys Cys Glu Gly Phe Val Cys Thr Leu Trp Cys Arg Lys Lys Leu Trp
   20 25 30
<210> 191
   <211> 32
   <212> PRT
   <213> Artificial sequence
<220>
   <223> Variant NV1D3360
<220>
   <221> MOD_RES
   <222> (32)..(32)
   <223> -COOH
<400> 191
   Gly Pro Gln Cys Gln Lys Trp Met Gln Thr Cys Arg Arg Glu Arg Lys
   1               5                   10                  15
   Cys Cys Glu Gly Phe Val Cys Thr Leu Trp Cys Arg Lys Lys Leu Trp
               20                  25                  30
<210> 192
   <211> 32
   <212> PRT
   <213> Artificial sequence
<220>
   <223> Variant NV1D3361
<220>
   <221> MOD_RES
   <222> (32)..(32)
   <223> -COOH
<400> 192
   Gly Pro Gln Cys Gln Lys Trp Met Gln Thr Cys Lys Arg Glu Arg Lys
   1               5                   10                  15
   Cys Cys Glu Gly Phe Val Cys Thr Leu Trp Cys Arg Lys Lys Leu Trp
               20                  25                  30
<210> 193
   <211> 32
   <212> PRT
   <213> Artificial sequence
<220>
   <223> Variant NV1D3362
<220>
   <221> MOD_RES
   <222> (32)..(32)
   <223> -COOH
<400> 193
   Gly Pro Gln Cys Gln Lys Trp Met Gln Thr Cys Thr Arg Glu Arg Lys
   1               5                   10                  15
   Cys Cys Glu Gly Phe Val Cys Thr Leu Trp Cys Arg Lys Lys Leu Trp
               20                  25                  30
<210> 194
   <211> 32
   <212> PRT
   <213> Artificial sequence
<220>
   <223> Variant NV1D3363
<220>
   <221> MOD_RES
   <222> (32)..(32)
   <223> -COOH
<400> 194
   Gly Pro Gln Cys Gln Lys Trp Met Gln Thr Cys Ala Arg Glu Arg Lys
   1               5                   10                  15
   Cys Cys Glu Gly Phe Val Cys Thr Leu Trp Cys Arg Lys Lys Leu Trp
               20                  25                  30
<210> 195
   <211> 32
   <212> PRT
   <213> Artificial sequence
<220>
   <223> Variant NV1D3365
<220>
   <221> MOD_RES
   <222> (32)..(32)
   <223> -COOH
<400> 195
   Gly Pro Gln Cys Gln Lys Trp Met Gln Thr Cys Gln Arg Glu Arg Lys
   1               5                   10                  15
   Cys Cys Glu Gly Phe Val Cys Thr Leu Trp Cys Arg Lys Lys Leu Trp
               20                  25                  30
<210> 196
   <211> 32
   <212> PRT
   <213> Artificial sequence
<220>
   <223> Variant NV1D3366
<220>
   <221> MOD_RES
   <222> (32)..(32)
   <223> -COOH
<400> 196
   Gly Pro Gln Cys Gln Lys Trp Met Gln Thr Cys Ser Arg Glu Arg Lys
   1               5                   10                  15
   Cys Cys Glu Gly Phe Val Cys Thr Leu Trp Cys Arg Lys Lys Leu Trp
               20                  25                  30
<210> 197
   <211> 32
   <212> PRT
   <213> Artificial sequence
<220>
   <223> Variant NV1D3367
<220>
   <221> MOD_RES
   <222> (32)..(32)
   <223> -COOH
<400> 197
   Gly Pro Gln Cys Gln Lys Trp Met Gln Thr Cys Asp Arg Gln Arg Lys
   1               5                   10                  15
   Cys Cys Glu Gly Phe Val Cys Thr Leu Trp Cys Arg Lys Lys Leu Trp
               20                  25                  30
<210> 198
   <211> 32
   <212> PRT
   <213> Artificial sequence
<220>
   <223> Variant NV1D3368
<220>
   <221> MOD_RES
   <222> (32)..(32)
   <223> -COOH
<400> 198
   Gly Pro Gln Cys Gln Lys Trp Met Gln Thr Cys Asp Arg Thr Arg Lys
   1               5                   10                  15
   Cys Cys Glu Gly Phe Val Cys Thr Leu Trp Cys Arg Lys Lys Leu Trp
               20                  25                  30
<210> 199
   <211> 32
   <212> PRT
   <213> Artificial sequence
<220>
   <223> Variant NV1D3369
<220>
   <221> MOD_RES
   <222> (32)..(32)
   <223> -COOH
<400> 199
   Gly Pro Gln Cys Gln Lys Trp Met Gln Thr Cys Asp Arg Glu Lys Lys
   1               5                   10                  15
   Cys Cys Glu Gly Phe Val Cys Thr Leu Trp Cys Arg Lys Lys Leu Trp
               20                  25                  30
<210> 200
   <211> 32
   <212> PRT
   <213> Artificial sequence
<220>
   <223> Variant NV1D3370
<220>
   <221> MOD_RES
   <222> (32)..(32)
   <223> -COOH
<400> 200
   Gly Pro Gln Cys Gln Lys Trp Met Gln Thr Cys Asp Arg Glu Thr Lys
   1               5                   10                  15
   Cys Cys Glu Gly Phe Val Cys Thr Leu Trp Cys Arg Lys Lys Leu Trp
               20                  25                  30
<210> 201
   <211> 32
   <212> PRT
   <213> Artificial sequence
<220>
   <223> Variant NV1D3371
<220>
   <221> MOD_RES
   <222> (32)..(32)
   <223> -COOH
<400> 201
   Gly Pro Gln Cys Gln Lys Trp Met Gln Thr Cys Asp Arg Glu Ala Lys
   1               5                   10                  15
   Cys Cys Glu Gly Phe Val Cys Thr Leu Trp Cys Arg Lys Lys Leu Trp
               20                  25                  30
<210> 202
   <211> 32
   <212> PRT
   <213> Artificial sequence
<220>
   <223> Variant NV1D3372
<220>
   <221> MOD_RES
   <222> (32)..(32)
   <223> -COOH
<400> 202
   Gly Pro Gln Cys Gln Lys Trp Met Gln Thr Cys Asp Arg Glu Asp Lys
   1               5                   10                  15
   Cys Cys Glu Gly Phe Val Cys Thr Leu Trp Cys Arg Lys Lys Leu Trp
               20                  25                  30
<211> 32
   <212> PRT
   <213> Artificial sequence
<220>
   <223> Variant NV1D3374
<220>
   <221> MOD_RES
   <222> (32)..(32)
   <223> -COOH
<400> 203
<210> 204
   <211> 32
   <212> PRT
   <213> Artificial sequence
<220>
   <223> Variant NV1D3375
<220>
   <221> MOD_RES
   <222> (32)..(32)
   <223> -COOH
<400> 204
   Gly Pro Gln Cys Gln Lys Trp Met Gln Thr Cys Asp Arg Glu Ser Lys
   1               5                   10                  15
   Cys Cys Glu Gly Phe Val Cys Thr Leu Trp Cys Arg Lys Lys Leu Trp
               20                  25                  30
<210> 205
   <211> 32
   <212> PRT
   <213> Artificial sequence
<220>
   <223> Variant NV1D3376
<220>
   <221> MOD_RES
   <222> (32)..(32)
   <223> -COOH
<400> 205
   Gly Pro Gln Cys Gln Lys Trp Met Gln Thr Cys Asp Arg Glu Arg Arg
   1               5                   10                  15
   Cys Cys Glu Gly Phe Val Cys Thr Leu Trp Cys Arg Lys Lys Leu Trp
               20                  25                  30
<210> 206
   <211> 32
   <212> PRT
   <213> Artificial sequence
<220>
   <223> Variant NV1D3377
<220>
   <221> MOD_RES
   <222> (32)..(32)
   <223> -COOH
<400> 206
   Gly Pro Gln Cys Gln Lys Trp Met Gln Thr Cys Asp Arg Glu Arg Thr
   1               5                   10                  15
   Cys Cys Glu Gly Phe Val Cys Thr Leu Trp Cys Arg Lys Lys Leu Trp
               20                  25                  30
<210> 207
   <211> 32
   <212> PRT
   <213> Artificial sequence
<220>
   <223> Variant NV1D3378
<220>
   <221> MOD_RES
   <222> (32)..(32)
   <223> -COOH
<400> 207
   Gly Pro Gln Cys Gln Lys Trp Met Gln Thr Cys Asp Arg Glu Arg Ala
   1               5                   10                  15
   Cys Cys Glu Gly Phe Val Cys Thr Leu Trp Cys Arg Lys Lys Leu Trp
               20                  25                  30
<210> 208
   <211> 32
   <212> PRT
   <213> Artificial sequence
<220>
   <223> Variant NV1D3379
<220>
   <221> MOD_RES
   <222> (32)..(32)
   <223> -COOH
<400> 208
   Gly Pro Gln Cys Gln Lys Trp Met Gln Thr Cys Asp Arg Glu Arg Asp
   1               5                   10                  15
   Cys Cys Glu Gly Phe Val Cys Thr Leu Trp Cys Arg Lys Lys Leu Trp
               20                  25                  30
<210> 209
   <211> 32
   <212> PRT
   <213> Artificial sequence
<220>
   <223> Variant NV1D3380
<220>
   <221> MOD_RES
   <222> (32)..(32)
   <223> -COOH
<400> 209
   Gly Pro Gln Cys Gln Lys Trp Met Gln Thr Cys Asp Arg Glu Arg Glu
   1 5 10 15
   Cys Cys Glu Gly Phe Val Cys Thr Leu Trp Cys Arg Lys Lys Leu Trp
   20 25 30
<210> 210
   <211> 32
   <212> PRT
   <213> Artificial sequence
<220>
   <223> Variant NV1D3381
<220>
   <221> MOD_RES
   <222> (32)..(32)
   <223> -COOH
<400> 210
   Gly Pro Gln Cys Gln Lys Trp Met Gln Thr Cys Asp Arg Glu Arg Gln
   1 5 10 15
   Cys Cys Glu Gly Phe Val Cys Thr Leu Trp Cys Arg Lys Lys Leu Trp
   20 25 30
<210> 211
   <211> 32
   <212> PRT
   <213> Artificial sequence
<220>
   <223> Variant NV1D3382
<220>
   <221> MOD_RES
   <222> (32)..(32)
   <223> -COOH
<400> 211
   Gly Pro Gln Cys Gln Lys Trp Met Gln Thr Cys Asp Arg Glu Arg Ser
   1 5 10 15
   Cys Cys Glu Gly Phe Val Cys Thr Leu Trp Cys Arg Lys Lys Leu Trp
   20 25 30
<210> 212
   <211> 32
   <212> PRT
   <213> Artificial sequence
<220>
   <223> Variant NV1D3383
<220>
   <221> MOD_RES
   <222> (32)..(32)
   <223> -COOH
<400> 212
   Gly Pro Gln Cys Gln Lys Trp Met Gln Thr Cys Asp Arg Glu Arg Lys
   1               5                   10                  15
   Cys Cys Arg Gly Phe Val Cys Thr Leu Trp Cys Arg Lys Lys Leu Trp
               20                  25                  30
<210> 213
   <211> 32
   <212> PRT
   <213> Artificial sequence
<220>
   <223> Variant NV1D3384
<220>
   <221> MOD_RES
   <222> (32)..(32)
   <223> -COOH
<400> 213
   Gly Pro Gln Cys Gln Lys Trp Met Gln Thr Cys Asp Arg Glu Arg Lys
   1               5                   10                  15
   Cys Cys Lys Gly Phe Val Cys Thr Leu Trp Cys Arg Lys Lys Leu Trp
               20                  25                  30
<210> 214
   <211> 32
   <212> PRT
   <213> Artificial sequence
<220>
   <223> Variant NV1D3385
<220>
   <221> MOD_RES
   <222> (32)..(32)
   <223> -COOH
<400> 214
   Gly Pro Gln Cys Gln Lys Trp Met Gln Thr Cys Asp Arg Glu Arg Lys
   1               5                   10                  15
   Cys Cys Thr Gly Phe Val Cys Thr Leu Trp Cys Arg Lys Lys Leu Trp
               20                  25                  30
<210> 215
   <211> 32
   <212> PRT
   <213> Artificial sequence
<220>
   <223> Variant NV1D3386
<220>
   <221> MOD_RES
   <222> (32)..(32)
   <223> -COOH
<400> 215
   Gly Pro Gln Cys Gln Lys Trp Met Gln Thr Cys Asp Arg Glu Arg Lys
   1               5                   10                  15
   Cys Cys Ala Gly Phe Val Cys Thr Leu Trp Cys Arg Lys Lys Leu Trp
               20                  25                  30
<210> 216
   <211> 32
   <212> PRT
   <213> Artificial sequence
<220>
   <223> Variant NV1D3387
<220>
   <221> MOD_RES
   <222> (32)..(32)
   <223> -COOH
<400> 216
   Gly Pro Gln Cys Gln Lys Trp Met Gln Thr Cys Asp Arg Glu Arg Lys
   1               5                   10                  15
   Cys Cys Asp Gly Phe Val Cys Thr Leu Trp Cys Arg Lys Lys Leu Trp
               20                  25                  30
<210> 217
   <211> 32
   <212> PRT
   <213> Artificial sequence
<220>
   <223> Variant NV1D3388
<220>
   <221> MOD_RES
   <222> (32)..(32)
   <223> -COOH
<400> 217
   Gly Pro Gln Cys Gln Lys Trp Met Gln Thr Cys Asp Arg Glu Arg Lys
   1 5 10 15
   Cys Cys Gln Gly Phe Val Cys Thr Leu Trp Cys Arg Lys Lys Leu Trp
   20 25 30
<210> 218
   <211> 32
   <212> PRT
   <213> Artificial sequence
<220>
   <223> Variant NV1D3389
<220>
   <221> MOD_RES
   <222> (32)..(32)
   <223> -COOH
<400> 218
   Gly Pro Gln Cys Gln Lys Trp Met Gln Thr Cys Asp Arg Glu Arg Lys
   1 5 10 15
   Cys Cys Ser Gly Phe Val Cys Thr Leu Trp Cys Arg Lys Lys Leu Trp
   20 25 30
<210> 219
   <211> 32
   <212> PRT
   <213> Artificial sequence
<220>
   <223> Variant NV1D3390
<220>
   <221> MOD_RES
   <222> (32)..(32)
   <223> -COOH
<400> 219
   Gly Pro Gln Cys Gln Lys Trp Met Gln Thr Cys Asp Arg Glu Arg Lys
   1 5 10 15
   Cys Cys Glu Arg Phe Val Cys Thr Leu Trp Cys Arg Lys Lys Leu Trp
   20 25 30
<210> 220
   <211> 32
   <212> PRT
   <213> Artificial sequence
<220>
   <223> Variant NV1D3391
<220>
   <221> MOD_RES
   <222> (32)..(32)
   <223> -COOH
<400> 220
   Gly Pro Gln Cys Gln Lys Trp Met Gln Thr Cys Asp Arg Glu Arg Lys
   1               5                   10                  15
   Cys Cys Glu Lys Phe Val Cys Thr Leu Trp Cys Arg Lys Lys Leu Trp
               20                  25                  30
<210> 221
   <211> 32
   <212> PRT
   <213> Artificial sequence
<220>
   <223> Variant NV1D3392
<220>
   <221> MOD_RES
   <222> (32)..(32)
   <223> -COOH
<400> 221
   Gly Pro Gln Cys Gln Lys Trp Met Gln Thr Cys Asp Arg Glu Arg Lys
   1               5                   10                  15
   Cys Cys Glu Thr Phe Val Cys Thr Leu Trp Cys Arg Lys Lys Leu Trp
               20                  25                  30
<210> 222
   <211> 32
   <212> PRT
   <213> Artificial sequence
<220>
   <223> Variant NV1D3393
<220>
   <221> MOD_RES
   <222> (32)..(32)
   <223> -COOH
<400> 222
   Gly Pro Gln Cys Gln Lys Trp Met Gln Thr Cys Asp Arg Glu Arg Lys
   1               5                   10                  15
   Cys Cys Glu Ala Phe Val Cys Thr Leu Trp Cys Arg Lys Lys Leu Trp
               20                  25                  30
<210> 223
   <211> 32
   <212> PRT
   <213> Artificial sequence
<220>
   <223> Variant NV1D3394
<220>
   <221> MOD_RES
   <222> (32)..(32)
   <223> -COOH
<400> 223
   Gly Pro Gln Cys Gln Lys Trp Met Gln Thr Cys Asp Arg Glu Arg Lys
   1               5                   10                  15
   Cys Cys Glu Asp Phe Val Cys Thr Leu Trp Cys Arg Lys Lys Leu Trp
               20                  25                  30
<210> 224
   <211> 32
   <212> PRT
   <213> Artificial sequence
<220>
   <223> Variant NV1D3396
<220>
   <221> MOD_RES
   <222> (32)..(32)
   <223> -COOH
<400> 224
   Gly Pro Gln Cys Gln Lys Trp Met Gln Thr Cys Asp Arg Glu Arg Lys
   1               5                   10                  15
   Cys Cys Glu Gln Phe Val Cys Thr Leu Trp Cys Arg Lys Lys Leu Trp
               20                  25                  30
<210> 225
   <211> 32
   <212> PRT
   <213> Artificial sequence
<220>
   <223> Variant NV1D3397
<220>
   <221> MOD_RES
   <222> (32)..(32)
   <223> -COOH
<400> 225
   Gly Pro Gln Cys Gln Lys Trp Met Gln Thr Cys Asp Arg Glu Arg Lys
   1 5 10 15
   Cys Cys Glu Ser Phe Val Cys Thr Leu Trp Cys Arg Lys Lys Leu Trp
   20 25 30
<210> 226
   <211> 32
   <212> PRT
   <213> Artificial sequence
<220>
   <223> Variant NV1D3398
<220>
   <221> MOD_RES
   <222> (32)..(32)
   <223> -COOH
<400> 226
   Gly Pro Gln Cys Gln Lys Trp Met Gln Thr Cys Asp Arg Glu Arg Lys
   1 5 10 15
   Cys Cys Glu Gly Phe Ser Cys Thr Leu Trp Cys Arg Lys Lys Leu Trp
   20 25 30
<210> 227
   <211> 32
   <212> PRT
   <213> Artificial sequence
<220>
   <223> Variant NV1D3399
<220>
   <221> MOD_RES
   <222> (32)..(32)
   <223> -COOH
<400> 227
   Gly Pro Gln Cys Gln Lys Trp Met Gln Thr Cys Asp Arg Glu Arg Lys
   1 5 10 15
   Cys Cys Glu Gly Phe Thr Cys Thr Leu Trp Cys Arg Lys Lys Leu Trp
   20 25 30
<210> 228
   <211> 32
   <212> PRT
   <213> Artificial sequence
<220>
   <223> Variant NV1D3400
<220>
   <221> MOD_RES
   <222> (32)..(32)
   <223> -COOH
<400> 228
   Gly Pro Gln Cys Gln Lys Trp Met Gln Thr Cys Asp Arg Glu Arg Lys
   1               5                   10                  15
   Cys Cys Glu Gly Phe Gln Cys Thr Leu Trp Cys Arg Lys Lys Leu Trp
               20                  25                  30
<210> 229
   <211> 32
   <212> PRT
   <213> Artificial sequence
<220>
   <223> Variant NV1D3401
<220>
   <221> MOD_RES
   <222> (32)..(32)
   <223> -COOH
<400> 229
   Gly Pro Gln Cys Gln Lys Trp Met Gln Thr Cys Asp Arg Glu Arg Lys
   1               5                   10                  15
   Cys Cys Glu Gly Phe Val Cys Thr Leu Trp Cys Arg Arg Lys Leu Trp
               20                  25                  30
<210> 230
   <211> 32
   <212> PRT
   <213> Artificial sequence
<220>
   <223> Variant NV1D3403
<220>
   <221> MOD_RES
   <222> (32)..(32)
   <223> -COOH
<400> 230
   Gly Pro Gln Cys Gln Lys Trp Met Gln Thr Cys Asp Arg Glu Arg Lys
   1               5                   10                  15
   Cys Cys Glu Gly Phe Val Cys Thr Leu Trp Cys Arg Ala Lys Leu Trp
               20                  25                  30
<210> 231
   <211> 32
   <212> PRT
   <213> Artificial sequence
<220>
   <223> Variant NV1D3408
<220>
   <221> MOD_RES
   <222> (32)..(32)
   <223> -COOH
<400> 231
   Gly Pro Gln Cys Gln Lys Trp Met Gln Thr Cys Asp Arg Glu Arg Lys
   1               5                   10                  15
   Cys Cys Glu Gly Phe Val Cys Thr Leu Trp Cys Arg Lys Arg Leu Trp
               20                  25                  30
<210> 232
   <211> 32
   <212> PRT
   <213> Artificial sequence
<220>
   <223> Variant NV1D3409
<220>
   <221> MOD_RES
   <222> (32)..(32)
   <223> -COOH
<400> 232
   Gly Pro Gln Cys Gln Lys Trp Met Gln Thr Cys Asp Arg Glu Arg Lys
   1               5                   10                  15
   Cys Cys Glu Gly Phe Val Cys Thr Leu Trp Cys Arg Lys Thr Leu Trp
               20                  25                  30
<210> 233
   <211> 32
   <212> PRT
   <213> Artificial sequence
<220>
   <223> Variant NV1D3410
<220>
   <221> MOD_RES
   <222> (32)..(32)
   <223> -COOH
<400> 233
   Gly Pro Gln Cys Gln Lys Trp Met Gln Thr Cys Asp Arg Glu Arg Lys
   1               5                   10                  15
   Cys Cys Glu Gly Phe Val Cys Thr Leu Trp Cys Arg Lys Ala Leu Trp
               20                  25                  30
<210> 234
   <211> 32
   <212> PRT
   <213> Artificial sequence
<220>
   <223> Variant NV1D3413
<220>
   <221> MOD_RES
   <222> (32)..(32)
   <223> -COOH
<400> 234
   Gly Pro Gln Cys Gln Lys Trp Met Gln Thr Cys Asp Arg Glu Arg Lys
   1               5                   10                  15
   Cys Cys Glu Gly Phe Val Cys Thr Leu Trp Cys Arg Lys Gln Leu Trp
               20                  25                  30
<210> 235
   <211> 32
   <212> PRT
   <213> Artificial sequence
<220>
   <223> Variant NV1D3414
<220>
   <221> MOD_RES
   <222> (32)..(32)
   <223> -COOH
<400> 235
   Gly Pro Gln Cys Gln Lys Trp Met Gln Thr Cys Asp Arg Glu Arg Lys
   1               5                   10                  15
   Cys Cys Glu Gly Phe Val Cys Thr Leu Trp Cys Arg Lys Ser Leu Trp
               20                  25                  30
<210> 236
   <211> 32
   <212> PRT
   <213> Artificial sequence
<220>
   <223> Variant NV1D3419
<220>
   <221> MOD_RES
   <222> (32)..(32)
   <223> -COOH
<400> 236
   Gly Pro Gln Cys Gln Lys Trp Met Gln Thr Cys Asp Arg Glu Arg Lys
   1               5                   10                  15
   Cys Cys Glu Gly Phe Val Cys Thr Leu Trp Cys Arg Lys Lys Leu Asp
               20                  25                  30
<210> 237
   <211> 32
   <212> PRT
   <213> Artificial sequence
<220>
   <223> Variant NV1D3423
<220>
   <221> MOD_RES
   <222> (32)..(32)
   <223> -COOH
<400> 237
   Gly Pro Gln Cys Gln Lys Trp Met Gln Thr Cys Arg Arg Arg Arg Lys
   1               5                   10                  15
   Cys Cys Glu Gly Phe Val Cys Thr Leu Trp Cys Arg Lys Lys Leu Trp
               20                  25                  30
<210> 238
   <211> 32
   <212> PRT
   <213> Artificial sequence
<220>
   <223> Variant NV1D3424
<220>
   <221> MOD_RES
   <222> (32)..(32)
   <223> -COOH
<400> 238
   Gly Pro Gln Cys Gln Lys Trp Met Gln Thr Cys Lys Arg Lys Arg Lys
   1               5                   10                  15
   Cys Cys Glu Gly Phe Val Cys Thr Leu Trp Cys Arg Lys Lys Leu Trp
               20                  25                  30
<210> 239
   <211> 32
   <212> PRT
   <213> Artificial sequence
<220>
   <223> Variant NV1D3425
<220>
   <221> MOD_RES
   <222> (32)..(32)
   <223> -COOH
<400> 239
   Gly Pro Gln Cys Gln Lys Trp Met Gln Thr Cys Arg Arg Arg Asp Lys
   1               5                   10                  15
   Cys Cys Glu Gly Phe Val Cys Thr Leu Trp Cys Arg Lys Lys Leu Trp
               20                  25                  30
<210> 240
   <211> 32
   <212> PRT
   <213> Artificial sequence
<220>
   <223> Variant NV1D3426
<220>
   <221> MOD_RES
   <222> (32)..(32)
   <223> -COOH
<400> 240
   Gly Pro Gln Cys Gln Lys Trp Met Gln Thr Cys Lys Arg Lys Asp Lys
   1               5                   10                  15
   Cys Cys Glu Gly Phe Val Cys Thr Leu Trp Cys Arg Lys Lys Leu Trp
               20                  25                  30
<210> 241
   <211> 32
   <212> PRT
   <213> Artificial sequence
<220>
   <223> Variant NV1D3427
<220>
   <221> MOD_RES
   <222> (32)..(32)
   <223> -COOH
<400> 241
   Gly Pro Gln Cys Gln Lys Trp Met Gln Thr Cys Arg Arg Arg Glu Lys
   1               5                   10                  15
   Cys Cys Glu Gly Phe Val Cys Thr Leu Trp Cys Arg Lys Lys Leu Trp
               20                  25                  30
<210> 242
   <211> 32
   <212> PRT
   <213> Artificial sequence
<220>
   <223> Variant NV1D3428
<220>
   <221> MOD_RES
   <222> (32)..(32)
   <223> -COOH
<400> 242
   Gly Pro Gln Cys Gln Lys Trp Met Gln Thr Cys Lys Arg Lys Glu Lys
   1               5                   10                  15
   Cys Cys Glu Gly Phe Val Cys Thr Leu Trp Cys Arg Lys Lys Leu Trp
               20                  25                  30
<210> 243
   <211> 32
   <212> PRT
   <213> Artificial sequence
<220>
   <223> Variant NV1D3430
<220>
   <221> MOD_RES
   <222> (32)..(32)
   <223> -COOH
<400> 243
   Gly Pro Gln Cys Gln Asp Trp Met Gln Thr Cys Asp Arg Glu Arg Lys
   1               5                   10                  15
   Cys Cys Lys Gly Phe Val Cys Thr Leu Trp Cys Arg Lys Lys Leu Trp
               20                  25                  30
<210> 244
   <211> 32
   <212> PRT
   <213> Artificial sequence
<220>
   <223> Variant NV1D3431
<220>
   <221> MOD_RES
   <222> (32)..(32)
   <223> -COOH
<400> 244
   Gly Pro Gln Cys Gln Glu Trp Met Gln Thr Cys Asp Arg Glu Arg Lys
   1               5                   10                  15
   Cys Cys Lys Gly Phe Val Cys Thr Leu Trp Cys Arg Lys Lys Leu Trp
               20                  25                  30
<210> 245
   <211> 32
   <212> PRT
   <213> Artificial sequence
<220>
   <223> Variant NV1D3432
<220>
   <221> MOD_RES
   <222> (32)..(32)
   <223> -COOH
<400> 245
   Gly Pro Gln Cys Gln Glu Trp Met Gln Thr Cys Asp Arg Glu Arg Lys
   1               5                   10                  15
   Cys Cys Arg Gly Phe Val Cys Thr Leu Trp Cys Arg Lys Lys Leu Trp
               20                  25                  30
<210> 246
   <211> 32
   <212> PRT
   <213> Artificial sequence
<220>
   <223> Variant NV1D3439
<220>
   <221> MOD_RES
   <222> (32)..(32)
   <223> -COOH
<400> 246
   Gly Pro Gln Cys Gln Lys Trp Met Gln Thr Cys Asp Arg Glu Arg Lys
   1               5                   10                  15
   Cys Cys Glu Gly Phe Val Cys Thr Leu Trp Cys Arg Lys Lys Leu Gly
               20                  25                  30
<210> 247
   <211> 32
   <212> PRT
   <213> Artificial sequence
<220>
   <223> Variant NV1D3465
<220>
   <221> MOD_RES
   <222> (32)..(32)
   <223> -COOH
<400> 247
   Gly Pro Gln Cys Gln Lys Phe Met Gln Thr Cys Asp Arg Glu Arg Lys
   1               5                   10                  15
   Cys Cys Glu Gly Phe Val Cys Thr Leu Trp Cys Arg Lys Lys Leu Trp
               20                  25                  30
<210> 248
   <211> 32
   <212> PRT
   <213> Artificial sequence
<220>
   <223> Variant NV1D3466
<220>
   <221> MOD_RES
   <222> (32)..(32)
   <223> -COOH
<400> 248
   Gly Pro Gln Cys Gln Lys Trp Met Gln Thr Cys Asp Glu Glu Arg Lys
   1               5                   10                  15
   Cys Cys Glu Gly Phe Val Cys Thr Leu Trp Cys Arg Lys Lys Leu Trp
               20                  25                  30
<210> 249
   <211> 32
   <212> PRT
   <213> Artificial sequence
<220>
   <223> Variant NV1D3467
<220>
   <221> MOD_RES
   <222> (32)..(32)
   <223> -COOH
<400> 249
   Gly Pro Gln Cys Gln Lys Trp Met Gln Thr Cys Asp Arg Glu Arg Lys
   1 5 10 15
   Cys Cys Gly Gly Phe Val Cys Thr Leu Trp Cys Arg Lys Lys Leu Trp
   20 25 30
<210> 250
   <211> 32
   <212> PRT
   <213> Artificial sequence
<220>
   <223> Variant NV1D3470
<220>
   <221> MOD_RES
   <222> (32)..(32)
   <223> -COOH
<400> 250
   Gly Pro Gln Cys Gln Lys Trp Met Gln Thr Cys Asp Arg Glu Arg Lys
   1 5 10 15
   Cys Cys Glu Gly Leu Val Cys Thr Leu Trp Cys Arg Lys Lys Leu Trp
   20 25 30
<210> 251
   <211> 44
   <212> PRT
   <213> Artificial sequence
<220>
   <223> Variant NV1D3510
<220>
   <221> MOD_RES
   <222> (44)..(44)
   <223> -COOH
<400> 251
   Gly Pro Gln Cys Gln Lys Trp Met Gln Thr Cys Asp Arg Glu Arg Lys
   1 5 10 15
   Cys Cys Glu Gly Phe Val Cys Thr Leu Trp Cys Arg Lys Lys Leu Trp
   20 25 30
   Ala Pro Ala Pro Ala Ser Pro Gly Ala Arg Ala Phe
   35 40
<210> 252
   <211> 39
   <212> PRT
   <213> Artificial sequence
<220>
   <223> Variant NV1D3511
<220>
   <221> MOD_RES
   <222> (39)..(39)
   <223> -COOH
<400> 252
   Gly Pro Gln Cys Gln Lys Trp Met Gln Thr Cys Asp Arg Glu Arg Lys
   1               5                   10                  15
   Cys Cys Glu Gly Phe Val Cys Thr Leu Trp Cys Arg Lys Lys Leu Trp
               20                  25                  30
   Ser Pro Gly Ala Arg Ala Phe
           35
<210> 253
   <211> 49
   <212> PRT
   <213> Artificial sequence
<220>
   <223> Variant NV1D3512
<220>
   <221> MOD_RES
   <222> (49).. (49)
   <223> -COOH
<400> 253
   Gly Pro Gln Cys Gln Lys Trp Met Gln Thr Cys Asp Arg Glu Arg Lys
   1               5                   10                  15
   Cys Cys Glu Gly Phe Val Cys Thr Leu Trp Cys Arg Lys Lys Leu Trp
               20                  25                  30
   Ala Pro Ala Pro Ala Pro Ala Pro Ala Pro Asp Gly Pro Trp Arg Lys
           35                  40                  45
   Met
<210> 254
   <211> 44
   <212> PRT
   <213> Artificial sequence
<220>
   <223> Variant NV1D3513
<220>
   <221> MOD_RES
   <222> (44)..(44)
   <223> -COOH
<400> 254
   Gly Pro Gln Cys Gln Lys Trp Met Gln Thr Cys Asp Arg Glu Arg Lys
   1 5 10 15
   Cys Cys Glu Gly Phe Val Cys Thr Leu Trp Cys Arg Lys Lys Leu Trp
   20 25 30
   Ala Pro Ala Pro Ala Asp Gly Pro Trp Arg Lys Met
   35 40
<210> 255
   <211> 39
   <212> PRT
   <213> Artificial sequence
<220>
   <223> Variant NV1D3514
<220>
   <221> MOD_RES
   <222> (39)..(39)
   <223> -COOH
<400> 255
   Gly Pro Gln Cys Gln Lys Trp Met Gln Thr Cys Asp Arg Glu Arg Lys
   1 5 10 15
   Cys Cys Glu Gly Phe Val Cys Thr Leu Trp Cys Arg Lys Lys Leu Trp
   20 25 30
   Asp Gly Pro Trp Arg Lys Met
   35
<210> 256
   <211> 49
   <212> PRT
   <213> Artificial sequence
<220>
   <223> Variant NV1D3515
<220>
   <221> MOD_RES
   <222> (49).. (49)
   <223> -COOH
<400> 256
   Gly Pro Gln Cys Gln Lys Trp Met Gln Thr Cys Asp Arg Glu Arg Lys
   1               5                   10                  15
   Cys Cys Glu Gly Phe Val Cys Thr Leu Trp Cys Arg Lys Lys Leu Trp
               20                  25                  30
   Ala Pro Ala Pro Ala Pro Ala Pro Ala Pro Phe Gly Gln Lys Ala Ser
           35                  40                  45
   Ser
<210> 257
   <211> 44
   <212> PRT
   <213> Artificial sequence
<220>
   <223> Variant NV1D3516
<220>
   <221> MOD_RES
   <222> (44)..(44)
   <223> -COOH
<400> 257
   Gly Pro Gln Cys Gln Lys Trp Met Gln Thr Cys Asp Arg Glu Arg Lys
   1               5                   10                  15
   Cys Cys Glu Gly Phe Val Cys Thr Leu Trp Cys Arg Lys Lys Leu Trp
               20                  25                  30
   Ala Pro Ala Pro Ala Phe Gly Gln Lys Ala Ser Ser
           35                  40
<210> 258
   <211> 39
   <212> PRT
   <213> Artificial sequence
<220>
   <223> Variant NV1D3517
<220>
   <221> MOD_RES
   <222> (39)..(39)
   <223> -COOH
<400> 258
   Gly Pro Gln Cys Gln Lys Trp Met Gln Thr Cys Asp Arg Glu Arg Lys
   1               5                   10                  15
   Cys Cys Glu Gly Phe Val Cys Thr Leu Trp Cys Arg Lys Lys Leu Trp
               20                  25                  30
   Phe Gly Gln Lys Ala Ser Ser
           35
<210> 259
   <211> 58
   <212> PRT
   <213> Artificial sequence
<220>
   <223> Variant NV1D3518
<220>
   <221> MOD_RES
   <222> (58)..(58)
   <223> -COOH
<400> 259
   Gly Pro Gln Cys Gln Lys Trp Met Gln Thr Cys Asp Arg Glu Arg Lys
   1               5                   10                  15
   Cys Cys Glu Gly Phe Val Cys Thr Leu Trp Cys Arg Lys Lys Leu Trp
               20                  25                  30
   Ala Pro Ala Pro Ala Pro Ala Pro Ala Pro Gln Arg Phe Val Thr Gly
           35                  40                  45
   His Phe Gly Gly Leu Tyr Pro Ala Asn Gly
       50                  55
<210> 260
   <211> 53
   <212> PRT
   <213> Artificial sequence
<220>
   <223> Variant NV1D3519
<220>
   <221> MOD_RES
   <222> (53)..(53)
   <223> -COOH
<400> 260
   Gly Pro Gln Cys Gln Lys Trp Met Gln Thr Cys Asp Arg Glu Arg Lys
   1               5                   10                  15
   Cys Cys Glu Gly Phe Val Cys Thr Leu Trp Cys Arg Lys Lys Leu Trp
               20                  25                  30
   Ala Pro Ala Pro Ala Gln Arg Phe Val Thr Gly His Phe Gly Gly Leu
           35                  40                  45
   Tyr Pro Ala Asn Gly
       50
<210> 261
   <211> 48
   <212> PRT
   <213> Artificial sequence
<220>
   <223> Variant NV1D3520
<220>
   <221> MOD_RES
   <222> (48)..(48)
   <223> -COOH
<400> 261
   Gly Pro Gln Cys Gln Lys Trp Met Gln Thr Cys Asp Arg Glu Arg Lys
   1               5                   10                  15
   Cys Cys Glu Gly Phe Val Cys Thr Leu Trp Cys Arg Lys Lys Leu Trp
               20                  25                  30
   Gln Arg Phe Val Thr Gly His Phe Gly Gly Leu Tyr Pro Ala Asn Gly
           35                  40                  45
<210> 262
   <211> 53
   <212> PRT
   <213> Artificial sequence
<220>
   <223> Variant NV1D3521
<220>
   <221> MOD_RES
   <222> (53)..(53)
   <223> -COOH
<400> 262
   Gly Pro Gln Cys Gln Lys Trp Met Gln Thr Cys Asp Arg Glu Arg Lys
   1 5 10 15
   Cys Cys Glu Gly Phe Val Cys Thr Leu Trp Cys Arg Lys Lys Leu Trp
   20 25 30
   Ala Pro Ala Pro Ala Pro Ala Pro Ala Pro Arg Arg Arg Arg Arg Arg
   35 40 45
   Arg Arg Arg Arg Arg
   50
<210> 263
   <211> 43
   <212> PRT
   <213> Artificial sequence
<220>
   <223> Variant NV1D3523
<220>
   <221> MOD_RES
   <222> (43)..(43)
   <223> -COOH
<400> 263
   Gly Pro Gln Cys Gln Lys Trp Met Gln Thr Cys Asp Arg Glu Arg Lys
   1 5 10 15
   Cys Cys Glu Gly Phe Val Cys Thr Leu Trp Cys Arg Lys Lys Leu Trp
   20 25 30
   Arg Arg Arg Arg Arg Arg Arg Arg Arg Arg Arg
   35 40
<210> 264
   <211> 53
   <212> PRT
   <213> Artificial sequence
<220>
   <223> Variant NV1D3524
<220>
   <221> MOD_RES
   <222> (53)..(53)
   <223> -COOH
<400> 264
   Gly Pro Gln Cys Gln Lys Trp Met Gln Thr Cys Asp Arg Glu Arg Lys
   1 5 10 15
   Cys Cys Glu Gly Phe Val Cys Thr Leu Trp Cys Arg Lys Lys Leu Trp
   20 25 30
   Ala Pro Ala Pro Ala Pro Ala Pro Ala Pro Tyr Gly Arg Lys Lys Arg
   35 40 45
   Arg Gln Arg Arg Arg
   50
<210> 265
   <211> 48
   <212> PRT
   <213> Artificial sequence
<220>
   <223> Variant NV1D3525
<220>
   <221> MOD_RES
   <222> (48)..(48)
   <223> -COOH
<400> 265
   Gly Pro Gln Cys Gln Lys Trp Met Gln Thr Cys Asp Arg Glu Arg Lys
   1 5 10 15
   Cys Cys Glu Gly Phe Val Cys Thr Leu Trp Cys Arg Lys Lys Leu Trp
   20 25 30
   Ala Pro Ala Pro Ala Tyr Gly Arg Lys Lys Arg Arg Gln Arg Arg Arg
   35 40 45
<210> 266
   <211> 43
   <212> PRT
   <213> Artificial sequence
<220>
   <223> Variant NV1D3526
<220>
   <221> MOD_RES
   <222> (43)..(43)
   <223> -COOH
<400> 266
   Gly Pro Gln Cys Gln Lys Trp Met Gln Thr Cys Asp Arg Glu Arg Lys
   1               5                   10                  15
   Cys Cys Glu Gly Phe Val Cys Thr Leu Trp Cys Arg Lys Lys Leu Trp
               20                  25                  30
   Tyr Gly Arg Lys Lys Arg Arg Gln Arg Arg Arg
           35                  40
<210> 267
   <211> 42
   <212> PRT
   <213> Artificial sequence
<220>
   <223> Variant NV1D3527
<220>
   <221> MOD_RES
   <222> (42)..(42)
   <223> -COOH
<400> 267
   Gly Pro Gln Cys Gln Lys Trp Met Gln Thr Cys Asp Arg Glu Arg Lys
   1               5                   10                  15
   Cys Cys Glu Gly Phe Val Cys Thr Leu Trp Cys Arg Lys Lys Leu Trp
               20                  25                  30
   Ala Pro Ala Pro Ala Pro Ala Pro Ala Pro
           35                  40
<210> 268
   <211> 37
   <212> PRT
   <213> Artificial sequence
<220>
   <223> Variant NV1D3528
<220>
   <221> MOD_RES
   <222> (37)..(37)
   <223> -COOH
<400> 268
   Gly Pro Gln Cys Gln Lys Trp Met Gln Thr Cys Asp Arg Glu Arg Lys
   1 5 10 15
   Cys Cys Glu Gly Phe Val Cys Thr Leu Trp Cys Arg Lys Lys Leu Trp
   20 25 30
   Ala Pro Ala Pro Ala
   35
<210> 269
   <211> 32
   <212> PRT
   <213> Artificial sequence
<220>
   <223> Variant NV1D3529
<220>
   <221> MOD_RES
   <222> (32)..(32)
   <223> -COOH
<400> 269
   Gly Pro Arg Cys Gln Lys Trp Met Gln Thr Cys Asp Ala Lys Arg Lys
   1 5 10 15
   Cys Cys Glu Gly Phe Val Cys Thr Leu Trp Cys Arg Lys Lys Leu Trp
   20 25 30
<210> 270
   <211> 32
   <212> PRT
   <213> Artificial sequence
<220>
   <223> Variant NV1D3530
<220>
   <221> MOD_RES
   <222> (32)..(32)
   <223> -COOH
<400> 270
   Gly Pro Ser Cys Gln Lys Trp Met Gln Thr Cys Asp Ala Lys Arg Lys
   1 5 10 15
   Cys Cys Glu Gly Phe Val Cys Thr Leu Trp Cys Arg Lys Lys Leu Trp
   20 25 30
<210> 271
   <211> 32
   <212> PRT
   <213> Artificial sequence
<220>
   <223> Variant NV1D3531
<220>
   <221> MOD_RES
   <222> (32)..(32)
   <223> -COOH
<400> 271
   Gly Pro Tyr Cys Gln Lys Trp Met Gln Thr Cys Asp Ala Lys Arg Lys
   1               5                   10                  15
   Cys Cys Glu Gly Phe Val Cys Thr Leu Trp Cys Arg Lys Lys Leu Trp
               20                  25                  30
<210> 272
   <211> 32
   <212> PRT
   <213> Artificial sequence
<220>
   <223> Variant NV1D3532
<220>
   <221> MOD_RES
   <222> (32)..(32)
   <223> -COOH
<400> 272
   Gly Pro Ala Cys Gln Lys Trp Met Gln Thr Cys Asp Ala Lys Arg Lys
   1               5                   10                  15
   Cys Cys Glu Gly Phe Val Cys Thr Leu Trp Cys Arg Lys Lys Leu Trp
               20                  25                  30
<210> 273
   <211> 32
   <212> PRT
   <213> Artificial sequence
<220>
   <223> Variant NV1D3533
<220>
   <221> MOD_RES
   <222> (32)..(32)
   <223> -COOH
<400> 273
   Gly Pro Gln Cys Gln Lys Trp Met Gln Thr Cys Asp Ala Lys Arg Lys
   1               5                   10                  15
   Cys Cys Glu Gly Phe Ser Cys Thr Leu Trp Cys Arg Lys Lys Leu Trp
               20                  25                  30
<210> 274
   <211> 32
   <212> PRT
   <213> Artificial sequence
<220>
   <223> Variant NV1D3534
<220>
   <221> MOD_RES
   <222> (32)..(32)
   <223> -COOH
<400> 274
   Gly Pro Arg Cys Gln Lys Trp Met Gln Thr Cys Asp Ala Lys Arg Lys
   1               5                   10                  15
   Cys Cys Glu Gly Phe Ser Cys Thr Leu Trp Cys Arg Lys Lys Leu Trp
               20                  25                  30
<210> 275
   <211> 32
   <212> PRT
   <213> Artificial sequence
<220>
   <223> Variant NV1D3535
<220>
   <221> MOD_RES
   <222> (32)..(32)
   <223> -COOH
<400> 275
   Gly Pro Ser Cys Gln Lys Trp Met Gln Thr Cys Asp Ala Lys Arg Lys
   1               5                   10                  15
   Cys Cys Glu Gly Phe Ser Cys Thr Leu Trp Cys Arg Lys Lys Leu Trp
               20                  25                  30
<210> 276
   <211> 32
   <212> PRT
   <213> Artificial sequence
<220>
   <223> Variant NV1D3536
<220>
   <221> MOD_RES
   <222> (32)..(32)
   <223> -COOH
<400> 276
   Gly Pro Tyr Cys Gln Lys Trp Met Gln Thr Cys Asp Ala Lys Arg Lys
   1               5                   10                  15
   Cys Cys Glu Gly Phe Ser Cys Thr Leu Trp Cys Arg Lys Lys Leu Trp
               20                  25                  30
<210> 277
   <211> 32
   <212> PRT
   <213> Artificial sequence
<220>
   <223> Variant NV1D3537
<220>
   <221> MOD_RES
   <222> (32)..(32)
   <223> -COOH
<400> 277
   Gly Pro Ala Cys Gln Lys Trp Met Gln Thr Cys Asp Ala Lys Arg Lys
   1               5                   10                  15
   Cys Cys Glu Gly Phe Ser Cys Thr Leu Trp Cys Arg Lys Lys Leu Trp
               20                  25                  30
<210> 278
   <211> 32
   <212> PRT
   <213> Artificial sequence
<220>
   <223> Variant NV1D3538
<220>
   <221> MOD_RES
   <222> (32)..(32)
   <223> -COOH
<400> 278
   Gly Pro Arg Cys Gln Lys Trp Met Gln Thr Cys Asp Arg Asn Arg Lys
   1               5                   10                  15
   Cys Cys Glu Gly Phe Val Cys Thr Leu Trp Cys Arg Lys Lys Leu Trp
               20                  25                  30
<210> 279
   <211> 32
   <212> PRT
   <213> Artificial sequence
<220>
   <223> Variant NV1D3539
<220>
   <221> MOD_RES
   <222> (32)..(32)
   <223> -COOH
<400> 279
   Gly Pro Ser Cys Gln Lys Trp Met Gln Thr Cys Asp Arg Asn Arg Lys
   1               5                   10                  15
   Cys Cys Glu Gly Phe Val Cys Thr Leu Trp Cys Arg Lys Lys Leu Trp
               20                  25                  30
<210> 280
   <211> 32
   <212> PRT
   <213> Artificial sequence
<220>
   <223> Variant NV1D3540
<220>
   <221> MOD_RES
   <222> (32)..(32)
   <223> -COOH
<400> 280
   Gly Pro Tyr Cys Gln Lys Trp Met Gln Thr Cys Asp Arg Asn Arg Lys
   1               5                   10                  15
   Cys Cys Glu Gly Phe Val Cys Thr Leu Trp Cys Arg Lys Lys Leu Trp
               20                  25                  30
<210> 281
   <211> 32
   <212> PRT
   <213> Artificial sequence
<220>
   <223> Variant NV1D3541
<220>
   <221> MOD_RES
   <222> (32)..(32)
   <223> -COOH
<400> 281
   Gly Pro Ala Cys Gln Lys Trp Met Gln Thr Cys Asp Arg Asn Arg Lys
   1 5 10 15
   Cys Cys Glu Gly Phe Val Cys Thr Leu Trp Cys Arg Lys Lys Leu Trp
   20 25 30
<210> 282
   <211> 32
   <212> PRT
   <213> Artificial sequence
<220>
   <223> Variant NV1D3542
<220>
   <221> MOD_RES
   <222> (32)..(32)
   <223> -COOH
<400> 282
   Gly Pro Gln Cys Gln Lys Trp Met Gln Thr Cys Asp Arg Asn Arg Lys
   1 5 10 15
   Cys Cys Glu Gly Phe Ser Cys Thr Leu Trp Cys Arg Lys Lys Leu Trp
   20 25 30
<210> 283
   <211> 32
   <212> PRT
   <213> Artificial sequence
<220>
   <223> Variant NV1D3543
<220>
   <221> MOD_RES
   <222> (32)..(32)
   <223> -COOH
<400> 283
   Gly Pro Arg Cys Gln Lys Trp Met Gln Thr Cys Asp Arg Asn Arg Lys
   1 5 10 15
   Cys Cys Glu Gly Phe Ser Cys Thr Leu Trp Cys Arg Lys Lys Leu Trp
   20 25 30
<210> 284
   <211> 32
   <212> PRT
   <213> Artificial sequence
<220>
   <223> Variant NV1D3544
<220>
   <221> MOD_RES
   <222> (32)..(32)
   <223> -COOH
<400> 284
   Gly Pro Ser Cys Gln Lys Trp Met Gln Thr Cys Asp Arg Asn Arg Lys
   1               5                   10                  15
   Cys Cys Glu Gly Phe Ser Cys Thr Leu Trp Cys Arg Lys Lys Leu Trp
               20                  25                  30
<210> 285
   <211> 32
   <212> PRT
   <213> Artificial sequence
<220>
   <223> Variant NV1D3545
<220>
   <221> MOD_RES
   <222> (32)..(32)
   <223> -COOH
<400> 285
   Gly Pro Tyr Cys Gln Lys Trp Met Gln Thr Cys Asp Arg Asn Arg Lys
   1               5                   10                  15
   Cys Cys Glu Gly Phe Ser Cys Thr Leu Trp Cys Arg Lys Lys Leu Trp
               20                  25                  30
<210> 286
   <211> 32
   <212> PRT
   <213> Artificial sequence
<220>
   <223> Variant NV1D3546
<220>
   <221> MOD_RES
   <222> (32)..(32)
   <223> -COOH
<400> 286
   Gly Pro Ala Cys Gln Lys Trp Met Gln Thr Cys Asp Arg Asn Arg Lys
   1               5                   10                  15
   Cys Cys Glu Gly Phe Ser Cys Thr Leu Trp Cys Arg Lys Lys Leu Trp
               20                  25                  30
<210> 287
   <211> 32
   <212> PRT
   <213> Artificial sequence
<220>
   <223> Variant NV1D3547
<220>
   <221> MOD_RES
   <222> (32)..(32)
   <223> -COOH
<400> 287
   Gly Pro Arg Cys Gln Lys Trp Met Gln Thr Cys Asp Arg Glu Arg Lys
   1               5                   10                  15
   Cys Cys Glu Gly Phe Val Cys Thr Leu Trp Cys Arg Lys Lys Leu Trp
               20                  25                  30
<210> 288
   <211> 32
   <212> PRT
   <213> Artificial sequence
<220>
   <223> Variant NV1D3548
<220>
   <221> MOD_RES
   <222> (32)..(32)
   <223> -COOH
<400> 288
   Gly Pro Ser Cys Gln Lys Trp Met Gln Thr Cys Asp Arg Glu Arg Lys
   1               5                   10                  15
   Cys Cys Glu Gly Phe Val Cys Thr Leu Trp Cys Arg Lys Lys Leu Trp
               20                  25                  30
<210> 289
   <211> 32
   <212> PRT
   <213> Artificial sequence
<220>
   <223> Variant NV1D3549
<220>
   <221> MOD_RES
   <222> (32)..(32)
   <223> -COOH
<400> 289
   Gly Pro Tyr Cys Gln Lys Trp Met Gln Thr Cys Asp Arg Glu Arg Lys
   1 5 10 15
   Cys Cys Glu Gly Phe Val Cys Thr Leu Trp Cys Arg Lys Lys Leu Trp
   20 25 30
<210> 290
   <211> 32
   <212> PRT
   <213> Artificial sequence
<220>
   <223> Variant NV1D3550
<220>
   <221> MOD_RES
   <222> (32)..(32)
   <223> -COOH
<400> 290
   Gly Pro Ala Cys Gln Lys Trp Met Gln Thr Cys Asp Arg Glu Arg Lys
   1 5 10 15
   Cys Cys Glu Gly Phe Val Cys Thr Leu Trp Cys Arg Lys Lys Leu Trp
   20 25 30
<210> 291
   <211> 32
   <212> PRT
   <213> Artificial sequence
<220>
   <223> Variant NV1D3551
<220>
   <221> MOD_RES
   <222> (32)..(32)
   <223> -COOH
<400> 291
   Gly Pro Arg Cys Gln Lys Trp Met Gln Thr Cys Asp Arg Glu Arg Lys
   1 5 10 15
   Cys Cys Glu Gly Phe Ser Cys Thr Leu Trp Cys Arg Lys Lys Leu Trp
   20 25 30
<210> 292
   <211> 32
   <212> PRT
   <213> Artificial sequence
<220>
   <223> Variant NV1D3552
<220>
   <221> MOD_RES
   <222> (32)..(32)
   <223> -COOH
<400> 292
   Gly Pro Ser Cys Gln Lys Trp Met Gln Thr Cys Asp Arg Glu Arg Lys
   1               5                   10                  15
   Cys Cys Glu Gly Phe Ser Cys Thr Leu Trp Cys Arg Lys Lys Leu Trp
               20                  25                  30
<210> 293
   <211> 32
   <212> PRT
   <213> Artificial sequence
<220>
   <223> Variant NV1D3553
<220>
   <221> MOD_RES
   <222> (32)..(32)
   <223> -COOH
<400> 293
   Gly Pro Tyr Cys Gln Lys Trp Met Gln Thr Cys Asp Arg Glu Arg Lys
   1               5                   10                  15
   Cys Cys Glu Gly Phe Ser Cys Thr Leu Trp Cys Arg Lys Lys Leu Trp
               20                  25                  30
<210> 294
   <211> 32
   <212> PRT
   <213> Artificial sequence
<220>
   <223> Variant NV1D3554
<220>
   <221> MOD_RES
   <222> (32)..(32)
   <223> -COOH
<400> 294
   Gly Pro Ala Cys Gln Lys Trp Met Gln Thr Cys Asp Arg Glu Arg Lys
   1               5                   10                  15
   Cys Cys Glu Gly Phe Ser Cys Thr Leu Trp Cys Arg Lys Lys Leu Trp
               20                  25                  30
<210> 295
   <211> 32
   <212> PRT
   <213> Artificial sequence
<220>
   <223> Variant NV1D3555
<220>
   <221> MOD_RES
   <222> (32)..(32)
   <223> -COOH
<400> 295
   Gly Pro Arg Cys Gln Lys Trp Met Gln Thr Cys Asp Ala Glu Arg Lys
   1               5                   10                  15
   Cys Cys Glu Gly Phe Ser Cys Thr Leu Trp Cys Lys Lys Lys Leu Trp
               20                  25                  30
<210> 296
   <211> 32
   <212> PRT
   <213> Artificial sequence
<220>
   <223> Variant NV1D3556
<220>
   <221> MOD_RES
   <222> (32)..(32)
   <223> -COOH
<400> 296
   Gly Pro Tyr Cys Gln Lys Trp Met Gln Thr Cys Asp Ala Glu Arg Lys
   1               5                   10                  15
   Cys Cys Glu Gly Phe Ser Cys Thr Leu Trp Cys Lys Lys Lys Leu Trp
               20                  25                  30
<210> 297
   <211> 32
   <212> PRT
   <213> Artificial sequence
<220>
   <223> Variant NV1D3558
<220>
   <221> MOD_RES
   <222> (32)..(32)
   <223> -COOH
<400> 297
   Gly Pro Arg Cys Gln Lys Trp Met Gln Thr Cys Asp Ala Asn Arg Lys
   1               5                   10                  15
   Cys Cys Glu Gly Phe Ser Cys Thr Leu Trp Cys Lys Lys Lys Leu Trp
               20                  25                  30
<210> 298
   <211> 32
   <212> PRT
   <213> Artificial sequence
<220>
   <223> Variant NV1D3559
<220>
   <221> MOD_RES
   <222> (32)..(32)
   <223> -COOH
<400> 298
   Gly Pro Ser Cys Gln Lys Trp Met Gln Thr Cys Asp Ala Asn Arg Lys
   1               5                   10                  15
   Cys Cys Glu Gly Phe Ser Cys Thr Leu Trp Cys Lys Lys Lys Leu Trp
               20                  25                  30
<210> 299
   <211> 32
   <212> PRT
   <213> Artificial sequence
<220>
   <223> Variant NV1D3560
<220>
   <221> MOD_RES
   <222> (32)..(32)
   <223> -COOH
<400> 299
   Gly Pro Tyr Cys Gln Lys Trp Met Gln Thr Cys Asp Ala Asn Arg Lys
   1               5                   10                  15
   Cys Cys Glu Gly Phe Ser Cys Thr Leu Trp Cys Lys Lys Lys Leu Trp
               20                  25                  30
<210> 300
   <211> 32
   <212> PRT
   <213> Artificial sequence
<220>
   <223> Variant NV1D3561
<220>
   <221> MOD_RES
   <222> (32)..(32)
   <223> -COOH
<400> 300
   Gly Pro Ala Cys Gln Lys Trp Met Gln Thr Cys Asp Ala Asn Arg Lys
   1               5                   10                  15
   Cys Cys Glu Gly Phe Ser Cys Thr Leu Trp Cys Lys Lys Lys Leu Trp
               20                  25                  30
<210> 301
   <211> 32
   <212> PRT
   <213> Artificial sequence
<220>
   <223> variant NV1D3562
<220>
   <221> MOD_RES
   <222> (32)..(32)
   <223> -COOH
<400> 301
   Gly Pro Arg Cys Gln Lys Trp Met Gln Thr Cys Asp Ala Arg Arg Lys
   1               5                   10                  15
   Cys Cys Glu Gly Phe Ser Cys Thr Leu Trp Cys Lys Lys Lys Leu Trp
               20                  25                  30
<210> 302
   <211> 32
   <212> PRT
   <213> Artificial sequence
<220>
   <223> Variant NV1D3563
<220>
   <221> MOD_RES
   <222> (32)..(32)
   <223> -COOH
<400> 302
   Gly Pro Ser Cys Gln Lys Trp Met Gln Thr Cys Asp Ala Arg Arg Lys
   1               5                   10                  15
   Cys Cys Glu Gly Phe Ser Cys Thr Leu Trp Cys Lys Lys Lys Leu Trp
               20                  25                  30
<210> 303
   <211> 32
   <212> PRT
   <213> Artificial sequence
<220>
   <223> Variant NV1D3564
<220>
   <221> MOD_RES
   <222> (32)..(32)
   <223> -COOH
<400> 303
   Gly Pro Tyr Cys Gln Lys Trp Met Gln Thr Cys Asp Ala Arg Arg Lys
   1               5                   10                  15
   Cys Cys Glu Gly Phe Ser Cys Thr Leu Trp Cys Lys Lys Lys Leu Trp
               20                  25                  30
<210> 304
   <211> 32
   <212> PRT
   <213> Artificial sequence
<220>
   <223> Variant NV1D3565
<220>
   <221> MOD_RES
   <222> (32)..(32)
   <223> -COOH
<400> 304
   Gly Pro Ala Cys Gln Lys Trp Met Gln Thr Cys Asp Ala Arg Arg Lys
   1               5                   10                  15
   Cys Cys Glu Gly Phe Ser Cys Thr Leu Trp Cys Lys Lys Lys Leu Trp
               20                  25                  30
<210> 305
   <211> 32
   <212> PRT
   <213> Artificial sequence
<220>
   <223> Variant NV1D3566
<220>
   <221> MOD_RES
   <222> (32)..(32)
   <223> -COOH
<400> 305
   Gly Pro Arg Cys Gln Lys Trp Met Gln Thr Cys Asp Ala Glu Arg Lys
   1               5                   10                  15
   Cys Cys Glu Gly Phe Val Cys Thr Leu Trp Cys Lys Lys Lys Leu Trp
               20                  25                  30
<210> 306
   <211> 32
   <212> PRT
   <213> Artificial sequence
<220>
   <223> Variant NV1D3568
<220>
   <221> MOD_RES
   <222> (32)..(32)
   <223> -COOH
<400> 306
   Gly Pro Ala Cys Gln Lys Trp Met Gln Thr Cys Asp Ala Glu Arg Lys
   1               5                   10                  15
   Cys Cys Glu Gly Phe Val Cys Thr Leu Trp Cys Lys Lys Lys Leu Trp
               20                  25                  30
<210> 307
   <211> 32
   <212> PRT
   <213> Artificial sequence
<220>
   <223> Variant NV1D3569
<220>
   <221> MOD_RES
   <222> (32)..(32)
   <223> -COOH
<400> 307
   Gly Pro Arg Cys Gln Lys Trp Met Gln Thr Cys Asp Ala Arg Arg Lys
   1               5                   10                  15
   Cys Cys Glu Gly Phe Val Cys Thr Leu Trp Cys Lys Lys Lys Leu Trp
               20                  25                  30
<210> 308
   <211> 32
   <212> PRT
   <213> Artificial sequence
<220>
   <223> Variant NV1D3570
<220>
   <221> MOD_RES
   <222> (32)..(32)
   <223> -COOH
<400> 308
   Gly Pro Ser Cys Gln Lys Trp Met Gln Thr Cys Asp Ala Arg Arg Lys
   1               5                   10                  15
   Cys Cys Glu Gly Phe Val Cys Thr Leu Trp Cys Lys Lys Lys Leu Trp
               20                  25                  30
<210> 309
   <211> 32
   <212> PRT
   <213> Artificial sequence
<220>
   <223> Variant NV1D3571
<220>
   <221> MOD_RES
   <222> (32)..(32)
   <223> -COOH
<400> 309
   Gly Pro Tyr Cys Gln Lys Trp Met Gln Thr Cys Asp Ala Arg Arg Lys
   1               5                   10                  15
   Cys Cys Glu Gly Phe Val Cys Thr Leu Trp Cys Lys Lys Lys Leu Trp
               20                  25                  30
<210> 310
   <211> 32
   <212> PRT
   <213> Artificial sequence
<220>
   <223> variant NV1D3572
<220>
   <221> MOD_RES
   <222> (32)..(32)
   <223> -COOH
<400> 310
   Gly Pro Ala Cys Gln Lys Trp Met Gln Thr Cys Asp Ala Arg Arg Lys
   1               5                   10                  15
   Cys Cys Glu Gly Phe Val Cys Thr Leu Trp Cys Lys Lys Lys Leu Trp
               20                  25                  30
<210> 311
   <211> 32
   <212> PRT
   <213> Artificial sequence
<220>
   <223> Variant NV1D3573
<220>
   <221> MOD_RES
   <222> (32)..(32)
   <223> -COOH
<400> 311
   Gly Pro Arg Cys Gln Lys Trp Met Gln Thr Cys Asp Ala Asn Arg Lys
   1               5                   10                  15
   Cys Cys Glu Gly Phe Val Cys Thr Leu Trp Cys Lys Lys Lys Leu Trp
               20                  25                  30
<210> 312
   <211> 32
   <212> PRT
   <213> Artificial sequence
<220>
   <223> Variant NV1D3574
<220>
   <221> MOD_RES
   <222> (32)..(32)
   <223> -COOH
<400> 312
   Gly Pro Ser Cys Gln Lys Trp Met Gln Thr Cys Asp Ala Asn Arg Lys
   1               5                   10                  15
   Cys Cys Glu Gly Phe Val Cys Thr Leu Trp Cys Lys Lys Lys Leu Trp
               20                  25                  30
<210> 313
   <211> 32
   <212> PRT
   <213> Artificial sequence
<220>
   <223> Variant NV1D3575
<220>
   <221> MOD_RES
   <222> (32)..(32)
   <223> -COOH
<400> 313
   Gly Pro Tyr Cys Gln Lys Trp Met Gln Thr Cys Asp Ala Asn Arg Lys
   1 5 10 15
   Cys Cys Glu Gly Phe Val Cys Thr Leu Trp Cys Lys Lys Lys Leu Trp
   20 25 30
<210> 314
   <211> 32
   <212> PRT
   <213> Artificial sequence
<220>
   <223> Variant NV1D3576
<220>
   <221> MOD_RES
   <222> (32)..(32)
   <223> -COOH
<400> 314
   Gly Pro Ala Cys Gln Lys Trp Met Gln Thr Cys Asp Ala Asn Arg Lys
   1 5 10 15
   Cys Cys Glu Gly Phe Val Cys Thr Leu Trp Cys Lys Lys Lys Leu Trp
   20 25 30
<210> 315
   <211> 32
   <212> PRT
   <213> Artificial sequence
<220>
   <223> Variant NV1D3577
<220>
   <221> MOD_RES
   <222> (32)..(32)
   <223> -COOH
<400> 315
   Gly Pro Arg Cys Gln Lys Trp Met Gln Thr Cys Asp Ala Glu Arg Lys
   1 5 10 15
   Cys Cys Glu Gly Phe Ser Cys Arg Leu Trp Cys Lys Lys Lys Leu Trp
   20 25 30
<210> 316
   <211> 32
   <212> PRT
   <213> Artificial sequence
<220>
   <223> Variant NV1D3578
<220>
   <221> MOD_RES
   <222> (32)..(32)
   <223> -COOH
<400> 316
   Gly Pro Tyr Cys Gln Lys Trp Met Gln Thr Cys Asp Ala Glu Arg Lys
   1               5                   10                  15
   Cys Cys Glu Gly Phe Ser Cys Arg Leu Trp Cys Lys Lys Lys Leu Trp
               20                  25                  30
<210> 317
   <211> 32
   <212> PRT
   <213> Artificial sequence
<220>
   <223> Variant NV1D3579
<220>
   <221> MOD_RES
   <222> (32)..(32)
   <223> -COOH
<400> 317
   Gly Pro Ala Cys Gln Lys Trp Met Gln Thr Cys Asp Ala Glu Arg Lys
   1               5                   10                  15
   Cys Cys Glu Gly Phe Ser Cys Arg Leu Trp Cys Lys Lys Lys Leu Trp
               20                  25                  30
<210> 318
   <211> 32
   <212> PRT
   <213> Artificial sequence
<220>
   <223> Variant NV1D3580
<220>
   <221> MOD_RES
   <222> (32)..(32)
   <223> -COOH
<400> 318
   Gly Pro Arg Cys Gln Lys Trp Met Gln Thr Cys Asp Ala Arg Arg Lys
   1               5                   10                  15
   Cys Cys Glu Gly Phe Ser Cys Arg Leu Trp Cys Lys Lys Lys Leu Trp
               20                  25                  30
<210> 319
   <211> 32
   <212> PRT
   <213> Artificial sequence
<220>
   <223> Variant NV1D3581
<220>
   <221> MOD_RES
   <222> (32)..(32)
   <223> -COOH
<400> 319
   Gly Pro Ser Cys Gln Lys Trp Met Gln Thr Cys Asp Ala Arg Arg Lys
   1               5                   10                  15
   Cys Cys Glu Gly Phe Ser Cys Arg Leu Trp Cys Lys Lys Lys Leu Trp
               20                  25                  30
<210> 320
   <211> 32
   <212> PRT
   <213> Artificial sequence
<220>
   <223> Variant NV1D3582
<220>
   <221> MOD_RES
   <222> (32)..(32)
   <223> -COOH
<400> 320
   Gly Pro Tyr Cys Gln Lys Trp Met Gln Thr Cys Asp Ala Arg Arg Lys
   1               5                   10                  15
   Cys Cys Glu Gly Phe Ser Cys Arg Leu Trp Cys Lys Lys Lys Leu Trp
               20                  25                  30
<210> 321
   <211> 32
   <212> PRT
   <213> Artificial sequence
<220>
   <223> Variant NV1D3583
<220>
   <221> MOD_RES
   <222> (32)..(32)
   <223> -COOH
<400> 321
   Gly Pro Ala Cys Gln Lys Trp Met Gln Thr Cys Asp Ala Arg Arg Lys
   1 5 10 15
   Cys Cys Glu Gly Phe Ser Cys Arg Leu Trp Cys Lys Lys Lys Leu Trp
   20 25 30
<210> 322
   <211> 32
   <212> PRT
   <213> Artificial sequence
<220>
   <223> Variant NV1D3584
<220>
   <221> MOD_RES
   <222> (32)..(32)
   <223> -COOH
<400> 322
   Gly Pro Arg Cys Gln Lys Trp Met Gln Thr Cys Asp Ala Asn Arg Lys
   1 5 10 15
   Cys Cys Glu Gly Phe Ser Cys Arg Leu Trp Cys Lys Lys Lys Leu Trp
   20 25 30
<210> 323
   <211> 32
   <212> PRT
   <213> Artificial sequence
<220>
   <223> Variant NV1D3585
<220>
   <221> MOD_RES
   <222> (32)..(32)
   <223> -COOH
<400> 323
   Gly Pro Ser Cys Gln Lys Trp Met Gln Thr Cys Asp Ala Asn Arg Lys
   1 5 10 15
   Cys Cys Glu Gly Phe Ser Cys Arg Leu Trp Cys Lys Lys Lys Leu Trp
   20 25 30
<210> 324
   <211> 32
   <212> PRT
   <213> Artificial sequence
<220>
   <223> Variant NV1D3586
<220>
   <221> MOD_RES
   <222> (32)..(32)
   <223> -COOH
<400> 324
   Gly Pro Tyr Cys Gln Lys Trp Met Gln Thr Cys Asp Ala Asn Arg Lys
   1               5                   10                  15
   Cys Cys Glu Gly Phe Ser Cys Arg Leu Trp Cys Lys Lys Lys Leu Trp
               20                  25                  30
<210> 325
   <211> 32
   <212> PRT
   <213> Artificial sequence
<220>
   <223> Variant NV1D3586-NH2
<220>
   <221> MOD_RES
   <222> (32)..(32)
   <223> -NH2
<400> 325
   Gly Pro Tyr Cys Gln Lys Trp Met Gln Thr Cys Asp Ala Asn Arg Lys
   1               5                   10                  15
   Cys Cys Glu Gly Phe Ser Cys Arg Leu Trp Cys Lys Lys Lys Leu Trp
               20                  25                  30
<210> 326
   <211> 32
   <212> PRT
   <213> Artificial sequence
<220>
   <223> Variant NV1D3587
<220>
   <221> MOD_RES
   <222> (32)..(32)
   <223> -COOH
<400> 326
   Gly Pro Ala Cys Gln Lys Trp Met Gln Thr Cys Asp Ala Asn Arg Lys
   1               5                   10                  15
   Cys Cys Glu Gly Phe Ser Cys Arg Leu Trp Cys Lys Lys Lys Leu Trp
               20                  25                  30
<210> 327
   <211> 48
   <212> PRT
   <213> Artificial sequence
<220>
   <223> Variant NV1D3772
<220>
   <221> MOD_RES
   <222> (48)..(48)
   <223> -COOH
<400> 327
   Gly Pro Gln Cys Gln Lys Trp Met Gln Thr Cys Asp Arg Glu Arg Lys
   1               5                   10                  15
   Cys Cys Glu Gly Phe Val Cys Thr Leu Trp Cys Arg Lys Lys Leu Trp
               20                  25                  30
   Ser His Ser Asn Thr Gln Thr Leu Ala Lys Ala Pro Glu His Thr Gly
           35                  40                  45
<210> 328
   <211> 68
   <212> PRT
   <213> Artificial sequence
<220>
   <223> Variant NV1D3774
<220>
   <221> MOD_RES
   <222> (68)..(68)
   <223> -COOH
<400> 328
   Gly Pro Ser His Ser Asn Thr Gln Thr Leu Ala Lys Ala Pro Glu His
   1 5 10 15
   Thr Gly Ala Pro Ala Pro Ala Pro Ala Pro Ala Pro Ala Pro Ala Pro
   20 25 30
   Ala Pro Ala Pro Ala Pro Gln Cys Gln Lys Trp Met Gln Thr Cys Asp
   35 40 45
   Arg Glu Arg Lys Cys Cys Glu Gly Phe Val Cys Thr Leu Trp Cys Arg
   50 55 60
   Lys Lys Leu Trp
   65
<210> 329
   <211> 58
   <212> PRT
   <213> Artificial sequence
<220>
   <223> Variant NV1D3775
<220>
   <221> MOD_RES
   <222> (58)..(58)
   <223> -COOH
<400> 329
   Gly Pro Ser His Ser Asn Thr Gln Thr Leu Ala Lys Ala Pro Glu His
   1 5 10 15
   Thr Gly Ala Pro Ala Pro Ala Pro Ala Pro Ala Pro Gln Cys Gln Lys
   20 25 30
   Trp Met Gln Thr Cys Asp Arg Glu Arg Lys Cys Cys Glu Gly Phe Val
   35 40 45
   Cys Thr Leu Trp Cys Arg Lys Lys Leu Trp
   50 55
<210> 330
   <211> 48
   <212> PRT
   <213> Artificial sequence
<220>
   <223> Variant NV1D3777
<220>
   <221> MOD_RES
   <222> (48)..(48)
   <223> -COOH
<400> 330
   Gly Pro Ser His Ser Asn Thr Gln Thr Leu Ala Lys Ala Pro Glu His
   1               5                   10                  15
   Thr Gly Gln Cys Gln Lys Trp Met Gln Thr Cys Asp Arg Glu Arg Lys
               20                  25                  30
   Cys Cys Glu Gly Phe Val Cys Thr Leu Trp Cys Arg Lys Lys Leu Trp
           35                  40                  45
<210> 331
   <211> 32
   <212> PRT
   <213> Artificial sequence
<220>
   <223> Variant NV1D3782
<220>
   <221> MOD_RES
   <222> (32)..(32)
   <223> -COOH
<400> 331
   Gly Pro Gln Cys Gln Lys Trp Met Gln Thr Cys Asp Arg Glu Arg Lys
   1               5                   10                  15
   Cys Cys Glu Gly Phe Val Cys Thr Leu Trp Cys Arg Lys Lys Ala Trp
               20                  25                  30
<210> 332
   <211> 37
   <212> PRT
   <213> Artificial sequence
<220>
   <223> Variant NV1D3788
<220>
   <221> MOD_RES
   <222> (37)..(37)
   <223> -COOH
<400> 332
   Gly Pro Ala Ala Ala Ala Ala Gln Cys Gln Lys Trp Met Gln Thr Cys
   1               5                   10                  15
   Asp Arg Glu Arg Lys Cys Cys Glu Gly Phe Val Cys Thr Leu Trp Cys
               20                  25                  30
   Arg Lys Lys Leu Trp
           35
<210> 333
   <211> 37
   <212> PRT
   <213> Artificial sequence
<220>
   <223> Variant NV1D3789
<220>
   <221> MOD_RES
   <222> (37)..(37)
   <223> -COOH
<400> 333
   Gly Pro Ala Pro Ala Pro Ala Gln Cys Gln Lys Trp Met Gln Thr Cys
   1               5                   10                  15
   Asp Arg Glu Arg Lys Cys Cys Glu Gly Phe Val Cys Thr Leu Trp Cys
               20                  25                  30
   Arg Lys Lys Leu Trp
           35
<210> 334
   <211> 37
   <212> PRT
   <213> Artificial sequence
<220>
   <223> Variant NV1D3791
<220>
   <221> MOD_RES
   <222> (37)..(37)
   <223> -COOH
<400> 334
   Gly Pro Gln Cys Gln Lys Trp Met Gln Thr Cys Asp Arg Glu Arg Lys
   1               5                   10                  15
   Cys Cys Glu Gly Phe Val Cys Thr Leu Trp Cys Arg Lys Lys Leu Trp
               20                  25                  30
   Ala Ala Ala Ala Ala
           35
<210> 335
   <211> 37
   <212> PRT
   <213> Artificial sequence
<220>
   <223> Variant NV1D3792
<220>
   <221> MOD_RES
   <222> (37)..(37)
   <223> -COOH
<400> 335
   Gly Pro Gln Cys Gln Lys Trp Met Gln Thr Cys Asp Arg Glu Arg Lys
   1               5                   10                  15
   Cys Cys Glu Gly Phe Val Cys Thr Leu Trp Cys Arg Lys Lys Leu Trp
               20                  25                  30
   Gly Gly Gly Gly Gly
           35
<210> 336
   <211> 74
   <212> PRT
   <213> Artificial sequence
<220>
   <223> Variant NV1D3793
<220>
   <221> MOD_RES
   <222> (74)..(74)
   <223> -COOH
<400> 336
   Gly Pro Cys Cys Asn Cys Ser Ser Lys Trp Cys Arg Asp His Ser Arg
   1 5 10 15
   Cys Cys Gly Arg Gly Ser Ala Pro Ala Pro Ala Pro Ala Pro Ala Pro
   20 25 30
   Ala Pro Ala Pro Ala Pro Ala Pro Ala Pro Gly Ser Gln Cys Gln Lys
   35 40 45
   Trp Met Gln Thr Cys Asp Arg Glu Arg Lys Cys Cys Glu Gly Phe Val
   50 55 60
   Cys Thr Leu Trp Cys Arg Lys Lys Leu Trp
   65 70
<210> 337
   <211> 72
   <212> PRT
   <213> Artificial sequence
<220>
   <223> Variant NV1D3795
<220>
   <221> MOD_RES
   <222> (72)..(72)
   <223> -COOH
<400> 337
   Gly Pro Cys Cys Asn Cys Ser Ser Lys Trp Cys Arg Asp His Ser Arg
   1 5 10 15
   Cys Cys Gly Ser Ala Pro Ala Pro Ala Pro Ala Pro Ala Pro Ala Pro
   20 25 30
   Ala Pro Ala Pro Ala Pro Ala Pro Gly Ser Gln Cys Gln Lys Trp Met
   35 40 45
   Gln Thr Cys Asp Arg Glu Arg Lys Cys Cys Glu Gly Phe Val Cys Thr
   50 55 60
   Leu Trp Cys Arg Lys Lys Leu Trp
   65 70
<210> 338
   <211> 62
   <212> PRT
   <213> Artificial sequence
<220>
   <223> Variant NV1D3796
<220>
   <221> MOD_RES
   <222> (62)..(62)
   <223> -COOH
<400> 338
   Gly Pro Cys Cys Asn Cys Ser Ser Lys Trp Cys Arg Asp His Ser Arg
   1               5                   10                  15
   Cys Cys Gly Ser Ala Pro Ala Pro Ala Pro Ala Pro Ala Pro Gly Ser
               20                  25                  30
   Gln Cys Gln Lys Trp Met Gln Thr Cys Asp Arg Glu Arg Lys Cys Cys
           35                  40                  45
   Glu Gly Phe Val Cys Thr Leu Trp Cys Arg Lys Lys Leu Trp
       50                  55                  60
<210> 339
   <211> 72
   <212> PRT
   <213> Artificial sequence
<220>
   <223> Variant NV1D3797
<220>
   <221> MOD_RES
   <222> (72)..(72)
   <223> -COOH
<400> 339
   Gly Pro Gln Cys Gln Lys Trp Met Gln Thr Cys Asp Arg Glu Arg Lys
   1               5                   10                  15
   Cys Cys Glu Gly Phe Val Cys Thr Leu Trp Cys Arg Lys Lys Leu Trp
               20                  25                  30
   Gly Ser Ala Pro Ala Pro Ala Pro Ala Pro Ala Pro Ala Pro Ala Pro
           35                  40                  45
   Ala Pro Ala Pro Ala Pro Gly Ser Cys Cys Asn Cys Ser Ser Lys Trp
       50                  55                  60
   Cys Arg Asp His Ser Arg Cys Cys
   65                  70
<210> 340
   <211> 74
   <212> PRT
   <213> Artificial sequence
<220>
   <223> Variant NV1D3798
<220>
   <221> MOD_RES
   <222> (74)..(74)
   <223> -COOH
<400> 340
   Gly Pro Gln Cys Gln Lys Trp Met Gln Thr Cys Asp Arg Glu Arg Lys
   1               5                   10                  15
   Cys Cys Glu Gly Phe Val Cys Thr Leu Trp Cys Arg Lys Lys Leu Trp
               20                  25                  30
   Gly Ser Ala Pro Ala Pro Ala Pro Ala Pro Ala Pro Ala Pro Ala Pro
           35                  40                  45
   Ala Pro Ala Pro Ala Pro Gly Ser Cys Cys Asn Cys Ser Ser Lys Trp
       50                  55                  60
   Cys Arg Asp His Ser Arg Cys Cys Gly Arg
   65                  70
<210> 341
   <211> 62
   <212> PRT
   <213> Artificial sequence
<220>
   <223> Variant NV1D3799
<220>
   <221> MOD_RES
   <222> (62)..(62)
   <223> -COOH
<400> 341
   Gly Pro Gln Cys Gln Lys Trp Met Gln Thr Cys Asp Arg Glu Arg Lys
   1               5                   10                  15
   Cys Cys Glu Gly Phe Val Cys Thr Leu Trp Cys Arg Lys Lys Leu Trp
                   20                  25                  30
   Gly Ser Ala Pro Ala Pro Ala Pro Ala Pro Ala Pro Gly Ser Cys Cys
           35                  40                  45
   Asn Cys Ser Ser Lys Trp Cys Arg Asp His Ser Arg Cys Cys
       50                  55                  60
<210> 342
   <211> 64
   <212> PRT
   <213> Artificial sequence
<220>
   <223> Variant NV1D3800
<220>
   <221> MOD_RES
   <222> (64)..(64)
   <223> -COOH
<400> 342
   Gly Pro Gln Cys Gln Lys Trp Met Gln Thr Cys Asp Arg Glu Arg Lys
   1               5                   10                  15
   Cys Cys Glu Gly Phe Val Cys Thr Leu Trp Cys Arg Lys Lys Leu Trp
               20                  25                  30
   Gly Ser Ala Pro Ala Pro Ala Pro Ala Pro Ala Pro Gly Ser Cys Cys
           35                  40                  45
   Asn Cys Ser Ser Lys Trp Cys Arg Asp His Ser Arg Cys Cys Gly Arg
       50                  55                  60
<210> 343
   <211> 49
   <212> PRT
   <213> Artificial sequence
<220>
   <223> Variant NV1D3801
<220>
   <221> MOD_RES
   <222> (49).. (49)
   <223> -COOH
<400> 343
   Gly Pro Ser Pro Gly Ala Arg Ala Phe Ala Pro Ala Pro Ala Pro Ala
   1               5                   10                  15
   Pro Ala Pro Gln Cys Gln Lys Trp Met Gln Thr Cys Asp Arg Glu Arg
   20 25 30
   Lys Cys Cys Glu Gly Phe Val Cys Thr Leu Trp Cys Arg Lys Lys Leu
   35 40 45
   Trp
<210> 344
   <211> 44
   <212> PRT
   <213> Artificial sequence
<220>
   <223> Variant NV1D3802
<220>
   <221> MOD_RES
   <222> (44)..(44)
   <223> -COOH
<400> 344
   Gly Pro Ser Pro Gly Ala Arg Ala Phe Ala Pro Ala Pro Ala Gln Cys
   1 5 10 15
   Gln Lys Trp Met Gln Thr Cys Asp Arg Glu Arg Lys Cys Cys Glu Gly
   20 25 30
   Phe Val Cys Thr Leu Trp Cys Arg Lys Lys Leu Trp
   35 40
<210> 345
   <211> 39
   <212> PRT
   <213> Artificial sequence
<220>
   <223> Variant NV1D3803
<220>
   <221> MOD_RES
   <222> (39)..(39)
   <223> -COOH
<400> 345
   Gly Pro Ser Pro Gly Ala Arg Ala Phe Gln Cys Gln Lys Trp Met Gln
   1 5 10 15
   Thr Cys Asp Arg Glu Arg Lys Cys Cys Glu Gly Phe Val Cys Thr Leu
   20 25 30
   Trp Cys Arg Lys Lys Leu Trp
   35
<210> 346
   <211> 49
   <212> PRT
   <213> Artificial sequence
<220>
   <223> Variant NV1D3804
<220>
   <221> MOD_RES
   <222> (49).. (49)
   <223> -COOH
<400> 346
   Gly Pro Asp Gly Pro Trp Arg Lys Met Ala Pro Ala Pro Ala Pro Ala
   1 5 10 15
   Pro Ala Pro Gln Cys Gln Lys Trp Met Gln Thr Cys Asp Arg Glu Arg
   20 25 30
   Lys Cys Cys Glu Gly Phe Val Cys Thr Leu Trp Cys Arg Lys Lys Leu
   35 40 45
   Trp
<210> 347
   <211> 44
   <212> PRT
   <213> Artificial sequence
<220>
   <223> Variant NV1D3805
<220>
   <221> MOD_RES
   <222> (44)..(44)
   <223> -COOH
<400> 347
   Gly Pro Asp Gly Pro Trp Arg Lys Met Ala Pro Ala Pro Ala Gln Cys
   1 5 10 15
   Gln Lys Trp Met Gln Thr Cys Asp Arg Glu Arg Lys Cys Cys Glu Gly
   20 25 30
   Phe Val Cys Thr Leu Trp Cys Arg Lys Lys Leu Trp
   35 40
<210> 348
   <211> 39
   <212> PRT
   <213> Artificial sequence
<220>
   <223> Variant NV1D3806
<220>
   <221> MOD_RES
   <222> (39)..(39)
   <223> -COOH
<400> 348
   Gly Pro Asp Gly Pro Trp Arg Lys Met Gln Cys Gln Lys Trp Met Gln
   1               5                   10                  15
   Thr Cys Asp Arg Glu Arg Lys Cys Cys Glu Gly Phe Val Cys Thr Leu
               20                  25                  30
   Trp Cys Arg Lys Lys Leu Trp
           35
<210> 349
   <211> 44
   <212> PRT
   <213> Artificial sequence
<220>
   <223> Variant NV1D3808
<220>
   <221> MOD_RES
   <222> (44)..(44)
   <223> -COOH
<400> 349
   Gly Pro Phe Gly Gln Lys Ala Ser Ser Ala Pro Ala Pro Ala Gln Cys
   1               5                   10                  15
   Gln Lys Trp Met Gln Thr Cys Asp Arg Glu Arg Lys Cys Cys Glu Gly
               20                  25                  30
   Phe Val Cys Thr Leu Trp Cys Arg Lys Lys Leu Trp
           35                  40
<210> 350
   <211> 39
   <212> PRT
   <213> Artificial sequence
<220>
   <223> Variant NV1D3809
<220>
   <221> MOD_RES
   <222> (39)..(39)
   <223> -COOH
<400> 350
   Gly Pro Phe Gly Gln Lys Ala Ser Ser Gln Cys Gln Lys Trp Met Gln
   1               5                   10                  15
   Thr Cys Asp Arg Glu Arg Lys Cys Cys Glu Gly Phe Val Cys Thr Leu
               20                  25                  30
   Trp Cys Arg Lys Lys Leu Trp
           35
<210> 351
   <211> 58
   <212> PRT
   <213> Artificial sequence
<220>
   <223> Variant NV1D3810
<220>
   <221> MOD_RES
   <222> (58)..(58)
   <223> -COOH
<400> 351
   Gly Pro Gln Arg Phe Val Thr Gly His Phe Gly Gly Leu Tyr Pro Ala
   1               5                   10                  15
   Asn Gly Ala Pro Ala Pro Ala Pro Ala Pro Ala Pro Gln Cys Gln Lys
               20                  25                  30
   Trp Met Gln Thr Cys Asp Arg Glu Arg Lys Cys Cys Glu Gly Phe Val
           35                  40                  45
   Cys Thr Leu Trp Cys Arg Lys Lys Leu Trp
       50                  55
<210> 352
   <211> 53
   <212> PRT
   <213> Artificial sequence
<220>
   <223> Variant NV1D3811
<220>
   <221> MOD_RES
   <222> (53)..(53)
   <223> -COOH
<400> 352
   Gly Pro Gln Arg Phe Val Thr Gly His Phe Gly Gly Leu Tyr Pro Ala
   1               5                   10                  15
   Asn Gly Ala Pro Ala Pro Ala Gln Cys Gln Lys Trp Met Gln Thr Cys
               20                  25                  30
   Asp Arg Glu Arg Lys Cys Cys Glu Gly Phe Val Cys Thr Leu Trp Cys
           35                  40                  45
   Arg Lys Lys Leu Trp
       50
<210> 353
   <211> 48
   <212> PRT
   <213> Artificial sequence
<220>
   <223> Variant NV1D3812
<220>
   <221> MOD_RES
   <222> (48)..(48)
   <223> -COOH
<400> 353
   Gly Pro Gln Arg Phe Val Thr Gly His Phe Gly Gly Leu Tyr Pro Ala
   1               5                   10                  15
   Asn Gly Gln Cys Gln Lys Trp Met Gln Thr Cys Asp Arg Glu Arg Lys
               20                  25                  30
   Cys Cys Glu Gly Phe Val Cys Thr Leu Trp Cys Arg Lys Lys Leu Trp
           35                  40                  45
<210> 354
   <211> 53
   <212> PRT
   <213> Artificial sequence
<220>
   <223> Variant NV1D3813
<220>
   <221> MOD_RES
   <222> (53)..(53)
   <223> -COOH
<400> 354
   Gly Pro Arg Arg Arg Arg Arg Arg Arg Arg Arg Arg Arg Ala Pro Ala
   1               5                   10                  15
   Pro Ala Pro Ala Pro Ala Pro Gln Cys Gln Lys Trp Met Gln Thr Cys
               20                  25                  30
   Asp Arg Glu Arg Lys Cys Cys Glu Gly Phe Val Cys Thr Leu Trp Cys
           35                  40                  45
   Arg Lys Lys Leu Trp
       50
<210> 355
   <211> 48
   <212> PRT
   <213> Artificial sequence
<220>
   <223> Variant NV1D3814
<220>
   <221> MOD_RES
   <222> (48)..(48)
   <223> -COOH
<400> 355
   Gly Pro Arg Arg Arg Arg Arg Arg Arg Arg Arg Arg Arg Ala Pro Ala
   1               5                   10                  15
   Pro Ala Gln Cys Gln Lys Trp Met Gln Thr Cys Asp Arg Glu Arg Lys
               20                  25                  30
   Cys Cys Glu Gly Phe Val Cys Thr Leu Trp Cys Arg Lys Lys Leu Trp
           35                  40                  45
<210> 356
   <211> 43
   <212> PRT
   <213> Artificial sequence
<220>
   <223> Variant NV1D3815
<220>
   <221> MOD_RES
   <222> (43)..(43)
   <223> -COOH
<400> 356
   Gly Pro Arg Arg Arg Arg Arg Arg Arg Arg Arg Arg Arg Gln Cys Gln
   1               5                   10                  15
   Lys Trp Met Gln Thr Cys Asp Arg Glu Arg Lys Cys Cys Glu Gly Phe
               20                  25                  30
   Val Cys Thr Leu Trp Cys Arg Lys Lys Leu Trp
           35                  40
<210> 357
   <211> 43
   <212> PRT
   <213> Artificial sequence
<220>
   <223> Variant NV1D3818
<220>
   <221> MOD_RES
   <222> (43)..(43)
   <223> -COOH
<400> 357
   Gly Pro Tyr Gly Arg Lys Lys Arg Arg Gln Arg Arg Arg Gln Cys Gln
   1               5                   10                  15
   Lys Trp Met Gln Thr Cys Asp Arg Glu Arg Lys Cys Cys Glu Gly Phe
               20                  25                  30
   Val Cys Thr Leu Trp Cys Arg Lys Lys Leu Trp
           35                  40
<210> 358
   <211> 42
   <212> PRT
   <213> Artificial sequence
<220>
   <223> Variant NV1D3819
<220>
   <221> MOD_RES
   <222> (42)..(42)
   <223> -COOH
<400> 358
   Gly Pro Ala Pro Ala Pro Ala Pro Ala Pro Ala Pro Gln Cys Gln Lys
   1 5 10 15
   Trp Met Gln Thr Cys Asp Arg Glu Arg Lys Cys Cys Glu Gly Phe Val
   20 25 30
   Cys Thr Leu Trp Cys Arg Lys Lys Leu Trp
   35 40
<210> 359
   <211> 107
   <212> PRT
   <213> Artificial sequence
<220>
   <223> Variant NV1D3822
<220>
   <221> MOD_RES
   <222> (107)..(107)
   <223> -COOH
<400> 359
   Gly Pro Gly Trp Cys Gly Asp Pro Gly Ala Thr Cys Gly Lys Leu Arg
   1 5 10 15
   Leu Tyr Cys Cys Ser Gly Phe Cys Asp Ser Tyr Thr Lys Thr Cys Lys
   20 25 30
   Asp Lys Ser Ser Ala Gly Gly Gly Gly Ser Ala Pro Ala Pro Ala Pro
   35 40 45
   Ala Pro Ala Pro Ala Pro Ala Pro Ala Pro Ala Pro Ala Pro Ala Pro
   50 55 60
   Ala Pro Ala Pro Ala Pro Ala Pro Gly Gly Gly Gly Ser Gln Cys Gln
   65 70 75 80
   Lys Trp Met Gln Thr Cys Asp Arg Glu Arg Lys Cys Cys Glu Gly Phe
   85 90 95
   Val Cys Thr Leu Trp Cys Arg Lys Lys Leu Trp
   100 105
<210> 360
   <211> 107
   <212> PRT
   <213> Artificial sequence
<220>
   <223> Variant NV1D3823
<220>
   <221> MOD_RES
   <222> (107)..(107)
   <223> -COOH
<400> 360
   Gly Pro Gln Cys Gln Lys Trp Met Gln Thr Cys Asp Arg Glu Arg Lys
   1               5                   10                  15
   Cys Cys Glu Gly Phe Val Cys Thr Leu Trp Cys Arg Lys Lys Leu Trp
               20                  25                  30
   Gly Gly Gly Gly Ser Ala Pro Ala Pro Ala Pro Ala Pro Ala Pro Ala
           35                  40                  45
   Pro Ala Pro Ala Pro Ala Pro Ala Pro Ala Pro Ala Pro Ala Pro Ala
       50                  55                  60
   Pro Ala Pro Gly Gly Gly Gly Ser Gly Trp Cys Gly Asp Pro Gly Ala
   65                  70                  75                  80
   Thr Cys Gly Lys Leu Arg Leu Tyr Cys Cys Ser Gly Phe Cys Asp Ser
                   85                  90                  95
   Tyr Thr Lys Thr Cys Lys Asp Lys Ser Ser Ala
               100                 105
<210> 361
   <211> 107
   <212> PRT
   <213> Artificial sequence
<220>
   <223> Variant NV1D3824
<220>
   <221> MOD_RES
   <222> (107)..(107)
   <223> -COOH
<400> 361
   Gly Pro Gly Trp Cys Gly Asp Pro Gly Ala Thr Cys Gly Lys Leu Arg
   1 5 10 15
   Leu Tyr Cys Cys Ser Gly Phe Cys Asp Ala Tyr Thr Lys Thr Cys Lys
   20 25 30
   Asp Lys Ser Ser Ala Gly Gly Gly Gly Ser Ala Pro Ala Pro Ala Pro
   35 40 45
   Ala Pro Ala Pro Ala Pro Ala Pro Ala Pro Ala Pro Ala Pro Ala Pro
   50 55 60
   Ala Pro Ala Pro Ala Pro Ala Pro Gly Gly Gly Gly Ser Gln Cys Gln
   65 70 75 80
   Lys Trp Met Gln Thr Cys Asp Arg Glu Arg Lys Cys Cys Glu Gly Phe
   85 90 95
   Val Cys Thr Leu Trp Cys Arg Lys Lys Leu Trp
   100 105
<210> 362
   <211> 107
   <212> PRT
   <213> Artificial sequence
<220>
   <223> Variant NV1D3825
<220>
   <221> MOD_RES
   <222> (107)..(107)
   <223> -COOH
<400> 362
   Gly Pro Gln Cys Gln Lys Trp Met Gln Thr Cys Asp Arg Glu Arg Lys
   1               5                   10                  15
   Cys Cys Glu Gly Phe Val Cys Thr Leu Trp Cys Arg Lys Lys Leu Trp
               20                  25                  30
   Gly Gly Gly Gly Ser Ala Pro Ala Pro Ala Pro Ala Pro Ala Pro Ala
           35                  40                  45
   Pro Ala Pro Ala Pro Ala Pro Ala Pro Ala Pro Ala Pro Ala Pro Ala
       50                  55                  60
   Pro Ala Pro Gly Gly Gly Gly Ser Gly Trp Cys Gly Asp Pro Gly Ala
   65                  70                  75                      80
   Thr Cys Gly Lys Leu Arg Leu Tyr Cys Cys Ser Gly Phe Cys Asp Ala
                   85                  90                  95
   Tyr Thr Lys Thr Cys Lys Asp Lys Ser Ser Ala
               100                 105
<210> 363
   <211> 107
   <212> PRT
   <213> Artificial sequence
<220>
   <223> Variant NV1D3826
<220>
   <221> MOD_RES
   <222> (107)..(107)
   <223> -COOH
<400> 363
   Gly Pro Gly Trp Cys Gly Asp Pro Gly Ala Thr Cys Gly Lys Leu Arg
   1 5 10 15
   Leu Tyr Cys Cys Ser Gly Phe Cys Asp Cys Tyr Thr Lys Thr Cys Lys
   20 25 30
   Asp Lys Ser Ser Ala Gly Gly Gly Gly Ser Ala Pro Ala Pro Ala Pro
   35 40 45
   Ala Pro Ala Pro Ala Pro Ala Pro Ala Pro Ala Pro Ala Pro Ala Pro
   50 55 60
   Ala Pro Ala Pro Ala Pro Ala Pro Gly Gly Gly Gly Ser Gln Cys Gln
   65 70 75 80
   Lys Trp Met Gln Thr Cys Asp Arg Glu Arg Lys Cys Cys Glu Gly Phe
   85 90 95
   Val Cys Thr Leu Trp Cys Arg Lys Lys Leu Trp
   100 105
<210> 364
   <211> 84
   <212> PRT
   <213> Artificial sequence
<220>
   <223> Variant NV1D3828
<220>
   <221> MOD_RES
   <222> (84)..(84)
   <223> -COOH
<400> 364
   Gly Pro Gln Cys Gln Lys Trp Met Gln Thr Cys Asp Arg Glu Arg Lys
   1 5 10 15
   Cys Cys Glu Gly Phe Val Cys Thr Leu Trp Cys Arg Lys Lys Leu Trp
   20 25 30
   Gly Ser Gly Gly Gly Gly Ser Ala Pro Ala Pro Ala Pro Ala Pro Ala
   35 40 45
   Pro Ala Pro Ala Pro Ala Pro Ala Pro Ala Pro Gly Gly Gly Gly Ser
   50 55 60
   Gly Ser Cys Cys Asn Cys Ser Ser Lys Trp Cys Arg Asp His Ser Arg
   65 70 75 80
   Cys Cys Gly Arg
<210> 365
   <211> 82
   <212> PRT
   <213> Artificial sequence
<220>
   <223> Variant NV1D3829
<220>
   <221> MOD_RES
   <222> (82)..(82)
   <223> -COOH
<400> 365
   Gly Pro Gln Cys Gln Lys Trp Met Gln Thr Cys Asp Arg Glu Arg Lys
   1 5 10 15
   Cys Cys Glu Gly Phe Val Cys Thr Leu Trp Cys Arg Lys Lys Leu Trp
   20 25 30
   Gly Ser Gly Gly Gly Gly Ser Ala Pro Ala Pro Ala Pro Ala Pro Ala
   35 40 45
   Pro Ala Pro Ala Pro Ala Pro Ala Pro Ala Pro Gly Gly Gly Gly Ser
   50 55 60
   Gly Ser Cys Cys Asn Cys Ser Ser Lys Trp Cys Arg Asp His Ser Arg
   65 70 75 80
   Cys Cys
<210> 366
   <211> 84
   <212> PRT
   <213> Artificial sequence
<220>
   <223> Variant NV1D3830
<220>
   <221> MOD_RES
   <222> (84)..(84)
   <223> -COOH
<400> 366
   Gly Pro Cys Cys Asn Cys Ser Ser Lys Trp Cys Arg Asp His Ser Arg
   1 5 10 15
   Cys Cys Gly Arg Gly Ser Gly Gly Gly Gly Ser Ala Pro Ala Pro Ala
   20 25 30
   Pro Ala Pro Ala Pro Ala Pro Ala Pro Ala Pro Ala Pro Ala Pro Gly
   35 40 45
   Gly Gly Gly Ser Gly Ser Gln Cys Gln Lys Trp Met Gln Thr Cys Asp
   50 55 60
   Arg Glu Arg Lys Cys Cys Glu Gly Phe Val Cys Thr Leu Trp Cys Arg
   65 70 75 80
   Lys Lys Leu Trp
<210> 367
   <211> 61
   <212> PRT
   <213> Artificial sequence
<220>
   <223> Variant NV1D3832
<220>
   <221> MOD_RES
   <222> (61)..(61)
   <223> -COOH
<400> 367
   Gly Pro Cys Arg Thr Ile Gly Pro Ser Val Cys Ala Pro Ala Pro Ala
   1 5 10 15
   Pro Ala Pro Ala Pro Ala Pro Ala Pro Ala Pro Ala Pro Ala Pro Gln
   20 25 30
   Cys Gln Lys Trp Met Gln Thr Cys Asp Arg Glu Arg Lys Cys Cys Glu
   35 40 45
   Gly Phe Val Cys Thr Leu Trp Cys Arg Lys Lys Leu Trp
   50 55 60
<210> 368
   <211> 51
   <212> PRT
   <213> Artificial sequence
<220>
   <223> Variant NV1D3833
<220>
   <221> MOD_RES
   <222> (51)..(51)
   <223> -COOH
<400> 368
   Gly Pro Cys Arg Thr Ile Gly Pro Ser Val Cys Ala Pro Ala Pro Ala
   1               5                   10                  15
   Pro Ala Pro Ala Pro Gln Cys Gln Lys Trp Met Gln Thr Cys Asp Arg
               20                  25                  30
   Glu Arg Lys Cys Cys Glu Gly Phe Val Cys Thr Leu Trp Cys Arg Lys
           35                  40                  45
   Lys Leu Trp
       50
<210> 369
   <211> 46
   <212> PRT
   <213> Artificial sequence
<220>
   <223> Variant NV1D3834
<220>
   <221> MOD_RES
   <222> (46).. (46)
   <223> -COOH
<400> 369
   Gly Pro Cys Arg Thr Ile Gly Pro Ser Val Cys Ala Pro Ala Pro Ala
   1               5                   10                  15
   Gln Cys Gln Lys Trp Met Gln Thr Cys Asp Arg Glu Arg Lys Cys Cys
               20                  25                  30
   Glu Gly Phe Val Cys Thr Leu Trp Cys Arg Lys Lys Leu Trp
           35                  40                  45
<210> 370
   <211> 41
   <212> PRT
   <213> Artificial sequence
<220>
   <223> Variant NV1D3835
<220>
   <221> MOD_RES
   <222> (41)..(41)
   <223> -COOH
<400> 370
   Gly Pro Cys Arg Thr Ile Gly Pro Ser Val Cys Gln Cys Gln Lys Trp
   1               5                   10                  15
   Met Gln Thr Cys Asp Arg Glu Arg Lys Cys Cys Glu Gly Phe Val Cys
               20                  25                  30
   Thr Leu Trp Cys Arg Lys Lys Leu Trp
           35                  40
<210> 371
   <211> 46
   <212> PRT
   <213> Artificial sequence
<220>
   <223> Variant NV1D3838
<220>
   <221> MOD_RES
   <222> (46).. (46)
   <223> -COOH
<400> 371
   Gly Pro Gln Cys Gln Lys Trp Met Gln Thr Cys Asp Arg Glu Arg Lys
   1               5                   10                  15
   Cys Cys Glu Gly Phe Val Cys Thr Leu Trp Cys Arg Lys Lys Leu Trp
               20                  25                  30
   Ala Pro Ala Pro Ala Cys Arg Thr Ile Gly Pro Ser Val Cys
           35                  40                  45
<210> 372
   <211> 7
   <212> PRT
   <213> Artificial sequence
<220>
   <223> N-terminal extension
<400> 372
   Gly Pro Ala Ala Ala Ala Ala
   1 5
<210> 373
   <211> 7
   <212> PRT
   <213> Artificial sequence
<220>
   <223> N-terminal extension
<400> 373
   Gly Pro Ala Pro Ala Pro Ala
   1 5
<210> 374
   <211> 6
   <212> PRT
   <213> Artificial sequence
<220>
   <223> N-terminal extension
<400> 374
   Gly Gly Gly Gly Gly Gly
   1 5
<210> 375
   <211> 18
   <212> PRT
   <213> Artificial sequence
<220>
   <223> N-terminal extension
<400> 375
   Gly Pro Cys Cys Asn Cys Ser Ser Lys Trp Cys Arg Asp His Ser Arg
   1 5 10 15
   Cys Cys
<210> 376
   <211> 9
   <212> PRT
   <213> Artificial sequence
<220>
   <223> N-terminal extension
<400> 376
   Gly Pro Ser Pro Gly Ala Arg Ala Phe
   1 5
<210> 377
   <211> 9
   <212> PRT
   <213> Artificial sequence
<220>
   <223> N-terminal extension
<400> 377
   Gly Pro Asp Gly Pro Trp Arg Lys Met
   1               5
<210> 378
   <211> 9
   <212> PRT
   <213> Artificial sequence
<220>
   <223> N-terminal extension
<400> 378
   Gly Pro Phe Gly Gln Lys Ala Ser Ser
   1               5
<210> 379
   <211> 11
   <212> PRT
   <213> Artificial sequence
<220>
   <223> N-terminal extension
<400> 379
   Gly Pro Cys Arg Thr Ile Gly Pro Ser Val Cys
   1               5                   10
<210> 380
   <211> 18
   <212> PRT
   <213> Artificial sequence
<220>
   <223> N-terminal extension
<400> 380
   Gly Pro Ser His Ser Asn Thr Gln Thr Leu Ala Lys Ala Pro Glu His
   1               5                   10                  15
   Thr Gly
<210> 381
   <211> 18
   <212> PRT
   <213> Artificial sequence
<220>
   <223> N-terminal extension
<400> 381
   Gly Pro Gln Arg Phe Val Thr Gly His Phe Gly Gly Leu Tyr Pro Ala
   1               5                   10                  15
   Asn Gly
<210> 382
   <211> 37
   <212> PRT
   <213> Artificial sequence
<220>
   <223> N-terminal extension
<400> 382
   Gly Pro Gly Trp Cys Gly Asp Pro Gly Ala Thr Cys Gly Lys Leu Arg
   1               5                   10                  15
   Leu Tyr Cys Cys Ser Gly Phe Cys Asp Ser Tyr Thr Lys Thr Cys Lys
               20                  25                  30
   Asp Lys Ser Ser Ala
           35
<210> 383
   <211> 10
   <212> PRT
   <213> Artificial sequence
<220>
   <223> N-terminal extension
<400> 383
   Ala Pro Ala Pro Ala Pro Ala Pro Ala Pro
   1               5                   10
<210> 384
   <211> 13
   <212> PRT
   <213> Artificial sequence
<220>
   <223> N-terminal extension
<400> 384
   Gly Pro Tyr Gly Arg Lys Lys Arg Arg Gln Arg Arg Arg
   1 5 10
<210> 385
   <211> 13
   <212> PRT
   <213> Artificial sequence
<220>
   <223> N-terminal extension
<400> 385
   Gly Pro Arg Arg Arg Arg Arg Arg Arg Arg Arg Arg Arg
   1 5 10
<210> 386
   <211> 9
   <212> PRT
   <213> Artificial sequence
<220>
   <223> C-terminal extension
<400> 386
   Cys Arg Thr Ile Gly Pro Ser Val Cys
   1 5
<210> 387
   <211> 11
   <212> PRT
   <213> Artificial sequence
<220>
   <223> C-terminal extension
<400> 387
   Tyr Gly Arg Lys Lys Arg Arg Gln Arg Arg Arg
   1 5 10
<210> 388
   <211> 7
   <212> PRT
   <213> Artificial sequence
<220>
   <223> C-terminal extension
<400> 388
   Asp Gly Pro Trp Arg Lys Met
   1 5
<210> 389
   <211> 16
   <212> PRT
   <213> Artificial sequence
<220>
   <223> C-terminal extension
<400> 389
   Cys Cys Asn Cys Ser Ser Lys Trp Cys Arg Asp His Ser Arg Cys Cys
   1 5 10 15
<210> 390
   <211> 11
   <212> PRT
   <213> Artificial sequence
<220>
   <223> C-terminal extension
<400> 390
   Arg Arg Arg Arg Arg Arg Arg Arg Arg Arg Arg
   1 5 10
<210> 391
   <211> 16
   <212> PRT
   <213> Artificial sequence
<220>
   <223> C-terminal extension
<400> 391
   Ser His Ser Asn Thr Gln Thr Leu Ala Lys Ala Pro Glu His Thr Gly
   1 5 10 15
<210> 392
   <211> 5
   <212> PRT
   <213> Artificial sequence
<220>
   <223> C-terminal extension
<400> 392
   Ala Pro Ala Pro Ala
   1 5
<210> 393
   <211> 5
   <212> PRT
   <213> Artificial sequence
<220>
   <223> C-terminal extension
<400> 393
   Ala Ala Ala Ala Ala
   1 5
<210> 394
   <211> 7
   <212> PRT
   <213> Artificial sequence
<220>
   <223> C-terminal extension
<400> 394
   Phe Gly Gln Lys Ala Ser Ser
   1 5
<210> 395
   <211> 16
   <212> PRT
   <213> Artificial sequence
<220>
   <223> C-terminal extension
<400> 395
   Gln Arg Phe Val Thr Gly His Phe Gly Gly Leu Tyr Pro Ala Asn Gly
   1 5 10 15
<210> 396
   <211> 7
   <212> PRT
   <213> Artificial sequence
<220>
   <223> C-terminal extension
<400> 396
   Ser Pro Gly Ala Arg Ala Phe
   1 5
<210> 397
   <211> 37
   <212> PRT
   <213> Artificial sequence
<220>
   <223> C-terminal extension
<400> 397
   Gly Pro Gly Trp Cys Gly Asp Pro Gly Ala Thr Cys Gly Lys Leu Arg
   1 5 10 15
   Leu Tyr Cys Cys Ser Gly Phe Cys Asp Ala Tyr Thr Lys Thr Cys Lys
   20 25 30
   Asp Lys Ser Ser Ala
   35
<210> 398
   <211> 14
   <212> PRT
   <213> Artificial sequence
<220>
   <223> Linker
<400> 398
   Gly Ser Ala Pro Ala Pro Ala Pro Ala Pro Ala Pro Gly Ser
   1 5 10
<210> 399
   <211> 24
   <212> PRT
   <213> Artificial sequence
<220>
   <223> Linker
<400> 399
   Gly Ser Ala Pro Ala Pro Ala Pro Ala Pro Ala Pro Ala Pro Ala Pro
   1 5 10 15
   Ala Pro Ala Pro Ala Pro Gly Ser
   20
<210> 400
   <211> 40
   <212> PRT
   <213> Artificial sequence
<220>
   <223> Linker
<400> 400
   Gly Gly Gly Gly Ser Ala Pro Ala Pro Ala Pro Ala Pro Ala Pro Ala
   1 5 10 15
   Pro Ala Pro Ala Pro Ala Pro Ala Pro Ala Pro Ala Pro Ala Pro Ala
   20 25 30
   Pro Ala Pro Gly Gly Gly Gly Ser
   35 40
<210> 401
   <211> 34
   <212> PRT
   <213> Artificial sequence
<220>
   <223> Linker
<400> 401
   Gly Ser Gly Gly Gly Gly Ser Ala Pro Ala Pro Ala Pro Ala Pro Ala
   1               5                   10                  15
   Pro Ala Pro Ala Pro Ala Pro Ala Pro Ala Pro Gly Gly Gly Gly Ser
               20                  25                  30
   Gly Ser
<210> 402
   <211> 20
   <212> PRT
   <213> Artificial sequence
<220>
   <223> Linker
<400> 402
   Ala Pro Ala Pro Ala Pro Ala Pro Ala Pro Ala Pro Ala Pro Ala Pro
   1               5                   10                  15
   Ala Pro Ala Pro
               20
<210> 403
   <211> 32
   <212> PRT
   <213> Artificial sequence
<220>
   <223> Genus protoxin-II sequence
<220>
   <221> MISC_FEATURE
   <222> (1)..(1)
   <223> Xaa is Gly, Pro, Ala or deleted
<220>
   <221> MISC_FEATURE
   <222> (2)..(2)
   <223> Xaa is Pro, Ala or deleted
<220>
   <221> MISC_FEATURE
   <222> (3)..(3)
   <223> Xaa is Ser, Gln, Ala, Arg or Tyr
<220>
   <221> MISC_FEATURE
   <222> (5)..(5)
   <223> Xaa is Gln, Arg, Lys, Ala or Ser
<220>
   <221> MISC_FEATURE
   <222> (6)..(6)
   <223> Xaa is Lys, Ser, Gln or Arg
<220>
   <221> MISC_FEATURE
   <222> (8)..(8)
   <223> Xaa is Met or Phe
<220>
   <221> MISC_FEATURE
   <222> (10)..(10)
   <223> Xaa is Thr, Ser, Arg, Lys or Gln
<220>
   <221> MISC_FEATURE
   <222> (12)..(12)
   <223> Xaa is Asp or Thr
<220>
   <221> MISC_FEATURE
   <222> (13)..(13)
   <223> Xaa is Ser, Ala or Arg
<220>
   <221> MISC_FEATURE
   <222> (14)..(14)
   <223> Xaa is Glu, Arg, Asn, Lys, Thr or Gln
<220>
   <221> MISC_FEATURE
   <222> (15)..(15)
   <223> Xaa is Arg or Lys
<220>
   <221> MISC_FEATURE
   <222> (16)..(16)
   <223> Xaa is Lys, Gln, Ser or Ala
<220>
   <221> MISC_FEATURE
   <222> (19)..(19)
   <223> Xaa is Glu, Gln or Asp
<220>
   <221> MISC_FEATURE
   <222> (20)..(20)
   <223> Xaa is Glu or Gln
<220>
   <221> MISC_FEATURE
   <222> (22)..(22)
   <223> Xaa is Val or Ser
<220>
   <221> MISC_FEATURE
   <222> (24)..(24)
   <223> Xaa is Arg or Thr
<220>
   <221> MISC_FEATURE
   <222> (28)..(28)
   <223> Xaa is Lys or Arg
<400> 403
   Xaa Xaa Xaa Cys Xaa Xaa Trp Xaa Gln Xaa Cys Xaa Xaa Xaa Xaa Xaa
   1               5                   10                  15
   Cys Cys Xaa Xaa Phe Xaa Cys Xaa Leu Trp Cys Xaa Lys Lys Leu Trp
               20                  25                  30
<210> 404
   <211> 32
   <212> PRT
   <213> Artificial sequence
<220>
   <223> Protoxin-II genus 2
<220>
   <221> MISC_FEATURE
   <222> (5)..(5)
   <223> Xaa is Gln, Arg, Lys, Ala or Ser
<220>
   <221> MISC_FEATURE
   <222> (6)..(6)
   <223> Xaa is Lys, Ser, Gln or Arg
<220>
   <221> MISC_FEATURE
   <222> (8)..(8)
   <223> Xaa is Met or Phe
<220>
   <221> MISC_FEATURE
   <222> (10)..(10)
   <223> Xaa is Thr, Ser, Arg, Lys or Gln
<220>
   <221> MISC_FEATURE
   <222> (12)..(12)
   <223> Xaa is Asp or Thr
<220>
   <221> MISC_FEATURE
   <222> (13)..(13)
   <223> Xaa is Ser, Ala or Arg
<220>
   <221> MISC_FEATURE
   <222> (14)..(14)
   <223> Xaa is Glu, Arg, Asn, Lys, Thr or Gln
<220>
   <221> MISC_FEATURE
   <222> (15)..(15)
   <223> Xaa is Arg or Lys
<220>
   <221> MISC_FEATURE
   <222> (16)..(16)
   <223> Xaa is Lys, Gln, Ser or Ala
<220>
   <221> MISC_FEATURE
   <222> (19)..(19)
   <223> Xaa is Glu, Gln or Asp
<220>
   <221> MISC_FEATURE
   <222> (20)..(20)
   <223> Xaa is is Gly or Gln
<220>
   <221> MISC_FEATURE
   <222> (22)..(22)
   <223> Xaa is Val or Ser
<220>
   <221> MISC_FEATURE
   <222> (24)..(24)
   <223> Xaa is Arg or Thr
<220>
   <221> MISC_FEATURE
   <222> (28)..(28)
   <223> Xaa is Lys or Arg
<400> 404
   Gly Pro Gln Cys Xaa Xaa Trp Xaa Gln Xaa Cys Xaa Xaa Xaa Xaa Xaa
   1               5                   10                  15
   Cys Cys Xaa Xaa Phe Xaa Cys Xaa Leu Trp Cys Xaa Lys Lys Leu Trp
               20                  25                  30
<210> 405
   <211> 32
   <212> PRT
   <213> Artificial sequence
<220>
   <223> Protoxin-II genus 3
<220>
   <221> MISC_FEATURE
   <222> (3)..(3)
   <223> Xaa is Tyr, Gln, Ala, Ser or Arg
<220>
   <221> MISC_FEATURE
   <222> (10)..(10)
   <223> Xaa is Thr or Ser
<220>
   <221> MISC_FEATURE
   <222> (13)..(13)
   <223> Xaa is Ser, Arg or Ala
<220>
   <221> MISC_FEATURE
   <222> (14)..(14)
   <223> Xaa is Glu, Thr or Asn
<220>
   <221> MISC_FEATURE
   <222> (19)..(19)
   <223> Xaa is Glu or Gln
<220>
   <221> MISC_FEATURE
   <222> (22)..(22)
   <223> Xaa is Val or Ser
<220>
   <221> MISC_FEATURE
   <222> (24)..(24)
   <223> Xaa is Arg or Thr
<220>
   <221> MISC_FEATURE
   <222> (28)..(28)
   <223> Xaa is Lys or Arg
<400> 405
   Gly Pro Xaa Cys Gln Lys Trp Met Gln Xaa Cys Asp Xaa Xaa Arg Lys
   1               5                   10                  15
   Cys Cys Xaa Gly Phe Xaa Cys Xaa Leu Trp Cys Xaa Lys Lys Leu Trp
               20                  25                  30
<210> 406
   <211> 32
   <212> PRT
   <213> Artificial sequence
<220>
   <223> Protoxin-II genus 4
<220>
   <221> MISC_FEATURE
   <222> (10)..(10)
   <223> Xaa is Thr or Ser
<220>
   <221> MISC_FEATURE
   <222> (13)..(13)
   <223> Xaa is Ser, Arg or Ala
<220>
   <221> MISC_FEATURE
   <222> (14)..(14)
   <223> Xaa is Glu, Thr or Asn
<220>
   <221> MISC_FEATURE
   <222> (19)..(19)
   <223> Xaa is Glu or Gln
<220>
   <221> MISC_FEATURE
   <222> (22)..(22)
   <223> Xaa is Val or Ser
<220>
   <221> MISC_FEATURE
   <222> (24)..(24)
   <223> Xaa is Arg or Thr
<220>
   <221> MISC_FEATURE
   <222> (28)..(28)
   <223> Xaa is Lys or Arg
<400> 406
   Gly Pro Gln Cys Gln Lys Trp Met Gln Xaa Cys Asp Xaa Xaa Arg Lys
   1               5                   10                  15
   Cys Cys Xaa Gly Phe Xaa Cys Xaa Leu Trp Cys Xaa Lys Lys Leu Trp
               20                  25                  30
<210> 407
   <211> 1980
   <212> PRT
   <213> Homo sapiens
<400> 407